(19) Europäisches Patentamt
European Patent Office
Office européen des brevets

(11) **EP 4 803 080 A1**

(12) **EUROPEAN PATENT APPLICATION**
published in accordance with Art. 153(4) EPC

(43) Date of publication:
**09.09.2026 Bulletin 2026/37**

(21) Application number: **24884884.8**

(22) Date of filing: **31.10.2024**

(51) International Patent Classification (IPC):
***A61K 31/4439*** *(2006.01)* ***A61K 31/444*** *(2006.01)*
***A61K 31/501*** *(2006.01)* ***C07D 401/14*** *(2006.01)*
***C07D 403/14*** *(2006.01)* ***A61P 35/02*** *(2006.01)*
***A61P 35/00*** *(2006.01)*

(52) Cooperative Patent Classification (CPC):
**A61K 31/4439; A61K 31/444; A61K 31/501; A61P 35/00; A61P 35/02; C07D 401/14; C07D 403/14**

(86) International application number:
**PCT/CN2024/128825**

(87) International publication number:
**WO 2025/092884 (08.05.2025 Gazette 2025/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.11.2023 CN 202311445361**
**24.10.2024 CN 202411499327**

(71) Applicant: **Shenzhen TargetRx Co., Ltd.**
**Shenzhen, Guangdong 518057 (CN)**

(72) Inventors:
- **WANG, Yihan**
  **Shenzhen, Guangdong 518057 (CN)**
- **CAO, Jingrong**
  **Shenzhen, Guangdong 518057 (CN)**
- **SHI, Yanxia**
  **Shenzhen, Guangdong 518057 (CN)**
- **LIU, Yun**
  **Shenzhen, Guangdong 518057 (CN)**
- **ZHAO, Jiuyang**
  **Shenzhen, Guangdong 518057 (CN)**

(74) Representative: **Novitas Patent AB**
**P.O. Box 55557**
**102 04 Stockholm (SE)**

(54) **METHOD FOR TREATING LEUKEMIA**

(57) Provided is a method for treating chronic-phase and/or accelerated-phase and/or blast-phase chronic myelogenous leukemia and/or acute lymphoblastic leukemia in a subject by administering to the subject a compound of formula (I) or a pharmaceutically acceptable salt, crystal form, solvate or hydrate thereof. Also provided is a use of the compound of formula (I) or the pharmaceutically acceptable salt, crystal form, solvate or hydrate thereof in combination with an ATP-competitive BCR-ABL1 inhibitor.

(I)

EP 4 803 080 A1

## Description

### FIELD OF THE INVENTION

The present disclosure belongs to the field of medicine, and specifically relates to the use and methods of a compound of formula (I) or a pharmaceutically acceptable salt, crystal form, solvate, or hydrate thereof in the treatment of chronic myeloid leukemia in the chronic phase and/or accelerated phase and/or blast phase and/or acute lymphoblastic leukemia. The present disclosure also relates to a pharmaceutical combination for treating diseases regulated by wild-type and/or mutant BCR-ABL1 kinase, comprising a BCR-ABL1 allosteric inhibitor and an ATP-competitive inhibitor, and its use in treating diseases regulated by wild-type and/or mutant BCR-ABL1 kinase.

### BACKGROUND OF THE INVENTION

Chronic Myeloid Leukemia (CML) and some cases of adult Acute Lymphoblastic Leukemia (ALL) are caused by the chromosomal translocation (9;22)(q34;q11), which results in the fusion of the BCR and ABL1 genes on the Philadelphia chromosome, forming the BCR-ABL1 fusion gene. The BCR-ABL1 fusion gene translates into the BCR-ABL1 fusion protein, leading to abnormally elevated BCR-ABL1 tyrosine kinase activity, which promotes cell proliferation, inhibits apoptosis, and impairs cell adhesion. CML has a slow onset, and its natural course includes the Chronic Phase (CP), Accelerated Phase (AP), and Blast phase (BP), with most patients diagnosed in the chronic phase. The advent of BCR-ABL1 Tyrosine Kinase Inhibitors (TKIs) has revolutionized the treatment of CML by inhibiting the BCR-ABL1 pathway and significantly reducing the proliferation of CML cells expressing BCR-ABL1. Currently, ATP-competitive BCR-ABL1 inhibitors for treating CML include the Type II tyrosine kinase inhibitors imatinib (Gleevec®) and nilotinib (Tasigna®), which bind to the DFG-out conformation, as well as the Type I inhibitors dasatinib (Sprycel®) and bosutinib (Bosulif®), which bind to the DFG-in conformation. However, continuous drug treatment often fails due to mutations in BCR-ABL1$^{T315I}$. Although the Type II inhibitor ponatinib (Iclusing®) can significantly inhibit the T315I mutation, it has adverse effects due to off-target effects on other kinases. Therefore, in Philadelphia chromosome-positive CML patients, the failure of ATP-competitive TKI therapy may be due to resistance to TKIs, unacceptable side effects, or both.

In 2003, the research groups of Kuriyan and Superti-Furga reported that myristoylation is involved in the autoregulation of the ABL kinase, inducing cross-linking of the SH3-SH2-Kinase structure and playing a key role in negatively regulating ABL kinase activity, thereby allowing normal cell proliferation. However, when the BCR-ABL1 fusion protein is formed, BCR occupies the myristoyl binding site at the N-terminal cap region of ABL. Consequently, the cross-linking of the SH3-SH2-Kinase domains cannot be induced. This leads to the structural activation of BCR-ABL1, resulting in persistent differentiation and proliferation of cells, leading to malignant transformation.

Asciminib (Scemblix®) is an allosteric inhibitor that binds to the myristoyl pocket of ABL. Asciminib is active against all catalytic ATP site mutations in BCR-ABL1 at low nanomolar concentrations, including the T315I mutation. Some mutations in the myristoyl binding site (such as P465S, V468F, I502L, A337V, A424T) confer resistance to asciminib.

Therefore, it is necessary to further develop BCR-ABL1 allosteric inhibitors for the treatment of CML, and it is also necessary to further develop combination therapies of BCR-ABL1 allosteric inhibitors and ATP-competitive inhibitors.

### SUMMARY OF THE INVENTION

The present disclosure provides the use and methods of a compound of formula (I) or a pharmaceutically acceptable salt, crystal form, solvate, or hydrate thereof for treating chronic phase and/or accelerated phase and/or blast phase CML and/or ALL, wherein the compound of formula (I) or a pharmaceutically acceptable salt, crystal form, solvate, or hydrate thereof is as described below.

The present disclosure also provides a pharmaceutical combination comprising a BCR-ABL1 allosteric inhibitor (e.g., a compound of formula (I) or a pharmaceutically acceptable salt, crystal form, solvate, or hydrate thereof) and an ATP-competitive inhibitor. This combination significantly inhibits BCR-ABL1 mutations, particularly refractory compound mutations, and can be used to treat diseases regulated by wild-type and/or mutant BCR-ABL1 kinase.

In this regard, the present disclosure adopts the following technical solutions:

Technical Solution 1. Use of a compound of formula (I) or a pharmaceutically acceptable salt, crystal form, solvate, or hydrate thereof in the manufacture of a medicament for treating chronic myeloid leukemia (CML) or acute lymphoblastic leukemia (ALL), wherein the compound of formula (I) has the following structure:

wherein:

$Y_1$ is independently selected from $CR_a$ or N;
Y is independently selected from $CR_a$ or N;
$R_1$ is selected from hydrogen, halogen, cyano, nitro, $C_{1-6}$ alkyl or $C_{1-6}$ haloalkyl, wherein the $C_{1-6}$ alkyl or $C_{1-6}$ haloalkyl is optionally substituted with an $R_{1a}$ group;
$R_2$ is selected from hydrogen, $C_{1-6}$ alkyl or $C_{1-6}$ haloalkyl, wherein the $C_{1-6}$ alkyl or $C_{1-6}$ haloalkyl is optionally substituted with an $R_{2a}$ group;
Z is a chemical bond, O, $S(O)_{0-2}$ or $NR_b$;
or, $-Z-R_2$ together represents $-SF_5$;
Ar is a six-membered heteroaryl containing at least one N atom, optionally substituted with R groups;
Het is

;

wherein $X_9$ is selected from O, S, $NR_b$ or $C(R)_2$;
m is 0, 1 or 2;
n is 0, 1, 2, 3, 4, 5 or 6;
$R_a$ is independently selected from hydrogen, halogen, cyano, nitro, hydroxy, $-NH_2$, $-NHC_{1-6}$ alkyl, $-N(C_{1-6}$ alkyl$)_2$, $C_{1-6}$ alkyl, $C_{1-6}$ haloalkyl or $C_{1-6}$ alkoxy;
$R_b$ is independently selected from hydrogen, $C_{1-6}$ alkyl or $C_{1-6}$ haloalkyl;
$R_{1a}$, $R_{2a}$ and R are independently selected from hydrogen, halogen, hydroxy, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{3-7}$ cycloalkyl, $C_{3-7}$ heterocycloalkyl, $C_{6-10}$ aryl or $C_{5-10}$ heteroaryl;
alternatively, two R groups on the same atom or adjacent atoms may together form a $C_{3-7}$ cycloalkyl, $C_{3-7}$ heterocycloalkyl, $C_{6-10}$ aryl or $C_{5-10}$ heteroaryl.

Technical Solution 2. A method for treating chronic myeloid leukemia (CML) or acute lymphoblastic leukemia (ALL) in a patient, comprising administering to the patient a therapeutically effective amount of a compound of formula (I) or a pharmaceutically acceptable salt, crystal form, solvate, or hydrate thereof, wherein the compound of formula (I) has the following structure:

(I)

wherein:

Yi is selected from $CR_a$ or N;
Y is independently selected from $CR_a$ or N;
$R_1$ is selected from hydrogen, halogen, cyano, nitro, $C_{1-6}$ alkyl or $C_{1-6}$ haloalkyl, wherein the $C_{1-6}$ alkyl or $C_{1-6}$ haloalkyl is optionally substituted with an $R_{1a}$ group;
$R_2$ is selected from hydrogen, $C_{1-6}$ alkyl or $C_{1-6}$ haloalkyl, wherein the $C_{1-6}$ alkyl or $C_{1-6}$ haloalkyl is optionally substituted with an $R_{2a}$ group;
Z is a chemical bond, O, $S(O)_{0-2}$ or $NR_b$;
or, $-Z-R_2$ together represents $-SF_5$;

Ar is a six-membered heteroaryl containing at least one N atom, optionally substituted with R groups;
Het is

;

wherein $X_9$ is selected from O, S, $NR_b$ or $C(R)_2$;
m is 0, 1 or 2;
n is 0, 1, 2, 3, 4, 5 or 6;
$R_a$ is independently selected from hydrogen, halogen, cyano, nitro, hydroxy, $-NH_2$, $-NHC_{1-6}$ alkyl, $-N(C_{1-6}$ alkyl$)_2$, $C_{1-6}$ alkyl, $C_{1-6}$ haloalkyl or $C_{1-6}$ alkoxy;
$R_b$ is independently selected from hydrogen, $C_{1-6}$ alkyl or $C_{1-6}$ haloalkyl;
$R_{1a}$, $R_{2a}$ and R are independently selected from hydrogen, halogen, hydroxy, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{3-7}$ cycloalkyl, $C_{3-7}$ heterocycloalkyl, $C_{6-10}$ aryl or $C_{5-10}$ heteroaryl;
alternatively, two R groups on the same atom or adjacent atoms may together form a $C_{3-7}$ cycloalkyl, $C_{3-7}$ heterocycloalkyl, $C_{6-10}$ aryl or $C_{5-10}$ heteroaryl.

Technical Solution 3. A compound of formula (I) or a pharmaceutically acceptable salt, crystal form, solvate, or hydrate thereof, for use in treating chronic myeloid leukemia (CML) or acute lymphoblastic leukemia (ALL), wherein the compound of formula (I) has the following structure:

(I)

wherein:

$Y_1$ is independently selected from $CR_a$ or N;
Y is independently selected from $CR_a$ or N;
$R_1$ is selected from hydrogen, halogen, cyano, nitro, $C_{1-6}$ alkyl or $C_{1-6}$ haloalkyl, wherein the $C_{1-6}$ alkyl or $C_{1-6}$ haloalkyl is optionally substituted with an $R_{1a}$ group;
$R_2$ is selected from hydrogen, $C_{1-6}$ alkyl or $C_{1-6}$ haloalkyl, wherein the $C_{1-6}$ alkyl or $C_{1-6}$ haloalkyl is optionally substituted with an $R_{2a}$ group;
Z is a chemical bond, O, $S(O)_{0-2}$ or $NR_b$;
or, $-Z-R_2$ together represents $-SF_5$;
Ar is a six-membered heteroaryl containing at least one N atom, optionally substituted with R groups;
Het is

;

wherein $X_9$ is selected from O, S, $NR_b$ or $C(R)_2$;
m is 0, 1 or 2;
n is 0, 1, 2, 3, 4, 5 or 6;
$R_a$ is independently selected from hydrogen, halogen, cyano, nitro, hydroxy, $-NH_2$, $-NHC_{1-6}$ alkyl, $-N(C_{1-6}$ alkyl$)_2$, $C_{1-6}$ alkyl, $C_{1-6}$ haloalkyl or $C_{1-6}$ alkoxy;
$R_b$ is independently selected from hydrogen, $C_{1-6}$ alkyl or $C_{1-6}$ haloalkyl;
$R_{1a}$, $R_{2a}$ and R are independently selected from hydrogen, halogen, hydroxy, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{3-7}$ cycloalkyl, $C_{3-7}$ heterocycloalkyl, $C_{6-10}$ aryl or $C_{5-10}$ heteroaryl;

alternatively, two R groups on the same atom or adjacent atoms may together form a $C_{3-7}$ cycloalkyl, $C_{3-7}$ heterocycloalkyl, $C_{6-10}$ aryl or $C_{5-10}$ heteroaryl.

Technical Solution 4. The use according to Technical Solution 1, the method according to Technical Solution 2, or the compound for use according to Technical Solution 3, wherein the compound of formula (I) is (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof.
Technical Solution 5. The use, method, or the compound for use according to any one of Technical Solutions 1-4, wherein the chronic myeloid leukemia is chronic myeloid leukemia in chronic phase, optionally central nervous system leukemia relapse thereof, such as brain metastasis.
Technical Solution 6. The use, method, or the compound for use according to any one of Technical Solutions 1-4, wherein the chronic myeloid leukemia is chronic myeloid leukemia in accelerated phase, optionally central nervous system leukemia relapse thereof, such as brain metastasis.
Technical Solution 7. The use, method, or the compound for use according to any one of Technical Solutions 1-4, wherein the chronic myeloid leukemia is chronic myeloid leukemia in blast phase, optionally central nervous system leukemia relapse thereof, such as brain metastasis.
Technical Solution 8. The use, method, or the compound for use according to any one of Technical Solutions 1-7, wherein the chronic myeloid leukemia is Philadelphia chromosome-positive (Ph+).
Technical Solution 9. The use, method, or the compound for use according to any one of Technical Solutions 1-4, wherein the acute lymphoblastic leukemia is Philadelphia chromosome-positive (Ph+), optionally central nervous system leukemia relapse thereof, such as brain metastasis.
Technical Solution 10. The use, method, or the compound for use according to any one of Technical Solutions 1-9, wherein the chronic myeloid leukemia is BCR-ABL1 fusion gene-positive;

optionally, the BCR-ABL1 fusion gene is wild-type;
optionally, the BCR-ABL1 fusion gene is mutant; optionally, the mutation is selected from G250E, Q252H, Y253H, E255K/V, M244V, H396P, F317L, E459K, E355G, V299L, T315I, M351T, F359V, P223S, and I502L, alternatively G250E, Q252H, Y253H, E255K/V, E459K, T315I, and M351T, alternatively T315I.

Technical Solution 11. The use, method, or the compound for use according to any one of Technical Solutions 1-10, wherein the chronic myeloid leukemia has progressed after treatment with another therapy;

optionally, wherein the other therapy is selected from radiation therapy, drug therapy, or surgical therapy;
optionally, wherein the drug therapy is chemotherapy, targeted therapy, or immunotherapy;
optionally, wherein the drug therapy is chemotherapy;
optionally, wherein the drug therapy is targeted therapy;
optionally, wherein the drug for the drug therapy is selected from one or more of imatinib, dasatinib, nilotinib, BCR-ABL tyrosine kinase inhibitors, olverembatinib, hydroxyurea, ponatinib, asciminib, interferon, cytarabine, azacitidine, interferon alfa-1b, homoharringtonine, decitabine, peginterferon alfa-2a, thalidomide, chidamide, or recombinant interferon alfa-2b.

Technical Solution 12. The use, method, or the compound for use according to any one of Technical Solutions 1-11, wherein the chronic myeloid leukemia has progressed after treatment with a BCR-ABL tyrosine kinase inhibitor; optionally, wherein the BCR-ABL tyrosine kinase inhibitor(s) is selected from imatinib, dasatinib, nilotinib, olverembatinib, ponatinib or asciminib.
Technical Solution 13. The use, method, or the compound for use according to any one of Technical Solutions 1-12, wherein the patient is an adult patient.
Technical Solution 14. The use, method, or the compound for use according to any one of Technical Solutions 1-13, wherein the patient is a pediatric patient.
Technical Solution 15. The use, method, or the compound for use according to any one of Technical Solutions 1-14, wherein the patient has received one or more lines of treatment; optionally, wherein the patient has received two or more lines of treatment; optionally, wherein the patient has received three or more lines of treatment; optionally, the patient has received one, two, three, four, or five lines of treatment.
Technical Solution 16. The use, method, or the compound for use according to any one of Technical Solutions 1-15, wherein the patient has previously received treatment with ≥1 different BCR-ABL1 tyrosine kinase inhibitor(s) and subsequently experienced disease progression to accelerated phase or blast phase, with treatment evaluation results being failure, warning, or intolerance;

optionally, wherein the patient has previously received treatment with ≥2 different BCR-ABL1 tyrosine kinase inhibitor(s) and subsequently experienced disease progression to accelerated phase or blast phase, with treatment evaluation results being failure, warning, or intolerance;
optionally, wherein the patient has previously received treatment with ≥3 different BCR-ABL1 tyrosine kinase inhibitor(s) and subsequently experienced disease progression to accelerated phase or blast phase, with treatment evaluation results being failure, warning, or intolerance;
optionally, wherein the patient has previously received treatment with ≥4 different BCR-ABL1 tyrosine kinase inhibitor(s) and subsequently experienced disease progression to accelerated phase or blast phase, with treatment evaluation results being failure, warning, or intolerance;
optionally, wherein the patient has previously received treatment with ≥1 different BCR-ABL1 ATP-competitive tyrosine kinase inhibitor(s) and subsequently experienced disease progression to accelerated phase or blast phase, with treatment evaluation results being failure, warning, or intolerance;
optionally, wherein the patient has previously received treatment with ≥2 different BCR-ABL1 ATP-competitive tyrosine kinase inhibitor(s) and subsequently experienced disease progression to accelerated phase or blast phase, with treatment evaluation results being failure, warning, or intolerance;
optionally, wherein the patient has previously received treatment with ≥3 different BCR-ABL1 ATP-competitive tyrosine kinase inhibitor(s) and subsequently experienced disease progression to accelerated phase or blast phase, with treatment evaluation results being failure, warning, or intolerance;
optionally, wherein the patient has previously received treatment with ≥4 different BCR-ABL1 ATP-competitive tyrosine kinase inhibitor(s) and subsequently experienced disease progression to accelerated phase or blast phase, with treatment evaluation results being failure, warning, or intolerance.

Technical Solution 17. The use, method, or the compound for use according to any one of Technical Solutions 1-16, wherein the patient has previously received treatment with ≥1 different BCR-ABL1 tyrosine kinase inhibitors and subsequently experienced relapse or became refractory;

optionally, wherein the patient has previously received treatment with ≥2 different BCR-ABL1 tyrosine kinase inhibitors and subsequently experienced relapse or became refractory;
optionally, wherein the patient has previously received treatment with ≥3 different BCR-ABL1 tyrosine kinase inhibitors and subsequently experienced relapse or became refractory;
optionally, wherein the patient has previously received treatment with ≥4 different BCR-ABL1 tyrosine kinase inhibitors and subsequently experienced relapse or became refractory;
optionally, wherein the patient has previously received treatment with ≥1 different BCR-ABL1 ATP-competitive tyrosine kinase inhibitors and subsequently experienced relapse or became refractory;
optionally, wherein the patient has previously received treatment with ≥2 different BCR-ABL1 ATP-competitive tyrosine kinase inhibitors and subsequently experienced relapse or became refractory;
optionally, wherein the patient has previously received treatment with ≥3 different BCR-ABL1 ATP-competitive tyrosine kinase inhibitors and subsequently experienced relapse or became refractory;
optionally, wherein the patient has previously received treatment with ≥4 different BCR-ABL1 ATP-competitive tyrosine kinase inhibitors and subsequently experienced relapse or became refractory.

Technical Solution 18. The use, method, or the compound for use according to any one of Technical Solutions 16-17, wherein the BCR-ABL1 tyrosine kinase inhibitor(s) is selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382;

alternatively, BCR-ABL1 tyrosine kinase inhibitor(s) is selected from imatinib, dasatinib, nilotinib, bosutinib, flumatinib, ponatinib, olverembatinib, radotinib or asciminib;
alternatively, BCR-ABL1 tyrosine kinase inhibitor(s) is selected from imatinib, dasatinib, nilotinib, ponatinib, olverembatinib or asciminib;
alternatively, BCR-ABL1 tyrosine kinase inhibitor(s) is selected from imatinib, dasatinib, nilotinib, bosutinib, flumatinib or radotinib;
alternatively, BCR-ABL1 tyrosine kinase inhibitor(s) is selected from imatinib, dasatinib or nilotinib;
alternatively, BCR-ABL1 tyrosine kinase inhibitor(s) is selected from ponatinib, olverembatinib, asciminib or HS-10382;
alternatively, BCR-ABL1 tyrosine kinase inhibitor(s) is selected from ponatinib or olverembatinib;
alternatively, BCR-ABL1 tyrosine kinase inhibitor(s) is selected from asciminib or HS-10382;
alternatively, BCR-ABL1 tyrosine kinase inhibitor(s) is a first-line, second-line, third-line, fourth-line, or fifth-line

therapeutic agent;
alternatively, BCR-ABL1 tyrosine kinase inhibitor(s) is a first-line, second-line, or third-line therapeutic agent.

Technical Solution 19. The use, method, or the compound for use according to any one of Technical Solutions 16-17, wherein BCR-ABL1 ATP-competitive tyrosine kinase inhibitor(s) is selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228;

alternatively, BCR-ABL1 ATP-competitive tyrosine kinase inhibitor(s) is selected from imatinib, dasatinib, nilotinib, bosutinib, flumatinib, ponatinib, olverembatinib or radotinib;
alternatively, BCR-ABL1 ATP-competitive tyrosine kinase inhibitor(s) is selected from imatinib, dasatinib, nilotinib, ponatinib or olverembatinib;
alternatively, BCR-ABL1 ATP-competitive tyrosine kinase inhibitor(s) is selected from imatinib, dasatinib, nilotinib, bosutinib, flumatinib or radotinib;
alternatively, BCR-ABL1 ATP-competitive tyrosine kinase inhibitor(s) is selected from imatinib, dasatinib or nilotinib;
alternatively, BCR-ABL1 ATP-competitive tyrosine kinase inhibitor(s) is selected from ponatinib or olverembatinib;
alternatively, BCR-ABL1 ATP-competitive tyrosine kinase inhibitor(s) is a first-line, second-line, third-line, fourth-line, or fifth-line therapeutic agent;
alternatively, BCR-ABL1 ATP-competitive tyrosine kinase inhibitor(s) is a first-line, second-line, or third-line therapeutic agent.

Technical Solution 20. The use, method, or the compound for use according to any one of Technical Solutions 1-19, wherein the patient has previously received treatment with ≥1 different first-generation and/or second-generation and/or third-generation BCR-ABL1 ATP-competitive tyrosine kinase inhibitors and subsequently developed resistance or intolerance;

alternatively, the patient has previously received treatment with ≥2 different first-generation and/or second-generation and/or third-generation BCR-ABL1 ATP-competitive tyrosine kinase inhibitors and subsequently developed resistance or intolerance;
alternatively, the patient has previously received treatment with ≥3 different first-generation and/or second-generation and/or third-generation BCR-ABL1 ATP-competitive tyrosine kinase inhibitors and subsequently developed resistance or intolerance;
alternatively, the patient has previously received treatment with ≥4 different first-generation and/or second-generation and/or third-generation BCR-ABL1 ATP-competitive tyrosine kinase inhibitors and subsequently developed resistance or intolerance.

Technical Solution 21. The use, method, or the compound for use according to any one of Technical Solutions 1-20, wherein the resistance arises from BCR-ABL1 kinase domain point mutation(s);

optionally, the BCR-ABL1 kinase domain point mutation(s) is selected from one or more of T315I/A, Y253F/H, E255V/K, M351T, G250E, F359V/I/C, H396P/R, M244V, E355G, F317L/V/I/C, F311L, M237I, Q252H/R, D276G, L248V, V379I, L384M, L387M, E279K, F486S, E459K, P223S, I502L or V299L;
optionally, the BCR-ABL1 kinase domain point mutation(s) is selected from one or more of T315I, Y253H, E255V/K, M351T, G250E, F359V/, H396P, M244V, E355G, F317L, Q252H, E459K, P223S, I502L or V299L.

Technical Solution 22. The use, method, or the compound for use according to any one of Technical Solutions 1-21, wherein the patient has previously received treatment with ≥1 different first-generation and/or second-generation and/or third-generation BCR-ABL1 ATP-competitive tyrosine kinase inhibitors and subsequently experienced relapse or became refractory;

alternatively, the patient has previously received treatment with ≥2 different first-generation and/or second-generation and/or third-generation BCR-ABL1 ATP-competitive tyrosine kinase inhibitors and subsequently experienced relapse or became refractory;
alternatively, the patient has previously received treatment with ≥3 different first-generation and/or second-generation and/or third-generation BCR-ABL1 ATP-competitive tyrosine kinase inhibitors and subsequently experienced relapse or became refractory;

alternatively, the patient has previously received treatment with ≥4 different first-generation and/or second-generation and/or third-generation BCR-ABL1 ATP-competitive tyrosine kinase inhibitors and subsequently experienced relapse or became refractory.

Technical Solution 23. The use, method, or the compound for use according to any one of Technical Solutions 20-22, wherein the first-generation and/or second-generation and/or third-generation BCR-ABL1 ATP-competitive tyrosine kinase inhibitor(s) is selected from imatinib, dasatinib, nilotinib, bosutinib, flumatinib or radotinib;

alternatively, the first-generation and/or second-generation and/or third-generation BCR-ABL1 ATP-competitive tyrosine kinase inhibitor(s) is selected from imatinib, dasatinib or nilotinib;
alternatively, the first-generation and/or second-generation and/or third-generation BCR-ABL1 ATP-competitive tyrosine kinase inhibitor(s) is selected from imatinib, dasatinib, nilotinib, bosutinib, flumatinib, radotinib, ponatinib or olverembatinib;
alternatively, the first-generation and/or second-generation and/or third-generation BCR-ABL1 ATP-competitive tyrosine kinase inhibitor(s) is selected from imatinib, dasatinib, nilotinib, flumatinib or, ponatinib or olverembatinib;
alternatively, the first-generation and/or second-generation and/or third-generation BCR-ABL1 ATP-competitive tyrosine kinase inhibitor(s) is a first-line, second-line, third-line, fourth-line, or fifth-line therapeutic agent;
alternatively, the first-generation and/or second-generation and/or third-generation BCR-ABL1 ATP-competitive tyrosine kinase inhibitor(s) is first-line or second-line therapeutic agent.

Technical Solution 24. The use, method, or the compound for use according to any one of Technical Solutions 1-23, wherein the dose of the compound of formula (I) or pharmaceutically acceptable salt, crystal form, solvate, or hydrate thereof (alternatively compound A) is about 20 mg/day to about 400 mg/day;

alternatively, the dose of the compound of formula (I) or pharmaceutically acceptable salt, crystal form, solvate, or hydrate thereof (alternatively compound A) is about 20 mg/day to about 240 mg/day;
alternatively, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day;
alternatively, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day.

Technical Solution 25. The use, method, or the compound for use according to any one of Technical Solutions 1-24, wherein the compound of formula (I) or a pharmaceutically acceptable salt, crystal form, solvate, or hydrate thereof (alternatively compound A) is administered orally or intranasally; alternatively, administered orally; alternatively, administered orally under fasting conditions.
Technical Solution 26. The use, method, or the compound for use according to any one of Technical Solutions 1-25, wherein the compound of formula (I) or a pharmaceutically acceptable salt, crystal form, solvate, or hydrate thereof (alternatively Compound A) is administered once daily (QD), twice daily (BID), or three times daily (TID).
Technical Solution 27. The use, method, or the compound for use according to any one of Technical Solutions 1-26, wherein the compound of formula (I) or a pharmaceutically acceptable salt, crystal form, solvate, or hydrate thereof (alternatively Compound A) is administered at about 10 mg BID, about 20 mg BID, about 40 mg QD, about 40 mg BID, about 80 mg QD, about 80 mg BID, about 160 mg QD, or about 240 mg QD.
Technical Solution 28. The use, method, or the compound for use according to any one of Technical Solutions 1-27, wherein the compound of formula (I) or a pharmaceutically acceptable salt, crystal form, solvate, or hydrate thereof (alternatively Compound A) is administered until disease progression, intolerable toxicity, or death.
Technical Solution 29. A pharmaceutical combination comprising:

(a) The compound of formula (I) according to technical solution 1, or a pharmaceutically acceptable salt, crystal form, solvate, or hydrate thereof; and
(b) an ATP-competitive BCR-ABL1 inhibitor, or a pharmaceutically acceptable salt, crystal form, solvate, or hydrate thereof.

Technical solution 30. The pharmaceutical combination according to technical solution 29, wherein the compound of formula (I) is (R)-N-(4-(chlorodifluoromethoxy)phenyl)-6-(3-fluoropyrrolidin-1-yl)-5-(pyrazin-2-yl)nicotinamide (Compound A), or a pharmaceutically acceptable salt, crystal form, solvate, or hydrate thereof.
Technical solution 31. The pharmaceutical combination according to technical solution 29 or 30, wherein the ATP-

competitive BCR-ABL1 inhibitor(s) is selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228;

alternatively, the ATP-competitive BCR-ABL1 inhibitor(s) is selected from imatinib, dasatinib, nilotinib, bosutinib, flumatinib, ponatinib, olverembatinib or PF-114;
alternatively, the ATP-competitive BCR-ABL1 inhibitor(s) is selected from imatinib, nilotinib, radotinib, flumatinib, ponatinib, bafetinib, CHMFL-ABL-039, CHMFL-ABL-053, CHMFL-ABL-KIT-155, vodobatinib, PF-114, olverembatinib or rebastinib;
alternatively, the ATP-competitive BCR-ABL1 inhibitor(s) is selected from ponatinib, PF-114, olverembatinib or rebastinib;
alternatively, the ATP-competitive BCR-ABL1 inhibitor(s) is selected from ponatinib, olverembatinib or PF-114;
alternatively, the ATP-competitive BCR-ABL1 inhibitor(s) is selected from ponatinib or olverembatinib;
alternatively, the ATP-competitive BCR-ABL1 inhibitor(s) is selected from ponatinib.

Technical Solution 32. The pharmaceutical combination according to any one of Technical Solutions 29-31, wherein the dose of the compound of formula (I) or pharmaceutically acceptable salt, crystal form, solvate, or hydrate thereof (alternatively compound A) is about 1-500 mg;

optionally, wherein the dose of the compound of formula (I) or pharmaceutically acceptable salt, crystal form, solvate, or hydrate thereof (alternatively compound A) is about 1-250 mg;
optionally, wherein the dose of the compound of formula (I) or pharmaceutically acceptable salt, crystal form, solvate, or hydrate thereof (alternatively compound A) is about 1 mg, 2 mg, 3 mg, 4 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg or 250 mg;
optionally, wherein the dose of the compound of formula (I) or pharmaceutically acceptable salt, crystal form, solvate, or hydrate thereof (alternatively compound A) is about 1 mg, 2 mg, 3 mg, 4 mg, 5 mg, 10 mg, 15 mg, 20 mg, 25 mg, 30 mg, 35 mg, 40 mg, 45 mg, 50 mg, 55 mg, 60 mg, 80 mg, 160 mg or 240 mg;
optionally, wherein the compound of formula (I) or a pharmaceutically acceptable salt, crystal form, solvate, or hydrate thereof (alternatively compound A) is administered once daily or twice daily.

Technical Solution 33. The pharmaceutical combination according to any one of Technical Solutions 29-32, wherein the dose of the ATP-competitive BCR-ABL1 inhibitor is about 0.1-200 mg, alternatively about 0.1-60 mg.
Technical Solution 34. The pharmaceutical combination according to Technical Solution 32, wherein the ATP-competitive BCR-ABL1 inhibitor is ponatinib at a dose of about 0.1-200 mg, alternatively about 0.1-60 mg;

alternatively, the dose of ponatinib is 0.1 mg, 0.5 mg, 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 20.5 mg, 21 mg, 21.5 mg, 22 mg, 22.5 mg, 23 mg, 23.5 mg, 24 mg, 24.5 mg, 25 mg, 25.5 mg, 26 mg, 26.5 mg, 27 mg, 27.5 mg, 28 mg, 28.5 mg, 29 mg, 29.5 mg, 30 mg, 30.5 mg, 31 mg, 31.5 mg, 32 mg, 32.5 mg, 33 mg, 33.5 mg, 34 mg, 34.5 mg, 35 mg, 35.5 mg, 36 mg, 36.5 mg, 37 mg, 37.5 mg, 38 mg, 38.5 mg, 39 mg, 39.5 mg, 40 mg, 40.5 mg, 41 mg, 41.5 mg, 42 mg, 42.5 mg, 43 mg, 43.5 mg, 44 mg, 44.5 mg, 45 mg, 45.5 mg, 46 mg, 46.5 mg, 47 mg, 47.5 mg, 48 mg, 48.5 mg, 49 mg, 49.5 mg, 50 mg, 50.5 mg, 51 mg, 51.5 mg, 52 mg, 52.5 mg, 53 mg, 53.5 mg, 54 mg, 54.5 mg, 55 mg, 55.5 mg, 56 mg, 56.5 mg, 57 mg, 57.5 mg, 58 mg, 58.5 mg, 59 mg, 59.5 mg or 60 mg;
alternatively, the dose of ponatinib is 2 mg, 4 mg, 8 mg, 15 mg, 30 mg, 45 mg or 60 mg;
alternatively, ponatinib is administered once daily or twice daily.

Technical Solution 35. The pharmaceutical combination according to Technical Solution 32, wherein the ATP-competitive BCR-ABL1 inhibitor is olverembatinib at a dose of about 0.1-200 mg, alternatively about 0.1-60 mg;

alternatively, the dose of olverembatinib is 0.1 mg, 0.5 mg, 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 20.5 mg, 21 mg, 21.5 mg, 22 mg, 22.5 mg, 23 mg, 23.5 mg, 24 mg, 24.5 mg, 25 mg, 25.5 mg, 26

mg, 26.5 mg, 27 mg, 27.5 mg, 28 mg, 28.5 mg, 29 mg, 29.5 mg, 30 mg, 30.5 mg, 31 mg, 31.5 mg, 32 mg, 32.5 mg, 33 mg, 33.5 mg, 34 mg, 34.5 mg, 35 mg, 35.5 mg, 36 mg, 36.5 mg, 37 mg, 37.5 mg, 38 mg, 38.5 mg, 39 mg, 39.5 mg, 40 mg, 40.5 mg, 41 mg, 41.5 mg, 42 mg, 42.5 mg, 43 mg, 43.5 mg, 44 mg, 44.5 mg, 45 mg, 45.5 mg, 46 mg, 46.5 mg, 47 mg, 47.5 mg, 48 mg, 48.5 mg, 49 mg, 49.5 mg, 50 mg, 50.5 mg, 51 mg, 51.5 mg, 52 mg, 52.5 mg, 53 mg, 53.5 mg, 54 mg, 54.5 mg, 55 mg, 55.5 mg, 56 mg, 56.5 mg, 57 mg, 57.5 mg, 58 mg, 58.5 mg, 59 mg, 59.5 mg or 60 mg;

alternatively, the dose of olverembatinib is 1 mg, 2 mg, 4 mg, 8 mg, 12 mg, 20 mg, 30 mg, 40 mg, 45 mg, 50 mg or 60 mg;

alternatively, olverembatinib is administered once daily or twice daily.

Technical Solution 36. The pharmaceutical combination according to any one of Technical Solutions 29-35, wherein the (a) compound of formula (I) or a pharmaceutically acceptable salt, crystal form, solvate, or hydrate thereof; and (b) ATP-competitive BCR-ABL1 inhibitor or a pharmaceutically acceptable salt, crystal form, solvate, or hydrate thereof are present in the same dosage form.

Technical Solution 37. The pharmaceutical combination according to any one of Technical Solutions 29-35, wherein the (a) compound of formula (I) or a pharmaceutically acceptable salt, crystal form, solvate, or hydrate thereof; and (b) ATP-competitive BCR-ABL1 inhibitor or a pharmaceutically acceptable salt, crystal form, solvate, or hydrate thereof are present in different dosage forms.

Technical Solution 38. A pharmaceutical composition comprising the pharmaceutical combination of any one of Technical Solutions 29-35.

Technical Solution 39. Use of (a) the compound of formula (I) of Technical Solution 1 or a pharmaceutically acceptable salt, crystal form, solvate, or hydrate thereof and (b) an ATP-competitive BCR-ABL1 inhibitor or a pharmaceutically acceptable salt, crystal form, solvate, or hydrate thereof in the manufacture of a medicament for treating a disease regulated by wild-type or mutant BCR-ABL1 kinase.

Technical Solution 40. Use of (a) the compound of formula (I) of Technical Solution 1 or a pharmaceutically acceptable salt, crystal form, solvate, or hydrate thereof in the manufacture of a medicament for use in combination with (b) an ATP-competitive BCR-ABL1 inhibitor or a pharmaceutically acceptable salt, crystal form, solvate, or hydrate thereof for treating a disease regulated by wild-type or mutant BCR-ABL1 kinase.

Technical Solution 41. Use of (b) an ATP-competitive BCR-ABL1 inhibitor or a pharmaceutically acceptable salt, crystal form, solvate, or hydrate thereof in the manufacture of a medicament for use in combination with (a) the compound of formula (I) of Technical Solution 1 or a pharmaceutically acceptable salt, crystal form, solvate, or hydrate thereof for treating a disease regulated by wild-type or mutant BCR-ABL1 kinase.

Technical Solution 42. A pharmaceutical combination of (a) the compound of formula (I) of Technical Solution 1 or a pharmaceutically acceptable salt, crystal form, solvate, or hydrate thereof and (b) an ATP-competitive BCR-ABL1 inhibitor or a pharmaceutically acceptable salt, crystal form, solvate, or hydrate thereof, for use in treating a disease regulated by wild-type or mutant BCR-ABL1 kinase.

Technical Solution 43. (a) The compound of formula (I) of Technical Solution 1 or a pharmaceutically acceptable salt, crystal form, solvate, or hydrate thereof, for use in combination with (b) an ATP-competitive BCR-ABL1 inhibitor or a pharmaceutically acceptable salt, crystal form, solvate, or hydrate thereof, for treating a disease regulated by wild-type or mutant BCR-ABL1 kinase.

Technical Solution 44. (b) An ATP-competitive BCR-ABL1 inhibitor or a pharmaceutically acceptable salt, crystal form, solvate, or hydrate thereof, for use in combination with (a) the compound of formula (I) of Technical Solution 1 or a pharmaceutically acceptable salt, crystal form, solvate, or hydrate thereof, for treating a disease regulated by wild-type or mutant BCR-ABL1 kinase.

Technical Solution 45. A method for treating a disease regulated by wild-type or mutant BCR-ABL1 kinase, comprising administering to a patient in need thereof (a) the compound of formula (I) of Technical Solution 1 or a pharmaceutically acceptable salt, crystal form, solvate, or hydrate thereof, and (b) an ATP-competitive BCR-ABL1 inhibitor or a pharmaceutically acceptable salt, crystal form, solvate, or hydrate thereof.

Technical Solution 46. The method according to Technical Solution 45, wherein (a) and (b) are administered simultaneously, separately, or sequentially.

Technical Solution 47. The use according to any one of Technical Solutions 39-41, or the compound and/or inhibitor for use according to any one of Technical Solutions 42-44, or the method according to Technical Solution 45 or 46, wherein the disease regulated by the wild-type and/or mutant BCR-ABL1 kinase is a hematological malignancy or central nervous system leukemia relapse thereof, such as brain metastasis;

alternatively, the disease regulated by the wild-type and/or mutant BCR-ABL1 kinase is leukemia, neurodegenerative disease, neurofibroma, melanoma, breast cancer, colon cancer, lung cancer, prostate cancer, Kaposi’s sarcoma, gastrointestinal stromal tumor, mesothelioma, systemic mastocytosis, hypereosinophilic syndrome,

liver fibrosis, renal fibrosis, rheumatoid arthritis, polyarthritis, scleroderma, lupus erythematosus, graft-versus-host disease, pulmonary arterial hypertension, seminoma, dysgerminoma, and psoriasis;
alternatively, the leukemia is acute lymphoblastic leukemia, acute myeloid leukemia, acute promyelocytic leukemia, chronic myeloid leukemia, chronic myelomonocytic leukemia, chronic neutrophilic leukemia, acute undifferentiated leukemia, prolymphocytic leukemia, juvenile myelomonocytic leukemia, adult T-cell leukemia, acute myeloid leukemia with trilineage myelodysplasia, and mixed lineage leukemia;
alternatively, the leukemia is chronic myeloid leukemia and Philadelphia chromosome-positive acute lymphoblastic leukemia;
alternatively, the neurodegenerative disease is Parkinson's disease, Alzheimer's disease, Down syndrome, memory and cognitive impairment, dementia, amyloid neuropathy, or cerebral inflammation;
alternatively, the disease regulated by wild-type and/or mutant BCR-ABL1 kinase is chronic myeloid leukemia, Philadelphia chromosome-positive acute lymphoblastic leukemia, Parkinson's disease, Alzheimer's disease, neurofibromatosis, gastrointestinal stromal tumor, systemic mastocytosis, hypereosinophilic syndrome, liver fibrosis, renal fibrosis, or scleroderma.

Technical Solution 48. The use or compound and/or inhibitor for use or method according to any one of Technical Solutions 39-47, wherein the mutation of the mutant BCR-ABL1 kinase is one or more of T315I, T315M, F317L, E355G, V299L, M244V, G250E, Q252H, Y253H, E255K, E255V, H396P, H396R, H396A, A397P, P223S, K294E, M351T, F359V, E459K, P465S, V468F, I502L, A337V or A424T;

alternatively, the mutation of the mutant BCR-ABL1 kinase is one or more of T315I, T315M, Y253H/T315I, E255V/T315I, Q252H/T315I, F317L/T315I, F359V/T315I, Y253H/E255K, Y253H/F359V;
alternatively, the mutation of the mutant BCR-ABL1 kinase is T315I, T315M, Y253H/T315I, E255V/T315I or Q252H/T315I;
alternatively, the mutation of the mutant BCR-ABL1 kinase is T315M, Y253H/T315I, E255V/T315I or Q252H/T315I.

Technical Solution 49. The use or compound and/or inhibitor for use or method according to any one of Technical Solutions 39-48, wherein the compound of formula (I) is (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof.
Technical Solution 50. The use or compound and/or inhibitor for use or method according to any one of Technical Solutions 39-49, wherein the ATP-competitive BCR-ABL1 inhibitor(s) is selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228;

alternatively, the ATP-competitive BCR-ABL1 inhibitor(s) is selected from imatinib, dasatinib, nilotinib, bosutinib, flumatinib, ponatinib, olverembatinib or PF-114;
alternatively, the ATP-competitive BCR-ABL1 inhibitor(s) is selected from imatinib, nilotinib, radotinib, flumatinib, ponatinib, bafetinib, CHMFL-ABL-039, CHMFL-ABL-053, CHMFL-ABL-KIT-155, vodobatinib, PF-114, olverembatinib or rebastinib;
alternatively, the ATP-competitive BCR-ABL1 inhibitor(s) is selected from ponatinib, PF-114, olverembatinib or rebastinib;
alternatively, the ATP-competitive BCR-ABL1 inhibitor(s) is selected from ponatinib, olverembatinib or PF-114;
alternatively, the ATP-competitive BCR-ABL1 inhibitor(s) is selected from ponatinib or olverembatinib;
alternatively, the ATP-competitive BCR-ABL1 inhibitor(s) is selected from ponatinib.

Technical Solution 51. The use or compound and/or inhibitor for use or method according to any one of Technical Solutions 39-50, wherein the dose of the compound of formula (I) or pharmaceutically acceptable salt, crystal form, solvate, or hydrate thereof (alternatively compound A) is about 1-500 mg;

optionally, wherein the dose of the compound of formula (I) or pharmaceutically acceptable salt, crystal form, solvate, or hydrate thereof (alternatively compound A) is about 1-250 mg;
optionally, wherein the dose of the compound of formula (I) or pharmaceutically acceptable salt, crystal form, solvate, or hydrate thereof (alternatively compound A) is about 1 mg, 2 mg, 3 mg, 4 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235

mg, 240 mg, 245 mg or 250 mg;
optionally, wherein the dose of the compound of formula (I) or pharmaceutically acceptable salt, crystal form, solvate, or hydrate thereof (alternatively compound A) is about 1 mg, 2 mg, 3 mg, 4 mg, 5 mg, 10 mg, 15 mg, 20 mg, 25 mg, 30 mg, 35 mg, 40 mg, 45 mg, 50 mg, 55 mg, 60 mg, 80 mg, 160 mg or 240 mg;
optionally, wherein the compound of formula (I) or a pharmaceutically acceptable salt, crystal form, solvate, or hydrate thereof (alternatively compound A) is administered once daily or twice daily.

Technical Solution 52. The use or compound and/or inhibitor for use or method according to any one of Technical Solutions 39-51, wherein the dose of the ATP-competitive BCR-ABL1 inhibitor is about 0.1-200 mg, alternatively about 0.1-60 mg.
Technical Solution 53. The use or compound and/or inhibitor for use or method according to Technical Solution 52, wherein the ATP-competitive BCR-ABL1 inhibitor is ponatinib at a dose of about 0.1-200 mg, alternatively about 0.1-60 mg;

alternatively, the dose of ponatinib is 0.1 mg, 0.5 mg, 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 20.5 mg, 21 mg, 21.5 mg, 22 mg, 22.5 mg, 23 mg, 23.5 mg, 24 mg, 24.5 mg, 25 mg, 25.5 mg, 26 mg, 26.5 mg, 27 mg, 27.5 mg, 28 mg, 28.5 mg, 29 mg, 29.5 mg, 30 mg, 30.5 mg, 31 mg, 31.5 mg, 32 mg, 32.5 mg, 33 mg, 33.5 mg, 34 mg, 34.5 mg, 35 mg, 35.5 mg, 36 mg, 36.5 mg, 37 mg, 37.5 mg, 38 mg, 38.5 mg, 39 mg, 39.5 mg, 40 mg, 40.5 mg, 41 mg, 41.5 mg, 42 mg, 42.5 mg, 43 mg, 43.5 mg, 44 mg, 44.5 mg, 45 mg, 45.5 mg, 46 mg, 46.5 mg, 47 mg, 47.5 mg, 48 mg, 48.5 mg, 49 mg, 49.5 mg, 50 mg, 50.5 mg, 51 mg, 51.5 mg, 52 mg, 52.5 mg, 53 mg, 53.5 mg, 54 mg, 54.5 mg, 55 mg, 55.5 mg, 56 mg, 56.5 mg, 57 mg, 57.5 mg, 58 mg, 58.5 mg, 59 mg, 59.5 mg or 60 mg;
alternatively, the dose of ponatinib is 2 mg, 4 mg, 8 mg, 15 mg, 30 mg, 45 mg or 60 mg;
alternatively, ponatinib is administered once daily or twice daily.

Technical Solution 54. The use or compound and/or inhibitor for use or method according to Technical Solution 52, wherein the ATP-competitive BCR-ABL1 inhibitor is olverembatinib at a dose of about 0.1-200 mg, alternatively about 0.1-60 mg;

alternatively, the dose of olverembatinib is 0.1 mg, 0.5 mg, 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 20.5 mg, 21 mg, 21.5 mg, 22 mg, 22.5 mg, 23 mg, 23.5 mg, 24 mg, 24.5 mg, 25 mg, 25.5 mg, 26 mg, 26.5 mg, 27 mg, 27.5 mg, 28 mg, 28.5 mg, 29 mg, 29.5 mg, 30 mg, 30.5 mg, 31 mg, 31.5 mg, 32 mg, 32.5 mg, 33 mg, 33.5 mg, 34 mg, 34.5 mg, 35 mg, 35.5 mg, 36 mg, 36.5 mg, 37 mg, 37.5 mg, 38 mg, 38.5 mg, 39 mg, 39.5 mg, 40 mg, 40.5 mg, 41 mg, 41.5 mg, 42 mg, 42.5 mg, 43 mg, 43.5 mg, 44 mg, 44.5 mg, 45 mg, 45.5 mg, 46 mg, 46.5 mg, 47 mg, 47.5 mg, 48 mg, 48.5 mg, 49 mg, 49.5 mg, 50 mg, 50.5 mg, 51 mg, 51.5 mg, 52 mg, 52.5 mg, 53 mg, 53.5 mg, 54 mg, 54.5 mg, 55 mg, 55.5 mg, 56 mg, 56.5 mg, 57 mg, 57.5 mg, 58 mg, 58.5 mg, 59 mg, 59.5 mg or 60 mg;
alternatively, the dose of olverembatinib is 1 mg, 2 mg, 4 mg, 8 mg, 12 mg, 20 mg, 30 mg, 40 mg, 45 mg, 50 mg or 60 mg;
alternatively, olverembatinib is administered once daily or twice daily.

Technical Solution 55. The use or compound and/or inhibitor for use or method according to any one of Technical Solutions 39-54, wherein the (a) compound of formula (I) or a pharmaceutically acceptable salt, crystal form, solvate, or hydrate thereof; and (b) ATP-competitive BCR-ABL1 inhibitor or a pharmaceutically acceptable salt, crystal form, solvate, or hydrate thereof are present in the same dosage form.
Technical Solution 56. The use or compound and/or inhibitor for use or method according to any one of Technical Solutions 39-54, wherein the (a) compound of formula (I) or a pharmaceutically acceptable salt, crystal form, solvate, or hydrate thereof; and (b) ATP-competitive BCR-ABL1 inhibitor or a pharmaceutically acceptable salt, crystal form, solvate, or hydrate thereof are present in different dosage forms.
Technical Solution 57. A method for inhibiting a mutant BCR-ABL1 kinase, comprising administering:

(a) a compound of formula (I) according to Technical Solution 1, or a pharmaceutically acceptable salt, crystal form, solvate, or hydrate thereof; and
(b) an ATP-competitive BCR-ABL1 inhibitor, or a pharmaceutically acceptable salt, crystal form, solvate, or

hydrate thereof.

Technical Solution 58. The method according to Technical Solution 57, wherein the mutant BCR-ABL1 kinase is selected from one or more of T315I, T315M, F317L, E355G, V299L, M244V, G250E, Q252H, Y253H, E255K, E255V, H396P, H396R, H396A, A397P, P223S, K294E, M351T, F359V, E459K, P465S, V468F, I502L, A337V or A424T;

alternatively, the mutant BCR-ABL1 kinase is T315I, T315M, Y253H/T315I, E255V/T315I, Q252H/T315I, F317L/T315I, F359V/T315I, Y253H/E255K or Y253H/F359V;
alternatively, the mutant BCR-ABL1 kinase is T315I, T315M, Y253H/T315I, E255V/T315I or Q252H/T315I;
alternatively, the mutant BCR-ABL1 kinase is T315M, Y253H/T315I, E255V/T315I or Q252H/T315I.

Technical Solution 59. A commercial package, comprising:

(a) Compound A of Technical Solution 4 or a pharmaceutically acceptable salt, crystal form, solvate, or hydrate thereof; and
(b) an ATP-competitive BCR-ABL1 inhibitor or a pharmaceutically acceptable salt, crystal form, solvate, or hydrate thereof;

wherein (a) and (b) are present in a single unit dosage form or in two separate unit dosage forms.
Technical Solution 60. A commercial package comprising:

(a) Compound A of Technical Solution 4 or a pharmaceutically acceptable salt, crystal form, solvate, or hydrate thereof; and
(b) Ponatinib or a pharmaceutically acceptable salt, crystal form, solvate, or hydrate thereof;

wherein (a) and (b) are present in a single unit dosage form or in two separate unit dosage forms.
Technical Solution 61. A commercial package comprising:

(a) Compound A of Technical Solution 4 or a pharmaceutically acceptable salt, crystal form, solvate, or hydrate thereof; and
(b) Olverembatinib or a pharmaceutically acceptable salt, crystal form, solvate, or hydrate thereof;

wherein (a) and (b) are present in a single unit dosage form or in two separate unit dosage forms.
Technical Solution 62. A commercial package comprising:

(a) Compound A of Technical Solution 4 or a pharmaceutically acceptable salt, crystal form, solvate, or hydrate thereof; and
(b) PF-114 or a pharmaceutically acceptable salt, crystal form, solvate, or hydrate thereof;

wherein (a) and (b) are present in a single unit dosage form or in two separate unit dosage forms.
Technical Solution 63. The commercial package according to any one of Technical Solutions 59-62, wherein the unit dosage form is a fixed combination.
Technical Solution 64. The commercial packaging according to any one of Technical Solutions 59-63, for use in treating a disease regulated by wild-type or mutant BCR-ABL1 kinase.

Other objects and advantages of the present disclosure will be apparent to those skilled in the art from the following detailed description, examples, and claims.

## BRIEF DESCRIPTION OF THE DRAWINGS

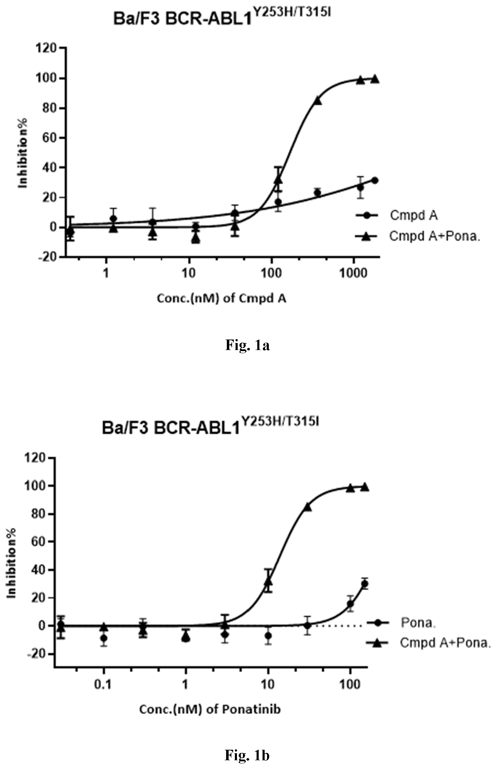

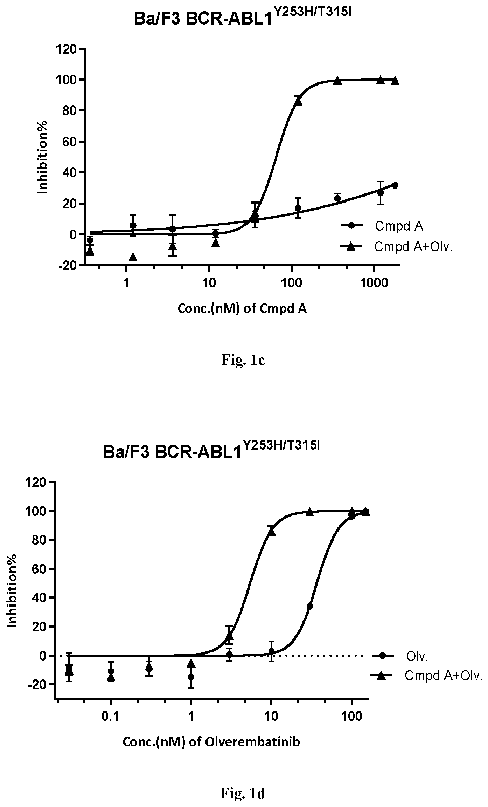

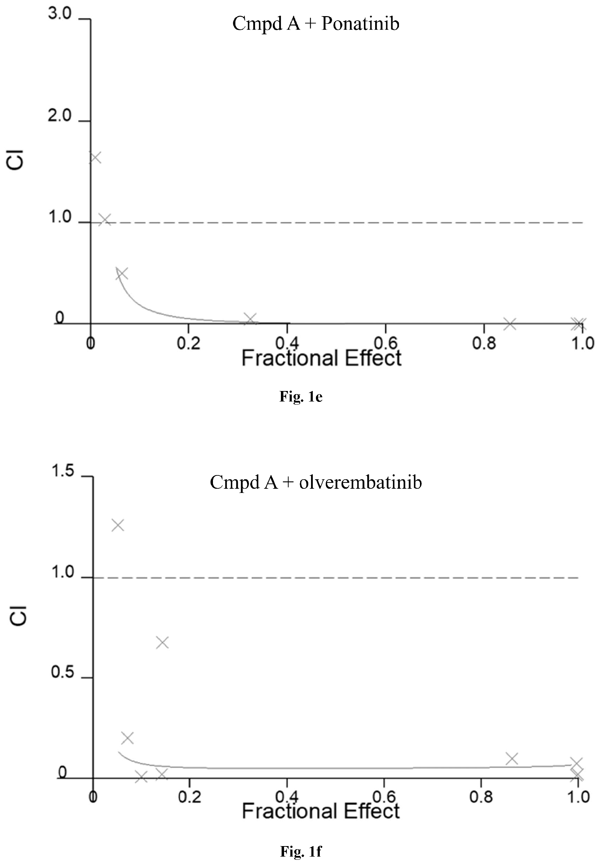

Fig. 1 shows the inhibitory effects of Compound A, ponatinib, and olverembatinib alone and in combination on BCR-ABL1 Y253H/T315I cells. Specifically, Figs. 1a and 1b show the effects of Compound A and ponatinib alone and in combination, Figs. 1c and 1d show the effects of Compound A and olverembatinib alone and in combination, and Figs. 1e and 1f show the CI coefficients of the two combinations, respectively (both less than 0.3).

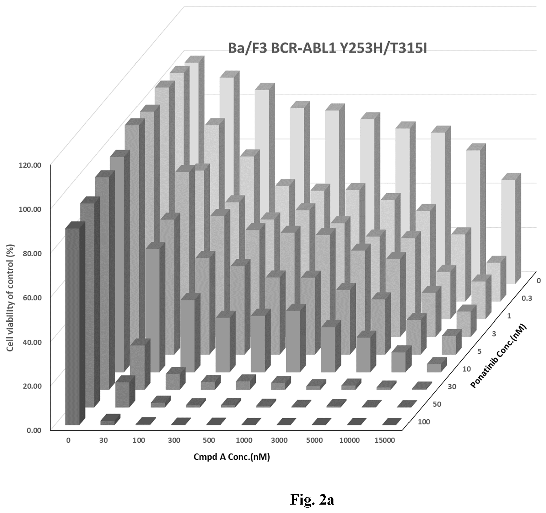

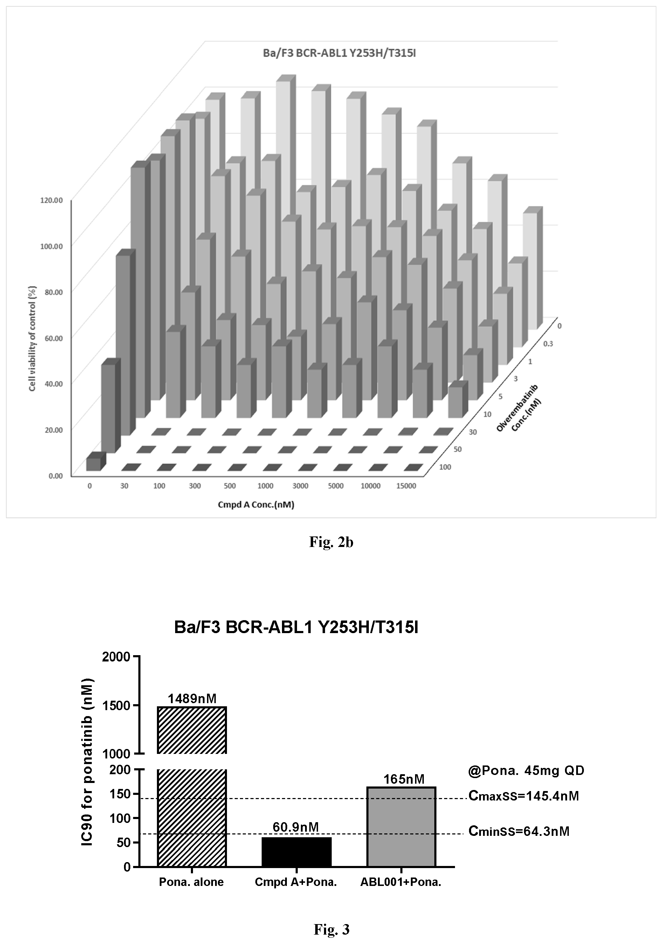

Fig. 2 shows the inhibitory effects of Compound A in combination with ponatinib and olverembatinib on the viability of Ba/F3 BCR-ABL1 Y253H/T315I cells. Specifically, Fig. 2a illustrates the combination of Compound A with ponatinib,

while Fig. 2b illustrates the combination of Compound A with olverembatinib.

Fig. 3 shows the inhibitory effects of ponatinib alone or in combination with Compound A and asciminib (ABL001) on the viability of Ba/F3 BCR-ABL1 Y253H/T315I cells.

Fig. 4 shows the inhibitory effects of ponatinib alone or in combination with Compound A and two concentrations of asciminib on the viability of Ba/F3 BCR-ABL11 E255V/T315I cells.

Fig. 5 shows the inhibitory effects of ponatinib alone or in combination with Compound A and two concentrations of asciminib on the viability of Ba/F3 BCR-ABL1 Q252H/T315I cells.

Fig. 6 shows the inhibitory effects of ponatinib alone or in combination with Compound A and two concentrations of asciminib on the viability of Ba/F3 BCR-ABL1 T315M cells.

Fig. 7 shows the inhibitory effects of ponatinib alone, Compound A alone, and their combination on the viability of Ba/F3 BCR-ABL11 T315I, Y253H/F359V, Y253H/E255K, and F359V cells.

Fig. 8 shows the changes in bioluminescence imaging (BLI) fluorescence intensity over time in each group of mice treated with Compound A in the K562-luc brain metastasis model.

## DETAILED DESCRIPTION OF THE INVENTION

The present disclosure has discovered that compounds of formula (I) can be used for treating CML in chronic phase and/or accelerated phase. Accordingly, disclosed herein are uses and methods of compounds of formula (I) or pharmaceutically acceptable salts thereof for treating CML in chronic phase and/or accelerated phase.

Abbreviations

| Abbreviation | Full English Name | Full Chinese Name |
|---|---|---|
| AE or AEs | Adverse Effect(s) | 不良反应 |
| AP | Accelerated phase | 加速期 |
| ATP | Adenosine Triphosphate | 腺苷三磷酸 |
| BCR-ABL1+ | Chimeric BCR-ABL1 oncogene positive | 嵌合 BCR-ABL 1癌基因阳性 |
| BID | bis in die/twice a day | 每日两次 |
| BP | Blast phase | 急变期 |
| CCyR | Complete cytogenetic response | 完全细胞遗传学反应 |
|  | Cell viability (% of control) | 细胞活力(相对于对照的百 分比) |
| CHR | Complete Hematologic response | 完全血液学反应 |
| CML | Chronic myeloid leukemia/Chronic mye-logenous leukemia | 慢性粒细胞白血病/慢性髓系白血病 |
| CML-AP | Chronic myeloid leukemia in accelerated phase | 加速期慢性粒细胞白血病 |
| CML-BP | Chronic myeloid leukemia in blast phase | 急变期慢性粒细胞白血病 |
| CML-CP | Chronic myeloid leukemia in chronic phase | 慢性期慢性粒细胞白血病 |
| Cmpd A | Compound A | 化合物 A |
| Conc. | Concentration | 浓度 |

(continued)

| Abbreviation | Full English Name | Full Chinese Name |
|---|---|---|
| CP | Chronic phase | 慢性期 |
| CyR | Cytogenetic response | 细胞遗传学反应 |
| DLT | Dose limited toxicity | 剂量限制毒性 |
| DOR | Duration of Overall Response | 缓解持续时间 |
| EFS | Event Free Survival | 无事件生存期 |
| FAS | Full Analysis Set | 全分析集 |
| HR | Hematologic response | 血液学反应 |
| | Inhibition % | 抑制 % |
| IMDM | Iscove's Modified Dulbecco's Medium | I scove改良杜氏培养基 |
| MaHR | Major Hematological Reactions | 主要血液学反应 |
| MCyR | Major Cytogenetic Response | 主要细胞遗传学反应 |
| MMR | Major Molecular Response | 主要分子学反应 |
| MR | Molecular Response | 分子学反应 |
| MTD | Maximum tolerated dose | 最大耐受剂量 |
| NOAEL | No Observed Adverse Effect Level | 未见明显毒性反应剂量 |
| Olv. | Olverembatinib | 奥雷巴替尼 |
| OS | Overall survival | 总生存期 |
| PBS | Phosphate Buffered Saline | 磷酸盐缓冲盐水 |
| PCyR | Partial Cytogenetic Response | 部分细胞遗传学反应 |
| PFS | Progression-Free-Survival | 无进展生存期 |
| Ph+ | Philadelphia chromosome positive | 费城染色体阳性 |
| Ph+ CML | Philadelphia chromosome-positive chronic myeloid leukemia | 费城染色体阳性慢性粒细胞白血病 |
| PK | Pharmacokinetic | 药代动力学 |
| Pona. | Ponatinib | 帕纳替尼 |
| QD | Quaque die/Once daily | 每日一次 |
| RES | Response Evaluation Set | 疗效可评估集 |
| RP2D | Recommended phase 2 dose | 二期推荐剂量 |

(continued)

| Abbreviation | Full English Name | Full Chinese Name |
|---|---|---|
| SAE or SAEs | Severe Adverse Effect(s) | 严重不良反应 |
| SPF | Specific pathogen Free | 无特定病原体 |
| TID | ter in die/three times a day | 每日三次 |
| TKI | Tyrosine kinase inhibitor | 酪氨酸激酶抑制剂 |
| TTR | Time to Response | 至起效时间 |

## Definition

The present disclosure may be more readily understood by reference to the following detailed description of embodiments of the present disclosure and the examples included herein. It is to be understood that the terminology used herein is for the purpose of describing specific embodiments only and is not intended to be limiting. It is further to be understood that unless specifically defined herein, the terminology used herein is to be given its ordinary meaning as known in the relevant art.

Chemical definitions

Definitions of specific functional groups and chemical terms are described in more detail below.

When a range of values is listed, it is intended to encompass each value and sub-range within the range. For example "$C_{1-6}$ alkyl" is intended to encompass, $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$ and $C_{5-6}$ alkyl.

It should be understood that when described herein any of the moieties defined forth below may be substituted with a variety of substituents, and that the respective definitions are intended to include such substituted moieties within their scope as set out below. Unless otherwise stated, the term "substituted" is to be defined as set out below.

"$C_{1-6}$ alkyl" refers to a radical of a straight-chain or branched saturated hydrocarbon group having from 1 to 6 carbon atoms, and it is also referred to herein as "lower alkyl". In some embodiments, $C_{1-4}$ alkyl is particularly alternative. Examples of alkyl groups include, but are not limited to: methyl ($C_1$), ethyl ($C_2$), n-propyl ($C_3$), isopropyl ($C_3$), n-butyl ($C_4$), tert-butyl ($C_4$), sec-butyl ($C_4$), iso-butyl ($C_4$), n-pentyl ($C_5$), 3-pentanyl ($C_5$), amyl ($C_5$), neopentyl ($C_5$), 3-methyl-2-butanyl ($C_5$), tertiary amyl ($C_5$), and n-hexyl ($C_6$). Unless otherwise specified, each instance of an alkyl group is independently optionally substituted, *i.e.*, unsubstituted (an "unsubstituted alkyl") or substituted (a "substituted alkyl") with one or more substituents; *e.g.,* for instance from 1 to 5 substituents, 1 to 3 substituents, or 1 substituent. In certain embodiments, the alkyl group is unsubstituted $C_{1-6}$ alkyl (*e.g.,* $-CH_3$). In certain embodiments, the alkyl group is substituted $C_{1-6}$ alkyl.

"$C_{1-6}$ alkoxy" refers to the group -OR wherein R is a substituted or unsubstituted $C_{1-6}$ alkyl group. In some embodiments, $C_{1-4}$ alkoxy group is particularly alternative. Specific alkoxy groups include, but are not limited to, methoxy, ethoxy, n-propoxy, isopropoxy, n-butoxy, tert-butoxy, sec-butoxy, n-pentyloxy, n-hexyloxy and 1,2-dimethylbutoxy.

"Halo" or "halogen" means fluorine (F), chlorine (Cl), bromine (Br) and iodine (I). In some embodiments, the halo group is F, -Cl or Br. In some embodiments, the halogen group is Cl. In some embodiments, the halogen group is F. In some embodiments, the halogen group is Br.

Thus, "$C_{1-6}$ haloalkyl" refers to the above "$C_{1-6}$ alkyl", which is substituted by one or more halo groups. In some embodiments, $C_{1-4}$ haloalkyl group is particularly alternative, and still alternatively $C_{1-2}$ haloalkyl group. Exemplary haloalkyl groups include, but are not limited to, $-CF_3$, $-CH_2F$, $-CHF_2$, $-CClF_2$, $-CHFCH_2F$, $-CH_2CHF_2$, $-CF_2CF_3$, $-CF_2CClF_2$, $-CF_2CH_3$, $-CCl_3$, $-CH_2Cl$, $-CHCl_2$, 2,2,2-trifluoro-1,1-dimethyl-ethyl, and the like.

"$C_{3-7}$ cycloalkyl" refers to a radical of a non-aromatic cyclic hydrocarbon group having from 3 to 7 ring carbon atoms and zero heteroatoms. In some embodiments, $C_{3-6}$ cycloalkyl is especially alternative, and $C_{5-6}$ cycloalkyl is still alternative. Cycloalkyl also includes ring systems wherein the cycloalkyl ring, as defined above, is fused with one or more aryl or heteroaryl groups wherein the point of attachment is on the cycloalkyl ring, and in such instances, the number of carbons continue to designate the number of carbons in the cycloalkyl ring system. Exemplary cycloalkyl groups include, but are not limited to, cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), cycloheptyl ($C_7$), cycloheptenyl ($C_7$), cycloheptadienyl ($C_7$), cycloheptatrienyl ($C_7$), and the like. Unless otherwise specified, each instance of a cycloalkyl group is independently optionally substituted, *i.e.*, unsubstituted (an "unsubstituted cycloalkyl") or substituted (a "substituted

cycloalkyl") with one or more substituents. In certain embodiments, the cycloalkyl group is unsubstituted $C_{3-7}$ cycloalkyl. In certain embodiments, the cycloalkyl group is a substituted $C_{3-7}$ cycloalkyl.

"$C_{3-7}$ heterocycloalkyl" refers to a radical of a 3- to 7-membered non-aromatic ring system having ring carbon atoms and 1 to 4 ring heteroatoms, wherein each heteroatom is independently selected from nitrogen, oxygen, sulfur, boron, phosphorus, and silicon. In heterocycloalkyl groups that contain one or more nitrogen atoms, the point of attachment can be a carbon or nitrogen atom, as valency permits. In some embodiments, $C_{3-6}$ heterocycloalkyl is especially alternative, which is a radical of a 3- to 6-membered non-aromatic ring system having ring carbon atoms and 1 to 3 ring heteroatoms. In some embodiments, $C_{5-6}$ heterocycloalkyl is still alternative, which is a radical of a 5- to 6-membered non-aromatic ring system having ring carbon atoms and 1 to 3 ring heteroatoms. Unless otherwise specified, each instance of heterocycloalkyl is independently optionally substituted, *i.e.*, unsubstituted (an "unsubstituted heterocycloalkyl") or substituted (a "substituted heterocycloalkyl") with one or more substituents. In certain embodiments, the heterocycloalkyl group is unsubstituted $C_{3-7}$ heterocycloalkyl. In certain embodiments, the heterocycloalkyl group is substituted $C_{3-7}$ heterocycloalkyl. Heterocycloalkyl also includes ring systems wherein the heterocycloalkyl ring, as defined above, is fused with one or more cycloalkyl groups, wherein the point of attachment is on the cycloalkyl ring; or wherein the heterocycloalkyl ring, as defined above, is fused with one or more aryl or heteroaryl groups, wherein the point of attachment is on the heterocycloalkyl ring; and in such instances, the number of ring members continue to designate the number of ring members in the heterocycloalkyl ring system. Exemplary 3-membered heterocycloalkyl groups containing one heteroatom include, without limitation, azirdinyl, oxiranyl, thiorenyl. Exemplary 4-membered heterocycloalkyl groups containing one heteroatom include, without limitation, azetidinyl, oxetanyl and thietanyl. Exemplary 5-membered heterocycloalkyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocycloalkyl groups containing two heteroatoms include, without limitation, dioxolanyl, oxasulfuranyl, disulfuranyl, and oxazolidin-2-one. Exemplary 5-membered heterocycloalkyl groups containing three heteroatoms include, without limitation, triazolinyl, oxadiazolinyl, and thiadiazolinyl. Exemplary 6-membered heterocycloalkyl groups containing one heteroatom include, without limitation, piperidinyl, tetrahydropyranyl, dihydropyridinyl, and thianyl. Exemplary 6-membered heterocycloalkyl groups containing two heteroatoms include, without limitation, piperazinyl, morpholinyl, dithianyl, dioxanyl. Exemplary 6-membered heterocycloalkyl groups containing three heteroatoms include, without limitation, triazinanyl. Exemplary 7-membered heterocycloalkyl groups containing one heteroatom include, without limitation, azepanyl, oxepanyl and thiepanyl. Exemplary 5-membered heterocycloalkyl groups fused to a $C_6$ aryl ring (also referred to herein as a 5,6-bicyclic heterocycloalkyl) include, without limitation, indolinyl, isoindolinyl, dihydrobenzofuranyl, dihydrobenzothienyl, benzoxazolinonyl, and the like. Exemplary 6-membered heterocycloalkyl groups fused to an $C_6$ aryl ring (also referred to herein as a 6,6-bicyclic heterocycloalkyl) include, without limitation, tetrahydroquinolinyl, tetrahydroisoquinolinyl, and the like.

"$C_{6-10}$ aryl" refers to a radical of a monocyclic or polycyclic (*e.g.,* bicyclic) 4n+2 aromatic ring system (*e.g.,* having 6 or 10 $\pi$ electrons shared in a cyclic array) having 6-10 ring carbon atoms and zero heteroatoms. In some embodiments, an aryl group has six ring carbon atoms ("$C_6$ aryl"; *e.g.,* phenyl). In some embodiments, an aryl group has ten ring carbon atoms ("$C_{10}$ aryl"; *e.g.,* naphthyl such as 1-naphthyl and 2-naphthyl). Aryl also includes ring systems wherein the aryl ring, as defined above, is fused with one or more cycloalkyl, or heterocycloalkyl groups wherein the point of attachment is on the aryl ring, and in such instances, the number of carbon atoms continue to designate the number of carbon atoms in the aryl ring system. Unless otherwise specified, each instance of an aryl group is independently optionally substituted, *i.e.*, unsubstituted (an "unsubstituted aryl") or substituted (a "substituted aryl") with one or more substituents. In certain embodiments, the aryl group is unsubstituted $C_{6-10}$ aryl. In certain embodiments, the aryl group is substituted $C_{6-10}$ aryl.

"$C_{5-10}$ heteroaryl" refers to a radical of a 5-10 membered monocyclic or bicyclic 4n+2 aromatic ring system (*e.g.,* having 6 or 10 $\pi$ electrons shared in a cyclic array) having ring carbon atoms and 1-4 ring heteroatoms, wherein each heteroatom is independently selected from nitrogen, oxygen and sulfur. In heteroaryl groups that contain one or more nitrogen atoms, the point of attachment can be a carbon or nitrogen atom, as valency permits. Heteroaryl bicyclic ring systems can include one or more heteroatoms in one or both rings. Heteroaryl further includes ring systems wherein the heteroaryl ring, as defined above, is fused with one or more cycloalkyl, or heterocycloalkyl groups wherein the point of attachment is on the heteroaryl ring, and in such instances, the number of ring members continue to designate the number of ring members in the heteroaryl ring system. In some embodiments, $C_{5-6}$ heteroaryl is especially alternative, which is a radical of a 5-6 membered monocyclic or bicyclic 4n+2 aromatic ring system having ring carbon atoms and 1-4 ring heteroatoms. Unless otherwise specified, each instance of a heteroaryl group is independently optionally substituted, *i.e.*, unsubstituted (an "unsubstituted heteroaryl") or substituted (a "substituted heteroaryl") with one or more substituents. In certain embodiments, the heteroaryl group is unsubstituted 5- to 10-membered heteroaryl. In certain embodiments, the heteroaryl group is substituted 5- to 10-membered heteroaryl. Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl,

oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

"Cyano group" represents the group -CN.

"Nitro group" represents the group $-NO_2$.

*Other definitions*

The term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, Berge et al., describes pharmaceutically acceptable salts in detail in J. Pharmaceutical Sciences (1977) 66:1-19. Pharmaceutically acceptable salts of the compounds of the present disclosure include those derived from suitable inorganic and organic acids and bases.

Pharmaceutically acceptable salts of the compounds disclosed herein include those derived from suitable inorganic and organic acids and bases. Examples of pharmaceutically acceptable, nontoxic acid addition salts are salts formed with inorganic acids such as hydrochloric acid, hydrobromic acid, phosphoric acid, sulfuric acid, and perchloric acid or with organic acids such as acetic acid, oxalic acid, maleic acid, tartaric acid, citric acid, succinic acid, or malonic acid. Salts formed using conventional methods in the art such as ion exchange are also included. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Pharmaceutically acceptable salts derived from appropriate bases include alkali metal, alkaline earth metal, ammonium and $N^+(C_{1-4}alkyl)_4$ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

Those skilled in the art will appreciate that an organic compound may form a complex with a solvent in which it reacts or from which it precipitates or crystallizes. These complexes are called "solvates". When the solvent is water, the complex is called a "hydrate". The present disclosure encompasses all solvates of the compounds of the present disclosure.

The term "solvate" refers to the form of a compound or a salt thereof in association with a solvent, usually formed by a solvolysis reaction. This physical association may involve hydrogen bonding. Conventional solvents include water, methanol, ethanol, acetic acid, DMSO, THF, diethyl ether, etc. The compounds described herein can be prepared, for example, in a crystal form, and can be solvated. Suitable solvates include pharmaceutically acceptable solvates and further include stoichiometric solvates and non-stoichiometric solvates. In some cases, the solvate will be capable of isolation, for example, when one or more solvent molecules are incorporated in the crystal lattice of the crystalline solid. The "solvate" includes both solution-phase and isolatable solvates. Representative solvates include hydrates, ethanolates and methanolates.

The term "hydrate" refers to a compound in combination with water. Usually, the ratio of the number of water molecules contained in a hydrate of a compound to the number of molecules of the compound in the hydrate is determined. Therefore, the hydrate of a compound can be represented, for example, by general formula $R \cdot x\ H_2O$, wherein R is the compound, and x is a number greater than 0. A given compound may form more than one type of hydrate, including, for example, a monohydrate (x is 1), a lower hydrate (x is a number greater than 0 and less than 1, for example, a hemihydrate ($R \cdot 0.5\ H_2O$)) and a polyhydrate (x is a number greater than 1, for example, a dihydrate ($R \cdot 2\ H_2O$) and a hexahydrate ($R \cdot 6\ H_2O$)).

The component compounds of the present disclosure may be in amorphous or crystal forms (crystal forms or polymorphs). Furthermore, the component compounds of the present disclosure may exist in one or more crystal forms.

Therefore, the present disclosure includes within its scope all amorphous or crystal forms of the component compounds of the present disclosure. The term "polymorph" refers to a crystal form of a compound (or a salt, hydrate, or solvate thereof) in a specific crystal packing arrangement. All polymorphs have the same elemental composition. Different crystal forms usually have different X-ray diffraction patterns, infrared spectra, melting points, density, hardness, crystal shape, optoelectronic properties, stability and solubility. The recrystallization solvent, crystallization rate, storage temperature and other factors may cause one crystal form to predominate. Various polymorphs of a compound can be prepared by crystallization under different conditions.

Each component compound of the pharmaceutical combination of the present disclosure also includes isotopically labeled compounds, which are equivalent to those of component (a) and component (b) active ingredients, but with one or more atoms replaced by atoms having an atomic mass or mass number different from the atomic mass or mass number commonly found in nature. Examples of isotopes that can be incorporated include isotopes of hydrogen, carbon, nitrogen, oxygen, phosphorus, sulfur, fluorine, and chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$, and $^{36}Cl$, respectively. Certain isotopically labeled component compounds of the present disclosure, such as those incorporating radioactive isotopes (e.g., $^{3}H$ and $^{14}C$), may be useful in drug and/or substrate tissue distribution assays. Tritium, i.e., $^{3}H$, and carbon-14, i.e., $^{14}C$, isotopes are particularly alternative due to their ease of preparation and detection. Furthermore, substitution with heavier isotopes, such as deuterium, i.e., $^{2}H$, may provide therapeutic benefits due to higher metabolic stability, such as prolonged in vivo half-life or reduced dosage requirements, and thus may be alternative in some cases.

As used herein, unless otherwise indicated, the singular forms "a," "an," and "the" include plural references. For example, "a" compound includes one or more compounds.

As used herein, unless otherwise indicated, the use of "or" means "and/or."

The terms "including", "comprising", "containing", "having" and other forms thereof, such as "include", "comprise", "contain", "have" are not limiting.

The section headings used herein are for organizational purposes only and are not to be construed as limiting the described subject matter. All documents or portions of documents cited in this application, including but not limited to patents, patent applications, articles, books, manuals, and discussions, are hereby expressly incorporated by reference in their entirety for any purpose.

The term "about" is used herein to mean approximately, within the range of, roughly, or around. When the term "about" is used in conjunction with a numerical range, it modifies the range by extending the boundaries above and below the specified numerical value. Generally, the term "about" is used herein to modify numerical values above and below the specified value by 10%.

The term "including" means "including but not limited to."

A "subject" includes, but is not limited to, humans (*i.e.,* a male or female of any age group, *e.g.,* a pediatric subject (*e.g,* infant, child, adolescent) or adult subject (*e.g.,* young adult, middle-aged adult or senior adult)) and/or a non-human animal, *e.g.*, a mammal such as primates (*e.g.,* cynomolgus monkeys, rhesus monkeys), cattle, pigs, horses, sheep, goats, rodents, cats, and/or dogs. In certain embodiments, the subject is a human. In certain embodiments, the subject is a non-human animal. The terms "human," "patient," and "subject" are used interchangeably herein.

Disease, disorder, and condition are used interchangeably herein.

"Fasting" is defined as not consuming any meals from 30 minutes before taking the medication until 60 minutes after taking the medication.

The term "administration" includes delivering a compound or a pharmaceutically acceptable salt, crystal form, solvate, or hydrate thereof, or a pharmaceutical composition containing them, to a subject using any suitable formulation or route of administration.

The "staging of CML (i.e., CP, AP, and BP)" refers to the ELN criteria disclosed in Table 1 of Baccarani et al., European LeukemiaNet recommendations for the management of chronic myeloid leukemia: 2013, Blood, 2013, Volume 122, Number 6, which article is incorporated herein by reference for all purposes.

"BCR-ABL1 TKIs" include "ATP-competitive BCR-ABL1 TKIs" and "BCR-ABL1 allosteric TKIs."

The terms "ATP-competitive BCR-ABL1 TKIs," "BCR-ABL1 orthosteric TKIs," and "BCR-ABL1 catalytic TKIs" are used interchangeably and are defined as binding to the ATP-binding site "hinge region," inhibiting the autophosphorylation and substrate phosphorylation of the BCR-ABL1 kinase, thereby suppressing the proliferation of cancer cells and tumor formation.

The term "BCR-ABL1 allosteric TKIs" refers to agents that bind to the myristoyl pocket, mimicking the role of the N-terminus in myristoylated ABL1, thereby restoring the negative regulatory function of BCR-ABL1 kinase activity.

In a specific embodiment, Y.-L Pan et al. (The progress of small-molecules and degraders against BCR-ABL1 for the treatment of CML, European Journal of Medicinal Chemistry 238 (2022) 114442) disclosed information on the structures, cellular activity, in vivo activity, and development status of certain BCR-ABL1 TKIs. This article is incorporated herein by reference for all purposes.

"First-line BCR-ABL1 TKIs" refer to the first-line therapeutic drugs approved by the FDA, EMA, and NMPA CDE,

including imatinib, nilotinib, dasatinib, bosutinib, flumatinib, and radotinib.

"Second-line BCR-ABL1 TKIs" refer to second-line therapeutic drugs selected based on the type of ABL mutation. For specific details, please refer to Table 1:

Table 1 Treatment Selection Based on ABL Mutation Status

| Mutation | Treatment Options |
|---|---|
| T315I | ponatinib, Hematopoietic Stem Cell Transplantation, |
| | Clinical Trials |
| V299L | ponatinib, nilotinib |
| T315A | ponatinib, nilotinib, imatinib*, bosutinib |
| F317L/V/I/C | ponatinib, nilotinib, bosutinib |
| Y253H, E225K/V, F359C/V/I | ponatinib, dasatinib, bosutinib |
| Any other mutations | ponatinib, dasatinib, nilotinib, bosutinib |
| Note: *, if it occurs during dasatinib treatment. Currently, there is limited clinical data on bosutinib for imatinib-resistant mutations, and some in vitro data suggest that the E255K/V mutation may have insufficient sensitivity to bosutinib. | |

The definitions of "failure" and "warning" in "treatment evaluation results after TKIs treatment are failure or warning" can be found in the "Diagnosis and Treatment Guidelines for Adult Chronic Myeloid Leukemia (2018 Edition)" issued by the National Health Commission of the People's Republic of China in December 2018. Specifically, please refer to Table 4 and Table 7 therein, excerpts as follows:

Table 4 of the "Diagnosis and Treatment Guidelines for Adult Chronic Myeloid Leukemia (2018 Edition)" - Evaluation Criteria for Treatment Response in CML-CP Patients Receiving First-Line TKI Therapy

| Time | Best Response Achieve CHR as the baseline | Warning Achieve CHR as the baseline | Failure |
|---|---|---|---|
| 3 month | - At least achieve-PCyR(Ph+ ≤ 35%)<br>- BCR-ABL$^{IS}$≤ 10% | - Did not achieve PCyR(Ph+ 36%-95%)<br>- BCR-ABL$^{IS}$> 10% | - Did not achieve CHR<br>- No CyR(Ph+ > 95%) |
| 6 month | - At least achieve-CCyR(Ph+=0)<br>- BCR-ABL$^{IS}$<1% | - Achieve PCyR but not-CCyR(Ph+ 1%-35%)<br>- BCR-ABL$^{IS}$ 1-10% | - Did not achieve PCyR(Ph+ >35%)<br>- BCR-ABL$^{IS}$> 10% |
| 12 month | -BCR-ABL$^{IS}$≤0.1% | BCR-ABL$^{IS}$> 0.1-1% | - Did not achieve CCyR(Ph+ > 0)<br>-BCR-ABL$^{IS}$ > 1 % |
| Anytime | Stable or MMR achieved | Ph = 0, presence of -7 or 7q- (CCA/Ph-) | Loss of CHR or CcyR or MMR, occurrence of imatinib or other TKI |
| resistance mutations, occurrence of other clonal chromosomal abnormalities on the basis of Ph chromosome | | | |
| Note: IS: International Standardization | | | |

Table 7 of the "Diagnosis and Treatment Guidelines for Adult Chronic Myeloid Leukemia (2018 Edition)" - Evaluation Criteria for Treatment Response in CML-CP Patients Receiving Second-Line TKI Therapy

| Time | Best Response | Warning | Failure |
|---|---|---|---|
| 3 month | - At least achieve-mCyR(Ph+ ≤65%)<br>- BCR-ABL$^{IS}$≤ 10% | - Did not achieve mCyR(Ph+ 66%-95%)<br>- BCR-ABL$^{IS}$ > 10% | - No CHR<br>- Not any CyR(Ph+ > 95%)<br>- New mutation |

(continued)

| Time | Best Response | Warning | Failure |
|---|---|---|---|
| 6 month | - At least achieve PCyR(Ph+ ≤ 35%)<br>- BCR-ABL$^{IS}$≤ 10% | - Achieve mCyR but not PCyR(Ph+ 36%-65%) | - Did not achieve mCyR(Ph+ >65%)<br>- BCR-ABL$^{IS}$ > 10% |
| 12 month | - Achieved CCyR<br>- BCR-ABL$^{IS}$< 1% | - BCR-ABL$^{IS}$ 1-10%<br>- Achieve PCyR(Ph+ 1%-35%) | - New mutation<br>- Did not achieve PCyR(Ph+ > 35%)<br>-BCR-ABL$^{IS}$ > 10%<br>- New mutation |
| At any time | Stable or MMR achieved | -Ph =0, appearance of 7 or 7q- (CCA/Ph-)<br>-BCR-ABL$^{IS}$ > 0.1 % | Loss of CHR or CcyR or PCyR or MMR, new -drug-resistant mutations, appearance of other clonal chromosomal abnormalities on the basis of Ph chromosome |

According to the standards of the "Diagnosis and Treatment Guidelines for Adult Chronic Myeloid Leukemia (2018 Edition)", the hematological response (HR), cytogenetic response (CyR), molecular response (MR), progression-free survival (PFS), and overall survival (OS) of CML patients are evaluated. The definitions of treatment responses for hematological response, cytogenetic response, and molecular response are shown in Table 3, excerpted as follows:

Table 3 from the "Diagnosis and Treatment Guidelines for Adult Chronic Myeloid Leukemia (2018 Edition)" - Definitions of Treatment Responses

| Hematological (HR) | ResponseCytogenetic (CyR) | | Response | Molecular Response (MR) | |
|---|---|---|---|---|---|
| Complete (CHR) | • Platelet count: <450×10$^9$/L | Complete CCyR | Ph+ 0% | Major Molecular Response (MMR) | BCR-ABL1(IS) ≤ 0.1% (ABL1 transcripts > 10,000) |
| | • White blood cell count: <10×10$^9$/L | Partial (PCyR) | Ph+ 1%-35% | Molecular Response MR4 | BCR-ABL1(IS) ≤0.01% (ABL1 transcripts >10000) |
| | • No immature myeloid cells in peripheral blood, basophils <5% | Minor mCyR | Ph+ 36%-65% | Molecular Response MR4.5 | BCR-ABL1(IS) ≤0.0032% (ABL1 transcript > 32000) |
| | • No symptoms or signs of disease, palpable splenomegaly has resolved | Micro mini-CyR | Ph+ 66%-95% | Molecular response | BCR-ABL1(IS) ≤ 0.001% (ABL1 MR5 transcripts > 100,000) |
| | | None | Ph+ >95% | Undetectable molecularly UMRD | BCR-ABL1 transcripts were undetectable at an expandable ABL1 transcript level. |

"Major Hematologic Response (MaHR)" includes Complete Hematologic Response (CHR) or No Evidence of Leukemia (NEL).

"Major Cytogenetic Response (MCyR)" includes Complete Cytogenetic Response (CCyR) or Partial Cytogenetic Response (PCyR). All patients with PcyR results both before and after administration are considered not to have achieved a major cytogenetic response.

"Major Molecular Response (MMR)" is defined as BCR-ABL1(IS) ≤ 0.1% (ABL1 transcripts > 10,000).

"Molecular Response MR4" is defined as BCR-ABL1(IS) ≤ 0.01% (ABL1 transcripts > 10,000).

"Molecular Response MR4.5" is defined as BCR-ABL1(IS) ≤ 0.0032% (ABL1 transcripts > 32,000).

"Molecular Response MR5" is defined as BCR-ABL1(IS) ≤ 0.001% (ABL1 transcripts > 100,000).

"Progression-Free Survival (PFS)" is defined as the time from the first administration of the test drug to disease progression (PD) or death from any cause, whichever occurs first. For subjects with CML in chronic phase, PD is defined as death or diagnosis of accelerated phase or blast phase CML by the investigator; for subjects with CML in accelerated phase, PD is defined as death or diagnosis of blast phase CML by the investigator.

"Overall Survival (OS)" is defined as the time from the first administration of the test drug to death from any cause.

"Event Free Survival (EFS)" is defined as the time from the day of randomization to the occurrence of any of the following "events" (whichever occurs first): progression to AP or BP; death from any cause (including but not limited to leukemia-related or treatment-related causes); loss of MCyR or CCyR; failure to achieve complete hematologic response after three cycles of treatment; loss of previously achieved CHR(confirmed by two consecutive assessments at least 7 days apart); doubling of peripheral blood WBC count exceeding 20,000/mm$^3$, sustained for ≥14 days (excluding secondary causes other than leukemia); discontinuation of treatment due to treatment toxicity (subjects judged to have withdrawn from treatment due to toxicity must be discussed and confirmed by the investigator and sponsor).

Unless otherwise specified, the term "treatment" as used herein includes actions occurring when a subject suffers from a specific disease, disorder, or condition, which reduce the severity of the disease, disorder, or condition, or delay or slow the progression of the disease, disorder, or condition ("therapeutic treatment"), as well as actions occurring before the subject begins to suffer from a specific disease, disorder, or condition ("preventive treatment").

Treatment

<u>Chronic Myeloid Leukemia in Chronic Phase (CML-CP)</u>

In one embodiment, the present disclosure relates to the use and method of a compound of formula (I) or a pharmaceutically acceptable salt, crystal form, solvate, or hydrate thereof for treating CML in chronic phase or central nervous system leukemia relapse thereof, such as brain metastasis. In another embodiment, the present disclosure relates to the use and method of (R)-N-(4-(chlorodifluoromethoxy)phenyl)-6-(3-fluoropyrrolidin-1-yl)-5-(pyrazin-2-yl) nicotinamide (Compound A) or a pharmaceutically acceptable salt, crystal form, solvate, or hydrate thereof for treating CML in chronic phase or central nervous system leukemia relapse thereof, such as brain metastasis.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-CP. In another more specific embodiment, the CML is Ph+ CML-CP with T315I mutation. In another more specific embodiment, the CML is Ph+ and BCR-ABL1+ CML-CP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the subject has previously received treatment with ≥1 different BCR-ABL1 TKIs and subsequently experienced disease progression to accelerated phase or blast phase, with treatment evaluation results being failure, warning, or intolerance. In another specific embodiment, the subject has previously received treatment with ≥2 different BCR-ABL1 TKIs and subsequently experienced disease progression to accelerated phase or blast phase, with treatment evaluation results being failure, warning, or intolerance. In another specific embodiment, the subject has previously received treatment with ≥3 different BCR-ABL1 TKIs and subsequently experienced disease progression to accelerated phase or blast phase, with treatment evaluation results being failure, warning, or intolerance. In another specific embodiment, the subject has previously received treatment with ≥4 different BCR-ABL1 TKIs and subsequently experienced disease progression to accelerated phase or blast phase, with treatment evaluation results being failure, warning, or intolerance. In another more specific embodiment, the BCR-ABL1 TKIs are first-line, second-line, third-line, fourth-line, or fifth-line therapeutic agents. In another more specific embodiment, the BCR-ABL1 TKIs are first-line, second-line, or third-line therapeutic agents. In another more specific embodiment, the BCR-ABL1 TKIs are first-line or second-line therapeutic agents. In another more specific embodiment, the BCR-ABL1 TKIs are first-line therapeutic agents. In another more specific embodiment, the BCR-ABL1 TKIs are second-line therapeutic agents. In another more specific embodiment, the BCR-ABL1 TKIs are second-line or higher-line therapeutic agents. In another more specific embodiment, the BCR-ABL1 TKIs include at least third-generation TKIs (e.g., ponatinib or olverembatinib). In another more specific embodiment, the BCR-ABL1 TKIs include at least third-generation TKIs (e.g., ponatinib or olverembatinib) and/or fourth-generation TKIs (e.g., asciminib or HS-10382). In another more specific embodiment, the BCR-ABL1 TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382. In another more specific embodiment, the BCR-ABL1 TKIs are selected from imatinib, dasatinib, nilotinib, bosutinib, flumatinib, ponatinib, olverembatinib, radotinib or asciminib. In another more specific embodiment, the BCR-ABL1 TKIs are selected from imatinib, dasatinib, nilotinib, ponatinib, olverembatinib or asciminib. In another more specific embodiment, the BCR-ABL1 TKIs are selected from imatinib, dasatinib, nilotinib, bosutinib, flumatinib or radotinib. In another more specific embodiment, the BCR-ABL1 TKIs are selected from ponatinib, olverembatinib, asciminib or HS-10382. In another more specific embodiment, the BCR-ABL1 TKIs are selected from imatinib, dasatinib or nilotinib. In another more specific embodiment, the BCR-ABL1 TKIs are

selected from ponatinib or olverembatinib. In another more specific embodiment, the BCR-ABL1 TKIs are selected from asciminib or HS-10382. In another more specific embodiment, the BCR-ABL1 TKIs are selected from asciminib.

In another specific embodiment, the subject has previously received treatment with ≥1 different BCR-ABL1 TKIs and subsequently experienced relapse or became refractory. In another specific embodiment, the subject has previously received treatment with ≥2 different BCR-ABL1 TKIs and subsequently experienced relapse or became refractory. In another specific embodiment, the subject has previously received treatment with ≥3 different BCR-ABL1 TKIs and subsequently experienced relapse or became refractory. In another specific embodiment, the subject has previously received treatment with ≥4 different BCR-ABL1 TKIs and subsequently experienced relapse or became refractory. In another more specific embodiment, the BCR-ABL1 TKIs are first-line, second-line, third-line, fourth-line, or fifth-line therapeutic agent. In another more specific embodiment, the BCR-ABL1 TKIs are first-line, second-line, or third-line therapeutic agent. In another more specific embodiment, the BCR-ABL1 TKIs are first-line or second-line therapeutic agents. In another more specific embodiment, the BCR-ABL1 TKIs are first-line therapeutic agents. In another more specific embodiment, the BCR-ABL1 TKIs are second-line therapeutic agents. In another more specific embodiment, the BCR-ABL1 TKIs are second-line or higher-line therapeutic agents. In another more specific embodiment, the BCR-ABL1 TKIs include at least third-generation TKIs (e.g., ponatinib or olverembatinib). In another more specific embodiment, the BCR-ABL1 TKIs include at least third-generation TKIs (e.g., ponatinib or olverembatinib) and/or fourth-generation TKIs (e.g., asciminib or HS-10382). In another more specific embodiment, the BCR-ABL1 TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382. In another more specific embodiment, the BCR-ABL1 TKIs are selected from imatinib, dasatinib, nilotinib, bosutinib, flumatinib, ponatinib, olverembatinib, radotinib or asciminib. In another more specific embodiment, the BCR-ABL1 TKIs are selected from imatinib, dasatinib, nilotinib, ponatinib, olverembatinib or asciminib. In another more specific embodiment, the BCR-ABL1 TKIs are selected from imatinib, dasatinib, nilotinib, bosutinib, flumatinib or radotinib. In another more specific embodiment, the BCR-ABL1 TKIs are selected from ponatinib, olverembatinib, asciminib or HS-10382. In another more specific embodiment, the BCR-ABL1 TKIs are selected from imatinib, dasatinib or nilotinib. In another more specific embodiment, the BCR-ABL1 TKIs are selected from ponatinib or olverembatinib. In another more specific embodiment, the BCR-ABL1 TKIs are selected from asciminib or HS-10382. In another more specific embodiment, the BCR-ABL1 TKIs are selected from asciminib.

In another specific embodiment, the subject has previously received treatment with ≥1 different BCR-ABL1 ATP-competitive TKIs and subsequently experienced disease progression to accelerated phase or blast phase, with treatment evaluation results being failure, warning, or intolerance. In another specific embodiment, the subject has previously received treatment with ≥2 different BCR-ABL1 ATP-competitive TKIs and subsequently experienced disease progression to accelerated phase or blast phase, with treatment evaluation results being failure, warning, or intolerance. In another specific embodiment, the subject has previously received treatment with ≥3 different BCR-ABL1 ATP-competitive TKIs and subsequently experienced disease progression to accelerated phase or blast phase, with treatment evaluation results being failure, warning, or intolerance. In another specific embodiment, the subject has previously received treatment with ≥4 different BCR-ABL1 ATP-competitive TKIs and subsequently experienced disease progression to accelerated phase or blast phase, with treatment evaluation results being failure, warning, or intolerance. In another more specific embodiment, the BCR-ABL1 ATP-competitive TKIs are a first-line, second-line, third-line, fourth-line, or fifth-line therapeutic agent. In another more specific embodiment, the BCR-ABL1 ATP-competitive TKIs are a first-line, second-line, or third-line therapeutic agent. In another more specific embodiment, the BCR-ABL1 ATP-competitive TKIs are first-line or second-line therapeutic agents. In another more specific embodiment, the BCR-ABL1 ATP-competitive TKIs are first-line therapeutic agents. In another more specific embodiment, the BCR-ABL1 ATP-competitive TKIs are second-line therapeutic agents. In another more specific embodiment, the BCR-ABL1 ATP-competitive TKIs are second-line or higher-line therapeutic agents. In another more specific embodiment, the BCR-ABL1 ATP-competitive TKIs include at least third-generation TKIs (e.g., ponatinib or olverembatinib). In another more specific embodiment, the BCR-ABL1 ATP-competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228. In another more specific embodiment, the BCR-ABL1 ATP-competitive TKIs are selected from imatinib, dasatinib, nilotinib, bosutinib, flumatinib, ponatinib, olverembatinib or radotinib. In another more specific embodiment, the BCR-ABL1 ATP-competitive TKIs are selected from imatinib, dasatinib, nilotinib, ponatinib or olverembatinib. In another more specific embodiment, the BCR-ABL1 ATP-competitive TKIs are selected from imatinib, dasatinib, nilotinib, bosutinib, flumatinib or radotinib. In another more specific embodiment, the BCR-ABL1 ATP-competitive TKIs are selected from imatinib, dasatinib or nilotinib. In another more specific embodiment, the BCR-ABL1 ATP-competitive TKIs are selected from ponatinib or olverembatinib.

In another specific embodiment, the subject has previously received treatment with ≥1 different BCR-ABL1 ATP-competitive TKIs and subsequently experienced relapse or became refractory. In another specific embodiment, the subject has previously received treatment with ≥2 different BCR-ABL1 ATP-competitive TKIs and subsequently experi-

enced relapse or became refractory. In another specific embodiment, the subject has previously received treatment with ≥3 different BCR-ABL1 ATP-competitive TKIs and subsequently experienced relapse or became refractory. In another specific embodiment, the subject has previously received treatment with ≥4 different BCR-ABL1 ATP-competitive TKIs and subsequently experienced relapse or became refractory. In another more specific embodiment, the BCR-ABL1 ATP-competitive TKIs are a first-line, second-line, third-line, fourth-line, or fifth-line therapeutic agent. In another more specific embodiment, the BCR-ABL1 ATP-competitive TKIs are a first-line, second-line, or third-line therapeutic agent. In another more specific embodiment, the BCR-ABL1 ATP-competitive TKIs are first-line or second-line therapeutic agents. In another more specific embodiment, the BCR-ABL1 ATP-competitive TKIs are first-line therapeutic agents. In another more specific embodiment, the BCR-ABL1 ATP-competitive TKIs are second-line therapeutic agents. In another more specific embodiment, the BCR-ABL1 ATP-competitive TKIs are second-line or higher-line therapeutic agents. In another more specific embodiment, the BCR-ABL1 ATP-competitive TKIs include at least third-generation TKIs (e.g., ponatinib or olverembatinib). In another more specific embodiment, the BCR-ABL1 ATP-competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228. In another more specific embodiment, the BCR-ABL1 ATP-competitive TKIs are selected from imatinib, dasatinib, nilotinib, bosutinib, flumatinib, ponatinib, olverembatinib or radotinib. In another more specific embodiment, the BCR-ABL1 ATP-competitive TKIs are selected from imatinib, dasatinib, nilotinib, ponatinib or olverembatinib. In another more specific embodiment, the BCR-ABL1 ATP-competitive TKIs are selected from imatinib, dasatinib, nilotinib, bosutinib, flumatinib or radotinib. In another more specific embodiment, the BCR-ABL1 ATP-competitive TKIs are selected from imatinib, dasatinib or nilotinib. In another more specific embodiment, the BCR-ABL1 ATP-competitive TKIs are selected from ponatinib or olverembatinib.

In another specific embodiment, the subject has previously received treatment with ≥1 different first- and/or second-generation BCR-ABL1 ATP-competitive TKIs and subsequently developed resistance or intolerance. In another specific embodiment, the subject has previously received treatment with ≥2 different first- and/or second-generation BCR-ABL1 ATP-competitive TKIs and subsequently developed resistance or intolerance. In another specific embodiment, the subject has previously received treatment with ≥3 different first- and/or second-generation BCR-ABL1 ATP-competitive TKIs and subsequently developed resistance or intolerance. In another specific embodiment, the subject has previously received treatment with ≥4 different first- and/or second-generation BCR-ABL1 ATP-competitive TKIs and subsequently developed resistance or intolerance. In another more specific embodiment, the resistance arises from BCR-ABL1 kinase domain point mutation(s), such as one or more of T315I/A, Y253F/H, E255V/K, M351T, G250E, F359V/I/C, H396P/R, M244V, E355G, F317L/V/I/C, F311L, M237I, Q252H/R, D276G, L248V, V379I, L384M, L387M, E279K, F486S, E459K, P223S, I502L, or V299L. In another more specific embodiment, the resistance involves BCR-ABL1 kinase domain point mutations, selected from one or more of T315I, Y253H, E255V/K, M351T, G250E, F359V/, H396P, M244V, E355G, F317L, Q252H, E459K, P223S, I502L, or V299L mutations. In another more specific embodiment, the resistance is T315I. In another more specific embodiment, the first- or second-generation BCR-ABL1 ATP-competitive TKIs are a first-line, second-line, third-line, fourth-line, or fifth-line therapeutic agent. In another more specific embodiment, the first- or second-generation BCR-ABL1 ATP-competitive TKIs are a first-line, second-line, or third-line therapeutic agent. In another more specific embodiment, the first- or second-generation BCR-ABL1 ATP-competitive TKIs are first-line or second-line therapeutic agents. In another more specific embodiment, the first- or second-generation BCR-ABL1 ATP-competitive TKIs are first-line therapeutic agents. In another more specific embodiment, the first- or second-generation BCR-ABL1 ATP-competitive TKIs are second-line therapeutic agents. In another more specific embodiment, the first- or second-generation BCR-ABL1 ATP-competitive TKIs are second-line or higher-line therapeutic agents. In another more specific embodiment, the first-generation BCR-ABL1 ATP-competitive TKI is imatinib. In another more specific embodiment, the second-generation BCR-ABL1 ATP-competitive TKIs are selected from dasatinib, nilotinib, bosutinib, flumatinib or radotinib. In another more specific embodiment, the first- and/or second-generation BCR-ABL1 ATP-competitive TKIs are selected from imatinib, dasatinib, nilotinib, bosutinib, flumatinib or radotinib. In another more specific embodiment, the first- and/or second-generation BCR-ABL1 ATP-competitive TKIs are selected from imatinib, dasatinib or nilotinib.

In another specific embodiment, the subject has previously received treatment with ≥1 different first- and/or second-generation BCR-ABL1 ATP-competitive TKIs and subsequently experienced relapse or became refractory. In another specific embodiment, the subject has previously received treatment with ≥2 different first- and/or second-generation BCR-ABL1 ATP-competitive TKIs and subsequently experienced relapse or became refractory. In another specific embodiment, the subject has previously received treatment with ≥3 different first- and/or second-generation BCR-ABL1 ATP-competitive TKIs and subsequently experienced relapse or became refractory. In another specific embodiment, the subject has previously received treatment with ≥4 different first- and/or second-generation BCR-ABL1 ATP-competitive TKIs and subsequently experienced relapse or became refractory. In another more specific embodiment, the first- or second-generation BCR-ABL1 ATP-competitive TKIs are a first-line, second-line, third-line, fourth-line, or fifth-line therapeutic agent. In another more specific embodiment, the first- or second-generation BCR-ABL1 ATP-competitive TKIs are a first-line, second-line, or third-line therapeutic agent. In another more specific embodiment, the first- or second-

generation BCR-ABL1 ATP-competitive TKIs are first-line or second-line therapeutic agents. In another more specific embodiment, the first- or second-generation BCR-ABL1 ATP-competitive TKIs are first-line therapeutic agents. In another more specific embodiment, the first- or second-generation BCR-ABL1 ATP-competitive TKIs are second-line therapeutic agents. In another more specific embodiment, the first- or second-generation BCR-ABL1 ATP-competitive TKIs are second-line or higher-line therapeutic agents. In another more specific embodiment, the first-generation BCR-ABL1 ATP-competitive TKI is imatinib. In another more specific embodiment, the second-generation BCR-ABL1 ATP-competitive TKIs are selected from dasatinib, nilotinib, bosutinib, flumatinib or radotinib. In another more specific embodiment, the first- and/or second-generation BCR-ABL1 ATP-competitive TKIs are selected from imatinib, dasatinib, nilotinib, bosutinib, flumatinib or radotinib. In another more specific embodiment, the first- and/or second-generation BCR-ABL1 ATP-competitive TKIs are selected from imatinib, dasatinib or nilotinib.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt,

crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the daily effective amount of the compound of formula (I) or a pharmaceutically acceptable salt, crystal form, solvate, or hydrate thereof (alternatively compound A) is about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in chronic phase (CML-CP) that has previously received treatment with ≥1 different BCR-ABL1 TKIs and subsequently experienced disease progression to accelerated phase or blast phase, with treatment evaluation results being failure, warning, or intolerance

In one embodiment, the present disclosure relates to the use and method of the compound of formula (I) or a pharmaceutically acceptable salt, crystal form, solvate, or hydrate thereof for treating CML in chronic phase or its central nervous system leukemia recurrence, such as brain metastasis, wherein the CML has previously received treatment with ≥1 different BCR-ABL1 TKIs and subsequently experienced disease progression to accelerated phase or blast phase, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥1 different BCR-ABL1 TKIs and subsequently experienced disease progression to accelerated phase or blast phase, with treatment evaluation results being failure, warning, or intolerance.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥2 different BCR-ABL1 TKIs and subsequently experienced disease progression to accelerated phase or blast phase, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥2 different BCR-ABL1 TKIs and subsequently experienced disease progression to accelerated phase or blast phase, with treatment evaluation results being failure, warning, or intolerance.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥3 different BCR-ABL1 TKIs and subsequently experienced disease progression to accelerated phase or blast phase, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥3 different BCR-ABL1 TKIs and subsequently experienced disease progression to accelerated phase or

blast phase, with treatment evaluation results being failure, warning, or intolerance.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥4 different BCR-ABL1 TKIs and subsequently experienced disease progression to accelerated phase or blast phase, with treatment evaluation results being failure, warning, or intolerance. In another embodiment, the present disclosure relates to the use and method of (R)-N-(4-(chlorodifluoromethoxy) phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥4 different BCR-ABL1 TKIs and subsequently experienced disease progression to accelerated phase or blast phase, with treatment evaluation results being failure, warning, or intolerance.

In one specific embodiment, the BCR-ABL1 TKIs are first-line, second-line, third-line, fourth-line, or fifth-line therapeutic agent. In another specific embodiment, the BCR-ABL1 TKIs are first-line, second-line, or third-line therapeutic agent. In another specific embodiment, the BCR-ABL1 TKIs are first-line or second-line therapeutic agents. In another specific embodiment, the BCR-ABL1 TKIs are first-line therapeutic agents. In another specific embodiment, the BCR-ABL1 TKIs are second-line therapeutic agents. In another specific embodiment, the BCR-ABL1 TKIs are second-line or higher-line therapeutic agents. In another more specific embodiment, the BCR-ABL1 TKIs include at least third-generation TKIs (e.g., ponatinib or olverembatinib). In another more specific embodiment, the BCR-ABL1 TKIs include at least third-generation TKIs (e.g., ponatinib or olverembatinib) and/or fourth-generation TKIs (e.g., asciminib or HS-10382). In another specific embodiment, the BCR-ABL1 TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382. In another specific embodiment, the BCR-ABL1 TKIs are selected from imatinib, dasatinib, nilotinib, bosutinib, flumatinib, ponatinib, olverembatinib, radotinib or asciminib. In another specific embodiment, the BCR-ABL1 TKIs are selected from imatinib, dasatinib, nilotinib, ponatinib, olverembatinib or asciminib. In another specific embodiment, the BCR-ABL1 TKIs are selected from imatinib, dasatinib, nilotinib, bosutinib, flumatinib or radotinib. In another more specific embodiment, the BCR-ABL1 TKIs are selected from ponatinib, olverembatinib, asciminib or HS-10382. In another specific embodiment, the BCR-ABL1 TKIs are selected from ponatinib, olverembatinib, asciminib. In another specific embodiment, the BCR-ABL1 TKIs are selected from imatinib, dasatinib or nilotinib. In another specific embodiment, the BCR-ABL1 TKIs are selected from ponatinib or olverembatinib. In another specific embodiment, the BCR-ABL1 TKIs are selected from asciminib or HS-10382. In another specific embodiment, the BCR-ABL1 TKIs are selected from asciminib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-CP. In another more specific embodiment, the CML is Ph+ CML-CP with T315I mutation. In another more specific embodiment, the CML is Ph+ and BCR-ABL1+ CML-CP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof

(alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in chronic phase (CML-CP) that has previously received treatment with ≥1 different BCR-ABL1 ATP-competitive TKIs and subsequently experienced disease progression to accelerated phase or blast phase, with treatment evaluation results being failure, warning, or intolerance

In one embodiment, the present disclosure relates to the use and method of the compound of formula (I) or a pharmaceutically acceptable salt, crystal form, solvate, or hydrate thereof for treating CML in chronic phase or its central nervous system leukemia recurrence, such as brain metastasis, wherein the CML has previously received treatment with ≥1 different BCR-ABL1 ATP-competitive TKIs and subsequently experienced disease progression to accelerated phase or blast phase, with treatment evaluation results being failure, warning, or intolerance. In another embodiment, the present disclosure relates to the method of (R)-N-(4-(chlorodifluoromethoxy)phenyl)-6-(3-fluoropyrrolidin-1-yl)-5-(pyrazin-2-yl) nicotinamide (Compound A) or a pharmaceutically acceptable salt, crystal form, solvate, or hydrate thereof for treating CML in chronic phase, wherein the CML has previously received treatment with ≥1 different BCR-ABL1 ATP-competitive TKIs and subsequently experienced disease progression to accelerated phase or blast phase, with treatment evaluation results being failure, warning, or intolerance.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥2 different BCR-ABL1 ATP-competitive TKIs and subsequently experienced disease progression to accelerated phase or blast phase, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥2 different BCR-ABL1 ATP-competitive TKIs and subsequently experienced disease progression to accelerated phase or blast phase, with treatment evaluation results being failure, warning, or intolerance.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥3 different BCR-ABL1 ATP-competitive TKIs and subsequently experienced disease progression to accelerated phase or blast phase, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥3 different BCR-ABL1 ATP-competitive TKIs and subsequently experienced disease progression to accelerated phase or blast phase, with treatment evaluation results being failure, warning, or intolerance.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥4 different BCR-ABL1 ATP-competitive TKIs and subsequently experienced disease progression to accelerated phase or blast phase, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥4 different BCR-ABL1 ATP-competitive TKIs and subsequently experienced disease progression to accelerated phase or blast phase, with treatment evaluation results being failure, warning, or intolerance.

In one specific embodiment, the BCR-ABL1 ATP-competitive TKIs are a first-line, second-line, third-line, fourth-line, or fifth-line therapeutic agent. In another specific embodiment, the BCR-ABL1 ATP-competitive TKIs are a first-line, second-line, or third-line therapeutic agent. In another specific embodiment, the BCR-ABL1 ATP-competitive TKIs are first-line or second-line therapeutic agents. In another specific embodiment, the BCR-ABL1 ATP-competitive TKIs are first-line therapeutic agents. In another specific embodiment, the BCR-ABL1 ATP-competitive TKIs are second-line therapeutic agents. In another specific embodiment, the BCR-ABL1 ATP-competitive TKIs are second-line or higher-line therapeutic agents. In another more specific embodiment, the BCR-ABL1 ATP-competitive TKIs include at least third-generation TKIs (e.g., ponatinib or olverembatinib). In another specific embodiment, the BCR-ABL1 ATP-competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, HS-10382, rebasinib, danusertib, tozasertib, AT9283, KW-2449 or XL228. In another specific embodiment, the BCR-ABL1 ATP-competitive TKIs are selected from imatinib, dasatinib, nilotinib, bosutinib, flumatinib, ponatinib, olverembatinib or radotinib. In another specific embodiment, the BCR-ABL1 ATP-competitive TKIs are selected from imatinib, dasatinib, nilotinib, ponatinib or olverembatinib. In another more specific embodiment, the BCR-ABL1 ATP-competitive TKIs are selected from imatinib, dasatinib, nilotinib, bosutinib, flumatinib or radotinib. In another specific embodiment, the BCR-ABL1 ATP-competitive TKIs are selected from imatinib, dasatinib or nilotinib. In another specific embodiment, the BCR-ABL1 ATP-competitive TKIs are selected from ponatinib or olverembatinib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-CP. In another more specific embodiment, the CML is Ph+ CML-CP with T315I mutation. In another more specific embodiment, the CML is Ph+ and BCR-ABL1+ CML-CP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively

compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in chronic phase (CML-CP) that has previously received treatment with ≥1 different first- and/or second-generation BCR-ABL1 ATP-competitive TKIs and subsequently developed resistance or intolerance

In one embodiment, the present disclosure relates to the use and method of the compound of formula (I) or a pharmaceutically acceptable salt, crystal form, solvate, or hydrate thereof for treating CML in chronic phase or its central nervous system leukemia recurrence, such as brain metastasis, wherein the CML has previously received treatment with ≥1 different first- and/or second-generation BCR-ABL1 ATP-competitive TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥1 different first- and/or second-generation BCR-ABL1 ATP-competitive TKIs and subsequently developed resistance or intolerance.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥2 different first- and/or second-generation BCR-ABL1 ATP-competitive TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥2 different first- and/or second-generation BCR-ABL1 ATP-competitive TKIs and subsequently developed resistance or intolerance.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥3 different first- and/or second-generation BCR-ABL1 ATP-competitive TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥3 different first- and/or second-generation BCR-ABL1 ATP-competitive TKIs and subsequently developed resistance or intolerance.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥4 different first- and/or second-generation BCR-ABL1 ATP-competitive TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Com-

pound A) or a pharmaceutically acceptable salt, crystal form, solvate, or hydrate thereof for treating CML in chronic phase, wherein the CML has previously received treatment with ≥4 different first- and/or second-generation BCR-ABL1 ATP-competitive TKIs and subsequently developed resistance or intolerance.

In one specific embodiment, the resistance arises from BCR-ABL1 kinase domain point mutation(s), such as one or more of T315I/A, Y253F/H, E255V/K, M351T, G250E, F359V/I/C, H396P/R, M244V, E355G, F317L/V/I/C, F311L, M237I, Q252H/R, D276G, L248V, V379I, L384M, L387M, E279K, F486S, E459K, P223S, I502L, or V299L. In another specific embodiment, the resistance involves BCR-ABL1 kinase domain point mutations, selected from one or more of T315I, Y253H, E255V/K, M351T, G250E, F359V/, H396P, M244V, E355G, F317L, Q252H, E459K, P223S, I502L, or V299L mutations. In another specific embodiment, the resistance is T315I.

In another specific embodiment, the first- or second-generation BCR-ABL1 ATP-competitive TKIs are a first-line, second-line, third-line, fourth-line, or fifth-line therapeutic agent. In another specific embodiment, the first- or second-generation BCR-ABL1 ATP-competitive TKIs are a first-line, second-line, or third-line therapeutic agent. In another specific embodiment, the first- or second-generation BCR-ABL1 ATP-competitive TKIs are first-line or second-line therapeutic agents. In another specific embodiment, the first- or second-generation BCR-ABL1 ATP-competitive TKIs are first-line therapeutic agents. In another specific embodiment, the first- or second-generation BCR-ABL1 ATP-competitive TKIs are second-line therapeutic agents. In another specific embodiment, the first- or second-generation BCR-ABL1 ATP-competitive TKIs are second-line or higher-line therapeutic agents. In another specific embodiment, the first-generation BCR-ABL1 ATP-competitive TKI is imatinib. In another specific embodiment, the second-generation BCR-ABL1 ATP-competitive TKIs are selected from dasatinib, nilotinib, bosutinib, flumatinib or radotinib. In another specific embodiment, the first- and/or second-generation BCR-ABL1 ATP-competitive TKIs are selected from imatinib, dasatinib, nilotinib, bosutinib, flumatinib or radotinib. In another specific embodiment, the first- and/or second-generation BCR-ABL1 ATP-competitive TKIs are selected from imatinib, dasatinib or nilotinib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-CP. In another more specific embodiment, the CML is Ph+ CML-CP with T315I mutation. In another more specific embodiment, the CML is Ph+ and BCR-ABL1+ CML-CP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt,

crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in chronic phase (CML-CP) that has previously received treatment with ≥1 different BCR-ABL1 TKIs and subsequently experienced relapse or became refractory

In one embodiment, the present disclosure relates to the use and method of the compound of formula (I) or a pharmaceutically acceptable salt, crystal form, solvate, or hydrate thereof for treating CML in chronic phase or its central nervous system leukemia recurrence, such as brain metastasis, wherein the CML has previously received treatment with ≥1 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥1 different BCR-ABL1 TKIs and subsequently experienced relapse or became refractory.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥2 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥2 different BCR-ABL1 TKIs and subsequently experienced relapse or became refractory.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥3 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥3 different BCR-ABL1 TKIs and subsequently experienced relapse or became refractory.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥4 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥4 different BCR-ABL1 TKIs and subsequently experienced relapse or became refractory.

In one specific embodiment, the BCR-ABL1 TKIs are first-line, second-line, third-line, fourth-line, or fifth-line therapeutic agent. In another specific embodiment, the BCR-ABL1 TKIs are first-line, second-line, or third-line therapeutic agent. In another specific embodiment, the BCR-ABL1 TKIs are first-line or second-line therapeutic agents. In another specific embodiment, the BCR-ABL1 TKIs are first-line therapeutic agents. In another specific embodiment, the BCR-ABL1 TKIs are second-line therapeutic agents. In another specific embodiment, the BCR-ABL1 TKIs are second-line or higher-line therapeutic agents. In another more specific embodiment, the BCR-ABL1 TKIs include at least third-generation TKIs (e.g., ponatinib or olverembatinib). In another more specific embodiment, the BCR-ABL1 TKIs include at least third-generation TKIs (e.g., ponatinib or olverembatinib) and/or fourth-generation TKIs (e.g., asciminib or HS-10382). In another specific embodiment, the BCR-ABL1 TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382. In another specific embodiment, the BCR-ABL1 TKIs are selected from imatinib, dasatinib, nilotinib, bosutinib, flumatinib, ponatinib, olverembatinib, radotinib or asciminib. In another specific embodiment, the BCR-ABL1 TKIs are selected from imatinib, dasatinib, nilotinib, ponatinib, olverembatinib or asciminib. In another specific embodiment, the BCR-ABL1 TKIs are selected from imatinib, dasatinib, nilotinib, bosutinib, flumatinib or radotinib. In another specific embodiment, the BCR-ABL1 TKIs are selected from ponatinib, olverembatinib, asciminib or HS-10382. In another specific embodiment, the BCR-ABL1 TKIs are selected from imatinib, dasatinib or nilotinib. In another specific embodiment, the BCR-ABL1 TKIs are selected from ponatinib or olverembatinib. In another specific embodiment, the BCR-ABL1 TKIs are selected from asciminib or HS-10382. In another specific embodiment, the BCR-ABL1 TKIs are selected from asciminib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-CP. In another more specific embodiment, the CML is Ph+ CML-CP with T315I mutation. In another more specific embodiment, the CML is Ph+ and BCR-ABL1+ CML-CP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively

compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I)

or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in chronic phase (CML-CP) that has previously received treatment with ≥1 different BCR-ABL1 ATP-competitive TKIs and subsequently experienced relapse or became refractory

In one embodiment, the present disclosure relates to the use and method of the compound of formula (I) or a pharmaceutically acceptable salt, crystal form, solvate, or hydrate thereof for treating CML in chronic phase or its central nervous system leukemia recurrence, such as brain metastasis, wherein the CML has previously received treatment with ≥1 different BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥1 different BCR-ABL1 ATP-competitive TKIs and subsequently experienced relapse or became refractory.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥2 different BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥2 different BCR-ABL1 ATP-competitive TKIs and subsequently experienced relapse or became refractory.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥3 different BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥3 different BCR-ABL1 ATP-competitive TKIs and subsequently experienced relapse or became refractory.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥4 different BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥4 different BCR-ABL1 ATP-competitive TKIs and subsequently experienced relapse or became refractory.

In one specific embodiment, the BCR-ABL1 ATP-competitive TKIs are a first-line, second-line, third-line, fourth-line, or fifth-line therapeutic agent. In one specific embodiment, the BCR-ABL1 ATP-competitive TKIs are a first-line, second-line, or third-line therapeutic agent. In another specific embodiment, the BCR-ABL1 ATP-competitive TKIs are first-line or second-line therapeutic agents. In another specific embodiment, the BCR-ABL1 ATP-competitive TKIs are first-line therapeutic agents. In another specific embodiment, the BCR-ABL1 ATP-competitive TKIs are second-line therapeutic agents. In another specific embodiment, the BCR-ABL1 ATP-competitive TKIs are second-line or higher-line therapeutic agents. In another more specific embodiment, the BCR-ABL1 ATP-competitive TKIs include at least third-generation TKIs (e.g., ponatinib or olverembatinib). In another specific embodiment, the BCR-ABL1 ATP-competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, HS-10382, rebasinib, danusertib, tozasertib, AT9283, KW-2449 or XL228. In another specific embodiment, the BCR-ABL1 ATP-competitive TKIs are selected from imatinib, dasatinib, nilotinib, bosutinib, flumatinib, ponatinib, olverembatinib or radotinib. In another specific embodiment, the BCR-ABL1 ATP-competitive TKIs are selected from imatinib, dasatinib, nilotinib, ponatinib or olverembatinib. In another specific embodiment, the BCR-ABL1 ATP-competitive TKIs are selected from imatinib, dasatinib, nilotinib, bosutinib, flumatinib or radotinib. In another specific embodiment, the BCR-ABL1 ATP-competitive TKIs are selected from imatinib, dasatinib or nilotinib. In another specific embodiment, the BCR-ABL1 ATP-competitive

TKIs are selected from ponatinib or olverembatinib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-CP. In another more specific embodiment, the CML is Ph+ CML-CP with T315I mutation. In another more specific embodiment, the CML is Ph+ and BCR-ABL1+ CML-CP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment,

the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in chronic phase (CML-CP) that has previously received treatment with ≥1 different first- and/or second-generation BCR-ABL1 ATP-competitive TKIs and subsequently experienced relapse or became refractory

In one embodiment, the present disclosure relates to the use and method of the compound of formula (I) or a pharmaceutically acceptable salt, crystal form, solvate, or hydrate thereof for treating CML in chronic phase or its central nervous system leukemia recurrence, such as brain metastasis, wherein the CML has previously received treatment with ≥1 different first- and/or second-generation BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥1 different first- and/or second-generation BCR-ABL1 ATP-competitive TKIs and subsequently experienced relapse or became refractory.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥2 different first- and/or second-generation BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥2 different first- and/or second-generation BCR-ABL1 ATP-competitive TKIs and subsequently experienced relapse or became refractory.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥3 different first- and/or second-generation BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥3 different first- and/or second-generation BCR-ABL1 ATP-competitive TKIs and subsequently experienced relapse or became refractory.

In another embodiment, the present disclosure relates to the use and method of a compound of formula (I) or a pharmaceutically acceptable salt, crystal form, solvate, or hydrate thereof for treating CML in chronic phase, wherein the CML has previously received treatment with ≥4 different first- and/or second-generation BCR-ABL1 ATP-competitive TKIs

and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with ≥4 different first- and/or second-generation BCR-ABL1 ATP-competitive TKIs and subsequently experienced relapse or became refractory.

In one specific embodiment, the first- or second-generation BCR-ABL1 ATP-competitive TKIs are a first-line, second-line, third-line, fourth-line, or fifth-line therapeutic agent. In another more specific embodiment, the first- or second-generation BCR-ABL1 ATP-competitive TKIs are a first-line, second-line, or third-line therapeutic agent. In another specific embodiment, the first- or second-generation BCR-ABL1 ATP-competitive TKIs are first-line or second-line therapeutic agents. In another specific embodiment, the first- or second-generation BCR-ABL1 ATP-competitive TKIs are first-line therapeutic agents. In another specific embodiment, the first- or second-generation BCR-ABL1 ATP-competitive TKIs are second-line therapeutic agents. In another specific embodiment, the first- or second-generation BCR-ABL1 ATP-competitive TKIs are second-line or higher-line therapeutic agents. In another specific embodiment, the first-generation BCR-ABL1 ATP-competitive TKI is imatinib. In another specific embodiment, the second-generation BCR-ABL1 ATP-competitive TKIs are selected from dasatinib, nilotinib, bosutinib, flumatinib or radotinib. In another specific embodiment, the first- and/or second-generation BCR-ABL1 ATP-competitive TKIs are selected from imatinib, dasatinib, nilotinib, bosutinib, flumatinib or radotinib. In another specific embodiment, the first- and/or second-generation BCR-ABL1 ATP-competitive TKIs are selected from imatinib, dasatinib or nilotinib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-CP. In another more specific embodiment, the CML is Ph+ CML-CP with T315I mutation. In another more specific embodiment, the CML is Ph+ and BCR-ABL1+ CML-CP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220

mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in chronic phase (CML-CP) that has previously received treatment with third or above generations of BCR-ABL1 TKIs and subsequently failed or was intolerant

In one embodiment, the present disclosure relates to the use and method of the compound of formula (I) or a pharmaceutically acceptable salt, crystal form, solvate, or hydrate thereof for treating CML in chronic phase or its central nervous system leukemia recurrence, such as brain metastasis, wherein the CML has previously received treatment with third or above generations of BCR-ABL1 TKIs and subsequently failed or was intolerant. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with third or above generations of BCR-ABL1 TKIs and subsequently failed or was intolerant.

In one specific embodiment, the BCR-ABL1 TKIs are first-line, second-line, third-line, fourth-line, or fifth-line therapeutic agent. In another specific embodiment, the BCR-ABL1 TKIs are first-line, second-line, or third-line therapeutic agent. In another specific embodiment, the BCR-ABL1 TKIs are first-line or second-line therapeutic agents. In another specific embodiment, the BCR-ABL1 TKIs are first-line or above therapeutic agents. In another specific embodiment, the

BCR-ABL1 TKIs are second-line or above therapeutic agents. In another specific embodiment, the BCR-ABL1 TKIs are third-line or above therapeutic agents. In another more specific embodiment, the BCR-ABL1 TKIs include at least third-generation TKIs (e.g., ponatinib or olverembatinib). In another more specific embodiment, the BCR-ABL1 TKIs include at least third-generation TKIs (e.g., ponatinib or olverembatinib) and/or fourth-generation TKIs (e.g., asciminib or HS-10382). In another specific embodiment, the BCR-ABL1 TKIs are selected from ponatinib, olverembatinib, PF-114, rebasinib, danusertib, tozasertib, AT9283, KW-2449, XL228, asciminib or HS-10382. In another specific embodiment, the BCR-ABL1 TKIs are selected from ponatinib, olverembatinib, PF-114, asciminib or HS-10382. In another specific embodiment, the BCR-ABL1 TKIs are selected from ponatinib, olverembatinib or PF-114. In another specific embodiment, the BCR-ABL1 TKIs are selected from ponatinib or olverembatinib. In another specific embodiment, the BCR-ABL1 TKIs are selected from ponatinib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-CP. In another more specific embodiment, the CML is Ph+ CML-CP with T315I mutation. In another more specific embodiment, the CML is Ph+ and BCR-ABL1+ CML-CP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof

(alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in chronic phase (CML-CP) that that has previously received treatment with third-generation BCR-ABL1 TKIs and subsequently developed resistance or intolerance

In one embodiment, the present disclosure relates to the use and method of the compound of formula (I) or a pharmaceutically acceptable salt, crystal form, solvate, or hydrate thereof for treating CML in chronic phase or its central nervous system leukemia recurrence, such as brain metastasis, wherein the CML has previously received treatment with third-generation BCR-ABL1 TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in chronic phase, wherein the CML has previously received treatment with third-generation BCR-ABL1 TKIs and subsequently developed resistance or intolerance.

In another specific embodiment, the definition of treatment resistance for CML-CP requires meeting at least one of the following criteria:

a. Failure to achieve a complete hematologic response (3 months prior to treatment treatment);
b. Failure to achieve a minor cytogenetic response (6 months prior to treatment treatment);
c. Failure to achieve a major cytogenetic response (12 months prior to treatment treatment);
d. Emergence of new BCR-ABL1 kinase domain mutations or new clonal evolution;
e. CML-CP patients are also considered resistant if, at any point during TKI treatment, they experience a loss of response, develop a kinase domain mutation while failing to achieve a complete cytogenetic response, or progress to CML-AP or CML-BP.

In another specific embodiment, intolerance to CML-CP treatment is defined as inability to tolerate the current

TKI therapy due to toxicity, or disease progression while on the current TKI, with inability to receive a higher dose due to toxicity.

In another specific embodiment, the CML has previously received treatment with first- and/or second-generation BCR-ABL1 TKI and subsequently developed resistance or intolerance. In another specific embodiment, the CML has previously received treatment with first- and/or second-generation BCR-ABL1 TKI before receiving third-generation BCR-ABL1 TKI therapy and subsequently developed resistance or intolerance. In another specific embodiment, the CML has not previously received treatment with first- and/or second-generation BCR-ABL1 TKI and developed resistance or intolerance. In another specific embodiment, the CML has also previously received treatment with fourth-generation BCR-ABL1 TKIs and subsequently developed resistance or intolerance. In another specific embodiment, the CML has not previously received treatment with fourth-generation BCR-ABL1 TKIs and subsequently developed resistance or intolerance. In another specific embodiment, the CML has also previously received treatment with first- and/or second-generation and/or fourth-generation BCR-ABL1 TKIs and subsequently developed resistance or intolerance.

In another specific embodiment, the first- or second-generation BCR-ABL1 TKIs are first-line or second-line therapeutic agents. In another more specific embodiment, the first- or second-generation BCR-ABL1 TKIs are first-line therapeutic agents. In another more specific embodiment, the first- or second-generation BCR-ABL1 TKIs are second-line therapeutic agents. In another more specific embodiment, the first- or second-generation BCR-ABL1 TKIs are second-line or higher-line therapeutic agents. In another more specific embodiment, the first-generation BCR-ABL1 TKI is imatinib. In another more specific embodiment, the second-generation BCR-ABL1 ATP-competitive TKIs are selected from dasatinib, nilotinib, bosutinib, flumatinib or radotinib. In another more specific embodiment, the first- and/or second-generation BCR-ABL1 TKIs are selected from imatinib, dasatinib, nilotinib, bosutinib, flumatinib or radotinib. In another more specific embodiment, the first- and/or second-generation BCR-ABL1 ATP-competitive TKIs are selected from imatinib, dasatinib, nilotinib or flumatinib. In another specific embodiment, the third-generation BCR-ABL1 TKIs are ponatinib or olverembatinib. In another specific embodiment, the fourth-generation BCR-ABL1 TKIs is asciminib or HS-10382.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-CP. In another more specific embodiment, the CML is Ph+ CML-CP with T315I mutation. In another more specific embodiment, the CML is Ph+ and BCR-ABL1+ CML-CP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160

mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in accelerated phase (CML-AP)

In one embodiment, the present disclosure relates to the use and method of the compound of formula (I) or a pharmaceutically acceptable salt, crystal form, solvate, or hydrate thereof for treating CML in accelerated phase or its central nervous system leukemia recurrence, such as brain metastasis. In another embodiment, the present disclosure relates to the use and method of (R)-N-(4-(chlorodifluoromethoxy)phenyl)-6-(3-fluoropyrrolidin-1-yl)-5-(pyrazin-2-yl) nicotinamide (Compound A) or a pharmaceutically acceptable salt, crystal form, solvate, or hydrate thereof for treating CML in accelerated phase.

In another specific embodiment, the CML is Ph+ CML. In another specific embodiment, the CML is BCR-ABL1+ CML. In another specific embodiment, the CML is CML with T315I mutation. In another specific embodiment, the CML is

CML without T315I mutation. In another more specific embodiment, the CML is Ph+ CML-AP. In another more specific embodiment, the CML is Ph+ CML-AP with T315I mutation. In another more specific embodiment, the CML is Ph+ and BCR-ABL1+ CML-AP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the CML has previously received treatment with ≥1 different BCR-ABL1 TKIs and subsequently experienced disease progression to blast phase, with treatment evaluation results being failure, warning, or intolerance. In another specific embodiment, the CML has previously received treatment with ≥2 different BCR-ABL1 TKIs and subsequently experienced disease progression to blast phase, with treatment evaluation results being failure, warning, or intolerance. In another specific embodiment, the CML has previously received treatment with ≥3 different BCR-ABL1 TKIs and subsequently experienced disease progression to blast phase, with treatment evaluation results being failure, warning, or intolerance. In another specific embodiment, the CML has previously received treatment with ≥4 different BCR-ABL1 TKIs and subsequently experienced disease progression to blast phase, with treatment evaluation results being failure, warning, or intolerance. In another more specific embodiment, the BCR-ABL1 TKIs are first-line, second-line, third-line, fourth-line, or fifth-line therapeutic agent. In another more specific embodiment, the BCR-ABL1 TKIs are first-line, second-line, or third-line therapeutic agent. In another more specific embodiment, the BCR-ABL1 TKIs are first-line or second-line therapeutic agents. In another more specific embodiment, the BCR-ABL1 TKIs are first-line therapeutic agents. In another more specific embodiment, the BCR-ABL1 TKIs are second-line therapeutic agents. In another more specific embodiment, the BCR-ABL1 TKIs are second-line or higher-line therapeutic agents. In another more specific embodiment, the BCR-ABL1 TKIs include at least third-generation TKIs (e.g., ponatinib or olverembatinib). In another more specific embodiment, the BCR-ABL1 TKIs include at least third-generation TKIs (e.g., ponatinib or olverembatinib) and/or fourth-generation TKIs (e.g., asciminib or HS-10382). In another more specific embodiment, the BCR-ABL1 TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382. In another more specific embodiment, the BCR-ABL1 TKIs are selected from imatinib, dasatinib, nilotinib, bosutinib, flumatinib, ponatinib, olverembatinib, radotinib or asciminib. In another more specific embodiment, the BCR-ABL1 TKIs are selected from imatinib, dasatinib, nilotinib, ponatinib, olverembatinib or asciminib. In another more specific embodiment, the BCR-ABL1 TKIs are selected from imatinib, dasatinib, nilotinib, bosutinib, flumatinib or radotinib. In another more specific embodiment, the BCR-ABL1 TKIs are selected from ponatinib, olverembatinib, asciminib or HS-10382. In another more specific embodiment, the BCR-ABL1 TKIs are selected from imatinib, dasatinib or nilotinib. In another more specific embodiment, the BCR-ABL1 TKIs are selected from ponatinib or olverembatinib. In another more specific embodiment, the BCR-ABL1 TKIs are selected from asciminib or HS-10382. In another more specific embodiment, the BCR-ABL1 TKIs are selected from asciminib.

In another specific embodiment, the CML has previously received treatment with ≥1 different BCR-ABL1 TKIs and subsequently experienced relapse or became refractory. In another specific embodiment, the CML has previously received treatment with ≥2 different BCR-ABL1 TKIs and subsequently experienced relapse or became refractory. In another specific embodiment, the CML has previously received treatment with ≥3 different BCR-ABL1 TKIs and subsequently experienced relapse or became refractory. In another specific embodiment, the CML has previously received treatment with ≥4 different BCR-ABL1 TKIs and subsequently experienced relapse or became refractory. In another more specific embodiment, the BCR-ABL1 TKIs are first-line, second-line, third-line, fourth-line, or fifth-line therapeutic agent. In another more specific embodiment, the BCR-ABL1 TKIs are first-line, second-line, or third-line therapeutic agent. In another more specific embodiment, the BCR-ABL1 TKIs are first-line or second-line therapeutic agents. In another more specific embodiment, the BCR-ABL1 TKIs are first-line therapeutic agents. In another more specific embodiment, the BCR-ABL1 TKIs are second-line therapeutic agents. In another more specific embodiment, the BCR-ABL1 TKIs are second-line or higher-line therapeutic agents. In another more specific embodiment, the BCR-ABL1 TKIs include at least third-generation TKIs (e.g., ponatinib or olverembatinib). In another more specific embodiment, the BCR-ABL1 TKIs include at least third-generation TKIs (e.g., ponatinib or olverembatinib) and/or fourth-generation TKIs (e.g., asciminib or HS-10382). In another more specific embodiment, the BCR-ABL1 TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382. In another more specific embodiment, the BCR-ABL1 TKIs are selected from imatinib, dasatinib, nilotinib, bosutinib, flumatinib, ponatinib, olverembatinib, radotinib or asciminib. In another more specific embodiment, the BCR-ABL1 TKIs are selected from imatinib, dasatinib, nilotinib, ponatinib, olverembatinib or asciminib. In another more specific embodiment, the BCR-ABL1 TKIs are selected from imatinib, dasatinib, nilotinib, bosutinib, flumatinib or radotinib. In another more specific embodiment, the BCR-ABL1 TKIs are selected from ponatinib, olverembatinib, asciminib or HS-10382. In another more specific embodiment, the BCR-ABL1 TKIs are selected from imatinib, dasatinib or nilotinib. In another more specific embodiment, the BCR-ABL1 TKIs are selected from ponatinib or

olverembatinib. In another more specific embodiment, the BCR-ABL1 TKIs are selected from asciminib or HS-10382. In another more specific embodiment, the BCR-ABL1 TKIs are selected from asciminib.

In another specific embodiment, the CML has previously received treatment with ≥1 different BCR-ABL1 ATP-competitive TKIs and subsequently experienced disease progression to blast phase, with treatment evaluation results being failure, warning, or intolerance. In another specific embodiment, the CML has previously received treatment with ≥2 different BCR-ABL1 ATP-competitive TKIs and subsequently experienced disease progression to blast phase, with treatment evaluation results being failure, warning, or intolerance. In another specific embodiment, the CML has previously received treatment with ≥3 different BCR-ABL1 ATP-competitive TKIs and subsequently experienced disease progression to blast phase, with treatment evaluation results being failure, warning, or intolerance. In another specific embodiment, the CML has previously received treatment with ≥4 different BCR-ABL1 ATP-competitive TKIs and subsequently experienced disease progression to blast phase, with treatment evaluation results being failure, warning, or intolerance. In another more specific embodiment, the BCR-ABL1 ATP-competitive TKIs are a first-line, second-line, third-line, fourth-line, or fifth-line therapeutic agent. In another more specific embodiment, the BCR-ABL1 ATP-competitive TKIs are a first-line, second-line, or third-line therapeutic agent. In another more specific embodiment, the BCR-ABL1 ATP-competitive TKIs are first-line or second-line therapeutic agents. In another more specific embodiment, the BCR-ABL1 ATP-competitive TKIs are first-line therapeutic agents. In another more specific embodiment, the BCR-ABL1 ATP-competitive TKIs are second-line therapeutic agents. In another more specific embodiment, the BCR-ABL1 ATP-competitive TKIs are second-line or higher-line therapeutic agents. In another more specific embodiment, the BCR-ABL1 ATP-competitive TKIs include at least third-generation TKIs (e.g., ponatinib or olverembatinib). In another more specific embodiment, the BCR-ABL1 ATP-competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228. In another more specific embodiment, the BCR-ABL1 ATP-competitive TKIs are selected from imatinib, dasatinib, nilotinib, bosutinib, flumatinib, ponatinib, olverembatinib or radotinib. In another more specific embodiment, the BCR-ABL1 ATP-competitive TKIs are selected from imatinib, dasatinib, nilotinib, ponatinib or olverembatinib. In another more specific embodiment, the BCR-ABL1 ATP-competitive TKIs are selected from imatinib, dasatinib, nilotinib, bosutinib, flumatinib or radotinib. In another more specific embodiment, the BCR-ABL1 TKIs are selected from ponatinib, olverembatinib, asciminib or HS-10382. In another more specific embodiment, the BCR-ABL1 ATP-competitive TKIs are selected from imatinib, dasatinib or nilotinib. In another more specific embodiment, the BCR-ABL1 ATP-competitive TKIs are selected from ponatinib or olverembatinib.

In another specific embodiment, the CML has previously received treatment with ≥1 different BCR-ABL1 ATP-competitive TKIs and subsequently experienced relapse or became refractory. In another specific embodiment, the CML has previously received treatment with ≥2 different BCR-ABL1 ATP-competitive TKIs and subsequently experienced relapse or became refractory. In another specific embodiment, the CML has previously received treatment with ≥3 different BCR-ABL1 ATP-competitive TKIs and subsequently experienced relapse or became refractory. In another specific embodiment, the CML has previously received treatment with ≥4 different BCR-ABL1 ATP-competitive TKIs and subsequently experienced relapse or became refractory. In another more specific embodiment, the BCR-ABL1 ATP-competitive TKIs are a first-line, second-line, third-line, fourth-line, or fifth-line therapeutic agent. In another more specific embodiment, the BCR-ABL1 ATP-competitive TKIs are a first-line, second-line, or third-line therapeutic agent. In another more specific embodiment, the BCR-ABL1 ATP-competitive TKIs are first-line or second-line therapeutic agents. In another more specific embodiment, the BCR-ABL1 ATP-competitive TKIs are first-line or second-line therapeutic agents. In another more specific embodiment, the BCR-ABL1 ATP-competitive TKIs are first-line therapeutic agents. In another more specific embodiment, the BCR-ABL1 ATP-competitive TKIs are second-line therapeutic agents. In another more specific embodiment, the BCR-ABL1 ATP-competitive TKIs are second-line or higher-line therapeutic agents. In another more specific embodiment, the BCR-ABL1 ATP-competitive TKIs include at least third-generation TKIs (e.g., ponatinib or olverembatinib). In another more specific embodiment, the BCR-ABL1 ATP-competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228. In another more specific embodiment, the BCR-ABL1 ATP-competitive TKIs are selected from imatinib, dasatinib, nilotinib, bosutinib, flumatinib, ponatinib, olverembatinib or radotinib. In another more specific embodiment, the BCR-ABL1 ATP-competitive TKIs are selected from imatinib, dasatinib, nilotinib, ponatinib or olverembatinib. In another more specific embodiment, the BCR-ABL1 ATP-competitive TKIs are selected from imatinib, dasatinib, nilotinib, bosutinib, flumatinib or radotinib. In another more specific embodiment, the BCR-ABL1 ATP-competitive TKIs are selected from ponatinib, olverembatinib, asciminib or HS-10382. In another more specific embodiment, the BCR-ABL1 ATP-competitive TKIs are selected from imatinib, dasatinib or nilotinib. In another more specific embodiment, the BCR-ABL1 ATP-competitive TKIs are selected from ponatinib or olverembatinib.

In another specific embodiment, the CML has previously received treatment with ≥1 different first- and/or second-generation BCR-ABL1 ATP-competitive TKIs and subsequently developed resistance or intolerance. In another specific

embodiment, the CML has previously received treatment with ≥2 different first- and/or second-generation BCR-ABL1 ATP-competitive TKIs and subsequently developed resistance or intolerance. In another specific embodiment, the CML has previously received treatment with ≥3 different first- and/or second-generation BCR-ABL1 ATP-competitive TKIs and subsequently developed resistance or intolerance. In another specific embodiment, the CML has previously received treatment with ≥4 different first- and/or second-generation BCR-ABL1 ATP-competitive TKIs and subsequently developed resistance or intolerance. In another more specific embodiment, the resistance arises from BCR-ABL1 kinase domain point mutation(s), such as one or more of T315I/A, Y253F/H, E255V/K, M351T, G250E, F359V/I/C, H396P/R, M244V, E355G, F317L/V/I/C, F311L, M237I, Q252H/R, D276G, L248V, V379I, L384M, L387M, E279K, F486S, E459K, P223S, I502L, or V299L. In another more specific embodiment, the resistance involves BCR-ABL1 kinase domain point mutations, selected from one or more of T315I, Y253H, E255V/K, M351T, G250E, F359V/, H396P, M244V, E355G, F317L, Q252H, E459K, P223S, I502L, or V299L mutations. In another more specific embodiment, the resistance is T315I. In another more specific embodiment, the first- or second-generation BCR-ABL1 ATP-competitive TKIs are a first-line, second-line, third-line, fourth-line, or fifth-line therapeutic agent. In another more specific embodiment, the first- or second-generation BCR-ABL1 ATP-competitive TKIs are a first-line, second-line, or third-line therapeutic agent. In another more specific embodiment, the first- or second-generation BCR-ABL1 ATP-competitive TKIs are first-line or second-line therapeutic agents. In another more specific embodiment, the first- or second-generation BCR-ABL1 ATP-competitive TKIs are first-line therapeutic agents. In another more specific embodiment, the first- or second-generation BCR-ABL1 ATP-competitive TKIs are second-line therapeutic agents. In another more specific embodiment, the first- or second-generation BCR-ABL1 ATP-competitive TKIs are second-line or higher-line therapeutic agents. In another more specific embodiment, the first-generation BCR-ABL1 ATP-competitive TKI is imatinib. In another more specific embodiment, the second-generation BCR-ABL1 ATP-competitive TKIs are selected from dasatinib, nilotinib, bosutinib, flumatinib or radotinib. In another more specific embodiment, the first- and/or second-generation BCR-ABL1 ATP-competitive TKIs are selected from imatinib, dasatinib, nilotinib, bosutinib, flumatinib or radotinib. In another more specific embodiment, the first- and/or second-generation BCR-ABL1 ATP-competitive TKIs are selected from imatinib, dasatinib or nilotinib.

In another specific embodiment, the CML has previously received treatment with ≥1 different first- and/or second-generation BCR-ABL1 ATP-competitive TKIs and subsequently experienced relapse or became refractory. In another specific embodiment, the CML has previously received treatment with ≥2 different first- and/or second-generation BCR-ABL1 ATP-competitive TKIs and subsequently experienced relapse or became refractory. In another specific embodiment, the CML has previously received treatment with ≥3 different first- and/or second-generation BCR-ABL1 ATP-competitive TKIs and subsequently experienced relapse or became refractory. In another specific embodiment, the CML has previously received treatment with ≥4 different first- and/or second-generation BCR-ABL1 ATP-competitive TKIs and subsequently experienced relapse or became refractory. In another more specific embodiment, the first- or second-generation BCR-ABL1 ATP-competitive TKIs are a first-line, second-line, third-line, fourth-line, or fifth-line therapeutic agent. In another more specific embodiment, the first- or second-generation BCR-ABL1 ATP-competitive TKIs are a first-line, second-line, or third-line therapeutic agent. In another more specific embodiment, the first- or second-generation BCR-ABL1 ATP-competitive TKIs are first-line or second-line therapeutic agents. In another more specific embodiment, the first- or second-generation BCR-ABL1 ATP-competitive TKIs are first-line therapeutic agents. In another more specific embodiment, the first- or second-generation BCR-ABL1 ATP-competitive TKIs are second-line therapeutic agents. In another more specific embodiment, the first- or second-generation BCR-ABL1 ATP-competitive TKIs are second-line or higher-line therapeutic agents. In another more specific embodiment, the first-generation BCR-ABL1 ATP-competitive TKI is imatinib. In another more specific embodiment, the second-generation BCR-ABL1 ATP-competitive TKIs are selected from dasatinib, nilotinib, bosutinib, flumatinib or radotinib. In another more specific embodiment, the first- and/or second-generation BCR-ABL1 ATP-competitive TKIs are selected from imatinib, dasatinib, nilotinib, bosutinib, flumatinib or radotinib. In another more specific embodiment, the first- and/or second-generation BCR-ABL1 ATP-competitive TKIs are selected from imatinib, dasatinib or nilotinib.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20

mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in accelerated phase (CML-AP) that has previously received treatment with ≥1 different BCR-ABL1 TKIs and subsequently experienced disease progression to blast phase, with treatment evaluation results being failure, warning, or intolerance

In one embodiment, the present disclosure relates to the use and method of the compound of formula (I) or a pharmaceutically acceptable salt, crystal form, solvate, or hydrate thereof for treating CML in accelerated phase or its central nervous system leukemia recurrence, such as brain metastasis, wherein the CML has previously received treatment with ≥1 different BCR-ABL1 TKIs and subsequently experienced disease progression to blast phase, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥1 different BCR-ABL1 TKIs and subsequently experienced disease progression to blast phase, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥2 different BCR-ABL1 TKIs and subsequently experienced disease progression to blast phase, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥2 different BCR-ABL1 TKIs and subsequently experienced disease progression to blast phase, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥3 different BCR-ABL1 TKIs and subsequently experienced disease progression to blast phase, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥3 different BCR-ABL1 TKIs and subsequently experienced disease progression to blast phase, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥4 different BCR-ABL1 TKIs and subsequently experienced disease progression to blast phase, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥4 different BCR-ABL1 TKIs and subsequently experienced disease progression to blast phase, with treatment evaluation results being failure, warning, or intolerance.

In one specific embodiment, the BCR-ABL1 TKIs are first-line, second-line, third-line, fourth-line, or fifth-line therapeutic agent. In another specific embodiment, the BCR-ABL1 TKIs are first-line, second-line, or third-line therapeutic agent. In another specific embodiment, the BCR-ABL1 TKIs are first-line or second-line therapeutic agents. In another specific embodiment, the BCR-ABL1 TKIs are first-line therapeutic agents. In another specific embodiment, the BCR-ABL1 TKIs are second-line therapeutic agents. In another specific embodiment, the BCR-ABL1 TKIs are second-line or higher-line therapeutic agents. In another more specific embodiment, the BCR-ABL1 TKIs include at least third-generation TKIs (e.g., ponatinib or olverembatinib). In another more specific embodiment, the BCR-ABL1 TKIs include at least third-generation TKIs (e.g., ponatinib or olverembatinib) and/or fourth-generation TKIs (e.g., asciminib or HS-10382). In another specific embodiment, the BCR-ABL1 TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382. In another more specific embodiment, the BCR-ABL1 TKIs are selected from imatinib, dasatinib, nilotinib, bosutinib, flumatinib, ponatinib, olverembatinib, radotinib or asciminib. In another more specific embodiment, the BCR-ABL1 TKIs are selected from

imatinib, dasatinib, nilotinib, ponatinib, olverembatinib or asciminib. In another more specific embodiment, the BCR-ABL1 TKIs are selected from imatinib, dasatinib, nilotinib, bosutinib, flumatinib or radotinib. In another more specific embodiment, the BCR-ABL1 TKIs are selected from ponatinib, olverembatinib, asciminib or HS-10382. In another more specific embodiment, the BCR-ABL1 TKIs are selected from imatinib, dasatinib or nilotinib. In another more specific embodiment, the BCR-ABL1 TKIs are selected from ponatinib or olverembatinib. In another more specific embodiment, the BCR-ABL1 TKIs are selected from asciminib or HS-10382. In another more specific embodiment, the BCR-ABL1 TKIs are selected from asciminib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-AP. In another more specific embodiment, the CML is Ph+ CML-AP with T315I mutation. In another more specific embodiment, the CML is Ph+ and BCR-ABL1+ CML-AP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt,

crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in accelerated phase (CML-AP) that has previously received treatment with ≥1 different BCR-ABL1 ATP-competitive TKIs and subsequently experienced disease progression to blast phase, with treatment evaluation results being failure, warning, or intolerance

In one embodiment, the present disclosure relates to the use and method of the compound of formula (I) or a pharmaceutically acceptable salt, crystal form, solvate, or hydrate thereof for treating CML in accelerated phase or its central nervous system leukemia recurrence, such as brain metastasis, wherein the CML has previously received treatment with ≥1 different BCR-ABL1 ATP-competitive TKIs and subsequently experienced disease progression to blast phase, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥1 different BCR-ABL1 ATP-competitive TKIs and subsequently experienced disease progression to blast phase, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥2 different BCR-ABL1 ATP-competitive TKIs and subsequently experienced disease progression to blast phase, with treatment evaluation results being failure, warning, or intolerance. In another embodiment, the present disclosure relates to the use and method of (R)-N-(4-(chlorodifluoromethoxy) phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥2 different BCR-ABL1 ATP-competitive TKIs and subsequently experienced disease progression to blast phase, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥3 different BCR-ABL1 ATP-competitive TKIs and subsequently

experienced disease progression to blast phase, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥3 different BCR-ABL1 ATP-competitive TKIs and subsequently experienced disease progression to blast phase, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥4 different BCR-ABL1 ATP-competitive TKIs and subsequently experienced disease progression to blast phase, with treatment evaluation results being failure, warning, or intolerance. In another embodiment, the present disclosure relates to the use and method of (R)-N-(4-(chlorodifluoromethoxy) phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥4 different BCR-ABL1 ATP-competitive TKIs and subsequently experienced disease progression to blast phase, with treatment evaluation results being failure, warning, or intolerance.

In another specific embodiment, the BCR-ABL1 ATP-competitive TKIs are a first-line, second-line, third-line, fourth-line, or fifth-line therapeutic agent. In another specific embodiment, the BCR-ABL1 ATP-competitive TKIs are a first-line, second-line, or third-line therapeutic agent. In another specific embodiment, the BCR-ABL1 ATP-competitive TKIs are first-line or second-line therapeutic agents. In another specific embodiment, the BCR-ABL1 ATP-competitive TKIs are first-line therapeutic agents. In another specific embodiment, the BCR-ABL1 ATP-competitive TKIs are second-line therapeutic agents. In another specific embodiment, the BCR-ABL1 ATP-competitive TKIs are second-line or higher-line therapeutic agents. In another more specific embodiment, the BCR-ABL1 ATP-competitive TKIs include at least third-generation TKIs (e.g., ponatinib or olverembatinib). In another specific embodiment, the BCR-ABL1 ATP-competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228. In another specific embodiment, the BCR-ABL1 ATP-competitive TKIs are selected from imatinib, dasatinib, nilotinib, bosutinib, flumatinib, ponatinib, olverembatinib or radotinib. In another specific embodiment, the BCR-ABL1 ATP-competitive TKIs are selected from imatinib, dasatinib, nilotinib, ponatinib or olverembatinib. In another specific embodiment, the BCR-ABL1 ATP-competitive TKIs are selected from imatinib, dasatinib, nilotinib, bosutinib, flumatinib or radotinib. In another more specific embodiment, the BCR-ABL1 ATP-competitive TKIs are selected from ponatinib, olverembatinib, asciminib or HS-10382. In another specific embodiment, the BCR-ABL1 ATP-competitive TKIs are selected from imatinib, dasatinib or nilotinib. In another specific embodiment, the BCR-ABL1 ATP-competitive TKIs are selected from ponatinib or olverembatinib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-AP. In another more specific embodiment, the CML is Ph+ CML-AP with T315I mutation. In another more specific embodiment, the CML is Ph+ and BCR-ABL1+ CML-AP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40

mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in accelerated phase (CML-AP) that has previously received treatment with ≥1 different first- and/or second-generation BCR-ABL1 ATP-competitive TKIs and subsequently developed resistance or intolerance

In one embodiment, the present disclosure relates to the use and method of the compound of formula (I) or a pharmaceutically acceptable salt, crystal form, solvate, or hydrate thereof for treating CML in accelerated phase or its central nervous system leukemia recurrence, such as brain metastasis, wherein the CML has previously received treatment with ≥1 different first- and/or second-generation BCR-ABL1 ATP-competitive TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥1 different first- and/or second-generation BCR-ABL1 ATP-competitive TKIs and subsequently developed resistance or intolerance.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥2 different first- and/or second-generation BCR-ABL1 ATP-competitive TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥2 different first- and/or second-generation BCR-ABL1 ATP-competitive TKIs and subsequently developed resistance or intolerance.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥3 different first- and/or second-generation BCR-ABL1 ATP-competitive TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥3 different first- and/or second-generation BCR-ABL1 ATP-competitive TKIs and subsequently developed resistance or intolerance.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥4 different first- and/or second-generation BCR-ABL1 ATP-competitive TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥4 different first- and/or second-generation BCR-ABL1 ATP-competitive TKIs and subsequently developed resistance or intolerance.

In another specific embodiment, the resistance arises from BCR-ABL1 kinase domain point mutation(s), such as one or more of T315I/A, Y253F/H, E255V/K, M351T, G250E, F359V/I/C, H396P/R, M244V, E355G, F317L/V/I/C, F311L, M237I, Q252H/R, D276G, L248V, V379I, L384M, L387M, E279K, F486S, E459K, P223S, I502L, or V299L. In another specific embodiment, the resistance involves BCR-ABL1 kinase domain point mutations, selected from one or more of T315I, Y253H, E255V/K, M351T, G250E, F359V/, H396P, M244V, E355G, F317L, Q252H, E459K, P223S, I502L, or V299L mutations. In another specific embodiment, the resistance is T315I. In another specific embodiment, the first- or second-generation BCR-ABL1 ATP-competitive TKIs are a first-line, second-line, third-line, fourth-line, or fifth-line therapeutic agent. In another specific embodiment, the first- or second-generation BCR-ABL1 ATP-competitive TKIs are a first-line, second-line, or third-line therapeutic agent. In another specific embodiment, the first- or second-generation BCR-ABL1 ATP-competitive TKIs are first-line or second-line therapeutic agents. In another specific embodiment, the first- or second-generation BCR-ABL1 ATP-competitive TKIs are first-line therapeutic agents. In another specific embodiment, the first- or second-generation BCR-ABL1 ATP-competitive TKIs are second-line therapeutic agents. In another specific embodiment, the first- or second-generation BCR-ABL1 ATP-competitive TKIs are second-line or higher-line therapeutic agents. In another specific embodiment, the first-generation BCR-ABL1 ATP-competitive TKI is imatinib. In another specific embodiment, the second-generation BCR-ABL1 ATP-competitive TKIs are selected from dasatinib, nilotinib, bosutinib, flumatinib or radotinib. In another specific embodiment, the first- and/or second-generation BCR-ABL1 ATP-competitive TKIs are selected from imatinib, dasatinib, nilotinib, bosutinib, flumatinib or radotinib. In another specific embodiment, the first- and/or second-generation BCR-ABL1 ATP-competitive TKIs are selected from imatinib, dasatinib or nilotinib.

In another specific embodiment, the CML is Ph+ CML. In another specific embodiment, the CML is BCR-ABL1+ CML. In another specific embodiment, the CML is CML with T315I mutation. In another specific embodiment, the CML is

CML without T315I mutation. In another more specific embodiment, the CML is Ph+ CML-AP. In another more specific embodiment, the CML is Ph+ CML-AP with T315I mutation. In another more specific embodiment, the CML is Ph+ and BCR-ABL1+ CML-AP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered

orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in accelerated phase (CML-AP) that has previously received treatment with ≥1 different BCR-ABL1 TKIs and subsequently experienced relapse or became refractory

In one embodiment, the present disclosure relates to the use and method of the compound of formula (I) or a pharmaceutically acceptable salt, crystal form, solvate, or hydrate thereof for treating CML in accelerated phase or its central nervous system leukemia recurrence, such as brain metastasis, wherein the CML has previously received treatment with ≥1 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥1 different BCR-ABL1 TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥2 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥2 different BCR-ABL1 TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥3 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥3 different BCR-ABL1 TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥4 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥4 different BCR-ABL1 TKIs and subsequently experienced relapse or became refractory.

In another specific embodiment, the BCR-ABL1 TKIs are first-line, second-line, third-line, fourth-line, or fifth-line therapeutic agent. In another specific embodiment, the BCR-ABL1 TKIs are first-line, second-line, or third-line therapeutic agent. In another specific embodiment, the BCR-ABL1 TKIs are first-line or second-line therapeutic agents. In another specific embodiment, the BCR-ABL1 TKIs are first-line therapeutic agents. In another specific embodiment, the BCR-ABL1 TKIs are second-line therapeutic agents. In another specific embodiment, the BCR-ABL1 TKIs are second-line or higher-line therapeutic agents. In another more specific embodiment, the BCR-ABL1 TKIs include at least third-generation TKIs (e.g., ponatinib or olverembatinib). In another more specific embodiment, the BCR-ABL1 TKIs include at least third-generation TKIs (e.g., ponatinib or olverembatinib) and/or fourth-generation TKIs (e.g., asciminib or HS-10382). In another specific embodiment, the BCR-ABL1 TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382. In another specific embodiment, the BCR-ABL1 TKIs are selected from imatinib, dasatinib, nilotinib, bosutinib, flumatinib, ponatinib, olverembatinib, radotinib or asciminib. In another specific embodiment, the BCR-ABL1 TKIs are selected from imatinib, dasatinib, nilotinib, ponatinib, olverembatinib or asciminib. In another specific embodiment, the BCR-ABL1 TKIs are selected from imatinib, dasatinib, nilotinib, bosutinib, flumatinib or radotinib. In another specific embodiment, the BCR-ABL1 TKIs are selected from ponatinib, olverembatinib, asciminib or HS-10382. In another specific embodiment, the BCR-ABL1 TKIs are selected from imatinib, dasatinib or nilotinib. In another specific embodiment, the BCR-ABL1 TKIs are selected from ponatinib or olverembatinib. In another specific embodiment, the BCR-ABL1 TKIs are selected from asciminib or HS-10382. In another specific embodiment, the BCR-ABL1 TKIs are selected from asciminib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-AP. In another more specific embodiment, the CML is Ph+ CML-AP with T315I mutation. In another more specific embodiment, the CML is Ph+ and BCR-ABL1+ CML-AP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I)

or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in accelerated phase (CML-AP) that has previously received treatment with ≥1 different BCR-ABL1 ATP-competitive TKIs and subsequently experienced relapse or became refractory

In one embodiment, the present disclosure relates to the use and method of the compound of formula (I) or a pharmaceutically acceptable salt, crystal form, solvate, or hydrate thereof for treating CML in accelerated phase or its central nervous system leukemia recurrence, such as brain metastasis, wherein the CML has previously received treatment with ≥1 different BCR-ABL1 ATP-competitive TKIs and subsequently experienced relapse or became refractory. In another embodiment, the present disclosure relates to the use and method of (R)-N-(4-(chlorodifluoromethoxy) phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥1 different BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥2 different BCR-ABL1 ATP-competitive TKIs and subsequently

experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥2 different BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥3 different BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥3 different BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥4 different BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥4 different BCR-ABL1 ATP-competitive TKIs and subsequently experienced relapse or became refractory.

In another specific embodiment, the BCR-ABL1 ATP-competitive TKIs are a first-line, second-line, third-line, fourth-line, or fifth-line therapeutic agent. In another specific embodiment, the BCR-ABL1 ATP-competitive TKIs are a first-line, second-line, or third-line therapeutic agent. In another specific embodiment, the BCR-ABL1 ATP-competitive TKIs are first-line or second-line therapeutic agents. In another specific embodiment, the BCR-ABL1 ATP-competitive TKIs are first-line therapeutic agents. In another specific embodiment, the BCR-ABL1 ATP-competitive TKIs are second-line therapeutic agents. In another specific embodiment, the BCR-ABL1 ATP-competitive TKIs are second-line or higher-line therapeutic agents. In another more specific embodiment, the BCR-ABL1 ATP-competitive TKIs include at least third-generation TKIs (e.g., ponatinib or olverembatinib). In another specific embodiment, the BCR-ABL1 ATP-competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, HS-10382, rebasinib, danusertib, tozasertib, AT9283, KW-2449 or XL228. In another specific embodiment, the BCR-ABL1 ATP-competitive TKIs are selected from imatinib, dasatinib, nilotinib, bosutinib, flumatinib, ponatinib, olverembatinib or radotinib. In another specific embodiment, the BCR-ABL1 ATP-competitive TKIs are selected from imatinib, dasatinib, nilotinib, ponatinib or olverembatinib. In another specific embodiment, the BCR-ABL1 ATP-competitive TKIs are selected from imatinib, dasatinib, nilotinib, bosutinib, flumatinib or radotinib. In another specific embodiment, the BCR-ABL1 ATP-competitive TKIs are selected from ponatinib, olverembatinib, asciminib or HS-10382. In another specific embodiment, the BCR-ABL1 ATP-competitive TKIs are selected from imatinib, dasatinib or nilotinib. In another specific embodiment, the BCR-ABL1 ATP-competitive TKIs are selected from ponatinib or olverembatinib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-AP. In another more specific embodiment, the CML is Ph+ CML-AP with T315I mutation. In another more specific embodiment, the CML is Ph+ and BCR-ABL1+ CML-AP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120

mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg

QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in accelerated phase (CML-AP) that has previously received treatment with ≥1 different first- and/or second-generation BCR-ABL1 ATP-competitive TKIs and subsequently experienced relapse or became refractory

In one embodiment, the present disclosure relates to the use and method of the compound of formula (I) or a pharmaceutically acceptable salt, crystal form, solvate, or hydrate thereof for treating CML in accelerated phase or its central nervous system leukemia recurrence, such as brain metastasis, wherein the CML has previously received treatment with ≥1 different first- and/or second-generation BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥1 different first- and/or second-generation BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥2 different first- and/or second-generation BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥2 different first- and/or second-generation BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥3 different first- and/or second-generation BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥3 different first- and/or second-generation BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in accelerated phase, wherein the CML has previously received treatment with ≥4 different first- and/or second-generation BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with ≥4 different first- and/or second-generation BCR-ABL1 ATP-competitive TKIs and subsequently experienced relapse or became refractory.

In another specific embodiment, the first-generation BCR-ABL1 ATP-competitive TKI is a first-line, second-line, third-line, fourth-line, or fifth-line therapeutic agent. In another specific embodiment, the first-generation BCR-ABL1 ATP-competitive TKI is a first-line, second-line, or third-line therapeutic agent. In another specific embodiment, the first-generation BCR-ABL1 ATP-competitive TKI is first-line or second-line therapeutic agent. In another specific embodiment, the first-generation BCR-ABL1 ATP-competitive TKI is imatinib. In another specific embodiment, the second-generation BCR-ABL1 ATP-competitive TKIs are selected from dasatinib, nilotinib, bosutinib, flumatinib or radotinib. In another specific embodiment, the first- and/or second-generation BCR-ABL1 ATP-competitive TKIs are selected from imatinib, dasatinib, nilotinib, bosutinib, flumatinib or radotinib. In another specific embodiment, the first- and/or second-generation BCR-ABL1 ATP-competitive TKIs are selected from imatinib, dasatinib or nilotinib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-AP. In another more specific embodiment, the CML is Ph+ CML-AP with T315I mutation. In another more specific embodiment, the CML is Ph+ and BCR-ABL1+ CML-AP with T315I mutation.

In another specific embodiment, the use and method are applied to adult subjects. In another specific embodi-

ment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg

QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in accelerated phase (CML-AP) that has previously received treatment with third or above generations of BCR-ABL1 TKIs and subsequently failed or was intolerant

In one embodiment, the present disclosure relates to the use and method of the compound of formula (I) or a pharmaceutically acceptable salt, crystal form, solvate, or hydrate thereof for treating CML in accelerated phase or its central nervous system leukemia recurrence, such as brain metastasis, wherein the CML has previously received treatment with third or above generations of BCR-ABL1 TKIs and subsequently failed or was intolerant. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with third or above generations of BCR-ABL1 TKIs and subsequently failed or was intolerant.

In another specific embodiment, the BCR-ABL1 TKIs are first-line, second-line, third-line, fourth-line, or fifth-line therapeutic agent. In another specific embodiment, the BCR-ABL1 TKIs are first-line, second-line, or third-line therapeutic agent. In another specific embodiment, the BCR-ABL1 TKIs are first-line or second-line therapeutic agents. In another specific embodiment, the BCR-ABL1 TKIs are first-line therapeutic agents. In another specific embodiment, the BCR-ABL1 TKIs are second-line therapeutic agents. In another more specific embodiment, the BCR-ABL1 TKIs include at least third-generation TKIs (e.g., ponatinib or olverembatinib). In another more specific embodiment, the BCR-ABL1 TKIs include at least third-generation TKIs (e.g., ponatinib or olverembatinib) and/or fourth-generation TKIs (e.g., asciminib or HS-10382). In another specific embodiment, the BCR-ABL1 TKIs is third-line therapeutic agent. In another specific embodiment, the BCR-ABL1 TKIs are selected from ponatinib, olverembatinib, PF-114, rebasinib, danusertib, tozasertib, AT9283, KW-2449, XL228, asciminib or HS-10382. In another specific embodiment, the BCR-ABL1 TKIs are selected from ponatinib, olverembatinib, PF-114, asciminib or HS-10382. In another specific embodiment, the BCR-ABL1 TKIs are selected from ponatinib, olverembatinib or PF-114. In another specific embodiment, the BCR-ABL1 TKIs are selected from ponatinib or olverembatinib. In another specific embodiment, the BCR-ABL1 TKIs are selected from ponatinib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-AP. In another more specific embodiment, the CML is Ph+ CML-AP with T315I mutation. In another more specific embodiment, the CML is Ph+ and BCR-ABL1+ CML-AP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about

240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or

hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in accelerated phase (CML-AP) that has previously received treatment with third-generation BCR-ABL1 TKIs and subsequently developed resistance or intolerance

In one embodiment, the present disclosure relates to the use and method of the compound of formula (I) or a pharmaceutically acceptable salt, crystal form, solvate, or hydrate thereof for treating CML in accelerated phase or its central nervous system leukemia recurrence, such as brain metastasis, wherein the CML has previously received treatment with third-generation BCR-ABL1 TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating CML in accelerated phase, wherein the CML has previously received treatment with third-generation BCR-ABL1 TKIs and subsequently developed resistance or intolerance.

In another specific embodiment, the definition of treatment resistance for CML-AP requires meeting at least one of the following criteria:

a. At 3 months after treatment initiation: Failure to achieve MaHR;
b. At any time after treatment initiation, loss of MaHR;
c. At any time after treatment initiation, development of a new BCR::ABL kinase domain mutation in the absence of MaHR.

In another specific embodiment, the definition of treatment intolerance for CML-AP requires meeting at least one of the following criteria:

a. Non-hematological intolerance: CML-AP subjects without MaHR who experience treatment-related grade 3 or 4 non-hematological toxicity; or those with persistent grade 2 toxicity that does not respond to optimal management, including dose adjustments (except when dose reduction is not performed to prioritize the subject's maximum benefit);
b. Hematological intolerance: CML-AP subjects without MaHR who experience treatment-related grade 4 hematological toxicity lasting more than 7 days; or those who experience recurrent grade 3 or 4 toxicity (ANC or platelets) after dose reduction to the minimum effective dose during treatment.

In another specific embodiment, the CML has also previously received treatment with first- and/or second-generation BCR-ABL1 TKI and subsequently developed resistance or intolerance. In another specific embodiment, the CML has previously received treatment with first- and/or second-generation BCR-ABL1 TKI before receiving third-generation BCR-ABL1 TKI therapy and subsequently developed resistance or intolerance. In another specific embodiment, the CML has not previously received treatment with first- and/or second-generation BCR-ABL1 TKI and developed resistance or intolerance. In another specific embodiment, the CML has also previously received treatment with fourth-generation BCR-ABL1 TKIs and subsequently developed resistance or intolerance. In another specific embodiment, the CML has not previously received treatment with fourth-generation BCR-ABL1 TKIs and subsequently developed resistance or intolerance. In another specific embodiment, the CML has also previously received treatment with first- and/or second-generation and/or fourth-generation BCR-ABL1 TKIs and subsequently developed resistance or intolerance.

In another specific embodiment, the first- or second-generation BCR-ABL1 TKIs are first-line or second-line therapeutic agents. In another more specific embodiment, the first- or second-generation BCR-ABL1 TKIs are first-line therapeutic agents. In another more specific embodiment, the first- or second-generation BCR-ABL1 TKIs are second-line therapeutic agents. In another more specific embodiment, the first- or second-generation BCR-ABL1 TKIs are second-line or higher-line therapeutic agents. In another more specific embodiment, the first-generation BCR-ABL1 TKI is imatinib. In another more specific embodiment, the second-generation BCR-ABL1 ATP-competitive TKIs are selected from dasatinib, nilotinib, bosutinib, flumatinib or radotinib. In another more specific embodiment, the first- and/or second-generation BCR-ABL1 TKIs are selected from imatinib, dasatinib, nilotinib, bosutinib, flumatinib or radotinib. In another more specific

embodiment, the first- and/or second-generation BCR-ABL1 ATP-competitive TKIs are selected from imatinib, dasatinib, nilotinib or flumatinib. In another specific embodiment, the third-generation BCR-ABL1 TKIs are ponatinib or olverembatinib. In another specific embodiment, the fourth-generation BCR-ABL1 TKIs is asciminib or HS-10382.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-AP. In another more specific embodiment, the CML is Ph+ CML-AP with T315I mutation. In another more specific embodiment, the CML is Ph+ and BCR-ABL1+ CML-AP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380

mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic Myeloid Leukemia in Blast Phase (CML-BP)

In one embodiment, the present disclosure relates to the use and method of a compound of formula (I) or a pharmaceutically acceptable salt, crystal form, solvate, or hydrate thereof for treating CML in blast phase or central nervous system leukemia relapse thereof, such as brain metastasis. In another embodiment, the present disclosure relates to the use and method of (R)-N-(4-(chlorodifluoromethoxy)phenyl)-6-(3-fluoropyrrolidin-1-yl)-5-(pyrazin-2-yl) nicotinamide (Compound A) or a pharmaceutically acceptable salt, crystal form, solvate, or hydrate thereof for treating CML in blast phase or central nervous system leukemia relapse thereof, such as brain metastasis.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-BP. In another more specific embodiment, the CML is Ph+ CML-BP with T315I mutation. In another more specific embodiment, the CML is Ph+ and BCR-ABL1+ CML-BP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the subject has previously received treatment with ≥1 different BCR-ABL1 TKIs and subsequently experienced disease progression, with treatment evaluation results being failure, warning, or intolerance. In another specific embodiment, the subject has previously received treatment with ≥2 different BCR-ABL1 TKIs and subsequently experienced disease progression, with treatment evaluation results being failure, warning, or intolerance. In another specific embodiment, the subject has previously received treatment with ≥3 different BCR-ABL1 TKIs and subsequently experienced disease progression, with treatment evaluation results being failure, warning, or intolerance. In another specific embodiment, the subject has previously received treatment with ≥4 different BCR-ABL1 TKIs and subsequently experienced disease progression, with treatment evaluation results being failure, warning, or intolerance. In another more specific embodiment, the BCR-ABL1 TKIs are first-line, second-line, third-line, fourth-line, or fifth-line therapeutic agent. In another more specific embodiment, the BCR-ABL1 TKIs are first-line, second-line, or third-line therapeutic agent. In another more specific embodiment, the BCR-ABL1 TKIs are first-line or second-line therapeutic agents. In another more specific embodiment, the BCR-ABL1 TKIs are first-line therapeutic agents. In another more specific embodiment, the BCR-ABL1 TKIs are second-line therapeutic agents. In another more specific embodiment, the BCR-ABL1 TKIs are second-line or higher-line therapeutic agents. In another more specific embodiment, the BCR-ABL1

TKIs include at least third-generation TKIs (e.g., ponatinib or olverembatinib). In another more specific embodiment, the BCR-ABL1 TKIs include at least third-generation TKIs (e.g., ponatinib or olverembatinib) and/or fourth-generation TKIs (e.g., asciminib or HS-10382). In another more specific embodiment, the BCR-ABL1 TKIs are selected from imatinib, dasatinib, nilotinib, bosutinib, flumatinib, ponatinib, olverembatinib(olverembatinib), radotinib, bafetinib(bafetinib), vodobatinib, CHMFL-ABL-039, CHMFL-ABL-053, CHMFL-ABL-KIT-155, PF-114, pacritinib(pacritinib), rebasinib, danusertib(danusertib), tozasertib(tozasertib), AT9283, KW-2449, XL228, asciminib(asciminib) or HS-10382. In another more specific embodiment, the BCR-ABL1 TKIs are selected from imatinib, dasatinib, nilotinib, bosutinib, flumatinib, ponatinib, olverembatinib, radotinib or asciminib. In another more specific embodiment, the BCR-ABL1 TKIs are selected from imatinib, dasatinib, nilotinib, ponatinib, olverembatinib or asciminib. In another more specific embodiment, the BCR-ABL1 TKIs are selected from imatinib, dasatinib, nilotinib, bosutinib, flumatinib or radotinib. In another more specific embodiment, the BCR-ABL1 TKIs are selected from ponatinib, olverembatinib, asciminib or HS-10382. In another more specific embodiment, the BCR-ABL1 TKIs are selected from imatinib, dasatinib or nilotinib. In another more specific embodiment, the BCR-ABL1 TKIs are selected from ponatinib or olverembatinib. In another more specific embodiment, the BCR-ABL1 TKIs are selected from asciminib or HS-10382. In another more specific embodiment, the BCR-ABL1 TKIs are selected from asciminib.

In another specific embodiment, the subject has previously received treatment with ≥1 different BCR-ABL1 TKIs and subsequently experienced relapse or became refractory. In another specific embodiment, the subject has previously received treatment with ≥2 different BCR-ABL1 TKIs and subsequently experienced relapse or became refractory. In another specific embodiment, the subject has previously received treatment with ≥3 different BCR-ABL1 TKIs and subsequently experienced relapse or became refractory. In another specific embodiment, the subject has previously received treatment with ≥4 different BCR-ABL1 TKIs and subsequently experienced relapse or became refractory. In another more specific embodiment, the BCR-ABL1 TKIs are first-line, second-line, third-line, fourth-line, or fifth-line therapeutic agent. In another more specific embodiment, the BCR-ABL1 TKIs are first-line, second-line, or third-line therapeutic agent. In another more specific embodiment, the BCR-ABL1 TKIs are first-line or second-line therapeutic agents. In another more specific embodiment, the BCR-ABL1 TKIs are first-line therapeutic agents. In another more specific embodiment, the BCR-ABL1 TKIs are second-line therapeutic agents. In another more specific embodiment, the BCR-ABL1 TKIs are second-line or higher-line therapeutic agents. In another more specific embodiment, the BCR-ABL1 TKIs include at least third-generation TKIs (e.g., ponatinib or olverembatinib(olverembatinib)). In another more specific embodiment, the BCR-ABL1 TKIs include at least third-generation TKIs (e.g., ponatinib or olverembatinib) and/or fourth-generation TKIs(e.g., asciminib(asciminib) or HS-10382). In another more specific embodiment, the BCR-ABL1 TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382. In another more specific embodiment, the BCR-ABL1 TKIs are selected from imatinib, dasatinib, nilotinib, bosutinib, flumatinib, ponatinib, olverembatinib, radotinib or asciminib. In another more specific embodiment, the BCR-ABL1 TKIs are selected from imatinib, dasatinib, nilotinib, ponatinib, olverembatinib or asciminib. In another more specific embodiment, the BCR-ABL1 TKIs are selected from imatinib, dasatinib, nilotinib, bosutinib, flumatinib or radotinib. In another more specific embodiment, the BCR-ABL1 TKIs are selected from ponatinib, olverembatinib, asciminib or HS-10382. In another more specific embodiment, the BCR-ABL1 TKIs are selected from imatinib, dasatinib or nilotinib. In another more specific embodiment, the BCR-ABL1 TKIs are selected from ponatinib or olverembatinib. In another more specific embodiment, the BCR-ABL1 TKIs are selected from asciminib or HS-10382. In another more specific embodiment, the BCR-ABL1 TKIs are selected from asciminib.

In another specific embodiment, the subject has previously received treatment with ≥1 different BCR-ABL1 ATP-competitive TKIs and subsequently experienced disease progression, with treatment evaluation results being failure, warning, or intolerance. In another specific embodiment, the subject has previously received treatment with ≥2 different BCR-ABL1 ATP-competitive TKIs and subsequently experienced disease progression, with treatment evaluation results being failure, warning, or intolerance. In another specific embodiment, the subject has previously received treatment with ≥3 different BCR-ABL1 ATP-competitive TKIs and subsequently experienced disease progression, with treatment evaluation results being failure, warning, or intolerance. In another specific embodiment, the subject has previously received treatment with ≥4 different BCR-ABL1 ATP-competitive TKIs and subsequently experienced disease progression, with treatment evaluation results being failure, warning, or intolerance. In another more specific embodiment, the BCR-ABL1 ATP-competitive TKIs are a first-line, second-line, third-line, fourth-line, or fifth-line therapeutic agent. In another more specific embodiment, the BCR-ABL1 ATP-competitive TKIs are a first-line, second-line, or third-line therapeutic agent. In another more specific embodiment, the BCR-ABL1 ATP-competitive TKIs are first-line or second-line therapeutic agents. In another more specific embodiment, the BCR-ABL1 ATP-competitive TKIs are first-line therapeutic agents. In another more specific embodiment, the BCR-ABL1 ATP-competitive TKIs are second-line therapeutic agents. In another more specific embodiment, the BCR-ABL1 ATP-competitive TKIs are second-line or higher-line therapeutic agents. In another more specific embodiment, the BCR-ABL1 ATP-competitive TKIs include at least third-generation TKIs (e.g., ponatinib or olverembatinib). In another more specific embodiment, the BCR-ABL1 ATP-

competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228. In another more specific embodiment, the BCR-ABL1 ATP-competitive TKIs are selected from imatinib, dasatinib, nilotinib, bosutinib, flumatinib, ponatinib, olverembatinib or radotinib. In another more specific embodiment, the BCR-ABL1 ATP-competitive TKIs are selected from imatinib, dasatinib, nilotinib, ponatinib or olverembatinib. In another more specific embodiment, the BCR-ABL1 ATP-competitive TKIs are selected from imatinib, dasatinib, nilotinib, bosutinib, flumatinib or radotinib. In another more specific embodiment, the BCR-ABL1 ATP-competitive TKIs are selected from imatinib, dasatinib or nilotinib. In another more specific embodiment, the BCR-ABL1 ATP-competitive TKIs are selected from ponatinib or olverembatinib.

In another specific embodiment, the subject has previously received treatment with ≥1 different BCR-ABL1 ATP-competitive TKIs and subsequently experienced relapse or became refractory. In another specific embodiment, the subject has previously received treatment with ≥2 different BCR-ABL1 ATP-competitive TKIs and subsequently experienced relapse or became refractory. In another specific embodiment, the subject has previously received treatment with ≥3 different BCR-ABL1 ATP-competitive TKIs and subsequently experienced relapse or became refractory. In another specific embodiment, the subject has previously received treatment with ≥4 different BCR-ABL1 ATP-competitive TKIs and subsequently experienced relapse or became refractory. In another more specific embodiment, the BCR-ABL1 ATP-competitive TKIs are a first-line, second-line, third-line, fourth-line, or fifth-line therapeutic agent. In another more specific embodiment, the BCR-ABL1 ATP-competitive TKIs are a first-line, second-line, or third-line therapeutic agent. In another more specific embodiment, the BCR-ABL1 ATP-competitive TKIs are first-line or second-line therapeutic agents. In another more specific embodiment, the BCR-ABL1 ATP-competitive TKIs are first-line therapeutic agents. In another more specific embodiment, the BCR-ABL1 ATP-competitive TKIs are second-line therapeutic agents. In another more specific embodiment, the BCR-ABL1 ATP-competitive TKIs are second-line or higher-line therapeutic agents. In another more specific embodiment, the BCR-ABL1 ATP-competitive TKIs include at least third-generation TKIs (e.g., ponatinib or olverembatinib). In another more specific embodiment, the BCR-ABL1 ATP-competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228. In another more specific embodiment, the BCR-ABL1 ATP-competitive TKIs are selected from imatinib, dasatinib, nilotinib, bosutinib, flumatinib, ponatinib, olverembatinib or radotinib. In another more specific embodiment, the BCR-ABL1 ATP-competitive TKIs are selected from imatinib, dasatinib, nilotinib, ponatinib or olverembatinib. In another more specific embodiment, the BCR-ABL1 ATP-competitive TKIs are selected from imatinib, dasatinib, nilotinib, bosutinib, flumatinib or radotinib. In another more specific embodiment, the BCR-ABL1 ATP-competitive TKIs are selected from imatinib, dasatinib or nilotinib. In another more specific embodiment, the BCR-ABL1 ATP-competitive TKIs are selected from ponatinib or olverembatinib.

In another specific embodiment, the subject has previously received treatment with ≥1 different first- and/or second-generation BCR-ABL1 ATP-competitive TKIs and subsequently developed resistance or intolerance. In another specific embodiment, the subject has previously received treatment with ≥2 different first- and/or second-generation BCR-ABL1 ATP-competitive TKIs and subsequently developed resistance or intolerance. In another specific embodiment, the subject has previously received treatment with ≥3 different first- and/or second-generation BCR-ABL1 ATP-competitive TKIs and subsequently developed resistance or intolerance. In another specific embodiment, the subject has previously received treatment with ≥4 different first- and/or second-generation BCR-ABL1 ATP-competitive TKIs and subsequently developed resistance or intolerance. In another more specific embodiment, the resistance arises from BCR-ABL1 kinase domain point mutation(s), such as one or more of T315I/A, Y253F/H, E255V/K, M351T, G250E, F359V/I/C, H396P/R, M244V, E355G, F317L/V/I/C, F311L, M237I, Q252H/R, D276G, L248V, V379I, L384M, L387M, E279K, F486S, E459K, P223S, I502L, or V299L. In another more specific embodiment, the resistance involves BCR-ABL1 kinase domain point mutations, selected from one or more of T315I, Y253H, E255V/K, M351T, G250E, F359V/, H396P, M244V, E355G, F317L, Q252H, E459K, P223S, I502L, or V299L mutations. In another more specific embodiment, the resistance is T315I. In another more specific embodiment, the first- or second-generation BCR-ABL1 ATP-competitive TKIs are a first-line, second-line, third-line, fourth-line, or fifth-line therapeutic agent. In another more specific embodiment, the first- or second-generation BCR-ABL1 ATP-competitive TKIs are a first-line, second-line, or third-line therapeutic agent. In another more specific embodiment, the first- or second-generation BCR-ABL1 ATP-competitive TKIs are first-line or second-line therapeutic agents. In another more specific embodiment, the first- or second-generation BCR-ABL1 ATP-competitive TKIs are first-line therapeutic agents. In another more specific embodiment, the first- or second-generation BCR-ABL1 ATP-competitive TKIs are second-line therapeutic agents. In another more specific embodiment, the first- or second-generation BCR-ABL1 ATP-competitive TKIs are second-line or higher-line therapeutic agents. In another more specific embodiment, the first-generation BCR-ABL1 ATP-competitive TKI is imatinib. In another more specific embodiment, the second-generation BCR-ABL1 ATP-competitive TKIs are selected from dasatinib, nilotinib, bosutinib, flumatinib or radotinib. In another more specific embodiment, the first- and/or second-generation BCR-ABL1 ATP-competitive TKIs are selected from imatinib, dasatinib, nilotinib, bosutinib, flumatinib or radotinib. In another more specific embodiment, the

first- and/or second-generation BCR-ABL1 ATP-competitive TKIs are selected from imatinib, dasatinib or nilotinib.

In another specific embodiment, the subject has previously received treatment with ≥1 different first- and/or second-generation BCR-ABL1 ATP-competitive TKIs and subsequently experienced relapse or became refractory. In another specific embodiment, the subject has previously received treatment with ≥2 different first- and/or second-generation BCR-ABL1 ATP-competitive TKIs and subsequently experienced relapse or became refractory. In another specific embodiment, the subject has previously received treatment with ≥3 different first-and/or second-generation BCR-ABL1 ATP-competitive TKIs and subsequently experienced relapse or became refractory. In another specific embodiment, the subject has previously received treatment with ≥4 different first- and/or second-generation BCR-ABL1 ATP-competitive TKIs and subsequently experienced relapse or became refractory. In another more specific embodiment, the first- or second-generation BCR-ABL1 ATP-competitive TKIs are a first-line, second-line, third-line, fourth-line, or fifth-line therapeutic agent. In another more specific embodiment, the first- or second-generation BCR-ABL1 ATP-competitive TKIs are a first-line, second-line, or third-line therapeutic agent. In another more specific embodiment, the first- or second-generation BCR-ABL1 ATP-competitive TKIs are first-line or second-line therapeutic agents. In another more specific embodiment, the first- or second-generation BCR-ABL1 ATP-competitive TKIs are first-line therapeutic agents. In another more specific embodiment, the first- or second-generation BCR-ABL1 ATP-competitive TKIs are second-line therapeutic agents. In another more specific embodiment, the first- or second-generation BCR-ABL1 ATP-competitive TKIs are second-line or higher-line therapeutic agents. In another more specific embodiment, the first-generation BCR-ABL1 ATP-competitive TKI is imatinib. In another more specific embodiment, the second-generation BCR-ABL1 ATP-competitive TKIs are selected from dasatinib, nilotinib, bosutinib, flumatinib or radotinib. In another more specific embodiment, the first- and/or second-generation BCR-ABL1 ATP-competitive TKIs are selected from imatinib, dasatinib, nilotinib, bosutinib, flumatinib or radotinib. In another more specific embodiment, the first- and/or second-generation BCR-ABL1 ATP-competitive TKIs are selected from imatinib, dasatinib or nilotinib.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I)

or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the daily effective amount of the compound of formula (I) or a pharmaceutically acceptable salt, crystal form, solvate, or hydrate thereof (alternatively compound A) is about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in blast phase (CML-BP) that has previously received treatment with ≥1 different BCR-ABL1 TKIs and subsequently experienced disease progression, with treatment evaluation results being failure, warning, or intolerance

In one embodiment, the present disclosure relates to the use and method of a compound of formula (I) or a pharmaceutically acceptable salt, crystal form, solvate, or hydrate thereof for treating CML in blast phase or central nervous system leukemia relapse thereof, such as brain metastasis, wherein the CML has previously received treatment with ≥1 different BCR-ABL1 TKIs and subsequently experienced disease progression, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥1 different BCR-ABL1 TKIs and subsequently experienced disease progression, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥2 different BCR-ABL1 TKIs and subsequently experienced disease progression, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for

treating CML in blast phase, wherein the CML has previously received treatment with ≥2 different BCR-ABL1 TKIs and subsequently experienced disease progression, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥3 different BCR-ABL1 TKIs and subsequently experienced disease progression, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥3 different BCR-ABL1 TKIs and subsequently experienced disease progression, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥4 different BCR-ABL1 TKIs and subsequently experienced disease progression, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥4 different BCR-ABL1 TKIs and subsequently experienced disease progression, with treatment evaluation results being failure, warning, or intolerance.

In one specific embodiment, the BCR-ABL1 TKIs are first-line, second-line, third-line, fourth-line, or fifth-line therapeutic agent. In another specific embodiment, the BCR-ABL1 TKIs are first-line, second-line, or third-line therapeutic agent. In another specific embodiment, the BCR-ABL1 TKIs are first-line or second-line therapeutic agents. In another specific embodiment, the BCR-ABL1 TKIs are first-line therapeutic agents. In another specific embodiment, the BCR-ABL1 TKIs are second-line therapeutic agents. In another specific embodiment, the BCR-ABL1 TKIs are second-line or higher-line therapeutic agents. In another specific embodiment, the BCR-ABL1 TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382. In another specific embodiment, the BCR-ABL1 TKIs are selected from imatinib, dasatinib, nilotinib, bosutinib, flumatinib, ponatinib, olverembatinib, radotinib or asciminib. In another specific embodiment, the BCR-ABL1 TKIs are selected from imatinib, dasatinib, nilotinib, ponatinib, olverembatinib or asciminib. In another specific embodiment, the BCR-ABL1 TKIs are selected from imatinib, dasatinib, nilotinib, bosutinib, flumatinib or radotinib. In another more specific embodiment, the BCR-ABL1 TKIs are selected from ponatinib, olverembatinib, asciminib or HS-10382. In another specific embodiment, the BCR-ABL1 TKIs are selected from ponatinib, olverembatinib, asciminib. In another specific embodiment, the BCR-ABL1 TKIs are selected from imatinib, dasatinib or nilotinib. In another specific embodiment, the BCR-ABL1 TKIs are selected from ponatinib or olverembatinib. In another specific embodiment, the BCR-ABL1 TKIs are selected from asciminib or HS-10382. In another specific embodiment, the BCR-ABL1 TKIs are selected from asciminib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-BP. In another more specific embodiment, the CML is Ph+ CML-BP with T315I mutation. In another more specific embodiment, the CML is Ph+ and BCR-ABL1+ CML-BP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the

dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is

administered until disease progression, intolerable toxicity, or death. In another specific embodiment, compound A or a pharmaceutically acceptable salt thereof is administered until disease progression, intolerable toxicity, or death.

Chronic myeloid leukemia in blast phase (CML-BP) that has previously received treatment with ≥1 different BCR-ABL1 ATP-competitive TKIs and subsequently experienced disease progression, with treatment evaluation results being failure, warning, or intolerance

In one embodiment, the present disclosure relates to the use and method of a compound of formula (I) or a pharmaceutically acceptable salt, crystal form, solvate, or hydrate thereof for treating CML in blast phase or central nervous system leukemia relapse thereof, such as brain metastasis, wherein the CML has previously received treatment with ≥1 different BCR-ABL1 ATP-competitive TKIs and subsequently experienced disease progression, with treatment evaluation results being failure, warning, or intolerance. In another embodiment, the present disclosure relates to the method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥1 different BCR-ABL1 ATP-competitive TKIs and subsequently experienced disease progression, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥2 different BCR-ABL1 ATP-competitive TKIs and subsequently experienced disease progression, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥2 different BCR-ABL1 ATP-competitive TKIs and subsequently experienced disease progression, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥3 different BCR-ABL1 ATP-competitive TKIs and subsequently experienced disease progression, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥3 different BCR-ABL1 ATP-competitive TKIs and subsequently experienced disease progression, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥4 different BCR-ABL1 ATP-competitive TKIs and subsequently experienced disease progression, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥4 different BCR-ABL1 ATP-competitive TKIs and subsequently experienced disease progression, with treatment evaluation results being failure, warning, or intolerance.

In one specific embodiment, the BCR-ABL1 ATP-competitive TKIs are a first-line, second-line, third-line, fourth-line, or fifth-line therapeutic agent. In another specific embodiment, the BCR-ABL1 ATP-competitive TKIs are a first-line, second-line, or third-line therapeutic agent. In another specific embodiment, the BCR-ABL1 ATP-competitive TKIs are first-line or second-line therapeutic agents. In another specific embodiment, the BCR-ABL1 ATP-competitive TKIs are first-line therapeutic agents. In another specific embodiment, the BCR-ABL1 ATP-competitive TKIs are second-line therapeutic agents. In another specific embodiment, the BCR-ABL1 ATP-competitive TKIs are second-line or higher-line therapeutic agents. In another specific embodiment, the BCR-ABL1 ATP-competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, HS-10382, rebasinib, danusertib, tozasertib, AT9283, KW-2449 or XL228. In another specific embodiment, the BCR-ABL1 ATP-competitive TKIs are selected from imatinib, dasatinib, nilotinib, bosutinib, flumatinib, ponatinib, olverembatinib or radotinib. In another specific embodiment, the BCR-ABL1 ATP-competitive TKIs are selected from imatinib, dasatinib, nilotinib, ponatinib or olverembatinib. In another more specific embodiment, the BCR-ABL1 ATP-competitive TKIs are selected from imatinib, dasatinib, nilotinib, bosutinib, flumatinib or radotinib. In another specific embodiment, the BCR-ABL1 ATP-competitive TKIs are selected from imatinib, dasatinib or nilotinib. In another specific embodiment, the BCR-ABL1 ATP-competitive TKIs are selected from ponatinib or

olverembatinib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-BP. In another more specific embodiment, the CML is Ph+ CML-BP with T315I mutation. In another more specific embodiment, the CML is Ph+ and BCR-ABL1+ CML-BP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment,

the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in blast phase (CML-BP) that has previously received treatment with ≥1 different first- and/or second-generation BCR-ABL1 ATP-competitive TKIs and subsequently developed resistance or intolerance

In one embodiment, the present disclosure relates to the use and method of a compound of formula (I) or a pharmaceutically acceptable salt, crystal form, solvate, or hydrate thereof for treating CML in blast phase or central nervous system leukemia relapse thereof, such as brain metastasis, wherein the CML has previously received treatment with ≥1 different first- and/or second-generation BCR-ABL1 ATP-competitive TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥1 different first- and/or second-generation BCR-ABL1 ATP-competitive TKIs and subsequently developed resistance or intolerance.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥2 different first- and/or second-generation BCR-ABL1 ATP-competitive TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥2 different first- and/or second-generation BCR-ABL1 ATP-competitive TKIs and subsequently developed resistance or intolerance.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥3 different first- and/or second-generation BCR-ABL1 ATP-competitive TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥3 different first- and/or second-generation BCR-ABL1 ATP-competitive TKIs and subsequently developed resistance or intolerance.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥4 different first- and/or second-generation BCR-ABL1 ATP-competitive TKIs and subsequently developed resistance or intolerance. In another embodiment, the present disclosure relates to the use

and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥4 different first- and/or second-generation BCR-ABL1 ATP-competitive TKIs and subsequently developed resistance or intolerance.

In one specific embodiment, the resistance arises from BCR-ABL1 kinase domain point mutation(s), such as one or more of T315I/A, Y253F/H, E255V/K, M351T, G250E, F359V/I/C, H396P/R, M244V, E355G, F317L/V/I/C, F311L, M237I, Q252H/R, D276G, L248V, V379I, L384M, L387M, E279K, F486S, E459K, P223S, I502L, or V299L. In another specific embodiment, the resistance involves BCR-ABL1 kinase domain point mutations, selected from one or more of T315I, Y253H, E255V/K, M351T, G250E, F359V/, H396P, M244V, E355G, F317L, Q252H, E459K, P223S, I502L, or V299L mutations. In another specific embodiment, the resistance is T315I.

In another specific embodiment, the first- or second-generation BCR-ABL1 ATP-competitive TKIs are a first-line, second-line, third-line, fourth-line, or fifth-line therapeutic agent. In another specific embodiment, the first- or second-generation BCR-ABL1 ATP-competitive TKIs are a first-line, second-line, or third-line therapeutic agent. In another specific embodiment, the first- or second-generation BCR-ABL1 ATP-competitive TKIs are first-line or second-line therapeutic agents. In another specific embodiment, the first- or second-generation BCR-ABL1 ATP-competitive TKIs are first-line therapeutic agents. In another specific embodiment, the first- or second-generation BCR-ABL1 ATP-competitive TKIs are second-line therapeutic agents. In another specific embodiment, the first- or second-generation BCR-ABL1 ATP-competitive TKIs are second-line or higher-line therapeutic agents. In another specific embodiment, the first-generation BCR-ABL1 ATP-competitive TKI is imatinib. In another specific embodiment, the second-generation BCR-ABL1 ATP-competitive TKIs are selected from dasatinib, nilotinib, bosutinib, flumatinib or radotinib. In another specific embodiment, the first- and/or second-generation BCR-ABL1 ATP-competitive TKIs are selected from imatinib, dasatinib, nilotinib, bosutinib, flumatinib or radotinib. In another specific embodiment, the first- and/or second-generation BCR-ABL1 ATP-competitive TKIs are selected from imatinib, dasatinib or nilotinib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-BP. In another more specific embodiment, the CML is Ph+ CML-BP with T315I mutation. In another more specific embodiment, the CML is Ph+ and BCR-ABL1+ CML-BP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160

mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in blast phase (CML-BP) that has previously received treatment with ≥1 different BCR-ABL1 TKIs and subsequently experienced relapse or became refractory

In one embodiment, the present disclosure relates to the use and method of a compound of formula (I) or a pharmaceutically acceptable salt, crystal form, solvate, or hydrate thereof for treating CML in blast phase or central nervous system leukemia relapse thereof, such as brain metastasis, wherein the CML has previously received treatment with ≥1 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥1 different BCR-ABL1

TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥2 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥2 different BCR-ABL1 TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥3 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥3 different BCR-ABL1 TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥4 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥4 different BCR-ABL1 TKIs and subsequently experienced relapse or became refractory.

In one specific embodiment, the BCR-ABL1 TKIs are first-line, second-line, third-line, fourth-line, or fifth-line therapeutic agent. In another specific embodiment, the BCR-ABL1 TKIs are first-line, second-line, or third-line therapeutic agent. In another specific embodiment, the BCR-ABL1 TKIs are first-line or second-line therapeutic agents. In another specific embodiment, the BCR-ABL1 TKIs are first-line therapeutic agents. In another specific embodiment, the BCR-ABL1 TKIs are second-line therapeutic agents. In another specific embodiment, the BCR-ABL1 TKIs are second-line or higher-line therapeutic agents. In another specific embodiment, the BCR-ABL1 TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382. In another specific embodiment, the BCR-ABL1 TKIs are selected from imatinib, dasatinib, nilotinib, bosutinib, flumatinib, ponatinib, olverembatinib, radotinib or asciminib. In another specific embodiment, the BCR-ABL1 TKIs are selected from imatinib, dasatinib, nilotinib, ponatinib, olverembatinib or asciminib. In another specific embodiment, the BCR-ABL1 TKIs are selected from imatinib, dasatinib, nilotinib, bosutinib, flumatinib or radotinib. In another specific embodiment, the BCR-ABL1 TKIs are selected from ponatinib, olverembatinib, asciminib or HS-10382. In another specific embodiment, the BCR-ABL1 TKIs are selected from imatinib, dasatinib or nilotinib. In another specific embodiment, the BCR-ABL1 TKIs are selected from ponatinib or olverembatinib. In another specific embodiment, the BCR-ABL1 TKIs are selected from asciminib or HS-10382. In another specific embodiment, the BCR-ABL1 TKIs are selected from asciminib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-BP. In another more specific embodiment, the CML is Ph+ CML-BP with T315I mutation. In another more specific embodiment, the CML is Ph+ and BCR-ABL1+ CML-BP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120

mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg

QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in blast phase (CML-BP) that has previously received treatment with ≥1 different BCR-ABL1 ATP-competitive TKIs and subsequently experienced relapse or became refractory

In one embodiment, the present disclosure relates to the use and method of a compound of formula (I) or a pharmaceutically acceptable salt, crystal form, solvate, or hydrate thereof for treating CML in blast phase or central nervous system leukemia relapse thereof, such as brain metastasis, wherein the CML has previously received treatment with ≥1 different BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥1 different BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥2 different BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥2 different BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥3 different BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥3 different BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥4 different BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥4 different BCR-ABL1 ATP-competitive TKIs and subsequently experienced relapse or became refractory.

In one specific embodiment, the BCR-ABL1 ATP-competitive TKIs are a first-line, second-line, third-line, fourth-line, or fifth-line therapeutic agent. In one specific embodiment, the BCR-ABL1 ATP-competitive TKIs are a first-line, second-line, or third-line therapeutic agent. In another specific embodiment, the BCR-ABL1 ATP-competitive TKIs are first-line or second-line therapeutic agents. In another specific embodiment, the BCR-ABL1 ATP-competitive TKIs are first-line therapeutic agents. In another specific embodiment, the BCR-ABL1 ATP-competitive TKIs are second-line therapeutic agents. In another specific embodiment, the BCR-ABL1 ATP-competitive TKIs are second-line or higher-line therapeutic agents. In another specific embodiment, the BCR-ABL1 ATP-competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, HS-10382, rebasinib, danusertib, tozasertib, AT9283, KW-2449 or XL228. In another specific embodiment, the BCR-ABL1 ATP-competitive TKIs are selected from imatinib, dasatinib, nilotinib, bosutinib, flumatinib, ponatinib, olverembatinib or radotinib. In another specific embodiment, the BCR-ABL1 ATP-competitive TKIs are selected from imatinib, dasatinib, nilotinib, ponatinib or olverembatinib. In another specific embodiment, the BCR-ABL1 ATP-competitive TKIs are selected from imatinib, dasatinib, nilotinib, bosutinib, flumatinib or radotinib. In another specific embodiment, the BCR-ABL1 ATP-competitive TKIs are selected from imatinib, dasatinib or nilotinib. In another specific embodiment, the BCR-ABL1 ATP-competitive TKIs are selected from ponatinib or olverembatinib.

In another specific embodiment, the CML is Ph+ CML. In another specific embodiment, the CML is BCR-ABL1+

CML. In another specific embodiment, the CML is CML with T315I mutation. In another specific embodiment, the CML is CML without T315I mutation. In another more specific embodiment, the CML is Ph+ CML-BP. In another more specific embodiment, the CML is Ph+ CML-BP with T315I mutation. In another more specific embodiment, the CML is Ph+ and BCR-ABL1+ CML-BP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a

pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in blast phase (CML-BP) that has previously received treatment with ≥1 different first- and/or second-generation BCR-ABL1 ATP-competitive TKIs and subsequently experienced relapse or became refractory

In one embodiment, the present disclosure relates to the use and method of a compound of formula (I) or a pharmaceutically acceptable salt, crystal form, solvate, or hydrate thereof for treating CML in blast phase or central nervous system leukemia relapse thereof, such as brain metastasis, wherein the CML has previously received treatment with ≥1 different first- and/or second-generation BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥1 different first- and/or second-generation BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥2 different first- and/or second-generation BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥2 different first- and/or second-generation BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥3 different first- and/or second-generation BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with ≥3 different first- and/or second-generation BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating CML in blast phase, wherein the CML has previously received treatment with ≥4 different first- and/or second-generation BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast

phase, wherein the CML has previously received treatment with ≥4 different first- and/or second-generation BCR-ABL1 ATP-competitive TKIs and subsequently experienced relapse or became refractory.

In one specific embodiment, the first- or second-generation BCR-ABL1 ATP-competitive TKIs are a first-line, second-line, third-line, fourth-line, or fifth-line therapeutic agent. In another more specific embodiment, the first- or second-generation BCR-ABL1 ATP-competitive TKIs are a first-line, second-line, or third-line therapeutic agent. In another specific embodiment, the first- or second-generation BCR-ABL1 ATP-competitive TKIs are first-line or second-line therapeutic agents. In another specific embodiment, the first- or second-generation BCR-ABL1 ATP-competitive TKIs are first-line therapeutic agents. In another specific embodiment, the first- or second-generation BCR-ABL1 ATP-competitive TKIs are second-line therapeutic agents. In another specific embodiment, the first- or second-generation BCR-ABL1 ATP-competitive TKIs are second-line or higher-line therapeutic agents. In another specific embodiment, the first-generation BCR-ABL1 ATP-competitive TKI is imatinib. In another specific embodiment, the second-generation BCR-ABL1 ATP-competitive TKIs are selected from dasatinib, nilotinib, bosutinib, flumatinib or radotinib. In another specific embodiment, the first- and/or second-generation BCR-ABL1 ATP-competitive TKIs are selected from imatinib, dasatinib, nilotinib, bosutinib, flumatinib or radotinib. In another specific embodiment, the first- and/or second-generation BCR-ABL1 ATP-competitive TKIs are selected from imatinib, dasatinib or nilotinib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-BP. In another more specific embodiment, the CML is Ph+ CML-BP with T315I mutation. In another more specific embodiment, the CML is Ph+ and BCR-ABL1+ CML-BP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof

(alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Chronic myeloid leukemia in blast phase (CML-BP) that has previously received treatment with third or above generations of BCR-ABL1 TKIs and subsequently failed or was intolerant

In one embodiment, the present disclosure relates to the use and method of a compound of formula (I) or a pharmaceutically acceptable salt, crystal form, solvate, or hydrate thereof for treating CML in blast phase or central nervous system leukemia relapse thereof, such as brain metastasis, wherein the CML has previously received treatment with third or above generations of BCR-ABL1 TKIs and subsequently failed or was intolerant. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (compound A) or a pharmaceutically acceptable salt, crystal form, solvate, or hydrate thereof for treating CML in blast phase, wherein the CML has previously received treatment with third or above generations of BCR-ABL1 TKIs and subsequently failed or was intolerant.

In one specific embodiment, the BCR-ABL1 TKIs are first-line, second-line, third-line, fourth-line, or fifth-line therapeutic agent. In another specific embodiment, the BCR-ABL1 TKIs are first-line, second-line, or third-line therapeutic agent. In another specific embodiment, the BCR-ABL1 TKIs are first-line or second-line therapeutic agents. In another specific embodiment, the BCR-ABL1 TKIs are first-line or above therapeutic agents. In another specific embodiment, the BCR-ABL1 TKIs are second-line or above therapeutic agents. In another specific embodiment, the BCR-ABL1 TKIs are third-line or above therapeutic agents. In another specific embodiment, the BCR-ABL1 TKIs are selected from ponatinib, olverembatinib, PF-114, rebasinib, danusertib, tozasertib, AT9283, KW-2449, XL228, asciminib or HS-10382. In another

specific embodiment, the BCR-ABL1 TKIs are selected from ponatinib, olverembatinib, PF-114, asciminib or HS-10382. In another specific embodiment, the BCR-ABL1 TKIs are selected from ponatinib, olverembatinib or PF-114. In another specific embodiment, the BCR-ABL1 TKIs are selected from ponatinib or olverembatinib. In another specific embodiment, the BCR-ABL1 TKIs are selected from ponatinib.

In another specific embodiment, the CML is Ph+ CML. In another specific embodiment, the CML is BCR-ABL1+ CML. In another specific embodiment, the CML is CML with T315I mutation. In another specific embodiment, the CML is CML without T315I mutation. In another more specific embodiment, the CML is Ph+ CML-BP. In another more specific embodiment, the CML is Ph+ CML-BP with T315I mutation. In another more specific embodiment, the CML is Ph+ and BCR-ABL1+ CML-BP with T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I)

or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Philadelphia chromosome-positive acute lymphoblastic leukemia (Ph+ ALL)

In one embodiment, the present disclosure relates to the use and method of the compound of formula (I) or a pharmaceutically acceptable salt, crystal form, solvate, or hydrate thereof for treating Ph+ ALL or central nervous system leukemia relapse thereof, such as brain metastasis. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL.

In another specific embodiment, the Ph+ ALL has BCR-ABL1+. In another specific embodiment, the Ph+ ALL is Ph+ ALL with T315I mutation. In another specific embodiment, the Ph+ ALL is Ph+ ALL without T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the Ph+ ALL has previously received treatment with ≥1 different BCR-ABL1 TKIs and subsequently experienced disease progression, with treatment evaluation results being failure, warning, or intolerance. In another specific embodiment, the Ph+ ALL has previously received treatment with ≥2 different BCR-ABL1 TKIs and subsequently experienced disease progression, with treatment evaluation results being failure, warning, or intolerance. In another specific embodiment, the Ph+ ALL has previously received treatment with ≥3 different BCR-ABL1 TKIs and subsequently experienced disease progression, with treatment evaluation results being failure, warning, or intolerance. In another specific embodiment, the Ph+ ALL has previously received treatment with ≥4 different BCR-ABL1 TKIs and subsequently experienced disease progression, with treatment evaluation results being failure, warning, or intolerance. In another more specific embodiment, the BCR-ABL1 TKIs are first-line, second-line, third-line, fourth-line, or fifth-line therapeutic agent. In another more specific embodiment, the BCR-ABL1 TKIs are first-line, second-line, or third-line therapeutic agent. In another more specific embodiment, the BCR-ABL1 TKIs are first-line or second-line therapeutic agents. In another more specific embodiment, the BCR-ABL1 TKIs are first-line therapeutic agents. In another more specific embodiment, the BCR-ABL1 TKIs are second-line therapeutic agents. In another more specific embodiment, the BCR-ABL1 TKIs are second-line or higher-line therapeutic agents. In another more specific embodiment, the BCR-ABL1 TKIs include at least third-generation TKIs (e.g., ponatinib or olverembatinib(olverembatinib)). In another more specific embodiment, the BCR-ABL1 TKIs include at least third-generation TKIs (e.g., ponatinib or olverembatinib) and/or fourth-generation TKIs(e.g., asciminib(asciminib) or HS-10382). In another more specific embodiment, the BCR-ABL1 TKIs are

selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382. In another more specific embodiment, the BCR-ABL1 TKIs are selected from imatinib, dasatinib, nilotinib, bosutinib, flumatinib, ponatinib, olverembatinib, radotinib or asciminib. In another more specific embodiment, the BCR-ABL1 TKIs are selected from imatinib, dasatinib, nilotinib, ponatinib, olverembatinib or asciminib. In another more specific embodiment, the BCR-ABL1 TKIs are selected from imatinib, dasatinib, nilotinib, bosutinib, flumatinib or radotinib. In another more specific embodiment, the BCR-ABL1 TKIs are selected from ponatinib, olverembatinib, asciminib or HS-10382. In another more specific embodiment, the BCR-ABL1 TKIs are selected from imatinib, dasatinib or nilotinib. In another more specific embodiment, the BCR-ABL1 TKIs are selected from ponatinib or olverembatinib. In another more specific embodiment, the BCR-ABL1 TKIs are selected from asciminib or HS-10382. In another more specific embodiment, the BCR-ABL1 TKIs are selected from asciminib.

In another specific embodiment, the Ph+ ALL has previously received treatment with ≥1 different BCR-ABL1 TKIs and subsequently experienced relapse or became refractory. In another specific embodiment, the Ph+ ALL has previously received treatment with ≥2 different BCR-ABL1 TKIs and subsequently experienced relapse or became refractory. In another specific embodiment, the Ph+ ALL has previously received treatment with ≥3 different BCR-ABL1 TKIs and subsequently experienced relapse or became refractory. In another specific embodiment, the Ph+ ALL has previously received treatment with ≥4 different BCR-ABL1 TKIs and subsequently experienced relapse or became refractory. In another more specific embodiment, the BCR-ABL1 TKIs are first-line, second-line, third-line, fourth-line, or fifth-line therapeutic agent. In another more specific embodiment, the BCR-ABL1 TKIs are first-line, second-line, or third-line therapeutic agent. In another more specific embodiment, the BCR-ABL1 TKIs are first-line or second-line therapeutic agents. In another more specific embodiment, the BCR-ABL1 TKIs are first-line therapeutic agents. In another more specific embodiment, the BCR-ABL1 TKIs are second-line therapeutic agents. In another more specific embodiment, the BCR-ABL1 TKIs are second-line or higher-line therapeutic agents. In another more specific embodiment, the BCR-ABL1 TKIs include at least third-generation TKIs (e.g., ponatinib or olverembatinib(olverembatinib)). In another more specific embodiment, the BCR-ABL1 TKIs include at least third-generation TKIs (e.g., ponatinib or olverembatinib) and/or fourth-generation TKIs(e.g., asciminib(asciminib) or HS-10382). In another more specific embodiment, the BCR-ABL1 TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382. In another more specific embodiment, the BCR-ABL1 TKIs are selected from imatinib, dasatinib, nilotinib, bosutinib, flumatinib, ponatinib, olverembatinib, radotinib or asciminib. In another more specific embodiment, the BCR-ABL1 TKIs are selected from imatinib, dasatinib, nilotinib, ponatinib, olverembatinib or asciminib. In another more specific embodiment, the BCR-ABL1 TKIs are selected from imatinib, dasatinib, nilotinib, bosutinib, flumatinib or radotinib. In another more specific embodiment, the BCR-ABL1 TKIs are selected from ponatinib, olverembatinib, asciminib or HS-10382. In another more specific embodiment, the BCR-ABL1 TKIs are selected from imatinib, dasatinib or nilotinib. In another more specific embodiment, the BCR-ABL1 TKIs are selected from ponatinib or olverembatinib. In another more specific embodiment, the BCR-ABL1 TKIs are selected from asciminib or HS-10382. In another more specific embodiment, the BCR-ABL1 TKIs are selected from asciminib.

In another specific embodiment, the Ph+ ALL has previously received treatment with ≥1 different BCR-ABL1 ATP-competitive TKIs and subsequently experienced disease progression, with treatment evaluation results being failure, warning, or intolerance. In another specific embodiment, the Ph+ ALL has previously received treatment with ≥2 different BCR-ABL1 ATP-competitive TKIs and subsequently experienced disease progression, with treatment evaluation results being failure, warning, or intolerance. In another specific embodiment, the Ph+ ALL has previously received treatment with ≥3 different BCR-ABL1 ATP-competitive TKIs and subsequently experienced disease progression, with treatment evaluation results being failure, warning, or intolerance. In another specific embodiment, the Ph+ ALL has previously received treatment with ≥4 different BCR-ABL1 ATP-competitive TKIs and subsequently experienced disease progression, with treatment evaluation results being failure, warning, or intolerance. In another more specific embodiment, the BCR-ABL1 ATP-competitive TKIs are a first-line, second-line, third-line, fourth-line, or fifth-line therapeutic agent. In another more specific embodiment, the BCR-ABL1 ATP-competitive TKIs are a first-line, second-line, or third-line therapeutic agent. In another more specific embodiment, the BCR-ABL1 ATP-competitive TKIs are first-line or second-line therapeutic agents. In another more specific embodiment, the BCR-ABL1 ATP-competitive TKIs are first-line therapeutic agents. In another more specific embodiment, the BCR-ABL1 ATP-competitive TKIs are second-line therapeutic agents. In another more specific embodiment, the BCR-ABL1 ATP-competitive TKIs are second-line or higher-line therapeutic agents. In another more specific embodiment, the BCR-ABL1 ATP-competitive TKIs include at least third-generation TKIs (e.g., ponatinib or olverembatinib). In another more specific embodiment, the BCR-ABL1 ATP-competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228. In another more specific embodiment, the BCR-ABL1 ATP-competitive TKIs are selected from imatinib, dasatinib, nilotinib, bosutinib, flumatinib, ponatinib, olverembatinib or radotinib. In another

more specific embodiment, the BCR-ABL1 ATP-competitive TKIs are selected from imatinib, dasatinib, nilotinib, ponatinib or olverembatinib. In another more specific embodiment, the BCR-ABL1 ATP-competitive TKIs are selected from imatinib, dasatinib, nilotinib, bosutinib, flumatinib or radotinib. In another more specific embodiment, the BCR-ABL1 TKIs are selected from ponatinib, olverembatinib, asciminib or HS-10382. In another more specific embodiment, the BCR-ABL1 ATP-competitive TKIs are selected from imatinib, dasatinib or nilotinib. In another more specific embodiment, the BCR-ABL1 ATP-competitive TKIs are selected from ponatinib or olverembatinib.

In another specific embodiment, the Ph+ ALL has previously received treatment with ≥1 different BCR-ABL1 ATP-competitive TKIs and subsequently experienced relapse or became refractory. In another specific embodiment, the Ph+ ALL has previously received treatment with ≥2 different BCR-ABL1 ATP-competitive TKIs and subsequently experienced relapse or became refractory. In another specific embodiment, the Ph+ ALL has previously received treatment with ≥3 different BCR-ABL1 ATP-competitive TKIs and subsequently experienced relapse or became refractory. In another specific embodiment, the Ph+ ALL has previously received treatment with ≥4 different BCR-ABL1 ATP-competitive TKIs and subsequently experienced relapse or became refractory. In another more specific embodiment, the BCR-ABL1 ATP-competitive TKIs are a first-line, second-line, third-line, fourth-line, or fifth-line therapeutic agent. In another more specific embodiment, the BCR-ABL1 ATP-competitive TKIs are a first-line, second-line, or third-line therapeutic agent. In another more specific embodiment, the BCR-ABL1 ATP-competitive TKIs are first-line or second-line therapeutic agents. In another more specific embodiment, the BCR-ABL1 ATP-competitive TKIs are first-line or second-line therapeutic agents. In another more specific embodiment, the BCR-ABL1 ATP-competitive TKIs are first-line therapeutic agents. In another more specific embodiment, the BCR-ABL1 ATP-competitive TKIs are second-line therapeutic agents. In another more specific embodiment, the BCR-ABL1 ATP-competitive TKIs are second-line or higher-line therapeutic agents. In another more specific embodiment, the BCR-ABL1 ATP-competitive TKIs include at least third-generation TKIs (e.g., ponatinib or olverembatinib). In another more specific embodiment, the BCR-ABL1 ATP-competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228. In another more specific embodiment, the BCR-ABL1 ATP-competitive TKIs are selected from imatinib, dasatinib, nilotinib, bosutinib, flumatinib, ponatinib, olverembatinib or radotinib. In another more specific embodiment, the BCR-ABL1 ATP-competitive TKIs are selected from imatinib, dasatinib, nilotinib, ponatinib or olverembatinib. In another more specific embodiment, the BCR-ABL1 ATP-competitive TKIs are selected from imatinib, dasatinib, nilotinib, bosutinib, flumatinib or radotinib. In another more specific embodiment, the BCR-ABL1 ATP-competitive TKIs are selected from ponatinib, olverembatinib, asciminib or HS-10382. In another more specific embodiment, the BCR-ABL1 ATP-competitive TKIs are selected from imatinib, dasatinib or nilotinib. In another more specific embodiment, the BCR-ABL1 ATP-competitive TKIs are selected from ponatinib or olverembatinib.

In another specific embodiment, the Ph+ ALL has previously received treatment with ≥1 different first- and/or second-generation BCR-ABL1 ATP-competitive TKIs and subsequently developed resistance or intolerance. In another specific embodiment, the Ph+ ALL has previously received treatment with ≥2 different first- and/or second-generation BCR-ABL1 ATP-competitive TKIs and subsequently developed resistance or intolerance. In another specific embodiment, the Ph+ ALL has previously received treatment with ≥3 different first-and/or second-generation BCR-ABL1 ATP-competitive TKIs and subsequently developed resistance or intolerance. In another specific embodiment, the Ph+ ALL has previously received treatment with ≥4 different first- and/or second-generation BCR-ABL1 ATP-competitive TKIs and subsequently developed resistance or intolerance. In another more specific embodiment, the resistance arises from BCR-ABL1 kinase domain point mutation(s), such as one or more of T315I/A, Y253F/H, E255V/K, M351T, G250E, F359V/I/C, H396P/R, M244V, E355G, F317L/V/I/C, F311L, M237I, Q252H/R, D276G, L248V, V379I, L384M, L387M, E279K, F486S, E459K, P223S, I502L, or V299L. In another more specific embodiment, the resistance involves BCR-ABL1 kinase domain point mutations, selected from one or more of T315I, Y253H, E255V/K, M351T, G250E, F359V/, H396P, M244V, E355G, F317L, Q252H, E459K, P223S, I502L, or V299L mutations. In another more specific embodiment, the resistance is T315I. In another more specific embodiment, the first- or second-generation BCR-ABL1 ATP-competitive TKIs are a first-line, second-line, third-line, fourth-line, or fifth-line therapeutic agent. In another more specific embodiment, the first- or second-generation BCR-ABL1 ATP-competitive TKIs are a first-line, second-line, or third-line therapeutic agent. In another more specific embodiment, the first- or second-generation BCR-ABL1 ATP-competitive TKIs are first-line or second-line therapeutic agents. In another more specific embodiment, the first- or second-generation BCR-ABL1 ATP-competitive TKIs are first-line therapeutic agents. In another more specific embodiment, the first- or second-generation BCR-ABL1 ATP-competitive TKIs are second-line therapeutic agents. In another more specific embodiment, the first- or second-generation BCR-ABL1 ATP-competitive TKIs are second-line or higher-line therapeutic agents. In another more specific embodiment, the first-generation BCR-ABL1 ATP-competitive TKI is imatinib. In another more specific embodiment, the second-generation BCR-ABL1 ATP-competitive TKIs are selected from dasatinib, nilotinib, bosutinib, flumatinib or radotinib. In another more specific embodiment, the first- and/or second-generation BCR-ABL1 ATP-competitive TKIs are selected from imatinib, dasatinib, nilotinib, bosutinib, flumatinib or radotinib. In another more specific embodiment, the first- and/or second-generation BCR-ABL1 ATP-competitive TKIs are selected from imatinib, dasatinib or nilotinib.

In another specific embodiment, the Ph+ ALL has previously received treatment with ≥1 different first- and/or second-generation BCR-ABL1 ATP-competitive TKIs and subsequently experienced relapse or became refractory. In another specific embodiment, the Ph+ ALL has previously received treatment with ≥2 different first- and/or second-generation BCR-ABL1 ATP-competitive TKIs and subsequently experienced relapse or became refractory. In another specific embodiment, the Ph+ ALL has previously received treatment with ≥3 different first- and/or second-generation BCR-ABL1 ATP-competitive TKIs and subsequently experienced relapse or became refractory. In another specific embodiment, the Ph+ ALL has previously received treatment with ≥4 different first- and/or second-generation BCR-ABL1 ATP-competitive TKIs and subsequently experienced relapse or became refractory. In another more specific embodiment, the first- or second-generation BCR-ABL1 ATP-competitive TKIs are a first-line, second-line, third-line, fourth-line, or fifth-line therapeutic agent. In another more specific embodiment, the first- or second-generation BCR-ABL1 ATP-competitive TKIs are a first-line, second-line, or third-line therapeutic agent. In another more specific embodiment, the first- or second-generation BCR-ABL1 ATP-competitive TKIs are first-line or second-line therapeutic agents. In another more specific embodiment, the first- or second-generation BCR-ABL1 ATP-competitive TKIs are first-line therapeutic agents. In another more specific embodiment, the first- or second-generation BCR-ABL1 ATP-competitive TKIs are second-line therapeutic agents. In another more specific embodiment, the first- or second-generation BCR-ABL1 ATP-competitive TKIs are second-line or higher-line therapeutic agents. In another more specific embodiment, the first-generation BCR-ABL1 ATP-competitive TKI is imatinib. In another more specific embodiment, the second-generation BCR-ABL1 ATP-competitive TKIs are selected from dasatinib, nilotinib, bosutinib, flumatinib or radotinib. In another more specific embodiment, the first- and/or second-generation BCR-ABL1 ATP-competitive TKIs are selected from imatinib, dasatinib, nilotinib, bosutinib, flumatinib or radotinib. In another more specific embodiment, the first- and/or second-generation BCR-ABL1 ATP-competitive TKIs are selected from imatinib, dasatinib or nilotinib.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270

mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Philadelphia chromosome-positive acute lymphoblastic leukemia (Ph+ ALL) that has previously received treatment with ≥1 different BCR-ABL1 TKIs and subsequently experienced disease progression, with treatment evaluation results being failure, warning, or intolerance

In one embodiment, the present disclosure relates to the use and method of the compound of formula (I) or a pharmaceutically acceptable salt, crystal form, solvate, or hydrate thereof for treating Ph+ ALL or central nervous system leukemia relapse thereof, such as brain metastasis, wherein the Ph+ ALL has previously received treatment with ≥1 different BCR-ABL1 TKIs and subsequently experienced disease progression, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥1 different BCR-ABL1 TKIs and subsequently experienced disease progression, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥2 different BCR-ABL1 TKIs and subsequently experienced disease progression, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥2 different BCR-ABL1 TKIs and subsequently experienced

disease progression, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥3 different BCR-ABL1 TKIs and subsequently experienced disease progression, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥3 different BCR-ABL1 TKIs and subsequently experienced disease progression, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥4 different BCR-ABL1 TKIs and subsequently experienced disease progression, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥4 different BCR-ABL1 TKIs and subsequently experienced disease progression, with treatment evaluation results being failure, warning, or intolerance.

In one specific embodiment, the BCR-ABL1 TKIs are first-line, second-line, third-line, fourth-line, or fifth-line therapeutic agent. In another specific embodiment, the BCR-ABL1 TKIs are first-line, second-line, or third-line therapeutic agent. In another specific embodiment, the BCR-ABL1 TKIs are first-line or second-line therapeutic agents. In another specific embodiment, the BCR-ABL1 TKIs are first-line therapeutic agents. In another specific embodiment, the BCR-ABL1 TKIs are second-line therapeutic agents. In another specific embodiment, the BCR-ABL1 TKIs are second-line or higher-line therapeutic agents. In another specific embodiment, the BCR-ABL1 TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382. In another more specific embodiment, the BCR-ABL1 TKIs are selected from imatinib, dasatinib, nilotinib, bosutinib, flumatinib, ponatinib, olverembatinib, radotinib or asciminib. In another more specific embodiment, the BCR-ABL1 TKIs are selected from imatinib, dasatinib, nilotinib, ponatinib, olverembatinib or asciminib. In another more specific embodiment, the BCR-ABL1 TKIs are selected from imatinib, dasatinib, nilotinib, bosutinib, flumatinib or radotinib. In another more specific embodiment, the BCR-ABL1 TKIs are selected from ponatinib, olverembatinib, asciminib or HS-10382. In another more specific embodiment, the BCR-ABL1 TKIs are selected from imatinib, dasatinib or nilotinib. In another more specific embodiment, the BCR-ABL1 TKIs are selected from ponatinib or olverembatinib. In another more specific embodiment, the BCR-ABL1 TKIs are selected from asciminib or HS-10382. In another more specific embodiment, the BCR-ABL1 TKIs are selected from asciminib.

In another specific embodiment, the Ph+ ALL has BCR-ABL1+. In another specific embodiment, the Ph+ ALL is Ph+ ALL with T315I mutation. In another specific embodiment, the Ph+ ALL is Ph+ ALL without T315I mutation. In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment,

the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Philadelphia chromosome-positive acute lymphoblastic leukemia (Ph+ ALL) that has previously received treatment with ≥1 different BCR-ABL1 ATP-competitive TKIs and subsequently experienced disease progression, with treatment evaluation results being failure, warning, or intolerance

In one embodiment, the present disclosure relates to the use and method of the compound of formula (I) or a pharmaceutically acceptable salt, crystal form, solvate, or hydrate thereof for treating Ph+ ALL or central nervous system leukemia relapse thereof, such as brain metastasis, wherein the Ph+ ALL has previously received treatment with ≥1 different BCR-ABL1 ATP-competitive TKIs and subsequently experienced disease progression, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥1 different BCR-ABL1 ATP-competitive TKIs and subsequently experienced disease progression, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥2 different BCR-ABL1 ATP-competitive TKIs and subsequently experienced disease progression, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥2 different BCR-ABL1 ATP-competitive TKIs and subsequently experienced disease progression, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥3 different BCR-ABL1 ATP-competitive TKIs and subsequently experienced disease progression, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥3 different BCR-ABL1 ATP-competitive TKIs and subsequently experienced disease progression, with treatment evaluation results being failure, warning, or intolerance.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥4 different BCR-ABL1 ATP-competitive TKIs and subsequently experienced disease progression, with treatment evaluation results being failure, warning, or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥4 different BCR-ABL1 ATP-competitive TKIs and subsequently experienced disease progression, with treatment evaluation results being failure, warning, or intolerance.

In another specific embodiment, the BCR-ABL1 ATP-competitive TKIs are a first-line, second-line, third-line, fourth-line, or fifth-line therapeutic agent. In another specific embodiment, the BCR-ABL1 ATP-competitive TKIs are a first-line, second-line, or third-line therapeutic agent. In another specific embodiment, the BCR-ABL1 ATP-competitive TKIs are first-line or second-line therapeutic agents. In another specific embodiment, the BCR-ABL1 ATP-competitive TKIs are first-line therapeutic agents. In another specific embodiment, the BCR-ABL1 ATP-competitive TKIs are second-line therapeutic agents. In another specific embodiment, the BCR-ABL1 ATP-competitive TKIs are second-line or higher-line therapeutic agents. In another specific embodiment, the BCR-ABL1 ATP-competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228. In another specific embodiment, the BCR-ABL1 ATP-competitive TKIs are selected from imatinib, dasatinib, nilotinib, bosutinib, flumatinib, ponatinib, olverembatinib or radotinib. In another specific embodiment, the BCR-ABL1 ATP-competitive TKIs are selected from imatinib, dasatinib, nilotinib, ponatinib or olverembatinib. In another specific embodiment, the BCR-ABL1 ATP-competitive TKIs are selected from imatinib, dasatinib, nilotinib, bosutinib, flumatinib or radotinib. In another more specific embodiment, the BCR-ABL1 ATP-competitive TKIs are selected from ponatinib, olverembatinib, asciminib or HS-10382. In another specific embodiment, the BCR-ABL1 ATP-competitive TKIs are selected from imatinib, dasatinib or nilotinib. In another specific embodiment, the BCR-ABL1 ATP-competitive TKIs are selected from ponatinib or olverembatinib.

In another specific embodiment, the Ph+ ALL has BCR-ABL1+. In another specific embodiment, the Ph+ ALL is

Ph+ ALL with T315I mutation. In another specific embodiment, the Ph+ ALL is Ph+ ALL without T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In

another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Philadelphia chromosome-positive acute lymphoblastic leukemia (Ph+ ALL) that has previously received treatment with ≥1 different first- and/or second-generation BCR-ABL1 ATP-competitive TKIs and subsequently developed resistance or intolerance

In one embodiment, the present disclosure relates to the use and method of the compound of formula (I) or a pharmaceutically acceptable salt, crystal form, solvate, or hydrate thereof for treating Ph+ ALL or central nervous system leukemia relapse thereof, such as brain metastasis, wherein the Ph+ ALL has previously received treatment with ≥1 different first-and/or second-generation BCR-ABL1 ATP-competitive TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥1 different first- and/or second-generation BCR-ABL1 ATP-competitive TKIs and subsequently developed resistance or intolerance.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥2 different first- and/or second-generation BCR-ABL1 ATP-competitive TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥2 different first- and/or second-generation BCR-ABL1 ATP-competitive TKIs and subsequently developed resistance or intolerance.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥3 different first- and/or second-generation BCR-ABL1 ATP-competitive TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥3 different first- and/or second-generation BCR-ABL1 ATP-competitive TKIs and subsequently developed resistance or intolerance.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥4 different first- and/or second-generation BCR-ABL1 ATP-competitive TKIs and subsequently developed resistance or intolerance. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥4 different first- and/or second-generation BCR-ABL1 ATP-competitive TKIs and subsequently developed resistance or intolerance.

In another specific embodiment, the resistance arises from BCR-ABL1 kinase domain point mutation(s), such as one or more of T315I/A, Y253F/H, E255V/K, M351T, G250E, F359V/I/C, H396P/R, M244V, E355G, F317L/V/I/C, F311L, M237I, Q252H/R, D276G, L248V, V379I, L384M, L387M, E279K, F486S, E459K, P223S, I502L, or V299L. In another specific embodiment, the resistance involves BCR-ABL1 kinase domain point mutations, selected from one or more of T315I, Y253H, E255V/K, M351T, G250E, F359V/, H396P, M244V, E355G, F317L, Q252H, E459K, P223S, I502L, or V299L mutations. In another specific embodiment, the resistance is T315I. In another specific embodiment, the first- or second-generation BCR-ABL1 ATP-competitive TKIs are a first-line, second-line, third-line, fourth-line, or fifth-line therapeutic agent. In another specific embodiment, the first- or second-generation BCR-ABL1 ATP-competitive TKIs are a first-line, second-line, or third-line therapeutic agent. In another specific embodiment, the first- or second-generation BCR-ABL1 ATP-competitive TKIs are first-line or second-line therapeutic agents. In another specific embodiment, the first- or second-generation BCR-ABL1 ATP-competitive TKIs are first-line therapeutic agents. In another specific embodiment, the first- or second-generation BCR-ABL1 ATP-competitive TKIs are second-line therapeutic agents. In another specific embodiment, the first- or second-generation BCR-ABL1 ATP-competitive TKIs are second-line or higher-line therapeutic agents. In another specific embodiment, the first-generation BCR-ABL1 ATP-competitive TKI is imatinib. In another specific embodiment, the second-generation BCR-ABL1 ATP-competitive TKIs are selected from dasatinib, nilotinib, bosutinib, flumatinib or radotinib. In another specific embodiment, the first- and/or second-generation BCR-ABL1 ATP-competitive TKIs are selected from imatinib, dasatinib, nilotinib, bosutinib, flumatinib or radotinib. In another specific embodiment, the first- and/or second-generation BCR-ABL1 ATP-competitive TKIs are selected from imatinib, dasatinib or nilotinib.

In another specific embodiment, the Ph+ ALL has BCR-ABL1+. In another specific embodiment, the Ph+ ALL is Ph+ ALL with T315I mutation. In another specific embodiment, the Ph+ ALL is Ph+ ALL without T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment,

the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Philadelphia chromosome-positive acute lymphoblastic leukemia (Ph+ ALL) that has previously received treatment with ≥1 different BCR-ABL1 TKIs and subsequently experienced relapse or became refractory

In one embodiment, the present disclosure relates to the use and method of the compound of formula (I) or a pharmaceutically acceptable salt, crystal form, solvate, or hydrate thereof for treating Ph+ ALL or central nervous system leukemia relapse thereof, such as brain metastasis, wherein the Ph+ ALL has previously received treatment with ≥1 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥1 different BCR-ABL1 TKIs and subsequently experienced relapse or became refractory.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥2 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥2 different BCR-ABL1 TKIs and subsequently experienced relapse or became refractory.

In another embodiment, the present disclosure relates to the use and method of the compound of formula (I) or a

pharmaceutically acceptable salt, crystal form, solvate, or hydrate thereof for treating Ph+ ALL, wherein the Ph+ ALL has previously received treatment with ≥3 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥3 different BCR-ABL1 TKIs and subsequently experienced relapse or became refractory.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥4 different BCR-ABL1 TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥4 different BCR-ABL1 TKIs and subsequently experienced relapse or became refractory.

In another specific embodiment, the BCR-ABL1 TKIs are first-line, second-line, third-line, fourth-line, or fifth-line therapeutic agent. In another specific embodiment, the BCR-ABL1 TKIs are first-line, second-line, or third-line therapeutic agent. In another specific embodiment, the BCR-ABL1 TKIs are first-line or second-line therapeutic agents. In another specific embodiment, the BCR-ABL1 TKIs are first-line therapeutic agents. In another specific embodiment, the BCR-ABL1 TKIs are second-line therapeutic agents. In another specific embodiment, the BCR-ABL1 TKIs are second-line or higher-line therapeutic agents. In another specific embodiment, the BCR-ABL1 TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382. In another specific embodiment, the BCR-ABL1 TKIs are selected from imatinib, dasatinib, nilotinib, bosutinib, flumatinib, ponatinib, olverembatinib, radotinib or asciminib. In another specific embodiment, the BCR-ABL1 TKIs are selected from imatinib, dasatinib, nilotinib, ponatinib, olverembatinib or asciminib. In another specific embodiment, the BCR-ABL1 TKIs are selected from imatinib, dasatinib, nilotinib, bosutinib, flumatinib or radotinib. In another specific embodiment, the BCR-ABL1 TKIs are selected from ponatinib, olverembatinib, asciminib or HS-10382. In another specific embodiment, the BCR-ABL1 TKIs are selected from imatinib, dasatinib or nilotinib. In another specific embodiment, the BCR-ABL1 TKIs are selected from ponatinib or olverembatinib. In another specific embodiment, the BCR-ABL1 TKIs are selected from asciminib or HS-10382. In another specific embodiment, the BCR-ABL1 TKIs are selected from asciminib.

In another specific embodiment, the Ph+ ALL has BCR-ABL1+. In another specific embodiment, the Ph+ ALL is Ph+ ALL with T315I mutation. In another specific embodiment, the Ph+ ALL is Ph+ ALL without T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form,

solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Philadelphia chromosome-positive acute lymphoblastic leukemia (Ph+ ALL) that has previously received treatment with ≥1 different BCR-ABL1 ATP-competitive TKIs and subsequently experienced relapse or became refractory

In one embodiment, the present disclosure relates to the use and method of the compound of formula (I) or a pharmaceutically acceptable salt, crystal form, solvate, or hydrate thereof for treating Ph+ ALL or central nervous system leukemia relapse thereof, such as brain metastasis, wherein the Ph+ ALL has previously received treatment with ≥1

different BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥1 different BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥2 different BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥2 different BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥3 different BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥3 different BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥4 different BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥4 different BCR-ABL1 ATP-competitive TKIs and subsequently experienced relapse or became refractory.

In another specific embodiment, the BCR-ABL1 ATP-competitive TKIs are a first-line, second-line, third-line, fourth-line, or fifth-line therapeutic agent. In another specific embodiment, the BCR-ABL1 ATP-competitive TKIs are a first-line, second-line, or third-line therapeutic agent. In another specific embodiment, the BCR-ABL1 ATP-competitive TKIs are first-line or second-line therapeutic agents. In another specific embodiment, the BCR-ABL1 ATP-competitive TKIs are first-line therapeutic agents. In another specific embodiment, the BCR-ABL1 ATP-competitive TKIs are second-line therapeutic agents. In another specific embodiment, the BCR-ABL1 ATP-competitive TKIs are second-line or higher-line therapeutic agents. In another specific embodiment, the BCR-ABL1 ATP-competitive TKIs are selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, HS-10382, rebasinib, danusertib, tozasertib, AT9283, KW-2449 or XL228. In another specific embodiment, the BCR-ABL1 ATP-competitive TKIs are selected from imatinib, dasatinib, nilotinib, bosutinib, flumatinib, ponatinib, olverembatinib or radotinib. In another specific embodiment, the BCR-ABL1 ATP-competitive TKIs are selected from imatinib, dasatinib, nilotinib, ponatinib or olverembatinib. In another specific embodiment, the BCR-ABL1 ATP-competitive TKIs are selected from imatinib, dasatinib, nilotinib, bosutinib, flumatinib or radotinib. In another specific embodiment, the BCR-ABL1 ATP-competitive TKIs are selected from ponatinib, olverembatinib, asciminib or HS-10382. In another specific embodiment, the BCR-ABL1 ATP-competitive TKIs are selected from imatinib, dasatinib or nilotinib. In another specific embodiment, the BCR-ABL1 ATP-competitive TKIs are selected from ponatinib or olverembatinib.

In another specific embodiment, the Ph+ ALL has BCR-ABL1+. In another specific embodiment, the Ph+ ALL is Ph+ ALL with T315I mutation. In another specific embodiment, the Ph+ ALL is Ph+ ALL without T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively

compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I)

or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Philadelphia chromosome-positive acute lymphoblastic leukemia (Ph+ ALL) that has previously received treatment with ≥1 different first- and/or second-generation BCR-ABL1 ATP-competitive TKIs and subsequently experienced relapse or became refractory

In one embodiment, the present disclosure relates to the use and method of the compound of formula (I) or a pharmaceutically acceptable salt, crystal form, solvate, or hydrate thereof for treating Ph+ ALL or central nervous system leukemia relapse thereof, such as brain metastasis, wherein the Ph+ ALL has previously received treatment with ≥1 different first-and/or second-generation BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥1 different first- and/or second-generation BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥2 different first- and/or second-generation BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥2 different first- and/or second-generation BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥3 different first- and/or second-generation BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥3 different first- and/or second-generation BCR-ABL1 ATP-competitive TKIs and subsequently experienced relapse or became refractory.

In another embodiment, the present disclosure relates to the use and method of the compound of formula (I) or a pharmaceutically acceptable salt, crystal form, solvate, or hydrate thereof for treating Ph+ ALL, wherein the Ph+ ALL has previously received treatment with ≥4 different first- and/or second-generation BCR-ABL1 ATP-competitive TKIs and subsequently experienced relapse or became refractory. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with ≥4 different first- and/or second-generation BCR-ABL1 ATP-competitive TKIs and subsequently experienced relapse or became refractory.

In another specific embodiment, the first-generation BCR-ABL1 ATP-competitive TKI is a first-line, second-line, third-line, fourth-line, or fifth-line therapeutic agent. In another specific embodiment, the first-generation BCR-ABL1 ATP-competitive TKI is a first-line, second-line, or third-line therapeutic agent. In another specific embodiment, the first-generation BCR-ABL1 ATP-competitive TKI is first-line or second-line therapeutic agent. In another specific embodiment, the first-generation BCR-ABL1 ATP-competitive TKI is imatinib. In another specific embodiment, the second-generation BCR-ABL1 ATP-competitive TKIs are selected from dasatinib, nilotinib, bosutinib, flumatinib or radotinib. In another specific embodiment, the first- and/or second-generation BCR-ABL1 ATP-competitive TKIs are selected from imatinib, dasatinib, nilotinib, bosutinib, flumatinib or radotinib. In another specific embodiment, the first- and/or second-generation BCR-ABL1 ATP-competitive TKIs are selected from imatinib, dasatinib or nilotinib.

In another specific embodiment, the Ph+ ALL has BCR-ABL1+. In another specific embodiment, the Ph+ ALL is Ph+ ALL with T315I mutation. In another specific embodiment, the Ph+ ALL is Ph+ ALL without T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is

about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is administered until disease progression, intolerable toxicity, or death. In another specific embodiment, compound A or a pharmaceutically acceptable salt thereof is administered until disease progression, intolerable toxicity, or death.

Philadelphia chromosome-positive acute lymphoblastic leukemia (Ph+ ALL) that has previously received treatment with third or above generations of BCR-ABL1 TKIs and subsequently failed or was intolerant

In one embodiment, the present disclosure relates to the use and method of the compound of formula (I) or a pharmaceutically acceptable salt, crystal form, solvate, or hydrate thereof for treating Ph+ ALL or central nervous system leukemia relapse thereof, such as brain metastasis, wherein the Ph+ ALL has previously received treatment with third or above generations of BCR-ABL1 TKIs and subsequently failed or was intolerant. In another embodiment, the present disclosure relates to the use and method of (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof for treating Ph+ ALL, wherein the Ph+ ALL has previously received treatment with third or above generations of BCR-ABL1 TKIs and subsequently failed or was intolerant.

In another specific embodiment, the BCR-ABL1 TKIs are first-line, second-line, third-line, fourth-line, or fifth-line therapeutic agent. In another specific embodiment, the BCR-ABL1 TKIs are first-line, second-line, or third-line therapeutic agent. In another specific embodiment, the BCR-ABL1 TKIs are first-line or second-line therapeutic agents. In another specific embodiment, the BCR-ABL1 TKIs are first-line therapeutic agents. In another specific embodiment, the BCR-ABL1 TKIs are second-line therapeutic agents. In another specific embodiment, the BCR-ABL1 TKIs is third-line therapeutic agent. In another specific embodiment, the BCR-ABL1 TKIs are selected from ponatinib, olverembatinib, PF-114, rebasinib, danusertib, tozasertib, AT9283, KW-2449, XL228, asciminib or HS-10382. In another specific embodiment, the BCR-ABL1 TKIs are selected from ponatinib, olverembatinib, PF-114, asciminib or HS-10382. In another specific embodiment, the BCR-ABL1 TKIs are selected from ponatinib, olverembatinib or PF-114. In another specific embodiment, the BCR-ABL1 TKIs are selected from ponatinib or olverembatinib. In another specific embodiment, the BCR-ABL1 TKIs are selected from ponatinib.

In another specific embodiment, the Ph+ ALL has BCR-ABL1+. In another specific embodiment, the Ph+ ALL is Ph+ ALL with T315I mutation. In another specific embodiment, the Ph+ ALL is Ph+ ALL without T315I mutation.

In another specific embodiment, the use and method are applied to adult subjects. In another specific embodiment, the use and method are applied to pediatric subjects. In another specific embodiment, the use and method are applied to subjects with brain metastases.

In another specific embodiment, the dose of the compound of formula (I) or pharmaceutically acceptable salt, crystal form, solvate, or hydrate thereof (alternatively compound A) is about 20 mg/day to about 400 mg/day. In another specific embodiment, the dose of the compound of formula (I) or pharmaceutically acceptable salt, crystal form, solvate, or hydrate thereof (alternatively compound A) is about 20 mg/day to about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 140 mg/day, about 160 mg/day, about 180 mg/day, about 200 mg/day, about 220 mg/day, about 240 mg/day, about 260 mg/day, about 270 mg/day, about 280 mg/day, about 300 mg/day, about 320 mg/day, about 340 mg/day, about 360 mg/day, about 380 mg/day, or about 400 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day. In another specific embodiment, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day. In another specific embodiment, the

dose of the compound of formula (I) or pharmaceutically acceptable salt, crystal form, solvate, or hydrate thereof (alternatively compound A) is about 20 mg/day. In another specific embodiment, the dose of the compound of formula (I) or pharmaceutically acceptable salt, crystal form, solvate, or hydrate thereof (alternatively compound A) is about 40 mg/day. In another specific embodiment, the dose of the compound of formula (I) or pharmaceutically acceptable salt, crystal form, solvate, or hydrate thereof (alternatively compound A) is about 60 mg/day. In another specific embodiment, the dose of the compound of formula (I) or pharmaceutically acceptable salt, crystal form, solvate, or hydrate thereof (alternatively compound A) is about 80 mg/day. In another specific embodiment, the dose of the compound of formula (I) or pharmaceutically acceptable salt, crystal form, solvate, or hydrate thereof (alternatively compound A) is about 100 mg/day. In another specific embodiment, the dose of the compound of formula (I) or pharmaceutically acceptable salt, crystal form, solvate, or hydrate thereof (alternatively compound A) is about 120 mg/day. In another specific embodiment, the dose of the compound of formula (I) or pharmaceutically acceptable salt, crystal form, solvate, or hydrate thereof (alternatively compound A) is about 140 mg/day. In another specific embodiment, the dose of the compound of formula (I) or pharmaceutically acceptable salt, crystal form, solvate, or hydrate thereof (alternatively compound A) is about 160 mg/day. In another specific embodiment, the dose of the compound of formula (I) or pharmaceutically acceptable salt, crystal form, solvate, or hydrate thereof (alternatively compound A) is about 180 mg/day. In another specific embodiment, the dose of the compound of formula (I) or pharmaceutically acceptable salt, crystal form, solvate, or hydrate thereof (alternatively compound A) is about 200 mg/day. In another specific embodiment, the dose of the compound of formula (I) or pharmaceutically acceptable salt, crystal form, solvate, or hydrate thereof (alternatively compound A) is about 220 mg/day. In another specific embodiment, the dose of the compound of formula (I) or pharmaceutically acceptable salt, crystal form, solvate, or hydrate thereof (alternatively compound A) is about 240 mg/day. In another specific embodiment, the dose of the compound of formula (I) or pharmaceutically acceptable salt, crystal form, solvate, or hydrate thereof (alternatively compound A) is about 260 mg/day. In another specific embodiment, the dose of the compound of formula (I) or pharmaceutically acceptable salt, crystal form, solvate, or hydrate thereof (alternatively compound A) is about 270 mg/day. In another specific embodiment, the dose of the compound of formula (I) or pharmaceutically acceptable salt, crystal form, solvate, or hydrate thereof (alternatively compound A) is about 280 mg/day. In another specific embodiment, the dose of the compound of formula (I) or pharmaceutically acceptable salt, crystal form, solvate, or hydrate thereof (alternatively compound A) is about 300 mg/day. In another specific embodiment, the dose of the compound of formula (I) or pharmaceutically acceptable salt, crystal form, solvate, or hydrate thereof (alternatively compound A) is about 320 mg/day. In another specific embodiment, the dose of the compound of formula (I) or pharmaceutically acceptable salt, crystal form, solvate, or hydrate thereof (alternatively compound A) is about 340 mg/day. In another specific embodiment, the dose of the compound of formula (I) or pharmaceutically acceptable salt, crystal form, solvate, or hydrate thereof (alternatively compound A) is about 360 mg/day. In another specific embodiment, the dose of the compound of formula (I) or pharmaceutically acceptable salt, crystal form, solvate, or hydrate thereof (alternatively compound A) is about 380 mg/day. In another specific embodiment, the dose of the compound of formula (I) or pharmaceutically acceptable salt, crystal form, solvate, or hydrate thereof (alternatively compound A) is about 400 mg/day. In another specific embodiment, the compound of formula (I) or a pharmaceutically acceptable salt, crystal form, solvate, or hydrate thereof (alternatively compound A) is administered orally or intranasally. In another specific embodiment, the compound of formula (I) or a pharmaceutically acceptable salt, crystal form, solvate, or hydrate thereof (alternatively compound A) is administered orally under fasting conditions. In another specific embodiment, the compound of formula (I) or a pharmaceutically acceptable salt, crystal form, solvate, or hydrate thereof (alternatively Compound A) is administered QD, BID, or TID. In another specific embodiment, the compound of formula (I) or a pharmaceutically acceptable salt, crystal form, solvate, or hydrate thereof (alternatively Compound A) is administered at about 10 mg BID, 20 mg BID, 40 mg QD, 40 mg BID, 80 mg QD, 80 mg BID, 160 mg QD or 240 mg QD. In another specific embodiment, the daily effective amount of the compound of formula (I) or a pharmaceutically acceptable salt, crystal form, solvate, or hydrate thereof (alternatively compound A) is about 10 mg BID. In another specific embodiment, the daily effective amount of the compound of formula (I) or a pharmaceutically acceptable salt, crystal form, solvate, or hydrate thereof (alternatively compound A) is about 20 mg BID. In another specific embodiment, the daily effective amount of the compound of formula (I) or a pharmaceutically acceptable salt, crystal form, solvate, or hydrate thereof (alternatively compound A) is about 40 mg QD. In another specific embodiment, the daily effective amount of the compound of formula (I) or a pharmaceutically acceptable salt, crystal form, solvate, or hydrate thereof (alternatively compound A) is about 40 mg BID. In another specific embodiment, the daily effective amount of the compound of formula (I) or a pharmaceutically acceptable salt, crystal form, solvate, or hydrate thereof (alternatively compound A) is about 80 mg QD. In another specific embodiment, the daily effective amount of the compound of formula (I) or a pharmaceutically acceptable salt, crystal form, solvate, or hydrate thereof (alternatively compound A) is about 80 mg BID. In another specific embodiment, the daily effective amount of the compound of formula (I) or a pharmaceutically acceptable salt, crystal form, solvate, or hydrate thereof (alternatively compound A) is about 160 mg QD. In another specific embodiment, the daily effective amount of the compound of formula (I) or a pharmaceutically acceptable salt, crystal form, solvate, or hydrate thereof (alternatively compound A) is about 240 mg QD.

In another specific embodiment, the compound of formula (I) or a pharmaceutically acceptable salt thereof is

administered until disease progression, intolerable toxicity, or death. In another specific embodiment, compound A or a pharmaceutically acceptable salt thereof is administered until disease progression, intolerable toxicity, or death.

Compound of formula (I) (BCR-ABL1 allosteric inhibitor)

The compound of formula (I) involved in the present disclosure is a potent and specific BCR-ABL1 TKI, which can selectively bind to the myristoyl pocket of BCR-ABL1 to mimic the role of the N-terminus in myristoylated ABL1, thereby restoring the negative regulatory function of BCR-ABL1 kinase activity. Due to the unique conformation of the myristoyl pocket, the compound of formula (I) involved in the present disclosure exhibits high selectivity only for ABL1 and possesses nanomolar-range activity against both unmutated BCR-ABL1 and all clinically observed ATP-site mutations (including T315I). For CML subjects who have failed treatment with multiple approved TKIs, the compound of formula (I) can achieve clinically meaningful remission in these subjects.

In some embodiments, the compound of formula (I) for use in the present disclosure also has any of the structures of formulas (Ia), (Ia'), (Ib), (Ib'), (Ic), (Ic'), (Id), and (Id'). Also described herein are pharmaceutically acceptable salts, stereoisomers, crystal forms, solvates, or hydrates of such compounds. Also provided are pharmaceutically acceptable compositions comprising at least one of the compounds or a pharmaceutically acceptable salt, stereoisomer, crystal form, solvate, or hydrate thereof.

In one embodiment, the present disclosure relates to a compound of formula (I), or a pharmaceutically acceptable salt, stereoisomer, crystal form, solvate, or hydrate thereof:

(I)

wherein:

$Y_1$ is independently selected from $CR_a$ or N;
Y is independently selected from $CR_a$ or N;
$R_1$ is selected from hydrogen, halogen, cyano, nitro, $C_{1-6}$ alkyl or $C_{1-6}$ haloalkyl, wherein the $C_{1-6}$ alkyl or $C_{1-6}$ haloalkyl is optionally substituted with an $R_{1a}$ group;
$R_2$ is selected from hydrogen, $C_{1-6}$ alkyl or $C_{1-6}$ haloalkyl, wherein the $C_{1-6}$ alkyl or $C_{1-6}$ haloalkyl is optionally substituted with an $R_{2a}$ group;
Z is a chemical bond, O, $S(O)_{0-2}$ or $NR_b$;
or, $-Z-R_2$ together represents $-SF_5$;
Ar is a six-membered heteroaryl containing at least one N atom, optionally substituted with R groups;
Het is

;

wherein $X_9$ is selected from O, S, $NR_b$ or $C(R)_2$;
m is 0, 1 or 2;
n is 0, 1, 2, 3, 4, 5 or 6;
$R_a$ is independently selected from hydrogen, halogen, cyano, nitro, hydroxy, $-NH_2$, $-NHC_{1-6}$ alkyl, $-N(C_{1-6}\text{ alkyl})_2$, $C_{1-6}$ alkyl, $C_{1-6}$ haloalkyl or $C_{1-6}$ alkoxy;
$R_b$ is independently selected from hydrogen, $C_{1-6}$ alkyl or $C_{1-6}$ haloalkyl;
$R_{1a}$, $R_{2a}$ and R are independently selected from hydrogen, halogen, hydroxy, $-NH_2$, $-NHC_{1-6}$ alkyl, $-N(C_{1-6}\text{ alkyl})_2$, $C_{1-6}$ alkyl, $C_{1-6}$ haloalkyl, $C_{1-6}$ alkoxy, $C_{3-7}$ cycloalkyl, $C_{3-7}$ heterocycloalkyl, $C_{6-10}$ aryl or $C_{5-10}$ heteroaryl;
alternatively, two R groups on the same atom or adjacent atoms may together form a $C_{3-7}$ cycloalkyl, $C_{3-7}$ heterocycloalkyl, $C_{6-10}$ aryl or $C_{5-10}$ heteroaryl.

In another embodiment, the present disclosure relates to the aforementioned compound, which is of formula (Ia)

and formula (Ia'), or a pharmaceutically acceptable salt, stereoisomer, crystal form, solvate, or hydrate thereof:

(Ia) or (Ia')

wherein Ar and Het are as defined herein.

In another specific embodiment, Ar is selected from the following groups that are optionally substituted by one, two, or three R groups:

, , , , , , , , , , or ,

wherein R is as defined herein.

In another specific embodiment, Ar is selected from the following groups that are optionally substituted by one, two, or three R groups:

, , , , , or ,

wherein R is as defined herein.

In another specific embodiment, Ar is selected from the following groups that are optionally substituted by one, two, or three R groups:

, ,

wherein R is as defined herein.

In another specific embodiment, Ar is

;

wherein $X_1$ is CR, $X_2$ to $X_4$ are independently selected from CR or N, and at least one of $X_2$, $X_3$, and $X_4$ is N, and R is as defined herein.

In another specific embodiment, Ar is selected from the following groups that are optionally substituted by one, two, or three R groups:

wherein R is as defined herein.

In another specific embodiment, Ar is selected from the following groups that are optionally substituted by one, two, or three R groups:

wherein R is as defined herein.

In another specific embodiment, Ar is

;

wherein $X_5$ to $X_8$ are independently selected from CR or N, and at least one of $X_5$ to $X_8$ is N, and R is as defined herein.

In another specific embodiment, Ar is selected from the following groups that are optionally substituted by one, two, or three R groups:

, , , , , , ,

, or ,

wherein R is as defined herein.

In another specific embodiment, Het is

;

wherein, $X_9$ is $C(R)_2$, and m, n and R as defined herein;

or, Het is selected from the following groups optionally substituted with one, two, three, or more R:

, or ,

where R is as defined herein;

or, Het is selected from:

, , , , , ,

, , , , ,

, , , , , ,

or .

In another specific embodiment, Het is

,

wherein, wherein one $X_9$ is selected from O, S or $NR_b$, and the optionally present other $X_9$ is $C(R)_2$, and m, n, R and $R_b$ are as defined herein;
or, Het is selected from the following groups:

, , , or .

In another embodiment, the present disclosure relates to the aforementioned compound, which is of formula (Ib) or formula (Ib'), or a pharmaceutically acceptable salt, stereoisomer, crystal form, solvate, or hydrate thereof:

(Ib) or (Ib')

wherein:

Ar is selected from the following groups that are optionally substituted by one, two, or three R groups:

, , , ,

, , , , , ,

or

;

R is selected from hydrogen, halogen, hydroxy, $-NH_2$, $-NHC_{1-6}$ alkyl or $-N(C_{1-6}\text{ alkyl})_2$.

In another embodiment, the present disclosure relates to the above compound, which is of formula (Ib) or formula (Ib'), or a pharmaceutically acceptable salt, stereoisomer, crystal form, solvate, or hydrate thereof:

(Ib) or (Ib')

wherein:

Ar is selected from the following groups that are optionally substituted by one, two, or three R groups:

, , , ,

, or ;

R is selected from hydrogen, halogen, hydroxy, $-NH_2$, $-NHC_{1-6}$ alkyl or $-N(C_{1-6}\text{ alkyl})_2$.

In another embodiment, the present disclosure relates to the above compound, which is of formula (Ib) or formula (Ib'), or a pharmaceutically acceptable salt, stereoisomer, crystal form, solvate, or hydrate thereof:

(Ib) or (Ib')

wherein:

Ar is selected from the following groups that are optionally substituted by one, two, or three R groups:

, , , ,

or ;

R is selected from hydrogen, halogen, hydroxy, $-NH_2$, $-NHC_{1-6}$ alkyl or $-N(C_{1-6}$ alkyl$)_2$.

In another embodiment, the present disclosure relates to the above compound, which is of formula (Ib) or formula (Ib'), or a pharmaceutically acceptable salt, stereoisomer, crystal form, solvate, or hydrate thereof:

(Ib) or (Ib')

wherein:

Ar is

,

$X_1$ to $X_4$ are as defined herein;
or, Ar is selected from the following groups that are optionally substituted by one, two, or three R groups:

, , , , , , , , , , , or

R is selected from hydrogen, halogen, hydroxy, $-NH_2$, $-NHC_{1-6}$ alkyl or $-N(C_{1-6}$ alkyl$)_2$.

In another embodiment, the present disclosure relates to the above compound, which is of formula (Ib) or formula (Ib'), or a pharmaceutically acceptable salt, stereoisomer, crystal form, solvate, or hydrate thereof:

(Ib) or (Ib')

wherein:

Ar is selected from the following groups that are optionally substituted by one, two, or three R groups:

, , , , , , or ,

R is selected from hydrogen, halogen, hydroxy, $-NH_2$, $-NHC_{1-6}$ alkyl or $-N(C_{1-6}$ alkyl$)_2$.

In another embodiment, the present disclosure relates to the above compound, which is of formula (Ib) or formula (Ib'), or a pharmaceutically acceptable salt, stereoisomer, crystal form, solvate, or hydrate thereof:

(Ib) or (Ib')

wherein:

Ar is selected from the following groups:

, or ,

R is selected from hydrogen, halogen, hydroxy, $-NH_2$, $-NHC_{1-6}$ alkyl or $-N(C_{1-6}$ alkyl$)_2$.

In another embodiment, the present disclosure relates to the above compound, which is of formula (Ib) or formula (Ib'), or a pharmaceutically acceptable salt, stereoisomer, crystal form, solvate, or hydrate thereof:

(Ib) or (Ib')

wherein:

Ar is selected from the following groups:

;

R is selected from hydrogen, halo or hydroxy.

In another embodiment, the present disclosure relates to the above compound, which is of formula (Ib) or formula (Ib'), or a pharmaceutically acceptable salt, stereoisomer, crystal form, solvate, or hydrate thereof:

(Ib) or (Ib')

wherein:

Ar is

,

wherein, $X_5$ to $X_8$ are as defined herein;
or, Ar is selected from the following groups that are optionally substituted by one, two, or three R groups:

, , , , , , ,

, or ,

R is selected from hydrogen, halogen, hydroxy, $-NH_2$, $-NHC_{1-6}$ alkyl or $-N(C_{1-6}$ alkyl$)_2$.

In another embodiment, the present disclosure relates to the aforementioned compound, which is of formula (Ic) or formula (Ic'), or a pharmaceutically acceptable salt, stereoisomer, crystal form, solvate, or hydrate thereof, wherein:

(Ic) or (Ic')

wherein:
Ar is selected from the following groups that are optionally substituted by one, two, or three R groups:

, , , ,

or

.

In another embodiment, the present disclosure relates to the aforementioned compound, which is of formula (Ic) or formula (Ic'), or a pharmaceutically acceptable salt, stereoisomer, crystal form, solvate, or hydrate thereof, wherein:

(Ic) or (Ic')

wherein:

Ar is selected from the following groups that are optionally substituted by one, two, or three R groups:

or .

In another embodiment, the present disclosure relates to the aforementioned compound, which is of formula (Ic) or formula (Ic'), or a pharmaceutically acceptable salt, stereoisomer, crystal form, solvate, or hydrate thereof, wherein:

(Ic) or (Ic')

wherein:

Ar is selected from the following groups that are optionally substituted by one, two, or three R groups:

or .

In another embodiment, the present disclosure relates to the aforementioned compound, which is of formula (Ic) or formula (Ic'), or a pharmaceutically acceptable salt, stereoisomer, crystal form, solvate, or hydrate thereof, wherein:

(Ic) or (Ic')

wherein:
Ar is selected from the following groups:

, , , , , , ,

, , , , , ,

or

.

In another embodiment, the present disclosure relates to the aforementioned compound, which is of formula (Ic) or formula (Ic'), or a pharmaceutically acceptable salt, stereoisomer, crystal form, solvate, or hydrate thereof, wherein:

(Ic) or (Ic')

wherein:
Ar is selected from the following groups:

, , , , , , or .

In another embodiment, the present disclosure relates to the aforementioned compound, which is of formula (Ic)

or formula (Ic'), or a pharmaceutically acceptable salt, stereoisomer, crystal form, solvate, or hydrate thereof, wherein:

(Ic) or (Ic')

wherein:
Ar is selected from the following groups:

, or .

In another embodiment, the present disclosure relates to the aforementioned compound, which is of formula (Ic) or formula (Ic'), or a pharmaceutically acceptable salt, stereoisomer, crystal form, solvate, or hydrate thereof, wherein:

(Ic) or (Ic')

wherein:
Ar is selected from the following groups:

or .

In another embodiment, the present disclosure relates to the aforementioned compound, which is of formula (Id) or formula (Id'), or a pharmaceutically acceptable salt, stereoisomer, crystal form, solvate, or hydrate thereof, wherein:

(Id) or (Id')

wherein:
Ar is selected from the following groups that are optionally substituted by one, two, or three R groups:

, , , ,

or

In another embodiment, the present disclosure relates to the aforementioned compound, which is of formula (Id) or formula (Id'), or a pharmaceutically acceptable salt, stereoisomer, crystal form, solvate, or hydrate thereof, wherein:

(Id) or (Id')

wherein:
Ar is selected from the following groups that are optionally substituted by one, two, or three R groups:

or

In another embodiment, the present disclosure relates to the aforementioned compound, which is of formula (Id) or formula (Id'), or a pharmaceutically acceptable salt, stereoisomer, crystal form, solvate, or hydrate thereof, wherein:

(Id) or (Id')

wherein:
Ar is selected from the following groups that are optionally substituted by one, two, or three R groups:

or .

In another embodiment, the present disclosure relates to the aforementioned compound, which is of formula (Id) or formula (Id'), or a pharmaceutically acceptable salt, stereoisomer, crystal form, solvate, or hydrate thereof, wherein:

(Id) or (Id')

wherein:
Ar is selected from the following groups:

, , , , , , , , , , , , ,

or

.

In another embodiment, the present disclosure relates to the aforementioned compound, which is of formula (Id) or formula (Id'), or a pharmaceutically acceptable salt, stereoisomer, crystal form, solvate, or hydrate thereof, wherein:

(Id) or (Id')

wherein:
Ar is selected from the following groups:

, , , , , , or .

In another embodiment, the present disclosure relates to the aforementioned compound, which is of formula (Id)

or formula (Id'), or a pharmaceutically acceptable salt, stereoisomer, crystal form, solvate, or hydrate thereof, wherein:

(Id) or (Id')

wherein:
Ar is selected from the following groups:

, or .

In another embodiment, the present disclosure relates to the aforementioned compound, which is of formula (Id) or formula (Id'), or a pharmaceutically acceptable salt, stereoisomer, crystal form, solvate, or hydrate thereof, wherein:

(Id) or (Id')

wherein:

Ar is selected from the following groups:

or .

$Y_1$ and Y

In one specific embodiment, one or two $Y_1$ are N; In another specific embodiment, one or two $Y_1$ are $CR_a$; In another specific embodiment, one or two $Y_1$ are CH; In another specific embodiment, one $Y_1$ is N and the other $Y_1$ is CH.

In one specific embodiment, one or two Y are $CR_a$; in another specific embodiment, one or two Y are N; in another specific embodiment, one or two Y are CH; in another specific embodiment, one Y is N and the other Y is CH.

$R_1$

In one specific embodiment, $R_1$ is selected from hydrogen, halo, cyano, nitro, $C_{1-6}$ alkyl or $C_{1-6}$ haloalkyl; in another specific embodiment, $R_1$ is selected from hydrogen, halo, cyano, nitro or $C_{1-6}$ alkyl; in another specific embodiment, $R_1$ is selected from hydrogen or halo; in another specific embodiment, $R_1$ is hydrogen; in another specific embodiment, $R_1$ is halo (F, Cl, Br or I); in another specific embodiment, $R_1$ is cyano; in another specific embodiment, $R_1$ is nitro; in another specific embodiment, $R_1$ is $C_{1-6}$ alkyl (methyl, ethyl, propyl, isopropyl, butyl, iso-butyl, tert-butyl, pentyl, iso-pentyl, hexyl, and the like); in another specific embodiment, $R_1$ is $C_{1-6}$ haloalkyl ($-CF_3$, $-CH_2F$, $-CHF_2$, $-CClF_2$, $-CHFCH_2F$, $-CH_2CHF_2$, $-CF_2CF_3$, - $CF_2CClF_2$, $-CF_2CH_3$, $-CC1_3$, $-CH_2Cl$, $-CHCl_2$, 2,2,2-trifluoro-1,1-dimethyl-ethyl, and the like).

$R_2$ and Z

In one specific embodiment, $R_2$ is selected from hydrogen, $C_{1-6}$ alkyl or $C_{1-6}$ haloalkyl; in another specific embodiment, $R_2$ is selected from $C_{1-6}$ alkyl or $C_{1-6}$ haloalkyl; in another specific embodiment, $R_2$ is hydrogen; in another specific embodiment, $R_2$ is $C_{1-6}$ alkyl (methyl, ethyl, propyl, isopropyl, butyl, iso-butyl, tert-butyl, pentyl, iso-pentyl, hexyl, and the like); in another specific embodiment, $R_2$ is $C_{1-6}$ haloalkyl ($-CF_3$, $-CH_2F$, $-CHF_2$, $-CClF_2$, $-CHFCH_2F$, $-CH_2CHF_2$, $-CF_2CF_3$, $-CF_2CClF_2$, $-CF_2CH_3$, $-CCl_3$, $-CH_2Cl$, $-CHCl_2$, 2,2,2-trifluoro-1,1-dimethyl-ethyl, and the like).

In one specific embodiment, Z is a chemical bond; in another specific embodiment, Z is O; in another specific embodiment, Z is $S(O)_{0-2}$; in another specific embodiment, Z is $NR_b$; in another specific embodiment, Z is NH.

In one specific embodiment, $-Z-R_2$ together form $-SF_5$.

Ar, $X_1$ to $X_8$

In one specific embodiment, Ar is a six-membered heteroaryl containing at least one N atom, optionally substituted with R groups.

In a more specific embodiment, Ar is selected from the following groups that are optionally substituted by one, two, or three R groups:

wherein R is as defined herein.

In a more specific embodiment, Ar is selected from the following groups that are optionally substituted by one, two, or three R groups:

wherein R is as defined herein.

In a more specific embodiment, Ar is selected from the following groups that are optionally substituted by one, two, or three R groups:

wherein R is as defined herein.

In one specific embodiment, Ar is

wherein $X_1$ to $X_4$ are independently selected from CR or N, and at least one of $X_1$, $X_2$, $X_3$ and $X_4$ is N; in another specific embodiment, Ar is

wherein $X_5$ to $X_8$ are independently selected from CR or N, and at least one of $X_5$, $X_6$, $X_7$ and $X_8$ is N.

In the above specific embodiment regarding Ar, $X_1$ is CR; in another specific embodiment, $X_1$ is CH; in another specific embodiment, $X_1$ is N. In the above specific embodiment regarding Ar, $X_2$ is CR; in another specific embodiment, $X_2$ is CH; in another specific embodiment, $X_2$ is N. In the above specific embodiment regarding Ar, $X_3$ is CR; in another specific embodiment, $X_3$ is CH; in another specific embodiment, $X_3$ is N. In the above specific embodiment regarding Ar, $X_4$ is CR; in another specific embodiment, $X_4$ is CH; in another specific embodiment, $X_4$ is N. In the above specific embodiment regarding Ar, $X_5$ is CR; in another specific embodiment, $X_5$ is CH; in another specific embodiment, $X_5$ is N. In the above specific embodiment regarding Ar, $X_6$ is CR; in another specific embodiment, $X_6$ is CH; in another specific embodiment, $X_6$ is N. In the above specific embodiment regarding Ar, $X_7$ is CR; in another specific embodiment, $X_7$ is CH; in another specific embodiment, $X_7$ is N. In the above specific embodiment regarding Ar, $X_8$ is CR; in another specific embodiment, $X_8$ is CH; in another specific embodiment, $X_8$ is N.

In more specific embodiment, Ar is selected from the following groups that are optionally substituted by one, two or three R:

wherein, R is as defined herein.

In more specific embodiment, Ar is selected from the following groups that are optionally substituted by one, two or three R:

wherein, R is as defined herein.

Het, $X_9$, m and n

In one specific embodiment, Het is

In the above specific embodiments regarding Het, $X_9$ is O; In another specific embodiment, $X_9$ is S; In another specific embodiment, $X_9$ is $NR_b$; In another specific embodiment, $X_9$ is $C(R)_2$. In the above specific embodiments regarding Het, m is 0; In another specific embodiment, m is 1; In another specific embodiment, m is 2. In the above specific embodiments regarding Het, n is 0; In another specific embodiment, n is 1; In another specific embodiment, n is 2; In another specific embodiment, n is 3; In another specific embodiment, n is 4; In another specific embodiment, n is 5; In another specific embodiment, n is 6.

In more specific embodiment, Het is

wherein, $X_9$ is $C(R)_2$. In more specific embodiment, Het is selected from the following groups that are optionally substituted by one, two, three or more R:

In more specific embodiment, Het is selected from the following groups:

, , , , , , or ;

In more specific embodiment, Het is

,

wherein, one $X_9$ is selected from O, S or $NR_b$, and the optionally present other $X_9$ is $C(R)_2$; In more specific embodiment, Het is selected from the following groups:

, , , or .

$R_a$, $R_b$, $R_{1a}$, $R_{2a}$ and R

In one specific embodiment, $R_a$ is independently selected from hydrogen, halo, cyano, nitro, hydroxy, $-NH_2$, $-NHC_{1-6}$ alkyl, $-N(C_{1-6}$ alkyl$)_2$, $C_{1-6}$ alkyl, $C_{1-6}$ haloalkyl or $C_{1-6}$ alkoxyl; in another specific embodiment, $R_a$ is hydrogen; in another specific embodiment, $R_a$ is halo; in another specific embodiment, $R_a$ is cyano; in another specific embodiment, $R_a$ is nitro; in another specific embodiment, $R_a$ is hydroxy; in another specific embodiment, $R_a$ is $-NH_2$; in another specific embodiment, $R_a$ is $-NHC_{1-6}$ alkyl; in another specific embodiment, $R_a$ is $-N(C_{1-6}$ alkyl$)_2$; in another specific embodiment, $R_a$ is $C_{1-6}$ alkyl; in another specific embodiment, $R_a$ is $C_{1-6}$ haloalkyl; in another specific embodiment, $R_a$ is $C_{1-6}$ alkoxyl.

In one specific embodiment, $R_b$ is independently selected from hydrogen, $C_{1-6}$ alkyl or $C_{1-6}$ haloalkyl; in another specific embodiment, $R_b$ is hydrogen; in another specific embodiment, $R_b$ is $C_{1-6}$ alkyl; in another specific embodiment, $R_b$ is $C_{1-6}$ haloalkyl.

In one specific embodiment, $R_{1a}$, $R_{2a}$ and R are independently selected from hydrogen, halo, hydroxy, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxyl, $C_{3-7}$ cycloalkyl, $C_{3-7}$ heterocycloalkyl, $C_{6-10}$ aryl or $C_{5-10}$ heteroaryl; in another specific embodiment, $R_{1a}$, $R_{2a}$ and R are hydrogen; in another specific embodiment, $R_{1a}$, $R_{2a}$ and R are halo; in another specific embodiment, $R_{1a}$, $R_{2a}$ and R are hydroxy; in another specific embodiment, $R_{1a}$, $R_{2a}$ and R are $-NH_2$; in another specific embodiment, $R_{1a}$, $R_{2a}$ and R are - $NHC_{1-6}$ alkyl; in another specific embodiment, $R_{1a}$, $R_{2a}$ and R are $-N(C_{1-6}$ alkyl$)_2$; in another specific embodiment, $R_{1a}$, $R_{2a}$ and R are $C_{1-6}$ alkyl; in another specific embodiment, $R_{1a}$, $R_{2a}$ and R are $C_{1-6}$ haloalkyl; in another specific embodiment, $R_{1a}$, $R_{2a}$ and R are $C_{1-6}$ alkoxyl; in another specific embodiment, $R_{1a}$, $R_{2a}$ and R are $C_{3-7}$ cycloalkyl; in another specific embodiment, $R_{1a}$, $R_{2a}$ and R are $C_{3-7}$ heterocycloalkyl; in another specific embodiment, $R_{1a}$, $R_{2a}$ and R are $C_{6-10}$ aryl; in another specific embodiment, $R_{1a}$, $R_{2a}$ and R are $C_{5-10}$ heteroaryl.

In one specific embodiment, two R groups on the same or adjacent atoms may together form $C_{3-7}$ cycloalkyl, or $C_{3-7}$ heterocycloalkyl; in another specific embodiment, two R groups on the same or adjacent atoms may together form $C_{3-7}$ cycloalkyl; in another specific embodiment, two R groups on the same or adjacent atoms may together form $C_{3-7}$ heterocycloalkyl.

Any technical solution in any one of the above specific embodiments, or any combination thereof, may be combined with any technical solution in other specific embodiments or any combination thereof. For example, any technical solution of $Y_1$ or any combination thereof may be combined with any technical solution of Y, $Y_1$, $R_1$, $R_2$, Z, Ar, $X_1$ to $X_8$, Het, m, n, $R_a$, $R_b$, $R_{1a}$, $R_{2a}$ and R or any combination thereof. The present disclosure is intended to include all

combination of such technical solutions, which are not exhaustively listed here to save space.

In some embodiments, the compounds disclosed herein are selected from the following compounds:

, ,

, ,

, ,

, ,

, ,

, ,

, ,

,

,

,

,

,

,

,

,

,

,

,

,

or a pharmaceutically acceptable salt, stereoisomer, crystal form, solvate, or hydrate thereof;

The preparation of some of these compounds can be found in WO2021/213380A1, WO2013/171640A1, WO2021/143927A1, and the Chinese invention patent application with application number 202311337298.0. The entire contents of these four patents are incorporated herein by reference for all purposes.

The compounds involved in the present disclosure may contain one or more asymmetric centers and thus may exist in various stereoisomeric forms, such as enantiomers and/or diastereomers. For example, the compounds involved in the present disclosure may be individual enantiomers, diastereomers, or geometric isomers (e.g., cis and trans isomers), or may be in the form of mixtures of stereoisomers, including racemic mixtures and mixtures enriched with one or more stereoisomers. Isomers can be separated from mixtures using methods known to those skilled in the art, including chiral high-performance liquid chromatography (HPLC) and the formation and crystallization of chiral salts; alternatively, alternative isomers can be prepared by asymmetric synthesis.

Those skilled in the art will understand that many organic compounds can form complexes with solvents in which they react or from which they precipitate or crystallize. These complexes are referred to as "solvates." When the solvent is water, the complex is called a "hydrate." The present disclosure encompasses all solvates of the compounds involved in the present disclosure.

In other embodiments, the compound involved in the present disclosure is (R)-N-(4-(chlorodifluoromethoxy) phenyl)-6-(3-fluoropyrrolidin-1-yl)-5-(pyrazin-2-yl)nicotinamide (Compound A) or a pharmaceutically acceptable salt, stereoisomer, crystal form, solvate, or hydrate thereof:

Compound A

Compound A and its preparation were first disclosed in WO/2018/133827A1, and subsequently, its salt forms, crystal forms, preparation, and pharmaceutical compositions were disclosed in WO2021/213380A1. The entire contents of these two patents are incorporated herein by reference for all purposes.

In one embodiment, Compound A, in an X-ray powder diffraction pattern obtained using CuK$\alpha$ radiation, includes at least characteristic peaks represented by the following °2$\theta$ values: 11.9$\pm$0.2, 20.5$\pm$0.2, 23.1$\pm$0.2, 23.9$\pm$0.2 and 24.8 $\pm$0.2. In one specific embodiment, in the X-ray powder diffraction pattern, Compound A further includes characteristic peaks represented by the following °2$\theta$ values: 9.7$\pm$0.2, 16.1$\pm$0.2, 19.3$\pm$0.2 and 21.2$\pm$0.2. In one specific embodiment, in the X-ray powder diffraction pattern, Compound A further includes characteristic peaks represented by the following °2$\theta$ values: 5.9+0.2, 11.0+0.2, 12.8+0.2, 14.7+0.2, 16.6+0.2, 17.8+0.2, 18.2+0.2, 18.6+0.2, 20.1+0.2, 22.0+0.2, 22.6+0.2, 26.2+0.2 and 29.0+0.2.

ATP-competitive BCR-ABL1 inhibitor

The terms "ATP-competitive BCR-ABL1 inhibitor," "BCR-ABL1 orthosteric inhibitor," and "BCR-ABL1 catalytic inhibitor" are used interchangeably, defined as binding to the ATP-binding site "hinge region," inhibiting the autophosphorylation and substrate phosphorylation of the BCR-ABL1 kinase, thereby suppressing the proliferation of cancer cells and tumor formation.

In one specific embodiment, the ATP-competitive BCR-ABL1 inhibitor(s) is selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228. In another specific embodiment, the ATP-competitive BCR-ABL1 inhibitor is imatinib. In another specific embodiment, the ATP-competitive BCR-ABL1 inhibitor is dasatinib. In another specific embodiment, the ATP-competitive BCR-ABL1

inhibitor is nilotinib. In another specific embodiment, the ATP-competitive BCR-ABL1 inhibitor is bosutinib. In another specific embodiment, the ATP-competitive BCR-ABL1 inhibitor is flumatinib. In another specific embodiment, the ATP-competitive BCR-ABL1 inhibitor is ponatinib. In another specific embodiment, the ATP-competitive BCR-ABL1 inhibitor is olverembatinib. In another specific embodiment, the ATP-competitive BCR-ABL1 inhibitor is radotinib. In another specific embodiment, the ATP-competitive BCR-ABL1 inhibitor is bafetinib. In another specific embodiment, the ATP-competitive BCR-ABL1 inhibitor is vodobatinib. In another specific embodiment, the ATP-competitive BCR-ABL1 inhibitor is CHMFL-ABL-039. In another specific embodiment, the ATP-competitive BCR-ABL1 inhibitor is CHMFL-ABL-053. In another specific embodiment, the ATP-competitive BCR-ABL1 inhibitor is CHMFL-ABL-KIT-155. In another specific embodiment, the ATP-competitive BCR-ABL1 inhibitor is PF-114. In another specific embodiment, the ATP-competitive BCR-ABL1 inhibitor is pacritinib. In another specific embodiment, the ATP-competitive BCR-ABL1 inhibitor is rebasinib. In another specific embodiment, the ATP-competitive BCR-ABL1 inhibitor is danusertib. In another specific embodiment, the ATP-competitive BCR-ABL1 inhibitor is tozasertib. In another specific embodiment, the ATP-competitive BCR-ABL1 inhibitor is AT9283. In another specific embodiment, the ATP-competitive BCR-ABL1 inhibitor is KW-2449. In another specific embodiment, the ATP-competitive BCR-ABL1 inhibitor is XL228. In another specific embodiment, the ATP-competitive BCR-ABL1 inhibitor is selected from imatinib, dasatinib, nilotinib, bosutinib, flumatinib, ponatinib, olverembatinib or PF-114. In another specific embodiment, the ATP-competitive BCR-ABL1 inhibitor is selected from imatinib, nilotinib, radotinib, flumatinib, ponatinib, bafetinib, CHMFL-ABL-039, CHMFL-ABL-053, CHMFL-ABL-KIT-155, vodobatinib, PF-114, olverembatinib or rebastinib. In another specific embodiment, the ATP-competitive BCR-ABL1 inhibitor is selected from ponatinib, PF-114, olverembatinib or rebastinib. In another specific embodiment, the ATP-competitive BCR-ABL1 inhibitor is selected from ponatinib, olverembatinib or PF-114. In another specific embodiment, the ATP-competitive BCR-ABL1 inhibitor is selected from ponatinib or olverembatinib.

Specifically, the structures of the aforementioned ATP-competitive BCR-ABL1 inhibitors are as follows:

| Inhibitor | Structure | Inhibitor | Structure |
|---|---|---|---|
| imatinib | | dasatinib | |
| nilotinib | | bosutinib | |
| flumatinib | | ponatinib | |

(continued)

| Inhibitor | Structure | Inhibitor | Structure |
|---|---|---|---|
| olverembatinib | | radotinib | |
| bafetinib | | vodobatinib | |
| CHMFL-ABL-039 | | CHMFL-ABL-053 | |
| CHMFL-ABL-KIT-155 | | PF-114 | |
| pacritinib | | rebasinib | |
| danusertib | | tozasertib | |

(continued)

| Inhibitor | Structure | Inhibitor | Structure |
|---|---|---|---|
| AT9283 | | KW-2449 | |
| XL228 | | asciminib | |

HS-10382, also known as TERN-701, is an allosteric BCR-ABL tyrosine kinase inhibitor developed by Terns Pharmaceuticals.

It is understood that references to component (a) and component (b) also include pharmaceutically acceptable salts, crystal forms, solvates, or hydrates of any active substance.

Pharmaceutical Composition

In one aspect, the present disclosure provides the use and method of a pharmaceutical composition for treating CML in chronic phase, wherein the pharmaceutical composition comprises a compound of formula (I) or a pharmaceutically acceptable salt, stereoisomer, crystal form, solvate, or hydrate thereof, and a pharmaceutically acceptable excipient.

In another aspect, the present disclosure provides the use and method of a pharmaceutical composition for treating CML in accelerated phase, wherein the pharmaceutical composition comprises a compound of formula (I) or a pharmaceutically acceptable salt, stereoisomer, crystal form, solvate, or hydrate thereof, and a pharmaceutically acceptable excipient.

In some embodiments, the pharmaceutical composition of the present disclosure comprises (i) a pharmaceutically active ingredient: a compound of formula (I) or a pharmaceutically acceptable salt, stereoisomer, solvate, or hydrate thereof, (ii) a diluent, (iii) a disintegrant, (iv) a glidant, and (v) a lubricant.

In one specific embodiment, the pharmaceutical composition of the present disclosure comprises (i) a pharmaceutically active ingredient: a crystal form of compound A free base or a crystal form of a pharmaceutically acceptable salt thereof, (ii) a diluent, (iii) a disintegrant, (iv) a glidant, and (v) a lubricant.

In another specific embodiment, the present disclosure provides the pharmaceutical composition described above, wherein the pharmaceutically active ingredient accounts for 1-30% by weight of the total weight of the pharmaceutical composition, alternatively 5-20%, yet alternatively 8-15%, and still alternatively about 10%, calculated based on the weight of the compound free base; optionally, wherein the content of the pharmaceutically active ingredient in a unit dose is 1-100 mg, alternatively 5-50 mg, yet alternatively 8-40 mg, and still alternatively about 10, 20, 30, or 40 mg.

In another specific embodiment, the present disclosure provides the pharmaceutical composition described above, wherein the diluent accounts for 65-95% by weight of the total weight of the pharmaceutical composition, alternatively 70-90%, yet alternatively about 80%, 81%, 82%, 83%, 84%, or 85%; optionally, wherein the content of the diluent in a unit dose is 65-380 mg, alternatively 70-360 mg, yet alternatively 80-350 mg, for example, about 83 mg or 332 mg.

In another specific embodiment, the present disclosure provides the pharmaceutical composition described above, wherein the diluent is selected from lactose monohydrate, microcrystalline cellulose, anhydrous dibasic calcium phosphate, mannitol, and pregelatinized starch, as well as mixtures thereof; alternatively, the microcrystalline cellulose is

microcrystalline cellulose 102; the lactose monohydrate is selected from lactose monohydrate Granulac 200, lactose monohydrate Tablettose 80, and lactose monohydrate FLOWLAC® 100; alternatively, when both lactose monohydrate and microcrystalline cellulose are present, the weight ratio of lactose monohydrate to microcrystalline cellulose is 5:1 to 1:5, alternatively 2:1 to 1:3, yet alternatively about 1:2, for example, 1:1.96.

In another specific embodiment, the present disclosure provides the aforementioned pharmaceutical composition, wherein the disintegrant accounts for 1-5% by weight of the total weight of the pharmaceutical composition, alternatively 2-4%, yet alternatively about 3%; optionally, wherein the content of the disintegrant in a unit dose is 1-20 mg, alternatively 2-16 mg, yet alternatively about 3, 6, 9, or 12 mg.

In another specific embodiment, the present disclosure provides the aforementioned pharmaceutical composition, wherein the disintegrant is croscarmellose sodium.

In another specific embodiment, the present disclosure provides the aforementioned pharmaceutical composition, wherein the glidant accounts for 1-5% by weight of the total weight of the pharmaceutical composition, alternatively 2-4%, yet alternatively about 3%; optionally, wherein the content of the glidant in a unit dose is 1-20 mg, alternatively 2-16 mg, yet alternatively about 3, 6, 9, or 12 mg.

In another specific embodiment, the present disclosure provides the aforementioned pharmaceutical composition, wherein the glidant is colloidal silicon dioxide.

In another specific embodiment, the present disclosure provides the aforementioned pharmaceutical composition, wherein the lubricant accounts for 0.1-5% by weight of the total weight of the pharmaceutical composition, alternatively 0.5-2%, alternatively about 1%; optionally, wherein the content of the lubricant in a unit dose is 0.1-20 mg, alternatively 0.5-8 mg, alternatively about 1, 2, 3, or 4 mg.

In another specific embodiment, the present disclosure provides the aforementioned pharmaceutical composition, wherein the lubricant is magnesium stearate or sodium stearyl fumarate.

In another specific embodiment, the present disclosure provides the aforementioned pharmaceutical composition, comprising the following components:

(i) 1-30% by weight of compound A,
(ii) 70-90% by weight of lactose monohydrate and microcrystalline cellulose (weight ratio 1:2),
(iii) 2-4% by weight of croscarmellose sodium,
(iv) 2-4% by weight of colloidal silicon dioxide, and
(v) 0.1-5% by weight of magnesium stearate.

In a more specific embodiment, Compound A in the aforementioned pharmaceutical composition has an X-ray powder diffraction pattern obtained using CuKα radiation, which includes at least characteristic peaks represented by the following °2θ: 11.9±0.2, 20.5±0.2, 23.1±0.2, 23.9±0.2 and 24.8±0.2. In one specific embodiment, the X-ray powder diffraction pattern further includes characteristic peaks represented by the following °2θ: 9.7±0.2, 16.1±0.2, 19.3±0.2 and 21.2±0.2. In a specific embodiment, the X-ray powder diffraction pattern further includes characteristic peaks represented by the following °2θ: 5.9±0.2, 11.0±0.2, 12.8±0.2, 14.7±0.2, 16.6±0.2, 17.8±0.2, 18.2±0.2, 18.6±0.2, 20.1±0.2, 22.0±0.2, 22.6±0.2, 26.2±0.2 and 29.0±0.2.

In another specific embodiment, the present disclosure provides the aforementioned pharmaceutical composition, wherein a unit dose comprises the following components:

(i) about 10 mg of Compound A,
(ii) about 28 mg of lactose monohydrate and about 55 mg of microcrystalline cellulose,
(iii) about 3 mg of croscarmellose sodium,
(iv) about 3 mg of colloidal silicon dioxide, and
(v) about 1 mg of magnesium stearate.

In a more specific embodiment, Compound A in the aforementioned pharmaceutical composition has an X-ray powder diffraction pattern obtained using CuKα radiation, which includes at least characteristic peaks represented by the following °2θ values: 11.9±0.2, 20.5±0.2, 23.1±0.2, 23.9±0.2 and 24.8±0.2. In one specific embodiment, the X-ray powder diffraction pattern further includes characteristic peaks represented by the following °2θ values: 9.7±0.2, 16.1 ±0.2, 19.3±0.2 and 21.2±0.2. In one specific embodiment, the X-ray powder diffraction pattern further includes characteristic peaks represented by the following °2θ values: 5.9±0.2, 11.0±0.2, 12.8±0.2, 14.7±0.2, 16.6±0.2, 17.8±0.2, 18.2±0.2, 18.6±0.2, 20.1±0.2, 22.0±0.2, 22.6±0.2, 26.2±0.2 and 29.0±0.2.

In another specific embodiment, the present disclosure provides the pharmaceutical composition described above, wherein the unit dose comprises the following components:

(i) about 40 mg of compound A,

(ii) about 112 mg of lactose monohydrate and about 220 mg of microcrystalline cellulose,
(iii) about 12 mg of croscarmellose sodium,
(iv) about 12 mg of colloidal silicon dioxide, and
(v) about 4 mg of magnesium stearate.

In a more specific embodiment, Compound A in the aforementioned pharmaceutical composition has an X-ray powder diffraction pattern obtained using CuKα radiation, which includes at least characteristic peaks represented by the following °2θ: 11.9±0.2, 20.5±0.2, 23.1±0.2, 23.9±0.2 and 24.8±0.2. In one specific embodiment, the X-ray powder diffraction pattern further includes characteristic peaks represented by the following °2θ values: 9.7±0.2, 16.1±0.2, 19.3 ±0.2 and 21.2±0.2. In one specific embodiment, the X-ray powder diffraction pattern further includes characteristic peaks represented by the following °2θ values: 5.9±0.2, 11.0±0.2, 12.8±0.2, 14.7±0.2, 16.6±0.2, 17.8±0.2, 18.2±0.2, 18.6 ±0.2, 20.1±0.2, 22.0±0.2, 22.6±0.2, 26.2±0.2 and 29.0±0.2.

The pharmaceutical compositions provided herein can be administered via various routes, including but not limited to oral (enteral) administration, parenteral (by injection) administration, rectal administration, transdermal administration, intradermal administration, intrathecal administration, subcutaneous (SC) administration, intravenous (IV) administration, intramuscular (IM) administration, and intranasal administration. In one embodiment, the pharmaceutical compositions disclosed herein are administered orally.

The pharmaceutical compositions provided herein can also be administered over an extended period ("long-term administration"). Long-term administration refers to the administration of the compound or its pharmaceutical composition over an extended period, for example, within 3 months, 6 months, 1 year, 2 years, 3 years, 5 years, etc., or may continue indefinitely, such as for the remainder of the subject's life. In one embodiment, long-term administration aims to provide a constant level of the compound in the blood, for example, within the therapeutic window over an extended period.

The pharmaceutical compositions provided herein can be presented in unit dosage forms to facilitate precise administration. The term "unit dosage form" refers to a physically discrete unit suitable as a unit dose for human subjects and other mammals, each unit containing a predetermined amount of active material, which is calculated to produce the desired therapeutic effect in combination with suitable pharmaceutical excipients. Typical unit dosage forms include prefilled, premeasured ampoules or syringes for liquid compositions, or in the case of solid compositions, pills, tablets, capsules, etc.

In another embodiment, the pharmaceutical compositions provided herein are administered to a subject in a solid dosage form. In a specific embodiment, the solid dosage form is a tablet.

In another embodiment, the compounds provided herein may be administered as a single active agent, or they may be administered in combination with other active agents.

Pharmaceutical Combinations

The present disclosure relates to the following pharmaceutical combinations:

(a) a compound of formula (I) or a pharmaceutically acceptable salt, crystal form, solvate, or hydrate thereof; and
(b) an ATP-competitive BCR-ABL1 inhibitor, or a pharmaceutically acceptable salt, crystal form, solvate, or hydrate thereof.

In one specific embodiment, the ATP-competitive BCR-ABL1 inhibitor(s) is selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228. In another specific embodiment, the ATP-competitive BCR-ABL1 inhibitor is imatinib. In another specific embodiment, the ATP-competitive BCR-ABL1 inhibitor is dasatinib. In another specific embodiment, the ATP-competitive BCR-ABL1 inhibitor is nilotinib. In another specific embodiment, the ATP-competitive BCR-ABL1 inhibitor is bosutinib. In another specific embodiment, the ATP-competitive BCR-ABL1 inhibitor is flumatinib. In another specific embodiment, the ATP-competitive BCR-ABL1 inhibitor is ponatinib. In another specific embodiment, the ATP-competitive BCR-ABL1 inhibitor is olverembatinib. In another specific embodiment, the ATP-competitive BCR-ABL1 inhibitor is radotinib. In another specific embodiment, the ATP-competitive BCR-ABL1 inhibitor is bafetinib. In another specific embodiment, the ATP-competitive BCR-ABL1 inhibitor is vodobatinib. In another specific embodiment, the ATP-competitive BCR-ABL1 inhibitor is CHMFL-ABL-039. In another specific embodiment, the ATP-competitive BCR-ABL1 inhibitor is CHMFL-ABL-053. In another specific embodiment, the ATP-competitive BCR-ABL1 inhibitor is CHMFL-ABL-KIT-155. In another specific embodiment, the ATP-competitive BCR-ABL1 inhibitor is PF-114. In another specific embodiment, the ATP-competitive BCR-ABL1 inhibitor is pacritinib. In another specific embodiment, the ATP-competitive BCR-ABL1 inhibitor is rebasinib. In another specific embodiment, the ATP-competitive BCR-ABL1 inhibitor is danusertib. In another specific embodiment, the ATP-competitive BCR-ABL1 inhibitor is tozasertib. In another specific embodiment, the ATP-competitive BCR-ABL1 inhibitor is

AT9283. In another specific embodiment, the ATP-competitive BCR-ABL1 inhibitor is KW-2449. In another specific embodiment, the ATP-competitive BCR-ABL1 inhibitor is XL228.

In another specific embodiment, the ATP-competitive BCR-ABL1 inhibitor is selected from imatinib, dasatinib, nilotinib, bosutinib, flumatinib, ponatinib, olverembatinib or PF-114. In another specific embodiment, the ATP-competitive BCR-ABL1 inhibitor is selected from imatinib, nilotinib, radotinib, flumatinib, ponatinib, bafetinib, CHMFL-ABL-039, CHMFL-ABL-053, CHMFL-ABL-KIT-155, vodobatinib, PF-114, olverembatinib or rebastinib. In another specific embodiment, the ATP-competitive BCR-ABL1 inhibitor is selected from ponatinib, PF-114, olverembatinib or rebastinib. In another specific embodiment, the ATP-competitive BCR-ABL1 inhibitor is selected from ponatinib, olverembatinib or PF-114. In another specific embodiment, the ATP-competitive BCR-ABL1 inhibitor is selected from ponatinib or olverembatinib.

In an alternative embodiment, the present disclosure relates to the following pharmaceutical combination:

(a) a compound of formula (I) or a pharmaceutically acceptable salt, crystal form, solvate, or hydrate thereof; and
(b) One or more ATP-competitive BCR-ABL1 inhibitors selected from: imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228, or a pharmaceutically acceptable salt, crystal form, solvate, or hydrate thereof.

In another alternative embodiment, the present disclosure relates to the following pharmaceutical combination:

(a) a compound of formula (I) or a pharmaceutically acceptable salt, crystal form, solvate, or hydrate thereof; and
(b) one or more ATP-competitive BCR-ABL1 inhibitors selected from: imatinib, dasatinib, nilotinib, bosutinib, flumatinib, ponatinib, olverembatinib or PF-114, or a pharmaceutically acceptable salt, crystal form, solvate, or hydrate thereof.

In another alternative embodiment, the present disclosure relates to the following pharmaceutical combination:

(a) a compound of formula (I) or a pharmaceutically acceptable salt, crystal form, solvate, or hydrate thereof; and
(b) one or more ATP-competitive BCR-ABL1 inhibitors selected from: imatinib, nilotinib, radotinib, flumatinib, ponatinib, bafetinib, CHMFL-ABL-039, CHMFL-ABL-053, CHMFL-ABL-KIT-155, vodobatinib, PF-114, olverembatinib or rebastinib, or a pharmaceutically acceptable salt, crystal form, solvate, or hydrate thereof.

In another alternative embodiment, the present disclosure relates to the following pharmaceutical combination:

(a) a compound of formula (I) or a pharmaceutically acceptable salt, crystal form, solvate, or hydrate thereof; and
(b) one or more ATP-competitive BCR-ABL1 inhibitors selected from: ponatinib, PF-114, olverembatinib or rebastinib, or a pharmaceutically acceptable salt, crystal form, solvate, or hydrate thereof.

In another alternative embodiment, the present disclosure relates to the following pharmaceutical combination:

(a) a compound of formula (I) or a pharmaceutically acceptable salt, crystal form, solvate, or hydrate thereof; and
(b) one or more ATP-competitive BCR-ABL1 inhibitors selected from: ponatinib, olverembatinib or PF-114, or a pharmaceutically acceptable salt, crystal form, solvate, or hydrate thereof.

In another alternative embodiment, the present disclosure relates to the following pharmaceutical combination:

(a) a compound of formula (I) or a pharmaceutically acceptable salt, crystal form, solvate, or hydrate thereof; and
(b) one or more ATP-competitive BCR-ABL1 inhibitors selected from: ponatinib or olverembatinib, or a pharmaceutically acceptable salt, crystal form, solvate, or hydrate thereof.

In another alternative embodiment, the present disclosure relates to the following pharmaceutical combination:

(a) a compound of formula (I) or a pharmaceutically acceptable salt, crystal form, solvate, or hydrate thereof; and
(b) ponatinib or a pharmaceutically acceptable salt, crystal form, solvate, or hydrate thereof.

In another alternative embodiment, the present disclosure relates to the following pharmaceutical combination:

(a) a compound of formula (I) or a pharmaceutically acceptable salt, crystal form, solvate, or hydrate thereof; and

(b) olverembatinib or a pharmaceutically acceptable salt, crystal form, solvate, or hydrate thereof.

In another alternative embodiment, the present disclosure relates to the following pharmaceutical combination:

(a) a compound of formula (I) or a pharmaceutically acceptable salt, crystal form, solvate, or hydrate thereof; and
(b) PF-114 or a pharmaceutically acceptable salt, crystal form, solvate, or hydrate thereof.

In a more specific alternative embodiment, the compound of formula (I) described above is compound A.

All combinations of any component (a) and component (b) as defined and mentioned above, including methods of administering these two components to treat subject, pharmaceutical compositions for the simultaneous, separate, or sequential use containing these two components, the combination for use in treating diseases regulated by wild-type or mutant BCR-ABL1 kinase or use in the manufacture of pharmaceutical formulations for these purposes, or commercial products containing this combination, are subsequently also referred to as "the combination of the present disclosure" (whereby this term refers to each of these embodiments, which may replace the term as appropriate).

Commercial Package

The term "commercial package" or "product", as used herein defines especially a "kit of parts" in the sense that the components (a) and (b) as defined above can be dosed independently or by use of different fixed combinations with distinguished amounts of the components (a) and (b), i.e., simultaneously or at different time points. Moreover, these terms comprise a commercial package comprising (especially combining) as active ingredients components (a) and (b), together with instructions for simultaneous, sequential (temporally staggered, in a time-specific sequence) or separate use thereof in the treatment of wild-type and/or mutant BCR-ABL1 kinase-regulated diseases. The parts of the kit of parts can then, e.g., be administered simultaneously or temporally staggered, that is at different time points and with equal or different time intervals for any part of the kit of parts. Yet alternatively, the time intervals are chosen such that the effect on the treated disease in the combined use of the parts is larger than the effect which would be obtained by use of only any one of the combination partners (a) and (b). The ratio of the total amounts of the combination partner (a) to the combination partner (b) to be administered in the combined preparation may vary, for example, to address the needs of a patient sub-population to be treated or the needs of the single patient which different needs can be due to the particular disease, age, sex, body weight, etc. of the patients. Alternatively, there is at least one beneficial effect, e.g., a mutual enhancing of the effect of the combination partners (a) and (b), in particular a more than additive effect, which hence could be achieved with lower doses of each of the combined drugs, respectively, than tolerable in the case of treatment with the individual drugs only without combination, producing additional advantageous effects, e.g., less side effects or a combined therapeutic effect in a non-effective dosage of one or both of the combination partners (components) (a) and (b), and very alternatively a strong synergism of the combination partners (a) and (b).

Both in the case of the use of the combination of components (a) and (b) and of the commercial package, any combination of simultaneous, sequential and separate use is also possible, meaning that the components (a) and (b) may be administered at one time point simultaneously, followed by administration of only one component with lower host toxicity either chronically, e.g., more than 3-4 weeks of daily dosing, at a later time point and subsequently the other component or the combination of both components at a still later time point (in subsequent drug combination treatment courses for an optimal anti-tumor effect) or the like.

In one embodiment, the present disclosure specifically relates to a commercial package comprising:

(a) Compound A or a pharmaceutically acceptable salt, crystal form, solvate, or hydrate thereof; and
(b) an ATP-competitive BCR-ABL1 inhibitor or a pharmaceutically acceptable salt, crystal form, solvate, or hydrate thereof;
wherein (a) and (b) are present in a single unit dosage form or in two separate unit dosage forms.

In another embodiment, the present disclosure specifically relates to a commercial package comprising:

(a)Compound A or a pharmaceutically acceptable salt, crystal form, solvate, or hydrate thereof; and
(b)ponatinib or a pharmaceutically acceptable salt, crystal form, solvate, or hydrate thereof;
wherein (a) and (b) are present in a single unit dosage form or in two separate unit dosage forms.

In another embodiment, the present disclosure specifically relates to a commercial package comprising:

(a)Compound A or a pharmaceutically acceptable salt, crystal form, solvate, or hydrate thereof; and
(b)olverembatinib or a pharmaceutically acceptable salt, crystal form, solvate, or hydrate thereof;

wherein (a) and (b) are present in a single unit dosage form or in two separate unit dosage forms.

In another embodiment, the present disclosure specifically relates to a commercial package comprising:

(a)Compound A or a pharmaceutically acceptable salt, crystal form, solvate, or hydrate thereof; and
(b) PF-114 or a pharmaceutically acceptable salt, crystal form, solvate, or hydrate thereof;
wherein (a) and (b) are present in a single unit dosage form or in two separate unit dosage forms.

In another specific embodiment, the unit dosage form in the commercial package of the present disclosure is a fixed combination.

In another specific embodiment, the commercial packaging of the present disclosure is used for treating diseases regulated by wild-type or mutant BCR-ABL1 kinase.

Formulation

The pharmaceutical compositions according to the present disclosure can be prepared by conventional methods and are those suitable for enteral administration, such as oral or rectal, and parenteral administration to mammals, including humans. These compositions contain a therapeutically effective amount of component (a) and (b), either alone or in combination with one or more pharmaceutically acceptable carriers, particularly those suitable for enteral or parenteral applications.

The pharmaceutical composition comprises about 0.00002% to about 100%, particularly, for example, 0.0001% to 0.02% in the case of infusion diluents to be used, or, for example, about 0.1% to 95% in the case of injection solutions or infusion concentrates, especially parenteral formulations, alternatively about 1% to about 90%, and yet alternatively about 20% to about 60%. The pharmaceutical compositions according to the present disclosure may be, for example, in unit dosage forms such as ampoules, vials, dragees, tablets, infusion bags, or capsules.

The effective amount of each combination partner used in the formulation of the present disclosure may vary depending on the specific compound or pharmaceutical composition used, the route of administration, the disease to be treated, and the severity of the disease to be treated. A general physician, clinician, or veterinarian can easily determine the effective amount of each active ingredient required to prevent, treat, or inhibit the progression of the disease.

Pharmaceutical formulations for enteral or parenteral administration in combination therapy are, for example, those in unit dosage forms, such as sugar-coated tablets, capsules, or suppositories, and further ampoules. Unless otherwise indicated, these formulations are prepared by conventional methods, for example, by means of conventional mixing, granulation, sugar-coating, dissolution, or lyophilization processes. It should be understood that the unit content of the combination partner contained in a single dose of each dosage form does not itself need to constitute an effective amount, as the required effective amount can be achieved by administering multiple dosage units. Those skilled in the art have the ability to determine appropriate pharmaceutically effective amounts of the combination components.

Alternatively, the compound or a pharmaceutically acceptable salt, crystal form, solvate, or hydrate thereof is administered as an oral pharmaceutical formulation in the form of tablets, capsules, or syrups; or, if suitable, as a parenteral injection.

In preparing compositions for oral administration, any pharmaceutically acceptable medium may be used, such as water, glycols, oils, alcohols, flavoring agents, preservatives, and coloring agents. Pharmaceutically acceptable carriers include starch, sugar, microcrystalline cellulose, diluents, granulating agents, lubricants, binders, and disintegrants.

Solutions and suspensions of the active ingredient, particularly isotonic aqueous solutions or suspensions, can be used for parenteral administration of the active ingredient, for example, in the case of lyophilized compositions containing the active ingredient alone or together with a pharmaceutically acceptable carrier such as mannitol, such solutions or suspensions may be prepared prior to use. The pharmaceutical composition may be sterilized and/or may contain excipients such as preservatives, stabilizers, wetting agents and/or emulsifiers, solubilizers, salts for adjusting osmotic pressure, and/or buffers, and is prepared in a manner known per se, for example, by means of conventional dissolution or lyophilization processes. The solution or suspension may contain viscosity-increasing substances such as sodium carboxymethylcellulose, carboxymethylcellulose, dextrose, polyvinylpyrrolidone, or gelatin. Suspensions in oil contain as the oil component vegetable oils, synthetic oils, or semisynthetic oils commonly used for injection purposes.

Isotonic agents may be selected from any of those known in the art, such as mannitol, glucose, dextrose, and sodium chloride. Infusion formulations can be diluted with aqueous media. The amount of aqueous medium used as a diluent is chosen according to the desired concentration of the active ingredient in the infusion solution. The infusion solution may contain other excipients commonly used in formulations for intravenous administration, such as antioxidants.

The present disclosure further relates to "combination preparations," which are used herein to particularly define "kit of parts," meaning that the component partners (a) and (b) as defined above can be administered independently or by

using different fixed combinations with varying amounts of component partners (a) and (b), i.e., administered simultaneously or at different time points. Thus, the parts of the kit of parts may be administered simultaneously or in a staggered manner over time, meaning that any part of the kit of parts may be administered at different time points with the same or different intervals. The ratio of the total amount of component partner (a) to component partner (b) administered in the combination preparation may vary, for example, to address the needs of subpopulations of patients to be treated or the needs of individual patients based on the severity of any side effects experienced.

In another embodiment, the present disclosure specifically relates to a combination preparation comprising:

(a) one or more unit dosage forms of Compound A or a pharmaceutically acceptable salt, crystal form, solvate, or hydrate thereof; and
(b) one or more unit dosage forms of an ATP-competitive BCR-ABL1 inhibitor or a pharmaceutically acceptable salt, crystal form, solvate, or hydrate thereof.

Administration and Treatment Regimens

In certain embodiments, the present disclosure provides a method for treating CML in chronic phase and/or accelerated phase in a subject in need thereof, comprising administering to the subject an effective amount of a compound of formula (I) or a pharmaceutically acceptable salt, crystal form, solvate, or hydrate thereof. In one embodiment, the amount of the compound of formula (I) or a pharmaceutically acceptable salt, crystal form, solvate, or hydrate thereof is sufficient to achieve CHR. In another embodiment, the amount of the compound of formula (I) or a pharmaceutically acceptable salt, crystal form, solvate, or hydrate thereof is sufficient to achieve MaHR. In another embodiment, the amount of the compound of formula (I) or a pharmaceutically acceptable salt, crystal form, solvate, or hydrate thereof is sufficient to achieve CCyR. In another embodiment, the amount of the compound of formula (I) or a pharmaceutically acceptable salt, crystal form, solvate, or hydrate thereof is sufficient to achieve PCyR. In another embodiment, the amount of the compound of formula (I) or a pharmaceutically acceptable salt, crystal form, solvate, or hydrate thereof is sufficient to achieve MCyR. In another embodiment, the amount of the compound of formula (I) or a pharmaceutically acceptable salt, crystal form, solvate, or hydrate thereof is sufficient to achieve MMR. In another embodiment, the amount of the compound of formula (I) or a pharmaceutically acceptable salt, crystal form, solvate, or hydrate thereof is sufficient to achieve MR4. In another embodiment, the amount of the compound of formula (I) or a pharmaceutically acceptable salt, crystal form, solvate, or hydrate thereof is sufficient to achieve MR4.5. In another embodiment, the amount of the compound of formula (I) or a pharmaceutically acceptable salt, crystal form, solvate, or hydrate thereof is about 20 mg/day to about 400 mg/day. In another embodiment, the amount of the compound of formula (I) or a pharmaceutically acceptable salt, crystal form, solvate, or hydrate thereof is about 20 mg/day to about 240 mg/day. In another embodiment, the amount of the compound of formula (I) or a pharmaceutically acceptable salt, crystal form, solvate, or hydrate thereof is about 20 mg/day, about 40 mg/day, about 60 mg/day, about 80 mg/day, about 100 mg/day, about 120 mg/day, about 160 mg/day, or about 240 mg/day. In another embodiment, the amount of the compound of formula (I) or a pharmaceutically acceptable salt, crystal form, solvate, or hydrate thereof is 20 mg/day, about 40 mg/day, about 80 mg/day, about 160 mg/day, or about 240 mg/day. In another embodiment, the $AUC_{0-24}$ of the compound of formula (I) or a pharmaceutically acceptable salt, crystal form, solvate, or hydrate thereof is between about 370 h*ng/mL and 6430 h*ng/mL. In another embodiment, the compound of formula (I) or a pharmaceutically acceptable salt, crystal form, solvate, or hydrate thereof is administered orally. In another embodiment, the compound of formula (I) or a pharmaceutically acceptable salt, crystal form, solvate, or hydrate thereof is administered orally under fasting conditions. In another embodiment, the compound of formula (I) or a pharmaceutically acceptable salt, crystal form, solvate, or hydrate thereof is administered once daily, twice daily, or three times daily. In another embodiment, the compound of formula (I) or a pharmaceutically acceptable salt, crystal form, solvate, or hydrate thereof is administered until disease progression, intolerable toxicity, or death. In another embodiment, the compound of formula (I) or a pharmaceutically acceptable salt, crystal form, solvate, or hydrate thereof is administered daily until disease progression, intolerable toxicity, or death. In another embodiment, the compound of formula (I) or a pharmaceutically acceptable salt, crystal form, solvate, or hydrate thereof is administered every other day until disease progression, intolerable toxicity, or death. In another embodiment, the compound of formula (I) or a pharmaceutically acceptable salt, crystal form, solvate, or hydrate thereof is used as maintenance therapy.

The amount of the compound disclosed herein depends on the severity and progression of the disease or condition, prior treatment, the health status and weight of the subject, the response to the drug, and the judgment of the treating physician. It is well known that those skilled in the art can determine such therapeutically effective amounts (including but not limited to dose-escalation clinical trials) through routine experimentation.

If the subject's condition indeed improves, the administration of the compound may continue at the discretion of the physician; alternatively, the dose of the administered drug may be temporarily reduced or temporarily suspended for a certain length of time (i.e., a "drug holiday"). The length of the drug holiday may vary between 2 days and 1 year, including, for example only, 2 days, 3 days, 4 days, 5 days, 6 days, 7 days, 10 days, 12 days, 15 days, 20 days, 28 days, 35 days, 50

days, 70 days, 100 days, 120 days, 150 days, 180 days, 200 days, 250 days, 280 days, 300 days, 320 days, 350 days, or 365 days. The dose reduction during the drug holiday may range from 10% to 100%, including, for example only, 10%, 15%, 20%, 25%, 30%, 35%, 40%, 45%, 50%, 55%, 60%, 65%, 70%, 75%, 80%, 85%, 90%, 95%, or 100%.

Once the subject's condition improves, a maintenance dose may be administered if necessary. Subsequently, the dose or frequency of administration, or both, may be reduced to a level that maintains the improvement of the disease, disorder, or condition, based on changes in symptoms. However, patients may require long-term intermittent treatment if any recurrence of symptoms occurs.

The amount of the specified agent corresponding to this quantity will vary depending on factors such as the specific compound, the severity of the disease, the characteristics of the individual or host requiring treatment (e.g., body weight), among others. However, it can still be adjusted according to the specific circumstances of the case, including, for example, the particular agent administered, the route of administration, and the individual or host being treated.

Simultaneous administration can be carried out, for example, in the form of a fixed combination containing two or more active ingredients, or by administering two or more independently formulated active ingredients simultaneously. Sequential administration alternatively refers to administering one (or more) component(s) of the combination at one time point and the other component(s) at different time points, i.e., in a staggered manner over time, alternatively such that the combination demonstrates greater efficacy (particularly synergistic effects) compared to the independent administration of individual compounds. Separate administration alternatively refers to administering the components of the combination independently of each other at different time points, alternatively components (a) and (b), such that they are administered in a way that there is no measurable overlap of blood levels of the two compounds (i.e., no simultaneous presence).

Combinations of two or more simultaneous, separate, or sequential administrations are also possible, alternatively resulting in a combined therapeutic effect of the combination components that exceeds the effect produced when the component drugs are used independently at such large time intervals that no interaction of their therapeutic effects is observed, particularly alternatively a synergistic effect.

"Combined therapeutic activity" or "combined therapeutic effect" refers to the ability to administer the compounds at such time intervals (in a staggered manner over time, particularly in a sequence-specific way) that they alternatively still exhibit (alternatively synergistic) interactions (combined therapeutic effects) in the subject to be treated, especially in humans. In any case, this can be determined, for example, by monitoring blood levels, showing that both compounds are present in the blood of the person to be treated at least during a certain time interval.

## Indications

In one aspect, the present disclosure relates to a method for treating a disease regulated by wild-type and/or mutant BCR-ABL1 kinase in a subject.

In one embodiment, the pharmaceutical combination of the present disclosure can be used to treat and/or prevent diseases regulated by mutant BCR-ABL1 kinase. In a specific embodiment, the mutation of the mutant BCR-ABL1 kinase may be located at the ATP-binding site. In a more specific embodiment, the mutation at the ATP-binding site is selected from T315I, T315M, F317L, E355G, or V299L. In a more specific embodiment, the mutation at the ATP-binding site is selected from T315I. In a more specific embodiment, the mutation at the ATP-binding site is selected from T315M. In another specific embodiment, the mutation of the mutant BCR-ABL1 kinase may be located at the P-loop. In a more specific embodiment, the mutation located at the P-loop is selected from M244V, K247R, L248V, G250E, G250R, Q252H, Q252R, Y253F, Y253H, E255K, or E255V. In a more specific embodiment, the mutation located at the P-loop is selected from M244V, G250E, Q252H, Y253H, E255K, or E255V. In another specific embodiment, the mutation of the mutant BCR-ABL1 kinase may be located at the A-loop. In a more specific embodiment, the mutation located at the A-loop is selected from V379I, A380T, F382L, L384M, L387M, L387F, L387V, M388L, Y393C, H396P, H396R, H396A, or A397P. In a more specific embodiment, the mutation located at the A-loop is selected from H396P, H396R, H396A, or A397P. In another specific embodiment, the mutation of the mutant BCR-ABL1 kinase may be located at the SH2 domain interface or the SH3 domain interface. In a more specific embodiment, the mutation located at the SH2 domain interface or the SH3 domain interface is selected from P223S, K294E, M351T, or F359V. In another specific embodiment, the mutation of the mutant BCR-ABL1 kinase is selected from T315I, Y253F, Y253H, E255K, E255V, M351T, G250E, F359C, F359V, H396P, H396R, M244V, E355G, F317L, M237I, Q252H, Q252R, D276G, L248V, or F486S.

In another embodiment, the pharmaceutical combination of the present disclosure can address the issue of resistance developed to asciminib.

In another embodiment, the pharmaceutical composition of the present disclosure can be used for treating and/or preventing diseases regulated by mutant BCR-ABL1 kinase. In a specific embodiment, the mutation of the mutant BCR-ABL1 kinase may be located at the myristoyl binding site. In a more specific embodiment, the mutation at the myristoyl binding site is selected from E459K, P465S, V468F, I502L, C464W, A337V, or A424T. In an even more specific embodiment, the mutation at the myristoyl binding site is selected from E459K, P465S, V468F, I502L, A337V, or A424T.

In another embodiment, the pharmaceutical combination of the present disclosure can be used to treat and/or

prevent diseases regulated by mutant BCR-ABL1 kinase. In a specific embodiment, the mutation of the mutant BCR-ABL1 kinase is a refractory compound mutation. In a more specific embodiment, the mutation of the mutant BCR-ABL1 kinase may be selected from Y253H/T315I, E255V/T315I, Q252H/T315I, F317L/T315I, F359V/T315I, Y253H/E255K, or Y253H/F359V. In an even more specific embodiment, the mutation of the mutant BCR-ABL1 kinase may be selected from Y253H/T315I, E255V/T315I, or Q252H/T315I.

In another embodiment, the pharmaceutical combination of the present disclosure can be used to treat and/or prevent diseases regulated by mutant BCR-ABL1 kinase. In a specific embodiment, the mutation of the mutant BCR-ABL1 kinase may be selected from one or more of T315I, T315M, F317L, E355G, V299L, M244V, G250E, Q252H, Y253H, E255K, E255V, H396P, H396R, H396A, A397P, P223S, K294E, M351T, F359V, E459K, P465S, V468F, I502L, A337V, or A424T. In a specific embodiment, the mutation of the mutant BCR-ABL1 kinase may be selected from T315I, T315M, Y253H/T315I, E255V/T315I, Q252H/T315I, F317L/T315I, F359V/T315I, Y253H/E255K, or Y253H/F359V. In a specific embodiment, the mutation of the mutant BCR-ABL1 kinase may be selected from T315I, T315M, Y253H/T315I, E255V/T315I, or Q252H/T315I. In a specific embodiment, the BCR-ABL1 mutation is T315M, Y253H/T315I, E255V/T315I, or Q252H/T315I.

In another embodiment, the pharmaceutical combination of the present disclosure can be used to treat and/or prevent diseases regulated by mutant BCR-ABL1 kinase. In a specific embodiment, the wild-type and/or mutant BCR-ABL1 kinase-regulated disease is selected from hematological malignancies.

In another specific embodiment, the wild-type and/or mutant BCR-ABL1 kinase-regulated disease is selected from leukemia, neurodegenerative disease, neurofibroma, melanoma, breast cancer, colon cancer, lung cancer, prostate cancer, Kaposi's sarcoma, gastrointestinal stromal tumor, mesothelioma, systemic mastocytosis, hypereosinophilic syndrome, liver fibrosis, renal fibrosis, rheumatoid arthritis, polyarthritis, scleroderma, lupus erythematosus, graft-versus-host disease, pulmonary arterial hypertension, seminoma, dysgerminoma, and psoriasis. In a more specific embodiment, the leukemia is acute lymphoblastic leukemia, acute myeloid leukemia, acute promyelocytic leukemia, chronic myeloid leukemia, chronic myelomonocytic leukemia, chronic neutrophilic leukemia, acute undifferentiated leukemia, prolymphocytic leukemia, juvenile myelomonocytic leukemia, adult T-cell leukemia, acute myeloid leukemia with trilineage myelodysplasia, and mixed lineage leukemia. In a more specific embodiment, the leukemia is chronic myeloid leukemia and Philadelphia chromosome-positive acute lymphoblastic leukemia. In a more specific embodiment, the neurodegenerative disease is Parkinson's disease, Alzheimer's disease, Down syndrome, memory and cognitive impairment, dementia, amyloid neuropathy, or cerebral inflammation.

In another specific embodiment, the disease regulated by wild-type and/or mutant BCR-ABL1 kinase is chronic myeloid leukemia, Philadelphia chromosome-positive acute lymphoblastic leukemia, Parkinson's disease, Alzheimer's disease, neurofibromatosis, gastrointestinal stromal tumors, systemic mastocytosis, hypereosinophilic syndrome, liver fibrosis, renal fibrosis, or scleroderma.

In another embodiment, the disease regulated by wild-type and/or mutant BCR-ABL1 kinase is selected from leukemia, non-Hodgkin lymphoma, Hodgkin lymphoma, myeloma, neurodegenerative diseases, solid tumors, immune disorders, or fibrosis.

In a specific implementation, diseases regulated by wild-type and/or mutant BCR-ABL1 kinase are selected from leukemia, non-Hodgkin lymphoma, Hodgkin lymphoma, or myeloma, for example, acute lymphoblastic leukemia (ALL), acute myeloid leukemia (AML), acute promyelocytic leukemia (APL), chronic lymphocytic lymphoma (CLL), chronic myeloid leukemia (CML), chronic myelomonocytic leukemia (CMML), chronic neutrophilic leukemia (CNL), acute undifferentiated leukemia (AUL), anaplastic large cell lymphoma (ALCL), prolymphocytic leukemia (PML), juvenile myelomonocytic leukemia (JMML), adult T-cell leukemia (ALL), acute myeloid leukemia with trilineage myelodysplastic syndrome (AML/TMDS), mixed lineage leukemia (MLL), myelodysplastic syndrome (MDS), myeloproliferative disorders (MPD), diffuse large B-cell lymphoma, follicular lymphoma, mantle cell lymphoma, marginal zone lymphoma (e.g., splenic marginal zone lymphoma, extranodal marginal zone B-cell lymphoma), Burkitt lymphoma, Waldenström macroglobulinemia (lymphoplasmacytic lymphoma), primary central nervous system lymphoma, small lymphocytic lymphoma, precursor B-cell lymphoblastic leukemia, hairy cell leukemia, mucosa-associated lymphoid tissue lymphoma, plasma cell myeloma, plasmacytoma, and multiple myeloma. Other examples of hematological cancers include myeloproliferative disorders (MPD), such as polycythemia vera (PV), essential thrombocythemia (ET), and idiopathic primary myelofibrosis (IMF/IPF/PMF). In another specific implementation, the disease regulated by wild-type BCR-ABL1 kinase and mutant BCR-ABL1 kinase is selected from acute myeloid leukemia (AML) or chronic myeloid leukemia (CML).

In another specific embodiment, the disease regulated by wild-type and/or mutant BCR-ABL1 kinase is selected from neurodegenerative diseases, such as amyotrophic lateral sclerosis (ALS), Parkinson's disease (PD), Alzheimer's disease (AD), frontotemporal dementia (FTD), Pick's disease, and Niemann-Pick disease type C (NPC).

In another specific embodiment, the disease regulated by wild-type and/or mutant BCR-ABL1 kinase is selected from solid tumors, such as bone cancer, bone metastasis, breast cancer, gastroesophageal cancer, pancreatic cancer, ovarian cancer, prostate cancer, lung cancer, colon cancer, and head and neck cancer.

In another specific embodiment, the disease regulated by wild-type and/or mutant BCR-ABL1 kinase is selected

from immune diseases, such as arthritis, multiple sclerosis, osteoporosis, irritable bowel syndrome, inflammatory bowel disease, Crohn's disease, lupus, rheumatoid arthritis, psoriatic arthritis, osteoarthritis, Still's disease, juvenile arthritis, diabetes, myasthenia gravis, Hashimoto's thyroiditis, Ord's thyroiditis, Graves' disease, Sjögren's syndrome, Guillain-Barré syndrome, acute disseminated encephalomyelitis, Addison's disease, opsoclonus-myoclonus syndrome, ankylosing spondylitis, antiphospholipid antibody syndrome, aplastic anemia, autoimmune hepatitis, celiac disease, Goodpasture syndrome, idiopathic thrombocytopenic purpura, optic neuritis, scleroderma, primary biliary cirrhosis, Reiter's syndrome, Takayasu's arteritis, warm autoimmune hemolytic anemia, Wegener's granulomatosis, psoriasis, alopecia universalis, Behçet's disease, chronic fatigue, dysautonomia, endometriosis, interstitial cystitis, neuromyotonia, scleroderma, vulvodynia, asthma, appendicitis, blepharitis, bronchiolitis, bronchitis, bursitis, cervicitis, cholangitis, cholecystitis, colitis, conjunctivitis, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, hepatitis, inflammatory bowel disease, hidradenitis suppurativa, laryngitis, mastitis, meningitis, myelitis, myocarditis, myositis, nephritis, oophoritis, orchitis, osteitis, otitis, pancreatitis, parotitis, pericarditis, peritonitis, pharyngitis, pleuritis, phlebitis, pneumonia, pneumonitis, proctitis, prostatitis, pyelonephritis, rhinitis, salpingitis, sinusitis, stomatitis, synovitis, tendinitis, tonsillitis, uveitis, vaginitis, vasculitis, vulvitis, graft-versus-host disease, transplantation, blood transfusion, anaphylaxis, allergy, type I hypersensitivity, allergic conjunctivitis, allergic rhinitis, and atopic dermatitis.

In another specific embodiment, the disease regulated by wild-type and/or mutant BCR-ABL1 kinase is selected from inflammatory diseases, such as arthritis, asthma, appendicitis, blepharitis, bronchiolitis, bronchitis, bursitis, cervicitis, cholangitis, cholecystitis, colitis, conjunctivitis, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, hepatitis, hidradenitis suppurativa, laryngitis, mastitis, meningitis, osteomyelitis, myocarditis, myositis, nephritis, oophoritis, orchitis, osteitis, otitis, pancreatitis, parotitis, pericarditis, peritonitis, pharyngitis, pleuritis, phlebitis, pneumonia, pneumonitis, proctitis, prostatitis, pyelonephritis, rhinitis, salpingitis, sinusitis, stomatitis, synovitis, tendinitis, tonsillitis, uveitis, vaginitis, vasculitis, and vulvitis.

In another specific embodiment, the disease regulated by wild-type and/or mutant BCR-ABL1 kinase is selected from autoimmune diseases, such as lupus and Sjögren's syndrome, rheumatoid arthritis, psoriatic arthritis, osteoarthritis, Still's disease, juvenile arthritis, diabetes, myasthenia gravis, Hashimoto's thyroiditis, Ord's thyroiditis, Graves' disease, Sjögren's syndrome, Guillain-Barré syndrome, acute disseminated encephalomyelitis, Addison's disease, opsoclonus-myoclonus syndrome, ankylosing spondylitis, antiphospholipid antibody syndrome, aplastic anemia, autoimmune hepatitis, celiac disease, Goodpasture's syndrome, idiopathic thrombocytopenic purpura, optic neuritis, scleroderma, primary biliary cirrhosis, Reiter's syndrome, Takayasu's arteritis, hemolytic anemia, Wegener's granulomatosis, psoriasis, alopecia universalis, Behçet's disease, chronic fatigue, autonomic dysfunction, endometriosis, interstitial cystitis, neuromyotonia, scleroderma, and vulvodynia.

In another specific embodiment, the disease regulated by wild-type and/or mutant BCR-ABL1 kinase is selected from xenogeneic immune diseases, such as graft-versus-host disease, transplantation, blood transfusion, anaphylaxis, allergies, type I hypersensitivity, allergic conjunctivitis, allergic rhinitis, and atopic dermatitis.

In another specific embodiment, the disease regulated by wild-type and/or mutant BCR-ABL1 kinase is selected from fibrosis, such as pulmonary fibrosis, idiopathic pulmonary fibrosis (IPF), usual interstitial pneumonia (UIP), interstitial lung disease, cryptogenic fibrosing alveolitis (CFA), bronchiolitis obliterans, bronchiectasis, fatty liver disease, steatosis (e.g., non-alcoholic steatohepatitis (NASH)), cholestatic liver disease (e.g., primary biliary cirrhosis (PBC)), cirrhosis, alcohol-induced liver fibrosis, bile duct injury, biliary fibrosis, cholestasis, and cholangiopathy.

## Example

The present disclosure is further described below in conjunction with specific examples. It should be understood that these examples are only used to illustrate the present disclosure and are not intended to limit the scope of the present disclosure. Experimental methods without specific conditions indicated in the following examples are generally carried out under conventional conditions or according to the conditions recommended by the manufacturer. Unless otherwise stated, parts and percentages are by weight.

### Example 1: Test of Cell Growth Inhibitory Activity

The inhibitory effects of the compounds of the examples on the activity of Ba/$F_3$ parental cells, Ba/$F_3$ Bcr-Abl wild-type and mutant cells were detected.

This test used the Celltiter-Glo (CTG) kit (a homogeneous method for cell viability detection) provided by Promega to determine the cell viability of cultured cells by quantifying ATP. Ba/$F_3$ parental cells were purchased from RIKEN BRC, catalog number RCB0805; other cells containing BCR-ABL1 wild-type and mutants were monoclonal engineered cell lines obtained by transfecting Ba/$F_3$ parental cells with lentiviral plasmids containing WT or corresponding

mutations of BCR-ABL1.

The steps for cell viability detection are as follows:

a) Collect cells in the logarithmic growth phase, prepare a cell suspension and count the cells to calculate the cell concentration.
b) After diluting the cell suspension to the desired concentration, add it to the inner wells of the 96-well plate, and add PBS to the edge wells. Wells containing only culture medium without cells serve as blank controls.
c) In addition to the cell plate used for compound analysis, prepare another 96-well plate as the T0 plate, adding 3-6 blank wells and 3-6 cell wells. Perform CTG assay the next day to measure cell viability, which serves as the T0 viability value for the cells.
d) Place the cell plate in a 37°C, 5% $CO_2$ incubator and culture overnight.
e) The next day, add gradient-diluted compounds at various concentrations, and place the cell plate in a 37°C, 5% $CO_2$ incubator for 72 hours. Wells containing only 0.5% DMSO without compounds serve as positive controls for cell growth.
f) Add CTG reagent (CellTiter-Glo kit) according to the manufacturer's instructions to measure cell viability.
g) Read the plate using a Biotek cytation 3.0 microplate reader and export the data.
h) Calculate the IC50 using GraphPad Prism 7.0 software.

The following nonlinear fitting formula was used to obtain the IC50 (half maximal inhibitory concentration) of the compounds:

$$Y=Bottom+(Top-Bottom)/(1+10^{\wedge}((LogIC_{50}-X)*HillSlope))$$

Y: Inhibition rate (%inhibition)
X: Logarithmic value of compound concentration
Top: Maximum response
Bottom: Baseline response
HillSlope: Slope of the curve

The formula for calculating the cell inhibition rate (inhibition rate %) is:

Inhibition rate % = 1 - (Lum of test drug - Lum of vehicle control) / (Lum of cell control - Lum of vehicle control) × 100%.

Lum of test drug: Luminescence signal value of cells with the test drug added
Lum of cell control: Luminescence signal value of cells in wells with only 0.5% DMSO added, without compounds
Lum of vehicle control: Luminescence signal value in wells with only culture medium added, without cells

The inhibitory effects of Compound A on the in vitro proliferation of $Ba/F_3$ Bcr-Abl wild-type and mutant cells are summarized in Table 2 below:

Table 2 Growth inhibitory activity of compound A against $Ba/F_3$ Bcr-Abl wild-type and mutant cells

| $Ba/F_3$ BCR-ABL1 | $IC_{50}$ values (nM) of compound A |
|---|---|
| Parental cells | 10000 |
| WT | 4.11±0.05 |
| G250E | 2.49±0.18 |
| Q252H | 2.38±0.43 |
| Y253H | 5.6±0.53 |
| E255K | 1.01±0.08 |
| E255V | 2.73±0.02 |

(continued)

| Ba/$F_3$ BCR-ABL1 | $IC_{50}$ values (nM) of compound A |
|---|---|
| M244V | 26.8±2.26 |
| H396P | 11.7±0.28 |
| F317L | 17.2±0.99 |
| E459K | 3.8±0.22 |
| E355G | 52.6±0.42 |
| V299L | 72.3±5.87 |
| M351T | 3.52±0.5 |
| F359V | 89.4±8.91 |
| P223S | 43.7±4.88 |
| I502L | 94.8±1.06 |

The test results indicate that compound A exhibits excellent inhibitory activity against cells expressing wild-type and mutant forms of Ba/$F_3$ Bcr-Abl (including, for example, G250E, Q252H, Y253H, E255K/V, M244V, H396P, F317L, E459K, E355G, V299L, M351T, F359V, P223S, and I502L).

**Example 2: A Phase I, Single-Arm, Open-Label, Dose Escalation and Dose Expansion Clinical Trial to Evaluate the Safety, Tolerability, Pharmacokinetics, and Preliminary Efficacy of Compound A in Patients with Chronic Myeloid Leukemia**

**Study Objectives**

Primary Objectives:

To explore the safety and tolerability of multiple doses of Compound A in humans, and to determine the maximum tolerated dose (MTD) and the recommended Phase II dose (RP2D).

Secondary Objectives:

1) To preliminarily evaluate the pharmacokinetic characteristics of Compound A after multiple oral administrations;
2) To preliminarily evaluate the antitumor effects of Compound A.

**Overall Design**

This study is a single-arm, open-label, dose-escalation and dose-expansion Phase I clinical trial. The trial plans to enroll patients with chronic myeloid leukemia (CML) who have developed resistance or intolerance after prior treatment with BCR-ABL1 tyrosine kinase inhibitor(s) (TKIs).

The dose-escalation phase of this trial consists of about 8 dose groups, with oral administration under fasting conditions. Subjects in all dose groups will receive a single dose on Day 1 (D1), followed by pharmacokinetic (PK) and tolerability observations. If no drug-related adverse events of Grade 3 or higher occur during the single-dose period, the subject will enter the multiple-dose period (i.e., the treatment period) after 3-5 days (for the first dose group, the multiple-dose period begins 5 days after the first dose [i.e., starting on Day 6]; for subsequent dose groups, the interval will be determined based on available tolerability and PK data). After entering the multiple-dose period, subjects in the first dose group will initially receive twice-daily (BID) dosing (if PK data from the single-dose period are available, the dosing frequency during the multiple-dose period may be adjusted based on the PK data). The dosing frequency for subjects in subsequent dose groups after entering the multiple-dose period will be determined based on available PK and tolerability data. Throughout the trial, dosing frequency for all subjects may be adjusted based on continuously updated safety and PK data.

A continuous reassessment method (CRM) based on the principle of the escalation with overdose control (EWOC) design will be used to recommend the next specific dose for each dose escalation, thereby completing dose

escalation and determining the maximum tolerated dose (MTD). If both the investigator and sponsor believe that higher dose groups may offer better efficacy and manageable safety, the EWOC-based CRM method may be used to explore higher doses. All enrolled subjects will receive long-term treatment with the test drug and undergo evaluations for safety, tolerability, and preliminary efficacy.

After obtaining certain research data from the dose-escalation phase, the investigator may comprehensively consider safety, efficacy, and other information to decide whether to enroll additional subjects at the MTD and/or lower dose levels to determine the recommended Phase II dose (RP2D). Two to four suitable dosing regimens will be selected for expansion in the target population (including subjects enrolled at the target dose levels during the escalation phase).

**Population Selection and Dose Groups**

The dose escalation phase included subjects who had previously received treatment with imatinib, dasatinib, and nilotinib. A total of 8 dose groups were established: 10 mg bid, 20 mg bid, 40 mg bid, 80 mg bid, 40 mg qd, 80 mg qd, 160 mg qd, and 240 mg qd.

The dose expansion phase was conducted in the following 3 cohorts:

Cohort 1: Subjects with chronic phase (CP) chronic myeloid leukemia (CML) who had previously received at least ≥2 different TKIs and were T315I-negative. A total of 3 dose groups were established: 40 mg qd, 80 mg qd, and 240 mg qd;

Cohort 2: Subjects with chronic phase (CP) chronic myeloid leukemia (CML) who have previously received ≥1 TKI and were T315I-positive. A total of 2 dose groups were established: 40 mg qd and 240 mg qd;

Cohort 3: Subjects with accelerated phase (AP) chronic myeloid leukemia (CML) who have previously received ≥1 TKI, regardless of T315I positivity. A total of 1 dose group was established: 240 mg qd.

**Inclusion Criteria**

Subjects who meet all of the following criteria may be considered for enrollment:

1) Sign a written informed consent form;

2) At the time of screening, the subject is between 18 and 75 years of age (inclusive of 18 and 75 years), regardless of gender;

3) During the screening period, the subject is confirmed as a chronic phase (CP) CML patient or accelerated phase (AP) CML patient through bone marrow cytomorphology, molecular biology, or cytogenetic testing (determined according to the ELN 2013 diagnostic criteria for chronic phase or accelerated phase CML patients);

4) As judged by the investigator, the subject is intolerant to or has not achieved satisfactory therapeutic outcomes with the following treatment drugs (i.e., the treatment response evaluation results are warning or failure [according to the 2018 edition of the "Diagnosis and Treatment Guidelines for Adult Chronic Myeloid Leukemia"]):

a) In dose escalation phase, patients who have previously received at least three drugs: imatinib, dasatinib, and nilotinib;

b) The dose expansion phase will be divided into 3 cohorts, each expanding in 1 to 3 dose groups:

Cohort 1: Patients with chronic phase (CP) chronic myeloid leukemia, T315I-negative, who have previously received at least ≥2 different TKIs;

Cohort 2: Patients with chronic phase (CP) chronic myeloid leukemia, T315I-positive, who have previously received at least ≥1 TKI;

Cohort 3: Patients with accelerated phase (AP) chronic myeloid leukemia who have previously received at least ≥1 TKI, regardless of T315I positivity;

5) Eastern Cooperative Oncology Group (ECOG) performance status ≤2;

6) Minimum expected life expectancy ≥3 months;

7) Good renal function, defined as serum creatinine <1.5 times the upper limit of normal (ULN);

8) Good liver function, defined as:

Total bilirubin <1.5×ULN;

Alanine aminotransferase (ALT [SGPT]) and aspartate aminotransferase (AST [SGOT]) <2.5×ULN respectively (if the liver is affected by leukemia, then ALT and AST <5×ULN respectively);

9) Good coagulation function: Prothrombin time (PT) <1.5×ULN; International normalized ratio (INR) <1.5×ULN, activated partial thromboplastin time (APTT) <1.5×ULN;
10) Normal pancreatic function, defined as lipase and amylase <1.5×ULN respectively;
11) Screening results assessed by electrocardiogram indicate a normal QTc interval, defined as ≤450 ms for males and ≤470 ms for females;
12) Negative pregnancy test during the screening period (for women of childbearing potential);
13) Agreement to use effective contraception with their sexual partners throughout the entire study period (from the date of signing the informed consent form until 28 days after the last dose) (only for male and female subjects of childbearing potential).

**Exclusion Criteria**

All subjects must not meet any of the following criteria:

1) Receiving TKI treatment within 7 days prior to the first administration of the test drug, or pre-existing drug-related AEs have not recovered to Grade 1 or below (NCI CTCAE V5.0) (except for alopecia);
2) Receiving other anti-tumor therapies and meet any of the following conditions: received hydroxyurea or anagrelide within 24 hours before the first dose of the test drug, interferon or immunotherapy within 14 days before the first dose, or any other cytotoxic chemotherapy, radiation therapy, or investigational therapy (excluding any TKI-based investigational therapies) within 28 days before the first dose;
3) Having undergone stem cell transplantation within 60 days before the first dose of the test drug, with evidence of graft-versus-host disease (GVHD) or requiring immunosuppressive therapy for GVHD;
4) Requiring concurrent immunosuppressive therapy (except for corticosteroids used for short-term treatment);
5) Having taken medications associated with torsades de pointes within 1 month of the screening period;
6) Subjects with cytological or pathological evidence of active central nervous system disease;
7) Chronic phase CML patients who have achieved complete cytogenetic response (CCyR);
8) Accelerated phase CML patients who have achieved major hematologic response (MaHR);
9) Having significant or uncontrolled cardiovascular disease, including but not limited to:

Myocardial infarction occurred within 3 months before the first dose;
Unstable angina occurred within 3 months before the first dose;
Congestive heart failure occurred within 3 months before the first dose;
Ejection fraction (EF) ≤ 50%, pulmonary artery systolic pressure > 50 mmHg;

10) Suffering from hypertension (diastolic blood pressure ≥ 90 mmHg and/or systolic blood pressure ≥ 140 mmHg) that cannot be reduced to normal levels with oral medication;
11) Use of Chinese herbal medicine preparations or over-the-counter medications containing Chinese herbal ingredients within 2 weeks before the first administration of the test drug;
12) Suffering from a severe bleeding disorder unrelated to CML;
13) Having history of pancreatitis or alcoholism;
14) Uncontrolled hypertriglyceridemia (triglycerides >450 mg/dL);
15) Having malabsorption syndrome or other gastrointestinal diseases that may affect the absorption of the test drug;
16) Having diagnosed with another primary malignancy within the past 3 years (except for non-melanoma skin cancer, in situ cervical cancer, or controlled prostate cancer that has been cured within 3 years);
17) Having underwent major surgery within 14 days prior to the first dose (excluding minor surgeries such as bone marrow biopsy);
18) Persistent or active infection (including known Treponema pallidum, human immunodeficiency virus [HIV], hepatitis B virus [HBV], or hepatitis C virus [HCV] infection; active hepatitis B virus infection is defined as: for subjects positive for hepatitis B surface antigen, HBV DNA ≥2000 copies/ml or ≥500 IU/ml; active hepatitis C virus infection is defined as: for subjects positive for HCV antibodies, hepatitis C virus RNA positive);
19) Any condition that, in the opinion of the investigator or medical monitor, makes the subject unsuitable for participation in this trial, such as the presence of a disease that affects the safety assessment of the study drug, poor patient compliance, etc.

**Trial Results 1**

1) As of August 24, 2023, 134 subjects received at least one dose of the test drug. The full analysis set, safety analysis set,

and efficacy analysis set all included 134 subjects. The DLT analysis set included 32 subjects, and the PK study analysis set included 85 subjects. Since the safety data cutoff date was June 2, 2023, this safety summary evaluated 118 subjects. For details, please refer to Table 3:

Table 3 Distribution of Subjects

| | 10 mg BID | 40 mg/day | | | 80 mg/day | | | 160 mg/day | | | 240mg QD | Total |
|---|---|---|---|---|---|---|---|---|---|---|---|---|
| | | 20 mg BID | 40 mg QD | Total | 40 mg BID | 80 mg QD | Total | 80 mg BID | 160mg QD | Total | | |
| Screening | | | | | | | | | | | | 20 |
| Screening failure | | | | | | | | | | | | 59 |
| Enrolled | 3 | 6 | 21 | 27 | 5 | 17 | 22 | 3 | 4 | 7 | 86 | 145 |
| Study phase at enrollment, n (%) | | | | | | | | | | | | |
| Dose escalation | 3 (100) | 6 (100) | 5 (23.8) | 11 (40.7) | 5 (100) | 5 (29.4) | 10 (45.5) | 3 (100) | 3 (75.0) | 6 (85.7) | 4 (4.7) | 34 (23.4) |
| Dose expansion | 0 | 0 | 16(76.2) | 16 (59.3) | 0 | 12 (70.6) | 12 (54.5) | 0 | 1 (25.0) | 1 (14.3) | 82 (95.3) | 111 (76.6) |
| Dose expansion (including dose-escalation subjects), n (%) | | | | | | | | | | | | |
| Cohort 1 | 0 | 0 | 15 (71.4) | 15 (55.6) | 0 | 11 (64.7) | 11 (50.0) | 0 | 0 | 0 | 33 (38.4) | 59 (40.7) |
| Cohort 2 | 0 | 0 | 3 (14.3) | 3 (11.1) | 0 | 0 | 0 | 0 | 0 | 0 | 20 (23.3) | 23 (15.9) |
| Cohort 3 | 0 | 0 | 0 | 0 | 0 | 0 | 0 | 0 | 0 | 0 | 32 (37.2) | 32 (22.1) |
| Received at least one dose of investigational drug. n (%) | 3 (100) | 6 (100) | 21 (100) | 27 (100) | 5 (100) | 16 (94.1) | 21 (95.5) | 3 (100) | 4 (100) | 7 (100) | 76 (88.4) | 134 (92.4) |
| Full analysis set. n (%) | 3 (100) | 6 (100) | 21 (100) | 27 (100) | 5 (100) | 16 (94.1) | 21 (95.5) | 3 (100) | 4 (100) | 7 (100) | 76 (88.4) | 134 (92.4) |
| Safety analysis set. n (%) | 3 (100) | 6 (100) | 21 (100) | 27 (100) | 5 (100) | 16 (94.1) | 21 (95.5) | 3 (100) | 4 (100) | 7 (100) | 76 (88.4) | 134 (92.4) |
| DLT analysis set. n (%) | 3 (100) | 5 (83.3) | 4 (19.0) | 9 (33.3) | 5 (100) | 5 (29.4) | 10 (45.5) | 3 (100) | 3 (75.0) | 6 (85.7) | 4 (4.7) | 32 (22.1) |
| PK analysis set. n (%) | 3 (100) | 6 (100) | 16(76.2) | 22 (81.5) | 5 (100) | 5 (29.4) | 10 (45.5) | 3 (100) | 4 (100) | 7 (100) | 43 (50.0) | 85 (58.6) |
| Efficacy analysis set. n (%) | 3 (100) | 6 (100) | 21(100) | 27 (100) | 5 (100) | 16 (94.1) | 21 (95.5) | 3 (100) | 4 (100) | 7 (100) | 76 (88.4) | 134 (92.4) |

2) The prior CML treatment history of 98 subjects was summarized. Specific results are shown in Table 4:

**[0541]**

Table 4: Prior CML Treatment History of Some Subjects

| | 10 mg BID (N = 3) n (%) | 20 mg BID (N = 6) n (%) | 40 mg/day 40 mg QD (N = 19) n (%) | Total (N = 25) n (%) | 40 mg BID (N = 5) n (%) | 80 mg/day 80 mg QD (N = 5) n (%) | Total (N = 10) n (%) | 80 mg BID (N = 3) n (%) | 160 mg/day 160mg QD (N= 4) n (%) | Total (N = 7) n (%) | 240mg QD (N = 53) n (%) | Total (N = 98) n (%) |
|---|---|---|---|---|---|---|---|---|---|---|---|---|
| At least one prior CML therapy | 3 (100) | 6 (100) | 19 (100) | 25 (100) | 5 (100) | 5 (100) | 10 (100) | 3 (100) | 4 (100) | 7 (100) | 53 (100) | 98 (100) |
| Therapy type | | | | | | | | | | | | |
| Molecular targeted therapy | 3 (100) | 6 (100) | 19 (100) | 25 (100) | 5 (100) | 5 (100) | 10 (100) | 3 (100) | 4 (100) | 7 (100) | 53 (100) | 98 (100) |
| Chemotherapy | 0 | 2 (33.3) | 1 (5.3) | 3 (12.0) | 0 | 0 | 0 | 0 | 1 (25.0) | 1 (14.3) | 25 (47.2) | 29 (29.6) |
| Immunotherapy | 0 | 0 | 1 (5.3) | 1 (4.0) | 0 | 0 | 0 | 0 | 0 | 0 | 7 (13.2) | 8 (8.2) |
| Other | 0 | 0 | 0 | 0 | 0 | 0 | 0 | 0 | 0 | 0 | 1 (1.9) | 1 (1.0) |
| Medication | | | | | | | | | | | | |
| Imatinib | 3 (100) | 6 (100) | 19 (100) | 25 (100) | 5 (100) | 5 (100) | 10 (100) | 3 (100) | 4 (100) | 7 (100) | 43 (81.1) | 88 (89.8) |
| Dasatinib | 3 (100) | 6 (100) | 17 (89.5) | 23 (92.0) | 5 (100) | 5 (100) | 10 (100) | 3 (100) | 4 (100) | 7 (100) | 45 (84.9) | 88 (89.8) |
| Nilotinib | 3 (100) | 6 (100) | 12 (63.2) | 18 (72.0) | 5 (100) | 5 (100) | 10 (100) | 3 (100) | 4 (100) | 7 (100) | 25 (47.2) | 63 (64.3) |
| BCR-ABL TKI | 1 (33.3) | 2 (33.3) | 8 (42.1) | 10 (40.0) | 0 | 3 (60.0) | 3 (30.0) | 1 (33.3) | 2 (50.0) | 3 (42.9) | 26 (49.1) | 43 (43.9) |
| Olverembatinib | 0 | 4 (66.7) | 5 (26.3) | 9 (36.0) | 1 (20.0) | 3 (60.0) | 4 (40.0) | 0 | 0 | 0 | 12 (22.6) | 25 (25.5) |
| Hydroxyurea | 0 | 0 | 1 (5.3) | 1 (4.0) | 0 | 0 | 0 | 0 | 0 | 0 | 20 (37.7) | 21 (21.4) |
| Ponatinib | 1 (33.3) | 0 | 1 (5.3) | 1 (4.0) | 1 (20.0) | 2 (40.0) | 3 (30.0) | 0 | 1 (25.0) | 1 (14.3) | 8 (15.1) | 14 (14.3) |
| Asciminib | 0 | 0 | 2 (10.5) | 2 (8.0) | 0 | 0 | 0 | 0 | 0 | 0 | 6 (11.3) | 8 (8.2) |
| Interferon | 0 | 0 | 0 | 0 | 0 | 0 | 0 | 0 | 0 | 0 | 5 (9.4) | 5 (5.1) |
| Cytarabine | 0 | 0 | 0 | 0 | 0 | 0 | 0 | 0 | 0 1 (25.0) | 0 | 3 (5.7) | 3 (3.1) |
| Azacitidine | 0 | 2 (33.3) | 0 | 2 (8.0) | 0 | 0 | 0 | 0 | 0 | 0 | 1 (1.9) | 3 (3.1) |

(continued)

| | 10 mg BID (N = 3) n (%) | 20 mg BID (N = 6) n (%) | 40 mg/day 40 mg QD (N = 19) n (%) | Total (N = 25) n (%) | 40 mg BID (N = 5) n (%) | 80 mg/day 80 mg QD (N = 5) n (%) | Total (N = 10) n (%) | 80 mg BID (N = 3) n (%) | 160 mg/day 160mg QD (N= 4) n (%) | Total (N = 7) n (%) | 240mg QD (N = 53) n (%) | Total (N = 98) n (%) |
|---|---|---|---|---|---|---|---|---|---|---|---|---|
| Interferon alfa-1b | 0 | 0 | 1 (5.3) | 1 (4.0) | 0 | 0 | 0 | 0 | 0 | 0 | 2 (3.8) | 3 (3.1) |
| Homoharringtonine | 0 | 0 | 0 | 0 | 0 | 0 | 0 | 0 | | 1 (14.3) | 2 (3.8) | 3 (3.1) |
| Decitabine | 0 | 0 | 0 | 0 | 0 | 0 | 0 | 0 | 0 | 0 | 2 (3.8) | 2 (2.0) |
| Peginterferon alfa-2a | 0 | 0 | 0 | 0 | 0 | 0 | 0 | 0 | 0 | 0 | 1 (1.9) | 1 (1.0) |
| Antineoplastic agent | 0 | 0 | 0 | 0 | 0 | 0 | 0 | 0 | 0 | 0 | 1 (1.9) | 1 (1.0) |
| Indigo naturalis; Radix Pseudostellariae; Realgar; Salvia miltiorrhiza root and rhizome | 0 | 0 | 0 | 0 | 0 | 0 | 0 | 0 | 0 | 0 | 1 (1.9) | 1 (1.0) |
| Arsenic trioxide: Sodium chloride | 0 | 0 | 0 | 0 | 0 | 0 | 0 | 0 | 0 | 0 | 1 (1.9) | 1 (1.0) |
| Thalidomide | | 0 | 0 | 0 | 0 | 0 | 0 | 0 | 0 | 0 | 1 (1.9) | 1 (1.0) |
| Chidamide | | 0 | 0 | 0 | 0 | 0 | 0 | 0 | 0 | 0 | 1 (1.9) | 1 (1.0) |
| Recombinant interferon A-2B | 0 | 0 | 0 | 0 | 0 | 0 | 0 | 0 | 0 | 0 | 1 (1.9) | 1 (1.0) |
| Treatment Lines | | | | | | | | | | | | |
| First-line | 0 | 0 | 0 | 0 | 0 | 0 | 0 | 0 | 0 | 0 | 1 (1.9) | 1 (1.0) |
| Second-line | 0 | 0 | 4 (21.1) | 4 (16.0) | 0 | 0 | 0 | 0 | 0 | 0 | 13 (24.5) | 17 (17.3) |
| Third-line | 2 (66.7) | 0 | 4 (21.1) | 4 (16.0) | 2 (40.0) | 0 | 2 (20.0) | 2 (66.7) | 1 (25.0) | 3 (42.9) | 12 (22.6) | 23 (23.5) |
| Other | 1 (33.3) | 6 (100) | 11 (57.9) | 17 (68.0) | 3 (60.0) | 5 (100) | 8 (80.0) | 1 (33.3) | 3 (75.0) | 4 (57.1) | 27 (50.9) | 57 (58.2) |

3) Based on the full analysis set, the demographic and baseline information of enrolled subjects is presented in Table 5 below:

Table 5: Demographic and Baseline Information (Full Analysis Set)

| | 10 mg BID (N=3) | 40 mg/day | | | 80 mg/day | | | 160 mg/day | | | 240 mg QD (N=76) | Total (N=134) |
|---|---|---|---|---|---|---|---|---|---|---|---|---|
| | | 20 mg BID (N=6) | 40 mg QD (N=21) | Total (N=27) | 40mgBID (N=5) | 80mg QD (N=16) | Total (N=21) | 80 mg BID (N=3) | 160 mg **QD** (N=4) | Total (N=7) | | |
| Age (years) | 45.7 (12.01) | 41.5 (15.02) | 46.2 (10.40) | 45.2 (11.43) | 45.6 (15.45) | 46.8 (13.65) | 46.5 (13.70) | 48.3 (16.92) | 41.3 (9.74) | 44.3 (12.54) | 47.3 (13.60) | 46.5 (12.97) |
| Gender, n(%) | | | | | | | | | | | | |
| Male | 1 (33.3) | 5 (83.3) | 13 (61.9) | 18 (66.7) | 5 (100) | 9 (56.3) | 14 (66.7) | 0 | 3 (75.0) | 3 (42.9) | 44 (57.9) | 80 (59.7) |
| Time from CML initial diagnosis to first administration of test drug (months) | 107.52 (82.862) | 88.44 (48.539) | 102.58 (72.806) | 99.44 (67.575) | 75.39 (62.890) | 92.34 (68.773) | 88.30 (66.280) | 183.35 (48.965) | 120.31 (66.836) | 147.33 (64.560) | 91.39 (66.404) | 95.81 (67.057) |
| Baseline ECOG score, n (%) | | | | | | | | | | | | |
| 1 | 0 | 1 (16.7) | 13 (61.9) | 14 (51.9) | 2 (40.0) | 10 (62.5) | 12 (57.1) | 1 (33.3) | 4 (100) | 5 (71.4) | 39 (51.3) | 70 (52.2) |
| Disease stage, n(%) | | | | | | | | | | | | |
| Chronic phase | 1 (33.3) | 3 (50.0) | 18 (85.7) | 21 (77.8) | 1 (20.0) | 12 (75.0) | 13 (61.9) | 3 (100) | 1 (25.0) | 4 (57.1) | 47 (61.8) | 86 (64.9) |
| Accelerated phase | 2 (66.7) | 3 (50.0) | 3 (14.3) | 6 (22.2) | 4 (80.0) | 4 (25.0) | 8 (38.1) | 0 | 3 (75.0) | 3 (42.9) | 29 (38.2) | 48 (35.1) |
| Screening period T315I mutation, n(%) | | | | | | | | | | | | |
| Negative | 3 (100) | 4 (66.7) | 16 (76.2) | 20 (74.1) | 3 (60.0) | 14 (87.5) | 17 (81.0) | 2 (66.7) | 1 (25.0) | 3 (42.9) | 46 (60.5) | 89 (66.4) |
| Positive | 0 | 2 (33.3) | 5 (23.8) | 7 (25.9) | 2 (40.0) | 2 (12.5) | 4 (19.0) | 1 (33.3) | 3 (75.0) | 4 (57.1) | 30 (39.5) | 45 (33.6) |

4) Efficacy Analysis

As of August 24, 2023, based on the efficacy analysis set, there were 86 subjects enrolled in the chronic phase, including 26 subjects enrolled in the chronic phase with T315I mutation; there were 48 subjects enrolled in the accelerated phase. As the trial is ongoing, this summary only includes the best efficacy assessment results after the first dose, and subsequent efficacy data will be followed up.

In terms of efficacy, a low dose of 10 mg bid showed efficacy signals. In the CP subject group, the CHR was 91.4%, MCyR was 39.7%, and MMR was 16.1%; in the CP with T315I group, the CHR was 92.3%, MCyR was 62.5%, and MMR was 35.3%; in the AP group, the MaHR was 84.8%, MCyR was 34.1%, and MMR was 4.5%. All subgroups of subjects (CP subjects, CP subjects with T315I, AP subjects) demonstrated favorable efficacy.

1. Hematological, Cytogenetic, and Molecular Responses in CP Subjects

Among the 86 CML-CP subjects who received the test drug treatment, 35 subjects had not achieved CHR at baseline and had at least one post-dose hematological response assessment. 32 subjects achieved CHR after dosing (91.4%, 95% CI: [76.9%, 98.2%]). There were 58 subjects with at least one post-dose cytogenetic response assessment, and 23 subjects achieved MCyR after dosing (39.7%, 95% CI: [27.0%, 53.4%]), with a median time to MCyR of 2.83 (2.76, 3.68) months. 20 subjects achieved CCyR (34.5%, 95% CI: [22.5%, 48.1%]), with a median time to CCyR of 2.81 (2.76, 3.30) months. There were 62 subjects with at least one post-dose molecular response assessment, and 10 subjects achieved a major molecular response (MMR) (16.1%, 95% CI: [8.0%, 27.7%]), with a median time to MMR of 2.81 (2.76, 5.49) months. Detailed results are presented in Table 6.

Table 6 Summary of Efficacy in CML-CP Subjects (Efficacy Analysis Set)

| | 10 mg BID (N=2) n (%) | 40 mg/day | | | 80 mg/day | | | 160 mg/day | | | 240 mg QD (N=46) n (%) | Total (N=86) n (%) |
|---|---|---|---|---|---|---|---|---|---|---|---|---|
| | | 20 mg BID (N=3) n (%) | 40 mg QD (N=18) n (%) | Total (N=21) n (%) | 40 mg BID (N=1) n (%) | 80 mg QD (N=12) n (%) | Total (N=13) n (%) | 80 mg BID (N=3) n (%) | 160 mg QD (N=1) n (%) | Total (N=4) n (%) | | |
| CHR eva-luable subjects | 0 | 2 | 7 | 9 | 1 | 3 | 4 | 2 | 1 | 3 | 19 | 35 |
| CHR | 0 | 2 (100) | 6 (85.7) | 8 (88.9) | 1 (100) | 3 (100) | 4 (100) | 2 (100) | 1 (100) | 3 (100) | 17 (89.5) | 32 (91.4) |
| 95% CI (%) | NE, NE | 15.8, 100.0 | 42.1, 99.6 | 51.8, 99.7 | 2.5, 100.0 | 29.2, 100.0 | 39.8, 100.0 | 15.8, 100.0 | 2.5, 100.0 | 29.2, 100.0 | 66.9, 98.7 | 76.9, 98.2 |
| McyR evaluable subjects | 2 | 3 | 15 | 18 | 1 | 2 | 3 | 3 | 1 | 4 | 31 | 58 |
| MCyR | 2 (100) | 2 (66.7) | 5 (33.3) | 7 (38.9) | 0 | 1 (50.0) | 1 (33.3) | 1 (33.3) | 1 (100) | 2 (50.0) | 11 (35.5) | 23 (39.7) |
| 95% CI (%) | 15.8, 100.0 | 9.4, 99.2 | 11.8, 61.6 | 17.3, 64.3 | 0.0, 97.5 | 1.3, 98.7 | 0.8, 90.6 | 0.8, 90.6 | 2.5, 100.0 | 6.8, 93.2 | 19.2, 54.6 | 27.0, 53.4 |
| CcyR evaluable subjects | 2 | 3 | 15 | 18 | 1 | 2 | 3 | 3 | 1 | 4 | 31 | 58 |
| CCyR | 1 (50.0) | 2 (66.7) | 5 (33.3) | 7 (38.9) | 0 | 1 (50.0) | 1 (33.3) | 1 (33.3) | 1 (100) | 2 (50.0) | 9 (29.0) | 20 (34.5) |
| 95% CI (%) | 1.3, 98.7 | 9.4, 99.2 | 11.8, 61.6 | 17.3, 64.3 | 0.0, 97.5 | 1.3, 98.7 | 0.8, 90.6 | 0.8, 90.6 | 2.5, 100.0 | 6.8, 93.2 | 14.2, 48.0 | 22.5, 48.1 |
| MMR evaluable subjects | 2 | 3 | 17 | 20 | 1 | 2 | 3 | 3 | 1 | 4 | 33 | 62 |
| MMR | 1 (50.0) | 0 | 3 (17.6) | 3 (15.0) | 0 | 0 | 0 | 1 (33.3) | 1 (100) | 2 (50.0) | 4 (12.1) | 10 (16.1) |
| 95% CI (%) | 1.3, 98.7 | 0.0, 70.8 | 3.8, 43.4 | 3.2, 37.9 | 0.0, 97.5 | 0.0, 84.2 | 0.0, 70.8 | 0.8, 90.6 | 2.5, 100.0 | 6.8, 93.2 | 3.4, 28.2 | 8.0, 27.7 |

2. Hematologic, Cytogenetic, and Molecular Responses in CP Patients with T315I Mutation

Among the 26 CML-CP subjects with T315I mutation who received the test drug treatment, 13 subjects had not achieved CHR at baseline and had at least one post-dose hematologic response assessment, and 12 subjects achieved CHR after dosing (92.3%, 95% CI: [64.0%, 99.8%]). There were 16 subjects with at least one post-dose cytogenetic response assessment, and 10 subjects achieved MCyR (62.5%, 95% CI: [35.4%, 84.8%]), with a median time to MCyR of 2.81 (2.76, 2.83) months; 9 subjects achieved CCyR (56.3%, 95% CI: [29.9%, 80.2%]), with a median time to CCyR of 2.83 (2.76, 2.92) months. There were 17 subjects with at least one post-dose molecular response assessment, and 6 subjects achieved MMR (35.3%, 95% CI: [14.2%, 61.7%]), with a median time to MMR of 2.81 (2.76, 3.68) months. For detailed results, please refer to Table 7:

Table 7 Summary of Efficacy in CML-CP Subjects with T315I Mutation (Efficacy Analysis Set)

| | 10 mg BID (N=0) n (%) | 40 mg/day | | | 80 mg/day | | | 160 mg/day | | | 240 mg QD (N=19) n (%) | Total (N=26) n (%) |
|---|---|---|---|---|---|---|---|---|---|---|---|---|
| | | 20 mg BID (N=1) n (%) | 40 mg QD (N=3) n (%) | Total (N=4) n (%) | 40 mg BID (N=1) n (%) | 80 mg QD (N=0) n (%) | Total (N=1) n (%) | 80 mg BID (N=1) n (%) | 160 mg QD (N=1) n (%) | Total (N=2) n (%) | | |
| CHR eva-luable subjects | 0 | 1 | 0 | 1 | 1 | 0 | 1 | 0 | 1 | 1 | 10 | 13 |
| CHR | 0 | 1 (100) | 0 | 1 (100) | 1 (100) | 0 | 1 (100) | 0 | 1 (100) | 1 (100) | 9 (90.0) | 12 (92.3) |
| 95% CI (%) | NE, NE | 2.5, 100.0 | NE, NE | 2.5, 100.0 | 2.5, 100.0 | NE, NE | 2.5, 100.0 | NE, NE | 2.5, 100.0 | 2.5, 100.0 | 55.5, 99.7 | 64.0, 99.8 |
| MCyR evaluable subjects | 0 | 1 | 2 | 3 | 1 | 0 | 1 | 1 | 1 | 2 | 10 | 16 |
| MCyR | 0 | 1 (100) | 2 (100) | 3 (100) | 0 | 0 | 0 | 1 (100) | 1 (100) | 2 (100) | 5 (50.0) | 10 (62.5) |
| 95% CI (%) | NE, NE | 2.5, 100.0 | 15.8, 100.0 | 29.2, 100.0 | 0.0, 97.5 | NE, NE | 0.0, 97.5 | 2.5, 100.0 | 2.5, 100.0 | 15.8, 100.0 | 18.7, 81.3 | 35.4, 84.8 |
| CCyR evaluable subjects | 0 | 1 | 2 | 3 | 1 | 0 | 1 | 1 | 1 | 2 | 10 | 16 |
| CCyR | 0 | 1 (100) | 2 (100) | 3 (100) | 0 | 0 | 0 | 1 (100) | 1 (100) | 2 (100) | 4 (40.0) | 9 (56.3) |
| 95% CI (%) | NE, NE | 2.5, 100.0 | 15.8, 100.0 | 29.2, 100.0 | 0.0, 97.5 | NE, NE | 0.0, 97.5 | 2.5, 100.0 | 2.5, 100.0 | 15.8, 100.0 | 12.2, 73.8 | 29.9, 80.2 |
| MMR evaluable subjects | 0 | 1 | 3 | 4 | 1 | 0 | 1 | 1 | 1 | 2 | 10 | 17 |
| MMR | 0 | 0 | 2 (66.7) | 2 (50.0) | 0 | 0 | 0 | 1 (100) | 1 (100) | 2 (100) | 2 (20.0) | 6 (35.3) |
| 95% CI (%) | NE, NE | 0.0, 97.5 | 9.4, 99.2 | 6.8, 93.2 | 0.0, 97.5 | NE, NE | 0.0, 97.5 | 2.5, 100.0 | 2.5, 100.0 | 15.8, 100.0 | 2.5, 55.6 | 14.2, 61.7 |

3. A Hematological, Cytogenetic, and Molecular Responses in AP Subjects

Among the 48 CML-AP subjects who received the test drug treatment, 46 AP subjects had not achieved MaHR (Major Hematological Response) at baseline and had at least one post-dose hematological response assessment, and 39 subjects (84.8%, 95% CI: [71.1%, 93.7%]) achieved MaHR after the first administration of the test drug. There were 44 subjects with at least one post-dose cytogenetic response assessment, and 15 subjects (34.1%, 95% CI: [20.5%, 49.9%]) achieved MCyR after the administration, with a median time to MCyR of 2.76 (0.95, 5.52) months. 8 subjects (18.2%, 95% CI: [8.2%, 32.7%]) achieved CCyR. There were 44 subjects with at least one post-dose molecular response assessment, and 2 subjects (4.5%, 95% CI: [0.6%, 15.5%]) achieved MMR, with a median time to MMR of 5.54 (2.79, 8.28) months. For detailed results, please refer to Table 8:

Table 8 Summary of Efficacy in CML-AP Subjects (Efficacy Analysis Set)

| | 10 mg BID (N=1) n (%) | 40 mg/day | | | 80 mg/day | | | 160 mg/day | | | 240 mg QD (N=30) n (%) | Total (N=48) n (%) |
|---|---|---|---|---|---|---|---|---|---|---|---|---|
| | | 20 mg BID (N=3) n (%) | 40 mg QD (N=3) n (%) | Total (N=6) n (%) | 40 mg BID (N=4) n (%) | 80 mg QD (N=4) n (%) | Total (N=8) n (%) | 80 mg BID (N=0) n (%) | 160 mg QD (N=3) n (%) | Total (N=3) n (%) | | |
| MaHR evaluable subjects | 1 | 3 | 2 | 5 | 4 | 4 | 8 | 0 | 3 | 3 | 29 | 46 |
| MaHR | 1 (100) | 3 (100) | 1 (50.0) | 4 (80.0) | 3 (75.0) | 3 (75.0) | 6 (75.0) | 0 | 3 (100) | 3 (100) | 25 (86.2) | 39 (84.8) |
| 95% CI (%) | 2.5, 100.0 | 29.2, 100.0 | 1.3, 98.7 | 28.4, 99.5 | 19.4, 99.4 | 19.4, 99.4 | 34.9, 96.8 | NE, NE | 29.2, 100.0 | 29.2, 100.0 | 68.3, 96.1 | 71.1, 93.7 |
| MCyR evaluable subjects | 1 | 3 | 2 | 5 | 4 | 4 | 8 | 0 | 3 | 3 | 27 | 44 |
| MCyR | 0 | 1 (33.3) | 1 (50.0) | 2 (40.0) | 1 (25.0) | 0 | 1 (12.5) | 0 | 1 (33.3) | 1 (33.3) | 11 (40.7) | 15 (34.1) |
| 95% CI (%) | 0.0, 97.5 | 0.8, 90.6 | 1.3, 98.7 | 5.3, 85.3 | 0.6, 80.6 | 0.0, 60.2 | 0.3, 52.7 | NE, NE | 0.8, 90.6 | 0.8, 90.6 | 22.4, 61.2 | 20.5, 49.9 |
| CCyR evaluable subjects | 1 | 3 | 2 | 5 | 4 | 4 | 8 | 0 | 3 | 3 | 27 | 44 |
| CCyR | 0 | 1 (33.3) | 0 | 1 (20.0) | 0 | 0 | 0 | 0 | 0 | 0 | 7 (25.9) | 8 (18.2) |
| 95% CI (%) | 0.0, 97.5 | 0.8, 90.6 | 0.0, 84.2 | 0.5, 71.6 | 0.0, 60.2 | 0.0, 60.2 | 0.0, 36.9 | NE, NE | 0.0, 70.8 | 0.0, 70.8 | 11.1, 46.3 | 8.2, 32.7 |
| MMR evaluable subjects | 1 | 3 | 2 | 5 | 4 | 4 | 8 | 0 | 3 | 3 | 27 | 44 |
| MMR | 0 | 0 | 0 | 0 | 0 | 0 | 0 | 0 | 0 | 0 | 2 (7.4) | 2 (4.5) |
| 95% CI (%) | 0.0, 97.5 | 0.0, 70.8 | 0.0, 84.2 | 0.0, 52.2 | 0.0, 60.2 | 0.0, 60.2 | 0.0, 36.9 | NE, NE | 0.0, 70.8 | 0.0, 70.8 | 0.9, 24.3 | 0.6, 15.5 |

Hematologic, cytogenetic, and molecular responses in subjects with CML-AP who have failed or are intolerant to third or above generations of TKI therapy

Among 30 subjects with CML-AP who had failed or were intolerant to third or above generations of TKI therapy, the median exposure time (range) was 8.8 (0.9, 28.3) months. There were 28 evaluable subjects who had not achieved MaHR at baseline and had at least one post-dose hematologic response assessment. 22 subjects achieved MaHR after the first administration of the test drug (78.6%, 95% CI: [59.0%, 91.7%]). The median duration of MaHR response was not reached (Q1, Q3: 4.83 months, NE). There were 28 evaluable subjects who had not achieved MCyR at baseline and had at least one post-dose cytogenetic response assessment, 7 subjects achieved MCyR after dosing (25.0%, 95% CI: [10.7%, 44.9%]), and 4 subjects achieved CCyR after dosing (14.3%, 95% CI: [4.0%, 32.7%]). The median time to achieve MCyR and CCyR was 2.69 (2.69, 5.55) months and 5.59 (4.16, 6.92) months, respectively. There were 28 evaluable subjects who had not achieved MMR at baseline and had at least one post-dose molecular response assessment. 1 subject achieved MMR (3.6%, 95% CI: [0.1%, 18.3%]), as detailed in Table 9. The median PFS was not reached, and the 12-month PFS rate was 79.9% (95% CI: [54.3%, 92.1%]) (estimated by the Kaplan-Meier method). The median EFS was 8.41 months (95% CI: 2.86, NE), and the 12-month EFS rate was 42.5% (95% CI: [17.9%, 65.4%]) (estimated by the Kaplan-Meier method).

Table 9 Summary of Efficacy in Patients with CML-AP Who Have Failed or Are Intolerant to Third or above generations of TKI Therapy

| | | 40 mg/day | | | 80 mg/day | | | 160 mg/day | | | | |
|---|---|---|---|---|---|---|---|---|---|---|---|---|
| | 10 mg BID (N=1) n (%) | 20 mg BID (N=1) n (%) | 40 mg QD (N=3) n (%) | Total (N=4) n (%) | 40 mg BID (N=2) n (%) | 80 mg QD (N=4) n (%) | Total (N=6) n (%) | 80 mg BID (N=0) n (%) | 160 mg QD (N=1) n (%) | Total (N=1) n (%) | 240 mg QD (N=18) n (%) | Total (N=30) n (%) |
| MaHR evaluable subjects | 1 | 1 | 2 | 3 | 2 | 4 | 6 | 0 | 1 | 1 | 17 | 28 |
| MaHR | 1 (100) | 1 (100) | 1 (50.0) | 2 (66.7) | 1 (50.0) | 3 (75.0) | 4 (66.7) | 0 | 1 (100) | 1 (100) | 14 (82.4) | 22 (78.6) |
| 95% CI (%) | 2.5, 100.0 | 2.5, 100.0 | 1.3, 98.7 | 9.4, 99.2 | 1.3, 98.7 | 19.4, 99.4 | 22.3, 95.7 | NE, NE | 2.5, 100.0 | 2.5, 100.0 | 56.6, 96.2 | 59.0, 91.7 |
| MCyR evaluable subjects | 1 | 1 | 2 | 3 | 2 | 4 | 6 | 0 | 1 | 1 | 17 | 28 |
| MCyR | 0 | 0 | 1 (50.0) | 1 (33.3) | 0 | 0 | 0 | 0 | 0 | 0 | 6 (35.3) | 7 (25.0) |
| 95% CI (%) | 0.0, 97.5 | 0.0, 97.5 | 1.3, 98.7 | 0.8, 90.6 | 0.0, 84.2 | 0.0, 60.2 | 0.0, 45.9 | NE, NE | 0.0, 97.5 | 0.0, 97.5 | 14.2, 61.7 | 10.7, 44.9 |
| CCyR evaluable subjects | 1 | 1 | 2 | 3 | 2 | 4 | 6 | 0 | 1 | 1 | 17 | 28 |
| CCyR | 0 | 0 | 0 | 0 | 0 | 0 | 0 | 0 | 0 | 0 | 4 (23.5) | 4 (14.3) |
| 95% CI (%) | 0.0, 97.5 | 0.0, 97.5 | 0.0, 84.2 | 0.0, 70.8 | 0.0, 84.2 | 0.0, 60.2 | 0.0, 45.9 | NE, NE | 0.0, 97.5 | 0.0, 97.5 | 6.8, 49.9 | 4.0, 32.7 |
| MMR evaluable subjects | 1 | 1 | 2 | 3 | 2 | 4 | 6 | 0 | 1 | 1 | 17 | 28 |
| MMR | 0 | 0 | 0 | 0 | 0 | 0 | 0 | 0 | 0 | 0 | 1 (5.9) | 1 (3.6) |
| 95% CI (%) | 0.0, 97.5 | 0.0, 97.5 | 0.0, 84.2 | 0.0, 70.8 | 0.0, 84.2 | 0.0, 60.2 | 0.0, 45.9 | NE, NE | 0.0, 97.5 | 0.0, 97.5 | 0.1, 28.7 | 0.1, 18.3 |

Among the 14 subjects with CML-AP and T315I mutation who had failed or were intolerant to third-generation or later TKI therapy, the median exposure time (range) was 8.4 (0.9, 18.4) months. There were 13 evaluable subjects who had not achieved MaHR at baseline and had at least one post-dose hematological response assessment. After the first administration of the test drug, 10 subjects achieved MaHR (76.9%, 95% CI: [46.2%, 95.0%]), with a median duration of MaHR response of 11.93 (Q1, Q3: 4.83, NE) months. There were 12 evaluable subjects who had not achieved MCyR at baseline and had at least one post-dose cytogenetic response assessment. After administration, 3 subjects achieved MCyR (25.0%, 95% CI: [5.5%, 57.2%]), and 2 subjects achieved CCyR (16.7%, 95% CI: [2.1%, 48.4%]). The median time to achieve MCyR and CCyR was 2.69 (2.69, 2.76) months and 4.19 (2.76, 5.62) months, respectively. There were 12 evaluable subjects who had not achieved MMR at baseline and had at least one post-dose molecular response assessment. One subject achieved major molecular response (MMR) (8.3%, 95% CI: [0.2%, 38.5%]), as detailed in Table 10. The median PFS was not reached, with a 12-month PFS rate of 75.8% (95% CI: [30.5%, 93.7%]) (estimated by the Kaplan-Meier method). The median EFS was 5.55 (95% CI: 1.58, 8.25) months, with a 12-month EFS rate of 18.0% (95% CI: [0.9%, 53.3%]) (estimated by the Kaplan-Meier method).

Table 10 Summary of Efficacy in Patients with CML-AP and T315I Mutation Who Failed or Were Intolerant to Third-Generation or Above TKI Therapy

| | | 40 mg/day | | | 80 mg/day | | | 160 mg/day | | | | |
|---|---|---|---|---|---|---|---|---|---|---|---|---|
| | 10 mg BID (N=0) n (%) | 20 mg BID (N=0) n (%) | 40 mg QD (N=2) n (%) | Total (N=2) n (%) | 40 mg BID (N=1) n (%) | 80 mg QD (N=2) n (%) | Total (N=3) n (%) | 80 mg BID (N=0) n (%) | 160 mg QD (N=1) n (%) | Total (N=1) n (%) | 240 mg QD (N=8) n (%) | Total (N=14) n (%) |
| MaHR evaluable subjects | 0 | 0 | 2 | 2 | 1 | 2 | 3 | 0 | 1 | 1 | 7 | 13 |
| MaHR | 0 | 0 | 1 (50.0) | 1 (50.0) | 1 (100) | 1 (50.0) | 2 (66.7) | 0 | 1 (100) | 1 (100) | 6 (85.7) | 10 (76.9) |
| 95% CI (%) | NE, NE | NE, NE | 1.3, 98.7 | 1.3, 98.7 | 2.5, 100.0 | 1.3, 98.7 | 9.4, 99.2 | NE, NE | 2.5, 100.0 | 2.5, 100.0 | 42.1, 99.6 | 46.2, 95.0 |
| MCyR evaluable subjects | 0 | 0 | 1 | 1 | 1 | 2 | 3 | 0 | 1 | 1 | 7 | 12 |
| MCyR | 0 | 0 | 0 | 0 | 0 | 0 | 0 | 0 | 0 | 0 | 3 (42.9) | 3 (25.0) |
| 95% CI (%) | NE, NE | NE, NE | 0.0, 97.5 | 0.0, 97.5 | 0.0, 97.5 | 0.0, 84.2 | 0.0, 70.8 | NE, NE | 0.0, 97.5 | 0.0, 97.5 | 9.9, 81.6 | 5.5, 57.2 |
| Complete CCyR evaluable subjects | 0 | 0 | 1 | 1 | 1 | 2 | 3 | 0 | 1 | 1 | 7 | 12 |
| Complete CCyR | 0 | 0 | 0 | 0 | 0 | 0 | 0 | 0 | 0 | 0 | 2 (28.6) | 2 (16.7) |
| 95% CI (%) | NE, NE | NE, NE | 0.0, 97.5 | 0.0, 97.5 | 0.0, 97.5 | 0.0, 84.2 | 0.0, 70.8 | NE, NE | 0.0, 97.5 | 0.0, 97.5 | 3.7, 71.0 | 2.1, 48.4 |
| MMR evaluable subjects | 0 | 0 | 1 | 1 | 1 | 2 | 3 | 0 | 1 | 1 | 7 | 12 |
| MMR | 0 | 0 | 0 | 0 | 0 | 0 | 0 | 0 | 0 | 0 | 1 (14.3) | 1 (8.3) |
| 95% CI (%) | NE, NE | NE, NE | 0.0, 97.5 | 0.0, 97.5 | 0.0, 97.5 | 0.0, 84.2 | 0.0, 70.8 | NE, NE | 0.0, 97.5 | 0.0, 97.5 | 0.4, 57.9 | 0.2, 38.5 |

5) Safety

As an allosteric site inhibitor with a novel mechanism of action, the test drug in this trial demonstrates significant improvement in non-hematological toxicity compared to ATP-competitive TKIs, with no reported pigmentation or severe arterial occlusive events and venous thromboembolic events; hematological toxicity remains largely consistent.

Grade ≥3 hematological adverse events associated with the test drug were mostly manageable with treatment interruption, with no occurrences of bleeding or serious systemic infectious complications.

The test drug demonstrated a favorable safety and tolerability profile, with no observed dose-dependent increase in the frequency of adverse events.

**Trial Results 2**

As of June 2, 2024, 108 subjects with chronic myeloid leukemia in chronic phase (CML-CP) had been enrolled and received treatment with the test drug. Among them: a) 71 subjects (66%) had previously received ≥3 different TKIs; b) 40 subjects (37%) had previously received ponatinib, olverembatinib, asciminib, and/or HS-10382 (a novel STAMP inhibitor); c) 91 subjects (84%) had BCR-ABL(IS) > 10%; d) 25 subjects (23%) had only the T315I mutation; e) 8 subjects (7%) had the T315I mutation along with additional mutations; f) 14 subjects (13%) had other mutations; g) 61 subjects (56%) had no mutations.

As of June 2, 2024, 50 subjects with chronic myeloid leukemia in accelerated phase (CML-AP) have been enrolled and received the test drug treatment. Among them: a) 44 subjects (88%) had previously received treatment with ≥3 different TKIs; b) 30 subjects (60%) had previously received ponatinib, olverembatinib, asciminib, and/or HS-10382; c) 46 subjects (92%) had BCR-ABL(IS) > 10%; d) 17 subjects (34%) had only the T315I mutation; e) 3 subjects (6%) had the T315I mutation along with additional mutations; f) 14 subjects (28%) had other mutations; g) 16 patients (32%) had no mutations.

1. Hematologic, Cytogenetic, and Molecular Responses in CML-CP Subjects

There were 49 CML-CP subjects who had not achieved CHR at baseline and had at least one post-dose hematologic response assessment, and 40 subjects (82%) achieved CHR within 3 treatment cycles. The 18-month cumulative incidence rates for MCyR, CCyR, and MMR were 52%, 40%, and 26%, respectively.

There were 20 CML-CP subjects with T315I mutation who had not achieved CHR at baseline and had at least one post-dose hematologic response assessment, 16 subjects (80%) achieved CHR within 3 treatment cycles. The 18-month cumulative incidences of MCyR, CCyR, and MMR were 76%, 69%, and 50%, respectively.

Among CML-CP subjects previously treated with ponatinib, olverembatinib, asciminib, and/or HS-10382, the 18-month cumulative incidences of MCyR and CCyR were 32% and 17%, respectively.

2. Hematologic, Cytogenetic, and Molecular Responses in AP Subjects

There were 42 CML-AP subjects who had not achieved MaHR and CHR at baseline and had at least one post-dose hematologic response assessment, 35 (83%) and 33 (79%) subjects achieved MaHR and CHR, respectively, within 3 treatment cycles. The 18-month cumulative incidences of MCyR, CCyR, and MMR were 35%, 30%, and 14%, respectively.

Among CML-AP subjects with T315I mutation who received the test drug treatment, the 18-month cumulative incidence rates of MCyR, CCyR, and MMR were 36%, 30%, and 15%, respectively.

Among CML-AP subjects previously treated with ponatinib, olverembatinib, asciminib, and/or HS-10382, the 18-month cumulative incidence rates of MCyR and CCyR were 23% and 17%, respectively.

With the increase in patient numbers and extended follow-up time, Compound A demonstrated good tolerability, with no new safety issues identified. Updated data indicate that Compound A shows good efficacy in both CML-CP and CML-AP patients, including those with T315I mutations and those who have previously failed treatment with ponatinib, olverembatinib, asciminib, and/or HS-10382.

PK results indicate that the exposure of Compound A is dose-proportional. Compound A has a long terminal half-life (>100 hours), which may lead to drug accumulation.

**Example 3 Cell Growth Inhibition Activity Test**

A series of cell lines expressing BCR-ABL1 single mutations and compound mutations were constructed based on the Ba/F3 mouse pro-B cell line. The effects of Compound A, asciminib, ATP-competitive BCR-ABL1 inhibitors (e.g., ponatinib) as single agents or in combination were analyzed using in vitro antiproliferation assays.

This assay utilized the CellTiter-Glo (CTG) kit provided by Promega, which is a homogeneous method for cell viability detection, measuring the viability of cultured cells by quantifying ATP. Cells were seeded in 96-well plates and treated with different concentrations of test substances. Using the Chou-Talalay median-effect method, the drug effects were tested with different concentrations of Compound A, asciminib, ATP-competitive BCR-ABL1 inhibitors, or their combinations, with each treatment performed in triplicate. The plates were then incubated at 37°C and 5% $CO_2$ for 72 hours. At the end of the treatment, CTG reagent (CellTiter-Glo kit) was added according to the manufacturer's instructions to detect cell viability values. Plates were read on a microplate reader (Biotek cytation 3.0). IC50 values were calculated using GraphPad Prism 7.0 software.

The following nonlinear fitting formula was used to obtain the IC50 (half-maximal inhibitory concentration) of the compounds:

$$Y=Bottom + (Top-Bottom)/(1+10^{((LogIC50-X)*HillSlope)})$$

Y: Inhibition rate (%inhibition)
X: Compound concentration log value
Top: Maximum response
Bottom: Baseline response
HillSlope: The slope of the curve
The formula for calculating the cell inhibition rate (Inhibition rate %) is:

Inhibition rate (%) = 1 - (Lum of test drug - Lum of vehicle control) / (Lum of cell control - Lum of vehicle control) × 100%.

Lum of test drug: Luminescence signal value of cells with the test drug added
Lum of cell control: Luminescence signal value of cells in wells with 0.5% DMSO added, without compounds
Lum of vehicle control: Luminescence signal value in wells with only culture medium added, without cells
Using Calcusyn software (Version 2.0, Zhou), the inhibition rate-combination (Fraction affected-Combination Index) curve under each combination of drugs was plotted, and the corresponding combination index (CI) was calculated. When the CI is greater than 0.7 and less than 1.0, it indicates a slight synergistic effect between the two compounds; when it is greater than 0.3 and less than 0.7, it is considered a synergistic effect between the two compounds; and when it is less than 0.3, it indicates a strong synergistic effect.

Cell-based anti-proliferation studies indicated that:
(1) Compound A and asciminib exhibit similar resistance profiles, showing significantly reduced anti-proliferative activity (IC50 values > 1000 nM) against Ba/F3 BCR-ABL1 T315M, Y253H/T315I, and Q252H/T315I mutations, and moderately reduced anti-proliferative activity (IC50 values of 151 nM and 446 nM, respectively) against the E255V/T315I mutation. The primary resistance mutations for ponatinib are T315M, Y253H/T315I, and E255V/T315I. The results are shown in Table 11.

Table 11: Effects of Compound A, asciminib, and ATP-competitive BCR-ABL1 inhibitors as single agents in Ba/F3 BCR-ABL11 cell lines (IC50 nM)

| Ba/F3 BCR-ABL11 mutant cell lines | Compo und A | asci mini b | ponati nib | olverem batinib | imat inib | dasat inib | nilot inib | bosuti nib | fluma tinib |
|---|---|---|---|---|---|---|---|---|---|
| T315M | 1549± 584 | 1000 0 | 137±2 .83 | 38.2±3.4 6 | 100 00 | 1000 0 | 1000 0 | 3968± 151 | 1000 0 |
| Y253H/T315I | 10000 | 1000 0 | 260.5 ±4.95 | 36.2±1.2 7 | 100 00 | 1000 0 | 1000 0 | 3715± 9.9 | 8422 ±245 |
| E255V/T315I | 151±1 3.4 | 446± 91.2 | 590±9 2.6 | 25.8±2.9 | 100 00 | 1000 0 | 1000 0 | 3922± 1377 | 1000 0 |
| Q252H/T315I | 10000 | 1000 0 | 33.2± 2.33 | 7.7±0.01 | 100 00 | 1000 0 | 1000 0 | 3130± 9.9 | 1000 0 |

(2) Compared to single drugs, the combination of Compound A with ponatinib or olverembatinib exhibits synergistic antitumor effects on BCR-ABL1 Y253H/T315I mutant cells.

We found that, compared to the corresponding monotherapies, the combination of Compound A with ponatinib or Compound A with olverembatinib significantly enhances the inhibition of BCR-ABL1 Y253H/T315I cells (as shown in Figs. 1a, 1b, 1c, 1d, and Table 12), and demonstrates strong synergistic effects, with combination index (CI) values all less than 0.3 (as shown in Figs. 1e and 1f).

Table 12 IC50 values (nM) of monotherapies and combination therapies with different inhibitors (I1 and I2)

| Cell Line | I1 | Treatment | I2 | $IC_{50}^{I1}$ | $IC_{50}^{I2}$ |
|---|---|---|---|---|---|
| BCR-ABL1 Y253H/T315I | Compound A | Monotherapy | None | 12255 | |
| | ponatinib | Monotherapy | None | 215.9 | |
| | olverembatinib | Monotherapy | None | 36.90 | |
| | Compound A | Combination | ponatinib | 166.2 | 13.85 |
| | Compound A | Combination | olverembatinib | 65.28 | 5.44 |

Fig. 2a indicates that the combination of Compound A (>300 nM) and ponatinib (>30 nM) achieved complete (100%) or near-complete (>95%) inhibition of Ba/F3 BCR-ABL1 Y253H/T315I cell viability.

Fig. 2b indicates that the combination of Compound A (>30 nM) and olverembatinib (>30 nM) achieved complete (100%) inhibition of Ba/F3 BCR-ABL1 Y253H/T315I cell viability.

Fig. 3 shows that by determining the IC90 values used alone or in combination, we found that compared to the IC90 value of 1489 nM for ponatinib alone, the combination of ponatinib and compound A significantly reduced the IC90 value of ponatinib to 60.9 nM, which is even lower than the equivalent minimum plasma concentration at steady state with a clinical dose of 45 mg QD (Cssmin = 64.3 nM); while the combination with asciminib only reduced the IC90 value of ponatinib to 165 nM.

(3) Compared to single agents, the combination of Compound A with ponatinib exhibits synergistic anti-tumor effects against cells harboring BCR-ABL1 mutations E255V/T315I, Q252H/T315I, T315M, T315I, Y253H/E255K, and F359V.

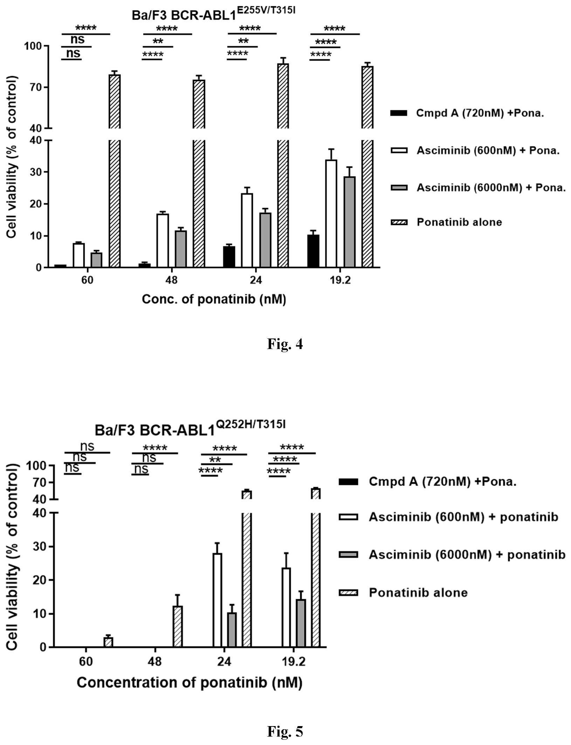

As shown in Fig. 4, single-agent ponatinib (≤ 60 nM) was unable to inhibit Ba/F3 BCR-ABL1 E255V/T315I cell viability. However, when combined with Compound A at a clinically achievable concentration (720 nM), the combination with 48 nM or 60 nM ponatinib reduced Ba/F3 BCR-ABL11 E255V/T315I cell viability by 98%; the combination with 24 nM or 19.2 nM ponatinib reduced Ba/F3 BCR-ABL11 E255V/T315I cell viability by about 90%. When asciminib was combined with ponatinib, even at concentrations of asciminib as high as 6000 nM, the inhibitory effect was still inferior to that of the combination of the Compound A and ponatinib.

As shown in Fig. 5, Ba/F3 BCR-ABL1 Q252H/T315I cell viability could be inhibited by single-agent ponatinib at 60 nM and 48 nM, but not by lower concentrations such as 24 nM or 19.2 nM of single-agent ponatinib. However, with the addition of 720 nM Compound A, its combination with ponatinib at all tested concentrations (e.g., 60 nM, 48 nM, 24 nM, or 19.2 nM) completely inhibited Ba/F3 BCR-ABL1 Q252H/T315I cell viability. The viability of Ba/F3 BCR-ABL1 Q252H/T315I cells was completely inhibited only when 600 nM or 6000 nM asciminib was used in combination with higher concentrations (60 nM and 48 nM) of ponatinib.

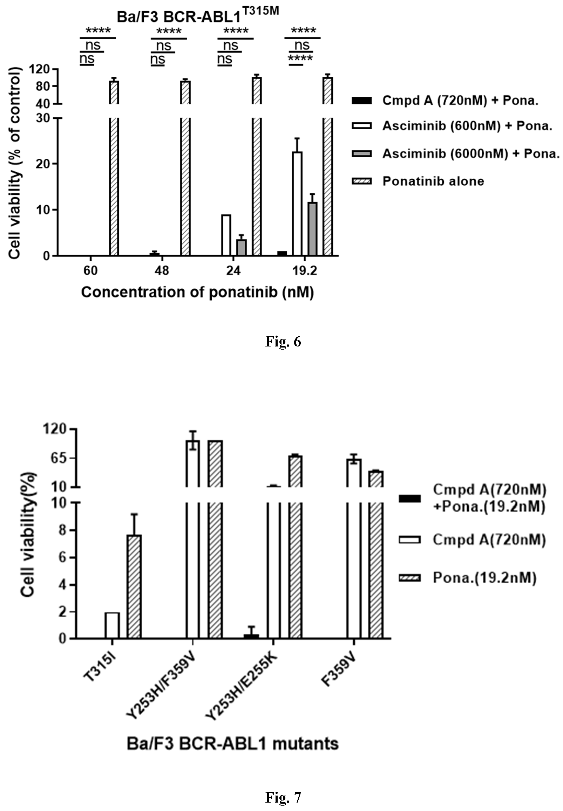

As shown in Fig. 6, single-agent ponatinib had no anti-proliferative effect on Ba/F3 BCR-ABL1 T315M, whereas Compound A or asciminib combined with 48 nM or 60 nM ponatinib restored anti-proliferative effects against Ba/F3 BCR-ABL1 T315M. However, when the concentration of ponatinib in the combination was reduced to 24 nM or 19.2 nM, only the combination with Compound A maintained the synergistic inhibitory effect.

As shown in Fig. 7, compared to the corresponding concentrations of single agents, the combination of 720 nM compound A and 19.2 nM ponatinib almost completely inhibited the viability of Ba/F3 BCR-ABL11 T315I, Y253H/F359V, Y253H/E255K, and F359V mutant cells by nearly 100%, demonstrating a strong synergistic effect.

Overall, the synergistic effect of compound A in combination with ponatinib/olverembatinib against the most challenging compound mutations and the ponatinib-resistant T315M mutation is highly significant and stronger than that of asciminib combined with ponatinib/olverembatinib.

## Example 4 Efficacy Study of Compound A in the SUP-B15 Subcutaneous Xenograft Model

SUP-B15 cells, a Philadelphia chromosome-positive acute lymphoblastic leukemia cell line, were cultured in vitro using IMDM medium supplemented with 20% fetal bovine serum. When the cell count reached the required level, the cells were harvested, counted, and inoculated.

Animals: NCG mice, female, aged 6-8 weeks, weighing 18-22 grams. A total of 35 mice (including 15 spare mice) were used. The mice were provided by Jiangsu GemPharmatech Co., Ltd. or other qualified suppliers.

Tumor inoculation: 0.1 mL (10×10^6 cells) SUP-B15 cells and 0.1 mL Matrigel (total volume 0.2 mL) were mixed thoroughly and subcutaneously inoculated into the right back of each mouse.

Animal grouping and dosing: Grouping and dosing began when the average tumor volume reached 150-200 $mm^3$. The test groups and dosing regimen are shown in the table below.

| Group | Treatment | Number | Dosage (mg/kg) | Dose volume (mL/kg) | Route of administration | Treatment duration |
|---|---|---|---|---|---|---|
| 1 | Vehicle control | 4 | - | 10 | p.o. | qd × 3 weeks |
| 2 | Compound A | 4 | 5 | 10 | p.o. | qd × 3 weeks |
| 3 | Compound A | 4 | 15 | 10 | p.o. | qd × 3 weeks |
| 4 | Compound A | 4 | 45 | 10 | p.o. | qd × 3 weeks |

Animal Husbandry: Animals must be acclimated in the experimental environment for 3-7 days after arrival before tests can commence. Animals were housed in IVC (Individually Ventilated Cages) systems within an SPF-grade animal facility, with four animals per cage. All cages, bedding, and drinking water must be sterilized prior to use. All personnel working in the animal facility must wear protective clothing and latex gloves. Each cage's information card should specify the number of animals, sex, strain, date of receipt, dosing regimen, experiment number, group, and experiment start date. Cages, feed, and drinking water were replaced twice weekly. The housing environment and lighting conditions are as follows: temperature: 20-26°C, humidity: 40-70%, light cycle: 12 hours of light, 12 hours of darkness.

Feed Composition: The feed complied with the experimental animal food identification standards. The maximum levels of contaminants were within controlled limits and are routinely inspected by the manufacturer. Drinking water was provided as high-pressure sterilized potable water.

Animal Grouping: Animals were weighed and tumor volumes were measured prior to drug administration. Grouping was performed randomly based on tumor volume.

Observation: Daily monitoring of animal health status and mortality, routine checks include observing tumor growth and the impact of drug treatment on daily behavioral performance such as activity levels, food and water intake, weight changes (measured twice a week or every other day), external signs, or other abnormalities. The number of animal deaths and side effects within each group based on the number of animals in each group were recorded.

Test indicators: The test indicators were to examine whether tumor growth is inhibited, delayed, or cured. Tumor diameter was measured three times a week using a vernier caliper. The formula for calculating tumor volume was: $V = 0.5a \times b^2$, where a and b represent the long and short diameters of the tumor, respectively.

The antitumor efficacy of the compounds was evaluated using TGI (%) or the relative tumor proliferation rate T/C (%). TGI (%) reflects the tumor growth inhibition rate. The calculation of TGI (%) is: TGI (%) = [1 - (average tumor volume at the end of treatment in a treatment group - average tumor volume at the start of administration in that group) / (average tumor volume at the end of treatment in the vehicle control group - average tumor volume at the start of treatment in the vehicle control group)] × 100%.

Relative Tumor Proliferation Rate T/C (%): The calculation formula is as follows: T/C % = TRTV / CRTV × 100% (TRTV: treatment group RTV; CRTV: negative control group RTV). The relative tumor volume (RTV) is calculated based on tumor measurement results, with the formula RTV = Vt / V0, where V0 is the average tumor volume measured at the time of grouping (i.e., d0), and Vt is the average tumor volume at a specific measurement time. TRTV and CRTV use data from the same day.

At the end of the test, tumor weight will be measured, and the Tweight/Cweight percentage will be calculated, where Tweight and Cweight represent the tumor weights of the treatment group and the vehicle control group, respectively.

Test termination: If the animal's health condition continues to deteriorate, or the tumor volume exceeds 3,000 $mm^3$, or there is severe illness, pain, or the need for euthanasia.

Data analysis: T-test is used for comparisons between two groups. One-way ANOVA is used for comparisons among three or more groups. If the F-value shows significant differences, multiple comparisons should be conducted following ANOVA analysis. All data analyses are performed using SPSS 17.0. A $p < 0.05$ is considered statistically significant.

Test results: The TGI (%) for the compound A treatment groups at doses of 5 mg/kg, 15 mg/kg, and 45 mg/kg after 21 days of administration were 119.5%, 124.7%, and 125.6%, respectively. No significant weight loss was observed in any treatment group, and tolerance was good during the treatment period.

**Example 5: A single-arm, open-label, multicenter Phase II clinical study evaluating the efficacy and safety of Compound A in the treatment of relapsed/refractory accelerated phase chronic myeloid leukemia following third-generation TKI therapy**

**Test Objectives**

Primary Objectives

Cohort 1: Evaluate the effectiveness of Compound A in treating subjects with relapsed/refractory CML-AP without T315I mutation after first-generation, second-generation, and third-generation TKI (ponatinib, olverembatinib) therapy.
Cohort 2: Evaluate the effectiveness of Compound A in treating subjects with relapsed/refractory CML-AP and T315I mutation who have received ≥2 TKI treatments (including at least one third-generation TKI).

Secondary Objectives

Further evaluate the efficacy of Compound A in CML-AP subjects in Cohort 1 and Cohort 2, including hematological response, cytogenetic response, molecular response, and clinical outcomes. The safety of Compound A in CML-AP subjects was assessed.

Exploratory Objectives

To explore the population pharmacokinetic (PopPK) characteristics of Compound A. To investigate the correlation between BCR-ABL1 kinase domain or other gene mutations and drug resistance/disease progression during Compound A treatment.

**Overall Design**

This study plans to enroll about 40 subjects with CML-AP, including 20 patients in Cohort 1 with CML-AP without T315I mutation and 20 patients in Cohort 2 with CML-AP with T315I mutation.

Subjects who pass screening will receive Compound A tablets at a dose of 240 mg once daily, taken orally on an empty stomach, with each 28-day period constituting one treatment cycle. Treatment will continue until intolerable toxicity, disease progression, death, withdrawal of informed consent by the subject, loss to follow-up, initiation of other antitumor therapy by the subject, reaching the maximum treatment cycle specified in the protocol, or termination of this study occurs, whichever comes first. During the treatment period, each subject will undergo regular assessments for hematological response, cytogenetic response, and molecular response. Safety information, including adverse events and laboratory tests, will also be evaluated.

All adverse events (AEs) and serious adverse events (SAEs) will be collected from subjects after administration of Compound A until the end of the study. The end of the study is defined as the subject completing up to 24 cycles of Compound A administration, early withdrawal, initiation of other antitumor therapy by the subject, disease progression, loss to follow-up, or death, whichever occurs first. If a subject discontinues study treatment for any reason, a final treatment follow-up will be conducted within 7 days after learning/confirming the discontinuation, and a safety follow-up will be performed 28 days (±7 days) after treatment ends. After treatment completion, subjects will undergo survival follow-up every 3 months (±7 days) until the end of this study. Once data collection for the primary endpoint is completed (about 6 months after the last subject is enrolled), a clinical study report for regulatory submission will be prepared. Further follow-up data will be summarized separately.

**Inclusion Criteria**

Patients must meet all of the following inclusion criteria to be eligible for enrollment in this study:

1. Sign a written informed consent form;
2. Subjects were aged 18 years or older at the time of screening, with no gender restrictions
3. CML-AP patients who are resistant or intolerant after third-generation TKI therapy: Cohort 1: CML-AP patients without T315I mutation, requiring prior treatment with at least first-generation, second-generation, and third-generation TKIs. Cohort 2: CML-AP patients with T315I mutation, requiring prior treatment with olverembatinib, ponatinib,

or other TKIs sensitive to T315I mutation after the T315I mutation (samples must be sent to the central laboratory before entering this study). Among these, first-generation TKIs refer to imatinib; second-generation TKIs include flumatinib, dasatinib, and nilotinib; third-generation TKIs include olverembatinib and ponatinib. Other TKIs approved overseas but not yet available domestically or undergoing clinical trials domestically may be counted as prior treatment with a corresponding line of TKI therapy. Patients must have received at least one TKI treatment during the AP phase, unless the investigator determines based on prior treatment history that no further benefit is expected. TKI treatments that were resistant or intolerant during the CP phase may be counted toward the required total number of TKIs, with the expectation that retreatment with the same TKI during the AP phase would yield a poor response. Resistance to CML-CP treatment is defined as (at least one criterion must be met): failure to achieve complete hematologic response (3 months before the treatment), minor cytogenetic response (6 months before the treatment), or major cytogenetic response (12 months before the treatment), or the emergence of new BCR-ABL1 kinase domain mutations or new clonal evolution. CML-CP patients are also considered resistant if they experience loss of response at any time during TKI treatment, develop kinase domain mutations without achieving complete cytogenetic response, or progress to CML-AP or CML-BP. Intolerance to CML-CP treatment is defined as: inability to tolerate the current TKI treatment due to toxicity, disease progression while on the current TKI, or inability to receive a higher dose due to toxicity.

4. Patients diagnosed with CML in accelerated phase according to the ELN 2020 criteria; the definition of resistance in CML-AP is as follows (at least one criterion must be met): a. At 3 months after treatment initiation: failure to achieve MaHR; b. At any time after treatment initiation, loss of MaHR; c. At any time after treatment initiation, development of new BCR::ABL kinase domain mutations in the absence of MaHR. The definition of intolerance to CML-AP treatment is as follows (at least one criterion must be met): a. Non-hematologic intolerance: Non-hematological intolerance: CML-AP subjects without MaHR who experience treatment-related grade 3 or 4 non-hematological toxicity; or those with persistent grade 2 toxicity that does not respond to optimal management, including dose adjustments (except when dose reduction is not performed to prioritize the subject's maximum benefit); b. Hematologic intolerance: CML-AP subjects without MaHR who experience treatment-related grade 4 hematological toxicity lasting more than 7 days; or those who experience recurrent grade 3 or 4 toxicity (ANC or platelets) after dose reduction to the minimum effective dose during treatment.

5. Eastern Cooperative Oncology Group (ECOG) performance status ≤ 2;

6. Minimum expected life expectancy ≥3 months;

7. Must meet the following hematological criteria (requires no blood transfusion or any blood products, and no use of cytokines within 14 days prior to testing): hemoglobin ≥ 60 g/L; absolute neutrophil count (ANC) ≥ $1.0 \times 10^9$ /L; platelets ≥ $100 \times 10^9$ /L (except those caused by the disease);

8. Good renal function, defined as serum creatinine < 1.5 times the upper limit of normal (ULN);

9. Good liver function, defined as:

- Total bilirubin <1.5×ULN;
- Alanine aminotransferase (ALT[SGPT]) and aspartate aminotransferase (AST[SGOT]) <2.5×ULN (if the liver is affected by leukemia, then ALT and AST <5×ULN);

10. Good coagulation function: Prothrombin time (PT) <1.5×ULN; International normalized ratio (INR) <1.5×ULN, activated partial thromboplastin time (APTT) <1.5×ULN;

11. Normal pancreatic function, defined as lipase and amylase <1.5×ULN;

12. Screening results assessed by electrocardiogram indicate a normal QTc interval, defined as ≤450 ms for males and ≤470 ms for females;

13. Negative pregnancy test during the screening period (for women of childbearing potential);

14. Agreement to use effective contraception with their sexual partners throughout the entire study period (from the date of signing the informed consent form until 28 days after the last dose) (only for male and female subjects of childbearing potential).

**Exclusion criteria**

Patients with any of the following conditions cannot be enrolled in this study:

1. Receiving TKI therapy within 5 half-lives prior to the first dose of the test drug, or non-hematological AEs related to previous TKI use have not recovered to Grade 1 or below (NCI CTCAE V5.0) (except for alopecia);

2. Receiving other anti-tumor treatments and meet any of the following conditions: received anagrelide within 24 hours before the first dose of the test drug, received interferon, cytarabine, or immunotherapy within 14 days before the first dose, or received any other cytotoxic chemotherapy (except hydroxyurea), radiation therapy, or investigational

therapy (excluding any TKI-based investigational therapies) within 28 days before the first dose;
3. Requiring concurrent immunosuppressive therapy (except for corticosteroids used for short-term treatment, defined as: within 7 days, with a daily hormone dose less than 30 mg of prednisone or an equivalent dose of other corticosteroids);
4. Having taken medications associated with torsades de pointes within 1 month of the screening period;
5. Having concomitant diseases requiring the use of drugs that may have drug interactions with the test drug (such as CYP2C8 substrates and moderate to strong CYP3A4 inducers or inhibitors);
6. Patients who have previously undergone autologous or allogeneic hematopoietic stem cell transplantation;
7. Subjects with active central nervous system disease;
8. CML-AP patients who have achieved a major hematologic response (MaHR);
9. CML-AP patients who have previously progressed to blast phase (BP);
10. Having significant or uncontrolled cardiovascular disease, including but not limited to:

- Myocardial infarction within 3 months prior to the first dose;
- Unstable angina within 3 months prior to the first dose;
- Congestive heart failure within 3 months prior to the first dose;
- Left ventricular ejection fraction (LVEF) ≤50%, with echocardiography showing pulmonary artery systolic pressure >50 mmHg; or presence of clinical symptoms related to pulmonary hypertension;

11. Having history of any cardiac or vascular disease, such as hypertension (systolic blood pressure >140 mmHg and/or diastolic blood pressure >90 mmHg), or subjects requiring medications known to potentially prolong the QT interval on electrocardiogram. Patients with primary hypertension may be enrolled if their blood pressure is stably controlled with antihypertensive medication (<140 mmHg/90 mmHg, and no blood pressure fluctuations exceeding 160 mmHg/100 mmHg within the past month);
12. Use of traditional Chinese medicine with clear anti-tumor indications within 2 weeks prior to the first administration of the test drug;
13. Suffering from a severe bleeding disorder unrelated to CML;
14. Having previous history of severe cardiovascular diseases (including myocardial infarction, unstable angina, severe arrhythmia, and congestive heart failure, etc.) during prior TKI treatment for CML;
15. Having history of pancreatitis or alcohol abuse within 3 years before the first dose;
16. Having uncontrolled hypertriglyceridemia (triglycerides >450 mg/dL, i.e., 5.1 mmol/L);
17. Having malabsorption syndrome or other gastrointestinal diseases that may affect the absorption of the test drug;
18. Having been diagnosed with another primary malignancy within the past 5 years (except for non-melanoma skin cancer, carcinoma in situ of the cervix, or controlled prostate cancer that has been cured within 5 years);
19. Having underwent major surgery within 14 days prior to the first dose (excluding minor surgeries such as bone marrow biopsy);
20. Persistent or active infection (including known Treponema pallidum, human immunodeficiency virus [HIV], hepatitis B virus [HBV], or hepatitis C virus [HCV] infection; active hepatitis B virus infection is defined as: for subjects positive for hepatitis B surface antigen, HBV DNA ≥2000 copies/ml or ≥500 IU/ml; active hepatitis C virus infection is defined as: for subjects positive for HCV antibodies, hepatitis C virus RNA positive);
21. Any condition that, in the opinion of the investigator or medical monitor, makes the subject unsuitable for participation in this trial, such as the presence of a disease that affects the safety assessment of the study drug, poor patient compliance, etc.

**Efficacy Analysis**

The efficacy analysis will use the RES for the primary analysis and the FAS for the supplementary analysis. For the primary estimation objective, the number and proportion of subjects achieving MaHR (CHR and NEL) within 6 months will be summarized, and the MaHR rate along with its two-sided 95% Clopper-Pearson confidence interval will be calculated.

Sensitivity analysis will be based on the FAS, using the same method as the primary estimation objective.

For the secondary endpoints, including MaHR rate, CHR rate, MCyR rate, CCyR rate, MMR rate, and MCyR/CCyR/MMR rates at the end of the first 6 cycles of treatment, the same method as for the primary endpoints will be used. The Kaplan-Meier method will be employed to estimate survival curves for TTR, DOR, PFS, and OS, calculating the first quartile, median, and third quartile, with their two-sided 95% confidence intervals estimated using the Brookmeyer-Crowley method.

If data permit, an exploratory analysis will also be conducted on the relationship between BCR::ABL1 T315I mutation status and clinical efficacy.

**Example 6 Study of Blood-Brain Ratio of Compound A in Male SD Rats**

A total of 15 male SD rats (3 per time point) were administered a single oral dose of Compound A via oral gavage at 50 mg/kg, with the vehicle being 0.5% sodium carboxymethyl cellulose (CMC-Na). The animals were not fasted. Whole blood, cerebrospinal fluid, and brain tissue were collected at 0.5, 2, 4, 8, and 24 hours post-dose. Whole blood samples (about 0.3 mL) were collected via puncture of the jugular venous sinus and transferred to dipotassium ethylenediaminetetraacetate (K2EDTA) anticoagulant tubes, mixed thoroughly, placed on wet ice, and centrifuged within 30 minutes for 5 minutes (6000 rpm, 4°C) to obtain plasma. Cerebrospinal fluid (about 0.10 mL) was collected via puncture of the cerebellomedullary cistern and placed in collection tubes. Brain tissue samples were rinsed with saline, dried with filter paper, blood vessels were removed, and the weight was weighed and recorded. Brain tissue was homogenized with 9 times its weight of homogenization buffer (30% MeOH, containing 0.2% FA/70% 10 mM PBS). All samples were stored in a freezer at -80°C or lower until LC-MS analysis. PK parameters were calculated using the non-compartmental model in WinNonlin and the compound concentrations in each matrix.

The assay results indicate that the Cmax and $AUC_{0\text{-}last}$ of compound A in the brain were 1130 ng/g and 16346 hr*ng/g, respectively. The brain-to-plasma concentration ratio of compound A ranged from 0.44 to 0.74, suggesting that compound A can cross the blood-brain barrier. Specific results are detailed in Table 13 below.

Table 13 Brain Concentration and Blood-Brain Ratio of Compound A in Male SD Rats at Different Time Points After a Single Oral Administration of 50 mg/kg

| Route | Rat # | Time (hr) | Brain Concentration (ng/g) | | | | Brain/Blood Ratio | | | |
|---|---|---|---|---|---|---|---|---|---|---|
| | | | Concentration | Mean | SD | CV (%) | Individual Value | Mean | SD | CV (%) |
| PO (50 mg/kg) | 1 | 0.5 | 415 | 366 | 58 | 15.8 | 0.624 | 0.526 | 0.132 | 25.1 |
| | 2 | | 302 | | | | 0.376 | | | |
| | 3 | | 381 | | | | 0.578 | | | |
| | 4 | 2 | 961 | 1130 | 206 | 18.2 | 0.439 | 0.450 | 0.047 | 10.5 |
| | 5 | | 1360 | | | | 0.410 | | | |
| | 6 | | 1070 | | | | 0.502 | | | |
| | 7 | 4 | 1190 | 1127 | 222 | 19.7 | 0.478 | 0.440 | 0.057 | 12.9 |
| | 8 | | 1310 | | | | 0.466 | | | |
| | 9 | | 880 | | | | 0.374 | | | |
| | 10 | 8 | 452 | 1049 | 626 | 59.7 | 0.511 | 0.474 | 0.033 | 7.06 |
| | 11 | | 1700 | | | | 0.447 | | | |
| | 12 | | 994 | | | | 0.462 | | | |
| | 13 | 24 | 40.4 | 16.9 | NA | NA | 0.488 | 0.739 | NA | NA |
| | 14 | | NA | | | | NA | | | |
| | 15 | | 10.2 | | | | 0.990 | | | |

**Example 7: Tissue Distribution Study Using Quantitative Whole-Body Autoradiography (QWBA) Following a Single Oral Gavage Administration of 50 mg/100 μCi/kg [$^{14}C$]-Compound A to Male Long-Evans Rats and Male and Female Sprague-Dawley Rats**

This test utilized a total of 8 male Long Evans (LE) rats, randomly designated as Gt1M01 to Gt1M08; and 4 Sprague-Dawley (SD) rats, with an equal number of males and females, randomly designated as Gt2M01 to Gt2M02 and Gt2F01 to Gt2F02. Each rat received a single oral dose of 50 mg/100 μCi/kg of [$^{14}C$]-Compound A via gavage. SD rats were euthanized at 4 hours and 72 hours post-dose (with the 72-hour rats serving as backups); LE rats were euthanized at 0.5 hours, 4 hours, 24 hours, 72 hours, 168 hours, 336 hours, 504 hours, and 840 hours post-dose (with the 504-hour and 840-hour rats serving as backups). According to the rat sequence numbers, one male and one female SD rat and one male LE rat were transferred to an induction chamber, euthanized with an overdose of carbon dioxide, and about 4 mL of whole blood was collected via cardiac puncture. Immediately after blood collection, the carcasses were immersed in a dry ice-hexane bath for freezing and fixation.

Plasma samples were obtained by low-temperature centrifugation of whole blood. After homogenization, aliquots of each plasma sample were mixed directly with scintillation fluid, and the radioactivity was measured using a liquid scintillation counter (LSC). Frozen carcasses were sectioned, dried, and exposed/developed to determine total radioactivity in tissues/regions (including gastrointestinal contents). Pharmacokinetic parameters for total radioactivity in plasma and tissues were calculated using a non-compartmental model with WinNonLin software (version 8.3, Certara). After development of sections at each time point, 46 tissues/regions (including gastrointestinal contents) were analyzed for LE rats, and 45 tissues/regions (including gastrointestinal contents) were analyzed for SD rats. The radioactivity concentrations in unanalyzed regions were all lower than the lowest concentration in the analyzed tissues or regions.

Test results: The quantifiable whole-brain-to-plasma radioactivity concentration ratio ranged from 0.58 to 0.69, indicating that Compound A can cross the blood-brain barrier. Specific results are presented in Table 14 below:

Table 14 Total radioactivity (ng Eq. /g) in plasma and whole brain following a single oral dose of 50 mg/100 μCi/kg of [$^{14}$C]-Compound A to rats via gavage

| Category | Time After Administration | Total Radioactivity (ng Eq./g) | |
|---|---|---|---|
| | | Plasma | Whole brain |
| Female SD rats | 4 hrs | 15320 | 9800 |
| Male SD rats | 4 hrs | 10656 | 6400 |
| Male LE rats | 0.5 hrs | 7131 | 4885 |
| Male LE rats | 4 hrs | 11981 | 6950 |

**Example 8: Pharmacodynamic Study on the Treatment of an Animal Model Inoculated via the Carotid Artery with K562-luc Human Leukemia Cells**

K562-luc tumor cells were cultured in IMDM medium containing 10% fetal bovine serum and 1500 ng/mL puromycin in an incubator at 37°C with 5% $CO_2$. The medium was replaced every 2 to 3 days. The cells were subcultured when the confluence reaches 80% - 90%. Tumor cells in the logarithmic growth phase were used for in vivo tumor inoculation.

Test animals: NCG mice, 6-8 weeks old, female, all handled according to SOP. The animals were housed in an SPF facility accredited by AAALAC (Association for Assessment and Accreditation of Laboratory Animal Care), in a constant-temperature and constant-humidity laminar flow clean room, using individually ventilated cages (IVC). Four mice were housed per cage, made of polycarbonate, with dimensions of 300 mm × 180 mm × 150 mm$^3$. The temperature was maintained at 22 ± 3°C, relative humidity at 40%-80%, and the light cycle was 12 hours. Autoclaved soft corn cob bedding was used and replaced once per week.

After intramuscular injection of Zoletil for anesthesia, the neck skin of each animal was disinfected with iodine and 75% alcohol. The mouse was placed supine on a neck surgery board with the upper incisors hooked and both forelimbs abducted and fixed. The skin over the sternoclavicular joint was grasped with forceps, and a 1 cm longitudinal incision was made through the skin and subcutaneous fat to expose the right submandibular gland. The right submandibular gland was separated medially, and the right common carotid artery was located along the lateral side of the trachea. The right common carotid artery was gently dissected, and sutures were placed at the distal and proximal ends for ligation. The proximal end of the common carotid artery was ligated first. The artery was gently lifted with forceps, and K562-luc human leukemia tumor cells ($2 \times 10^5$ cells/100 μL) resuspended in PBS were slowly injected into the carotid artery using an insulin syringe at a point between the two ligatures. Immediately after injection, the distal ligature was tightened. Excess suture material was trimmed, the muscle was repositioned to cover the wound, and the skin was closed using a wound clip. Tumor growth was monitored once weekly using an in vivo imaging system. Approximately 10 days after tumor cell inoculation, based on body weight and optical signal intensity at the tumor site, animals were randomly divided into 3 groups with 8 mice per group. The dosing regimen is shown in the table below:

| Group | Treatment | Number | Dose (mg/kg) | Dosing volume (mL/kg) | Route of Administration | Regimen |
|---|---|---|---|---|---|---|
| 1 | Vehicle control | 8 | - | 10 | p.o. | qd × 3 weeks |
| 2 | Compound A | 8 | 50 | 10 | p.o. | qd × 3 weeks |

After surgery, the analgesic meloxicam was administered subcutaneously at a dose of 5 mg/kg per animal. The

antibiotic levofloxacin (0.1 mg/mL) was added to the drinking water of the animals for 3-7 consecutive days.

For routine monitoring, not only tumor growth but also behavioral changes of the animals were observed, including animal movement, food and water consumption (by cage-side observation), body weight changes, whether the eyes/hair were glossy, and any other abnormal effects. Any deaths and/or clinical signs of abnormalities were recorded.

During the test, all animals were weighed three times per week. The percentage of body weight change (BW change, %) was calculated using the formula: BW change = (BW Day X / BW Day 0) × 100, where BW Day X is the body weight on the day of dosing, and BW Day 0 is the body weight on the day of grouping.

The small animal in vivo imaging system IVIS Lumina III (Perkin Elmer) was used to image the mice once per week (depending on mouse condition) to monitor the bioluminescence imaging (BLI) signal intensity at the tumor cell inoculation site (unit: photons/s) as the primary indicator for evaluating tumor growth and drug efficacy. The specific procedure was as follows:

After intraperitoneal injection of D-luciferin (Perkin Elmer, 15 mg/mL, injected at 5 μL/g body weight), mice were anesthetized by inhalation of 1%-2% isoflurane. Approximately 10 minutes after D-luciferin injection, imaging was performed using the IVIS Lumina III. The imaging results were analyzed using Living Image software (Perkin Elmer), and the optical signal intensity within the whole-body region of interest (ROI) was calculated for each animal. Percentage reduction of BLI was calculated as: BLI reduction % = (1 - BLI treatment / BLI control) × 100%, where BLI treatment and BLI control are the mean BLI values of the treatment group and control group, respectively.

Statistical analysis: Statistical analysis between groups was performed using SPSS 16.0 software. Mean and standard error were calculated for all measured parameters according to the experimental design. The significance level was set at $P < 0.05$.

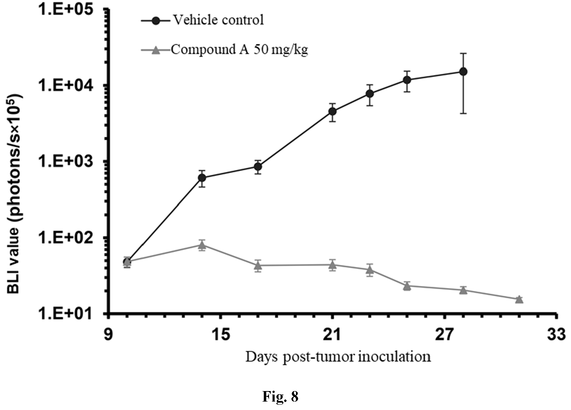

Results: In the K562-luc brain metastasis model, on day 28 after dosing, Compound A significantly inhibited the fluorescent signal, indicating that the BLI signal intensity of brain tumors in the treatment group was significantly reduced compared with that of the control group, as shown in Fig. 8. Furthermore, during the treatment period, the mice did not show significant body weight loss, indicating good tolerability.

The above content provides further detailed explanations of the present disclosure in conjunction with specific alternative examples, and it should not be construed that the specific implementations of the present disclosure are limited to these descriptions. For those of ordinary skill in the technical field to which the present disclosure pertains, without departing from the inventive concept, several simple deductions or substitutions can be made, all of which should be considered within the protection scope of the present disclosure.

## Claims

**1.** Use of a compound of formula (I) or a pharmaceutically acceptable salt, crystal form, solvate, or hydrate thereof in the manufacture of a medicament for treating chronic myeloid leukemia (CML) or acute lymphoblastic leukemia (ALL), wherein the compound of formula (I) has the following structure:

(I)

wherein:

$Y_1$ is independently selected from $CR_a$ or N;
Y is independently selected from $CR_a$ or N;
$R_1$ is selected from hydrogen, halogen, cyano, nitro, $C_{1-6}$ alkyl or $C_{1-6}$ haloalkyl, wherein the $C_{1-6}$ alkyl or $C_{1-6}$ haloalkyl is optionally substituted with an $R_{1a}$ group;
$R_2$ is selected from hydrogen, $C_{1-6}$ alkyl or $C_{1-6}$ haloalkyl, wherein the $C_{1-6}$ alkyl or $C_{1-6}$ haloalkyl is optionally substituted with an $R_{2a}$ group;
Z is a chemical bond, O, $S(O)_{0-2}$ or $NR_b$;
or, $-Z-R_2$ together represents $-SF_5$;
Ar is a six-membered heteroaryl containing at least one N atom, optionally substituted with R groups;
Het is

wherein $X_9$ is selected from O, S, $NR_b$ or $C(R)_2$;
m is 0, 1 or 2;
n is 0, 1, 2, 3, 4, 5 or 6;
$R_a$ is independently selected from hydrogen, halogen, cyano, nitro, hydroxy, $-NH_2$, $-NHC_{1-6}$ alkyl, $-N(C_{1-6}\text{ alkyl})_2$, $C_{1-6}$ alkyl, $C_{1-6}$ haloalkyl or $C_{1-6}$ alkoxy;
$R_b$ is independently selected from hydrogen, $C_{1-6}$ alkyl or $C_{1-6}$ haloalkyl;
$R_{1a}$, $R_{2a}$ and R are independently selected from hydrogen, halogen, hydroxy, $-NH_2$, $-NHC_{1-6}$ alkyl, $-N(C_{1-6}\text{ alkyl})_2$, $C_{1-6}$ alkyl, $C_{1-6}$ haloalkyl, $C_{1-6}$ alkoxy, $C_{3-7}$ cycloalkyl, $C_{3-7}$ heterocycloalkyl, $C_{6-10}$ aryl or $C_{5-10}$ heteroaryl;
alternatively, two R groups on the same atom or adjacent atoms may together form a $C_{3-7}$ cycloalkyl, $C_{3-7}$ heterocycloalkyl, $C_{6-10}$ aryl or $C_{5-10}$ heteroaryl.

**2.** A method for treating chronic myeloid leukemia (CML) or acute lymphoblastic leukemia (ALL) in a patient, comprising administering to the patient a therapeutically effective amount of a compound of formula (I) or a pharmaceutically acceptable salt, crystal form, solvate, or hydrate thereof, wherein the compound of formula (I) has the following structure:

(I)

wherein:

$Y_1$ is selected from $CR_a$ or N;
Y is independently selected from $CR_a$ or N;
$R_1$ is selected from hydrogen, halogen, cyano, nitro, $C_{1-6}$ alkyl or $C_{1-6}$ haloalkyl, wherein the $C_{1-6}$ alkyl or $C_{1-6}$ haloalkyl is optionally substituted with an $R_{1a}$ group;
$R_2$ is selected from hydrogen, $C_{1-6}$ alkyl or $C_{1-6}$ haloalkyl, wherein the $C_{1-6}$ alkyl or $C_{1-6}$ haloalkyl is optionally substituted with an $R_{2a}$ group;
Z is a chemical bond, O, $S(O)_{0-2}$ or $NR_b$;
or, $-Z-R_2$ together represents $-SF_5$;
Ar is a six-membered heteroaryl containing at least one N atom, optionally substituted with R groups;
Het is

;

wherein $X_9$ is selected from O, S, $NR_b$ or $C(R)_2$;
m is 0, 1 or 2;
n is 0, 1, 2, 3, 4, 5 or 6;
$R_a$ is independently selected from hydrogen, halogen, cyano, nitro, hydroxy, $-NH_2$, $-NHC_{1-6}$ alkyl, $-N(C_{1-6}\text{ alkyl})_2$, $C_{1-6}$ alkyl, $C_{1-6}$ haloalkyl or $C_{1-6}$ alkoxy;
$R_b$ is independently selected from hydrogen, $C_{1-6}$ alkyl or $C_{1-6}$ haloalkyl;
$R_{1a}$, $R_{2a}$ and R are independently selected from hydrogen, halogen, hydroxy, $-NH_2$, $-NHC_{1-6}$ alkyl, $-N(C_{1-6}\text{ alkyl})_2$, $C_{1-6}$ alkyl, $C_{1-6}$ haloalkyl, $C_{1-6}$ alkoxy, $C_{3-7}$ cycloalkyl, $C_{3-7}$ heterocycloalkyl, $C_{6-10}$ aryl or $C_{5-10}$ heteroaryl;
alternatively, two R groups on the same atom or adjacent atoms may together form a $C_{3-7}$ cycloalkyl, $C_{3-7}$ heterocycloalkyl, $C_{6-10}$ aryl or $C_{5-10}$ heteroaryl.

**3.** A compound of formula (I) or a pharmaceutically acceptable salt, crystal form, solvate, or hydrate thereof, for use in treating chronic myeloid leukemia (CML) or acute lymphoblastic leukemia (ALL), wherein the compound of formula (I) has the following structure:

(I)

wherein:

$Y_1$ is independently selected from $CR_a$ or N;
Y is independently selected from $CR_a$ or N;
$R_1$ is selected from hydrogen, halogen, cyano, nitro, $C_{1-6}$ alkyl or $C_{1-6}$ haloalkyl, wherein the $C_{1-6}$ alkyl or $C_{1-6}$ haloalkyl is optionally substituted with an $R_{1a}$ group;
$R_2$ is selected from hydrogen, $C_{1-6}$ alkyl or $C_{1-6}$ haloalkyl, wherein the $C_{1-6}$ alkyl or $C_{1-6}$ haloalkyl is optionally substituted with an $R_{2a}$ group;
Z is a chemical bond, O, $S(O)_{0-2}$ or $NR_b$;
or, $-Z-R_2$ together represents $-SF_5$;
Ar is a six-membered heteroaryl containing at least one N atom, optionally substituted with R groups;
Het is

;

wherein $X_9$ is selected from O, S, $NR_b$ or $C(R)_2$;
m is 0, 1 or 2;
n is 0, 1, 2, 3, 4, 5 or 6;
$R_a$ is independently selected from hydrogen, halogen, cyano, nitro, hydroxy, $-NH_2$, $-NHC_{1-6}$ alkyl, $-N(C_{1-6}$ alkyl$)_2$, $C_{1-6}$ alkyl, $C_{1-6}$ haloalkyl or $C_{1-6}$ alkoxy;
$R_b$ is independently selected from hydrogen, $C_{1-6}$ alkyl or $C_{1-6}$ haloalkyl;
$R_{1a}$, $R_{2a}$ and R are independently selected from hydrogen, halogen, hydroxy, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{3-7}$ cycloalkyl, $C_{3-7}$ heterocycloalkyl, $C_{6-10}$ aryl or $C_{5-10}$ heteroaryl;
alternatively, two R groups on the same atom or adjacent atoms may together form a $C_{3-7}$ cycloalkyl, $C_{3-7}$ heterocycloalkyl, $C_{6-10}$ aryl or $C_{5-10}$ heteroaryl.

**4.** The use according to claim 1, the method according to claim 2, or the compound for use according to claim 3, wherein the compound of formula (I) is (R)-N-(4-(chlorodifluoromethoxy)phenyl)-6-(3-fluoropyrrolidin-1-yl)-5-(pyrazin-2-yl) nicotinamide (Compound A) or a pharmaceutically acceptable salt, crystal form, solvate, or hydrate thereof.

**5.** The use, method, or the compound for use according to any one of claims 1-4, wherein the chronic myeloid leukemia is chronic myeloid leukemia in chronic phase, optionally central nervous system leukemia relapse thereof, such as brain metastasis.

**6.** The use, method, or the compound for use according to any one of claims 1-4, wherein the chronic myeloid leukemia is chronic myeloid leukemia in accelerated phase, optionally central nervous system leukemia relapse thereof, such as brain metastasis.

**7.** The use, method, or the compound for use according to any one of claims 1-4, wherein the chronic myeloid leukemia is chronic myeloid leukemia in blast phase, optionally central nervous system leukemia relapse thereof, such as brain metastasis.

**8.** The use, method, or the compound for use according to any one of claims 1-7, wherein the chronic myeloid leukemia is

Philadelphia chromosome-positive (Ph+).

**9.** The use, method, or the compound for use according to any one of claims 1-4, wherein the acute lymphoblastic leukemia is Philadelphia chromosome-positive (Ph+), optionally central nervous system leukemia relapse thereof, such as brain metastasis.

**10.** The use, method, or the compound for use according to any one of claims 1-9, wherein the chronic myeloid leukemia is BCR-ABL1 fusion gene-positive;

optionally, the BCR-ABL1 fusion gene is wild-type;
optionally, the BCR-ABL1 fusion gene is mutant; optionally, the mutation is selected from G250E, Q252H, Y253H, E255K/V, M244V, H396P, F317L, E459K, E355G, V299L, T315I, M351T, F359V, P223S, and I502L, alternatively G250E, Q252H, Y253H, E255K/V, E459K, T315I, and M351T, alternatively T315I.

**11.** The use, method, or the compound for use according to any one of claims 1-10, wherein the chronic myeloid leukemia has progressed after treatment with another therapy;

optionally, wherein the other therapy is selected from radiation therapy, drug therapy, or surgical therapy;
optionally, wherein the drug therapy is chemotherapy, targeted therapy, or immunotherapy;
optionally, wherein the drug therapy is chemotherapy;
optionally, wherein the drug therapy is targeted therapy;
optionally, wherein the drug for the drug therapy is selected from one or more of imatinib, dasatinib, nilotinib, BCR-ABL tyrosine kinase inhibitors, olverembatinib, hydroxyurea, ponatinib, asciminib, interferon, cytarabine, azacitidine, interferon alfa-1b, homoharringtonine, decitabine, peginterferon alfa-2a, thalidomide, chidamide, or recombinant interferon alfa-2b.

**12.** The use, method, or the compound for use according to any one of claims 1-11, wherein the chronic myeloid leukemia has progressed after treatment with a BCR-ABL tyrosine kinase inhibitor; optionally, wherein the BCR-ABL tyrosine kinase inhibitor(s) is selected from imatinib, dasatinib, nilotinib, olverembatinib, ponatinib or asciminib.

**13.** The use, method, or the compound for use according to any one of claims 1-12, wherein the patient is an adult patient.

**14.** The use, method, or the compound for use according to any one of claims 1-13, wherein the patient is a pediatric patient.

**15.** The use, method, or the compound for use according to any one of claims 1-14, wherein the patient has received one or more lines of treatment; optionally, wherein the patient has received two or more lines of treatment; optionally, wherein the patient has received three or more lines of treatment; optionally, the patient has received one, two, three, four, or five lines of treatment.

**16.** The use, method, or the compound for use according to any one of claims 1-15, wherein the patient has previously received treatment with ≥1 different BCR-ABL1 tyrosine kinase inhibitor(s) and subsequently experienced disease progression to accelerated phase or blast phase, with treatment evaluation results being failure, warning, or intolerance;

alternatively, wherein the patient has previously received treatment with ≥2 different BCR-ABL1 tyrosine kinase inhibitor(s) and subsequently experienced disease progression to accelerated phase or blast phase, with treatment evaluation results being failure, warning, or intolerance;
alternatively, wherein the patient has previously received treatment with ≥3 different BCR-ABL1 tyrosine kinase inhibitor(s) and subsequently experienced disease progression to accelerated phase or blast phase, with treatment evaluation results being failure, warning, or intolerance;
alternatively, wherein the patient has previously received treatment with ≥4 different BCR-ABL1 tyrosine kinase inhibitor(s) and subsequently experienced disease progression to accelerated phase or blast phase, with treatment evaluation results being failure, warning, or intolerance;
alternatively, wherein the patient has previously received treatment with ≥1 different BCR-ABL1 ATP-competitive tyrosine kinase inhibitor(s) and subsequently experienced disease progression to accelerated phase or blast phase, with treatment evaluation results being failure, warning, or intolerance;
alternatively, wherein the patient has previously received treatment with ≥2 different BCR-ABL1 ATP-competitive

tyrosine kinase inhibitor(s) and subsequently experienced disease progression to accelerated phase or blast phase, with treatment evaluation results being failure, warning, or intolerance;
alternatively, wherein the patient has previously received treatment with ≥3 different BCR-ABL1 ATP-competitive tyrosine kinase inhibitor(s) and subsequently experienced disease progression to accelerated phase or blast phase, with treatment evaluation results being failure, warning, or intolerance;
alternatively, wherein the patient has previously received treatment with ≥4 different BCR-ABL1 ATP-competitive tyrosine kinase inhibitor(s) and subsequently experienced disease progression to accelerated phase or blast phase, with treatment evaluation results being failure, warning, or intolerance.

**17.** The use, method, or the compound for use according to any one of claims 1-16, wherein the patient has previously received treatment with ≥1 different BCR-ABL1 tyrosine kinase inhibitors and subsequently experienced relapse or became refractory;

alternatively, wherein the patient has previously received treatment with ≥2 different BCR-ABL1 tyrosine kinase inhibitors and subsequently experienced relapse or became refractory; alternatively, wherein the patient has previously received treatment with ≥3 different BCR-ABL1 tyrosine kinase inhibitors and subsequently experienced relapse or became refractory; alternatively, wherein the patient has previously received treatment with ≥4 different BCR-ABL1 tyrosine kinase inhibitors and subsequently experienced relapse or became refractory;
alternatively, wherein the patient has previously received treatment with ≥1 different BCR-ABL1 ATP-competitive tyrosine kinase inhibitors and subsequently experienced relapse or became refractory;
alternatively, wherein the patient has previously received treatment with ≥2 different BCR-ABL1 ATP-competitive tyrosine kinase inhibitors and subsequently experienced relapse or became refractory;
alternatively, wherein the patient has previously received treatment with ≥3 different BCR-ABL1 ATP-competitive tyrosine kinase inhibitors and subsequently experienced relapse or became refractory;
alternatively, wherein the patient has previously received treatment with ≥4 different BCR-ABL1 ATP-competitive tyrosine kinase inhibitors and subsequently experienced relapse or became refractory.

**18.** The use, method, or the compound for use according to any one of claims 16-17, wherein the BCR-ABL1 tyrosine kinase inhibitor(s) is selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449, XL228, asciminib or HS-10382;

alternatively, BCR-ABL1 tyrosine kinase inhibitor(s) is selected from imatinib, dasatinib, nilotinib, bosutinib, flumatinib, ponatinib, olverembatinib, radotinib or asciminib;
alternatively, BCR-ABL1 tyrosine kinase inhibitor(s) is selected from imatinib, dasatinib, nilotinib, ponatinib, olverembatinib or asciminib;
alternatively, BCR-ABL1 tyrosine kinase inhibitor(s) is selected from imatinib, dasatinib, nilotinib, bosutinib, flumatinib or radotinib;
alternatively, BCR-ABL1 tyrosine kinase inhibitor(s) is selected from imatinib, dasatinib or nilotinib;
alternatively, BCR-ABL1 tyrosine kinase inhibitor(s) is selected from ponatinib, olverembatinib, asciminib or HS-10382;
alternatively, BCR-ABL1 tyrosine kinase inhibitor(s) is selected from ponatinib or olverembatinib;
alternatively, BCR-ABL1 tyrosine kinase inhibitor(s) is selected from asciminib or HS-10382; alternatively, BCR-ABL1 tyrosine kinase inhibitor(s) is a first-line, second-line, third-line, fourth-line, or fifth-line therapeutic agent;
alternatively, BCR-ABL1 tyrosine kinase inhibitor(s) is a first-line, second-line, or third-line therapeutic agent.

**19.** The use, method, or the compound for use according to any one of claims 16-17, wherein BCR-ABL1 ATP-competitive tyrosine kinase inhibitor(s) is selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228;

alternatively, BCR-ABL1 ATP-competitive tyrosine kinase inhibitor(s) is selected from imatinib, dasatinib, nilotinib, bosutinib, flumatinib, ponatinib, olverembatinib or radotinib;
alternatively, BCR-ABL1 ATP-competitive tyrosine kinase inhibitor(s) is selected from imatinib, dasatinib, nilotinib, ponatinib or olverembatinib;
alternatively, BCR-ABL1 ATP-competitive tyrosine kinase inhibitor(s) is selected from imatinib, dasatinib, nilotinib, bosutinib, flumatinib or radotinib;
alternatively, BCR-ABL1 ATP-competitive tyrosine kinase inhibitor(s) is selected from imatinib, dasatinib or

nilotinib;
alternatively, BCR-ABL1 ATP-competitive tyrosine kinase inhibitor(s) is selected from ponatinib or olverembatinib;
alternatively, BCR-ABL1 ATP-competitive tyrosine kinase inhibitor(s) is a first-line, second-line, third-line, fourth-line, or fifth-line therapeutic agent;
alternatively, BCR-ABL1 ATP-competitive tyrosine kinase inhibitor(s) is a first-line, second-line, or third-line therapeutic agent.

**20.** The use, method, or the compound for use according to any one of claims 1-19, wherein the patient has previously received treatment with ≥1 different first-generation and/or second-generation and/or third-generation BCR-ABL1 ATP-competitive tyrosine kinase inhibitors and subsequently developed resistance or intolerance;

alternatively, the patient has previously received treatment with ≥2 different first-generation and/or second-generation and/or third-generation BCR-ABL1 ATP-competitive tyrosine kinase inhibitors and subsequently developed resistance or intolerance;
alternatively, the patient has previously received treatment with ≥3 different first-generation and/or second-generation and/or third-generation BCR-ABL1 ATP-competitive tyrosine kinase inhibitors and subsequently developed resistance or intolerance;
alternatively, the patient has previously received treatment with ≥4 different first-generation and/or second-generation and/or third-generation BCR-ABL1 ATP-competitive tyrosine kinase inhibitors and subsequently developed resistance or intolerance.

**21.** The use, method, or the compound for use according to any one of claims 1-20, wherein the resistance arises from BCR-ABL1 kinase domain point mutation(s);

optionally, the BCR-ABL1 kinase domain point mutation(s) is selected from one or more of T315I/A, Y253F/H, E255V/K, M351T, G250E, F359V/I/C, H396P/R, M244V, E355G, F317L/V/I/C, F311L, M237I, Q252H/R, D276G, L248V, V379I, L384M, L387M, E279K, F486S, E459K, P223S, I502L or V299L;
optionally, the BCR-ABL1 kinase domain point mutation(s) is selected from one or more of T315I, Y253H, E255V/K, M351T, G250E, F359V/, H396P, M244V, E355G, F317L, Q252H, E459K, P223S, I502L or V299L.

**22.** The use, method, or the compound for use according to any one of claims 1-21, wherein the patient has previously received treatment with ≥1 different first-generation and/or second-generation and/or third-generation BCR-ABL1 ATP-competitive tyrosine kinase inhibitors and subsequently experienced relapse or became refractory;

alternatively, the patient has previously received treatment with ≥2 different first-generation and/or second-generation and/or third-generation BCR-ABL1 ATP-competitive tyrosine kinase inhibitors and subsequently experienced relapse or became refractory;
alternatively, the patient has previously received treatment with ≥3 different first-generation and/or second-generation and/or third-generation BCR-ABL1 ATP-competitive tyrosine kinase inhibitors and subsequently experienced relapse or became refractory;
alternatively, the patient has previously received treatment with ≥4 different first-generation and/or second-generation and/or third-generation BCR-ABL1 ATP-competitive tyrosine kinase inhibitors and subsequently experienced relapse or became refractory.

**23.** The use, method, or the compound for use according to any one of claims 20-22, wherein the first-generation and/or second-generation and/or third-generation BCR-ABL1 ATP-competitive tyrosine kinase inhibitor(s) is selected from imatinib, dasatinib, nilotinib, bosutinib, flumatinib or radotinib;

alternatively, the first-generation and/or second-generation and/or third-generation BCR-ABL1 ATP-competitive tyrosine kinase inhibitor(s) is selected from imatinib, dasatinib or nilotinib;
alternatively, the first-generation and/or second-generation and/or third-generation BCR-ABL1 ATP-competitive tyrosine kinase inhibitor(s) is selected from imatinib, dasatinib, nilotinib, bosutinib, flumatinib, radotinib, ponatinib or olverembatinib;
alternatively, the first-generation and/or second-generation and/or third-generation BCR-ABL1 ATP-competitive tyrosine kinase inhibitor(s) is selected from imatinib, dasatinib, nilotinib, flumatinib or, ponatinib or olverembatinib;
alternatively, the first-generation and/or second-generation and/or third-generation BCR-ABL1 ATP-competitive tyrosine kinase inhibitor(s) is a first-line, second-line, third-line, fourth-line, or fifth-line therapeutic agent;

alternatively, the first-generation and/or second-generation and/or third-generation BCR-ABL1 ATP-competitive tyrosine kinase inhibitor(s) is first-line or second-line therapeutic agent.

**24.** The use, method, or the compound for use according to any one of claims 1-23, wherein the dose of the compound of formula (I) or pharmaceutically acceptable salt, crystal form, solvate, or hydrate thereof (alternatively compound A) is about 20 mg/day to about 400 mg/day; alternatively, the dose of the compound of formula (I) or pharmaceutically acceptable salt, crystal form, solvate, or hydrate thereof (alternatively compound A) is about 20 mg/day to about 240 mg/day;

alternatively, the dose of the compound of formula (I) or pharmaceutically acceptable salt, crystal form, solvate, or hydrate thereof (alternatively compound A) is about 20 mg/day, about 40 mg/day, about 60 mg/day, about 80 mg/day, about 100 mg/day, about 120 mg/day, about 160 mg/day, or about 240 mg/day;
alternatively, the dose of the compound of formula (I) or pharmaceutically acceptable salt, crystal form, solvate, or hydrate thereof (alternatively compound A) is about 20 mg/day, about 40 mg/day, about 80 mg/day, about 160 mg/day, or about 240 mg/day.

**25.** The use, method, or the compound for use according to any one of claims 1-24, wherein the compound of formula (I) or a pharmaceutically acceptable salt, crystal form, solvate, or hydrate thereof (alternatively compound A) is administered orally or intranasally; alternatively, administered orally; alternatively, administered orally under fasting conditions.

**26.** The use, method, or the compound for use according to any one of claims 1-25, wherein the compound of formula (I) or a pharmaceutically acceptable salt, crystal form, solvate, or hydrate thereof (alternatively Compound A) is administered once daily (QD), twice daily (BID), or three times daily (TID).

**27.** The use, method, or the compound for use according to any one of claims 1-26, wherein the compound of formula (I) or a pharmaceutically acceptable salt, crystal form, solvate, or hydrate thereof (alternatively Compound A) is administered at about 10 mg BID, about 20 mg BID, about 40 mg QD, about 40 mg BID, about 80 mg QD, about 80 mg BID, about 160 mg QD, or about 240 mg QD.

**28.** The use, method, or the compound for use according to any one of claims 1-27, wherein the compound of formula (I) or a pharmaceutically acceptable salt, crystal form, solvate, or hydrate thereof (alternatively Compound A) is administered until disease progression, intolerable toxicity, or death.

**29.** A pharmaceutical combination comprising:

(a) the compound of formula (I) according to claim 1, or a pharmaceutically acceptable salt, crystal form, solvate, or hydrate thereof; and
(b) an ATP-competitive BCR-ABL1 inhibitor, or a pharmaceutically acceptable salt, crystal form, solvate, or hydrate thereof.

**30.** The pharmaceutical combination according to claim 29, wherein the compound of formula (I) is (R)-N-(4-(chlorodifluoromethoxy)phenyl)-6-(3-fluoropyrrolidin-1-yl)-5-(pyrazin-2-yl)nicotinamide (Compound A), or a pharmaceutically acceptable salt, crystal form, solvate, or hydrate thereof.

**31.** The pharmaceutical combination according to claim 29 or 30, wherein the ATP-competitive BCR-ABL1 inhibitor(s) is selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228;

alternatively, the ATP-competitive BCR-ABL1 inhibitor(s) is selected from imatinib, dasatinib, nilotinib, bosutinib, flumatinib, ponatinib, olverembatinib or PF-114;
alternatively, the ATP-competitive BCR-ABL1 inhibitor(s) is selected from imatinib, nilotinib, radotinib, flumatinib, ponatinib, bafetinib, CHMFL-ABL-039, CHMFL-ABL-053, CHMFL-ABL-KIT-155, vodobatinib, PF-114, olverembatinib or rebastinib;
alternatively, the ATP-competitive BCR-ABL1 inhibitor(s) is selected from ponatinib, PF-114, olverembatinib or rebastinib;
alternatively, the ATP-competitive BCR-ABL1 inhibitor(s) is selected from ponatinib, olverembatinib or PF-114;

alternatively, the ATP-competitive BCR-ABL1 inhibitor(s) is selected from ponatinib or olverembatinib; alternatively, the ATP-competitive BCR-ABL1 inhibitor(s) is selected from ponatinib.

**32.** The pharmaceutical combination according to any one of claims 29-31, wherein the dose of the compound of formula (I) or pharmaceutically acceptable salt, crystal form, solvate, or hydrate thereof (alternatively compound A) is about 1-500 mg;

alternatively, wherein the dose of the compound of formula (I) or pharmaceutically acceptable salt, crystal form, solvate, or hydrate thereof (alternatively compound A) is about 1-250 mg;
alternatively, wherein the dose of the compound of formula (I) or pharmaceutically acceptable salt, crystal form, solvate, or hydrate thereof (alternatively compound A) is about 1 mg, 2 mg, 3 mg, 4 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg or 250 mg;
alternatively, wherein the dose of the compound of formula (I) or pharmaceutically acceptable salt, crystal form, solvate, or hydrate thereof (alternatively compound A) is about 1 mg, 2 mg, 3 mg, 4 mg, 5 mg, 10 mg, 15 mg, 20 mg, 25 mg, 30 mg, 35 mg, 40 mg, 45 mg, 50 mg, 55 mg, 60 mg, 80 mg, 160 mg or 240 mg;
alternatively, wherein the compound of formula (I) or a pharmaceutically acceptable salt, crystal form, solvate, or hydrate thereof (alternatively compound A) is administered once daily or twice daily.

**33.** The pharmaceutical combination according to any one of claims 29-32, wherein the dose of the ATP-competitive BCR-ABL1 inhibitor is about 0.1-200 mg, alternatively about 0.1-60 mg.

**34.** The pharmaceutical combination according to claim 32, wherein the ATP-competitive BCR-ABL1 inhibitor is ponatinib at a dose of about 0.1-200 mg, alternatively about 0.1-60 mg;

alternatively, the dose of ponatinib is 0.1 mg, 0.5 mg, 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 20.5 mg, 21 mg, 21.5 mg, 22 mg, 22.5 mg, 23 mg, 23.5 mg, 24 mg, 24.5 mg, 25 mg, 25.5 mg, 26 mg, 26.5 mg, 27 mg, 27.5 mg, 28 mg, 28.5 mg, 29 mg, 29.5 mg, 30 mg, 30.5 mg, 31 mg, 31.5 mg, 32 mg, 32.5 mg, 33 mg, 33.5 mg, 34 mg, 34.5 mg, 35 mg, 35.5 mg, 36 mg, 36.5 mg, 37 mg, 37.5 mg, 38 mg, 38.5 mg, 39 mg, 39.5 mg, 40 mg, 40.5 mg, 41 mg, 41.5 mg, 42 mg, 42.5 mg, 43 mg, 43.5 mg, 44 mg, 44.5 mg, 45 mg, 45.5 mg, 46 mg, 46.5 mg, 47 mg, 47.5 mg, 48 mg, 48.5 mg, 49 mg, 49.5 mg, 50 mg, 50.5 mg, 51 mg, 51.5 mg, 52 mg, 52.5 mg, 53 mg, 53.5 mg, 54 mg, 54.5 mg, 55 mg, 55.5 mg, 56 mg, 56.5 mg, 57 mg, 57.5 mg, 58 mg, 58.5 mg, 59 mg, 59.5 mg or 60 mg;
alternatively, the dose of ponatinib is 2 mg, 4 mg, 8 mg, 15 mg, 30 mg, 45 mg or 60 mg;
alternatively, ponatinib is administered once daily or twice daily.

**35.** The pharmaceutical combination according to claim 32, wherein the ATP-competitive BCR-ABL1 inhibitor is olverembatinib at a dose of about 0.1-200 mg, alternatively about 0.1-60 mg;

alternatively, the dose of olverembatinib is 0.1 mg, 0.5 mg, 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 20.5 mg, 21 mg, 21.5 mg, 22 mg, 22.5 mg, 23 mg, 23.5 mg, 24 mg, 24.5 mg, 25 mg, 25.5 mg, 26 mg, 26.5 mg, 27 mg, 27.5 mg, 28 mg, 28.5 mg, 29 mg, 29.5 mg, 30 mg, 30.5 mg, 31 mg, 31.5 mg, 32 mg, 32.5 mg, 33 mg, 33.5 mg, 34 mg, 34.5 mg, 35 mg, 35.5 mg, 36 mg, 36.5 mg, 37 mg, 37.5 mg, 38 mg, 38.5 mg, 39 mg, 39.5 mg, 40 mg, 40.5 mg, 41 mg, 41.5 mg, 42 mg, 42.5 mg, 43 mg, 43.5 mg, 44 mg, 44.5 mg, 45 mg, 45.5 mg, 46 mg, 46.5 mg, 47 mg, 47.5 mg, 48 mg, 48.5 mg, 49 mg, 49.5 mg, 50 mg, 50.5 mg, 51 mg, 51.5 mg, 52 mg, 52.5 mg, 53 mg, 53.5 mg, 54 mg, 54.5 mg, 55 mg, 55.5 mg, 56 mg, 56.5 mg, 57 mg, 57.5 mg, 58 mg, 58.5 mg, 59 mg, 59.5 mg or 60 mg;
alternatively, the dose of olverembatinib is 1 mg, 2 mg, 4 mg, 8 mg, 12 mg, 20 mg, 30 mg, 40 mg, 45 mg, 50 mg or 60 mg;
alternatively, olverembatinib is administered once daily or twice daily.

**36.** The pharmaceutical combination according to any one of claims 29-35, wherein the (a) compound of formula (I) or a

pharmaceutically acceptable salt, crystal form, solvate, or hydrate thereof; and (b) ATP-competitive BCR-ABL1 inhibitor or a pharmaceutically acceptable salt, crystal form, solvate, or hydrate thereof are present in the same dosage form.

**37.** The pharmaceutical combination according to any one of claims 29-35, wherein the (a) compound of formula (I) or a pharmaceutically acceptable salt, crystal form, solvate, or hydrate thereof; and (b) ATP-competitive BCR-ABL1 inhibitor or a pharmaceutically acceptable salt, crystal form, solvate, or hydrate thereof are present in different dosage forms.

**38.** A pharmaceutical composition comprising the pharmaceutical combination of any one of claims 29-35.

**39.** Use of (a) the compound of formula (I) of claim 1 or a pharmaceutically acceptable salt, crystal form, solvate, or hydrate thereof and (b) an ATP-competitive BCR-ABL1 inhibitor or a pharmaceutically acceptable salt, crystal form, solvate, or hydrate thereof in the manufacture of a medicament for treating a disease regulated by wild-type or mutant BCR-ABL1 kinase.

**40.** Use of (a) the compound of formula (I) of claim 1 or a pharmaceutically acceptable salt, crystal form, solvate, or hydrate thereof in the manufacture of a medicament for use in combination with (b) an ATP-competitive BCR-ABL1 inhibitor or a pharmaceutically acceptable salt, crystal form, solvate, or hydrate thereof for treating a disease regulated by wild-type or mutant BCR-ABL1 kinase.

**41.** Use of (b) an ATP-competitive BCR-ABL1 inhibitor or a pharmaceutically acceptable salt, crystal form, solvate, or hydrate thereof in the manufacture of a medicament for use in combination with (a) the compound of formula (I) of claim 1 or a pharmaceutically acceptable salt, crystal form, solvate, or hydrate thereof for treating a disease regulated by wild-type or mutant BCR-ABL1 kinase.

**42.** A pharmaceutical combination of (a) the compound of formula (I) of claim 1 or a pharmaceutically acceptable salt, crystal form, solvate, or hydrate thereof and (b) an ATP-competitive BCR-ABL1 inhibitor or a pharmaceutically acceptable salt, crystal form, solvate, or hydrate thereof, for use in treating a disease regulated by wild-type or mutant BCR-ABL1 kinase.

**43.** (a) The compound of formula (I) of claim 1 or a pharmaceutically acceptable salt, crystal form, solvate, or hydrate thereof, for use in combination with (b) an ATP-competitive BCR-ABL1 inhibitor or a pharmaceutically acceptable salt, crystal form, solvate, or hydrate thereof, for treating a disease regulated by wild-type or mutant BCR-ABL1 kinase.

**44.** (b) An ATP-competitive BCR-ABL1 inhibitor or a pharmaceutically acceptable salt, crystal form, solvate, or hydrate thereof, for use in combination with (a) the compound of formula (I) of claim 1 or a pharmaceutically acceptable salt, crystal form, solvate, or hydrate thereof, for treating a disease regulated by wild-type or mutant BCR-ABL1 kinase.

**45.** A method for treating a disease regulated by wild-type or mutant BCR-ABL1 kinase, comprising administering to a patient in need thereof (a) the compound of formula (I) of claim 1 or a pharmaceutically acceptable salt, crystal form, solvate, or hydrate thereof, and (b) an ATP-competitive BCR-ABL1 inhibitor or a pharmaceutically acceptable salt, crystal form, solvate, or hydrate thereof.

**46.** The method according to claim 45, wherein (a) and (b) are administered simultaneously, separately, or sequentially.

**47.** The use according to any one of claims 39-41, or the compound and/or inhibitor for use according to any one of claims 42-44, or the method according to claim 45 or 46, wherein the disease regulated by the wild-type and/or mutant BCR-ABL1 kinase is a hematological malignancy or central nervous system leukemia relapse thereof, such as brain metastasis;

alternatively, the disease regulated by the wild-type and/or mutant BCR-ABL1 kinase is leukemia, neurodegenerative disease, neurofibroma, melanoma, breast cancer, colon cancer, lung cancer, prostate cancer, Kaposi's sarcoma, gastrointestinal stromal tumor, mesothelioma, systemic mastocytosis, hypereosinophilic syndrome, liver fibrosis, renal fibrosis, rheumatoid arthritis, polyarthritis, scleroderma, lupus erythematosus, graft-versus-host disease, pulmonary arterial hypertension, seminoma, dysgerminoma, and psoriasis;
alternatively, the leukemia is acute lymphoblastic leukemia, acute myeloid leukemia, acute promyelocytic leukemia, chronic myeloid leukemia, chronic myelomonocytic leukemia, chronic neutrophilic leukemia, acute

undifferentiated leukemia, prolymphocytic leukemia, juvenile myelomonocytic leukemia, adult T-cell leukemia, acute myeloid leukemia with trilineage myelodysplasia, and mixed lineage leukemia;
alternatively, the leukemia is chronic myeloid leukemia and Philadelphia chromosome-positive acute lymphoblastic leukemia;
alternatively, the neurodegenerative disease is Parkinson's disease, Alzheimer's disease, Down syndrome, memory and cognitive impairment, dementia, amyloid neuropathy, or cerebral inflammation;
alternatively, the disease regulated by wild-type and/or mutant BCR-ABL1 kinase is chronic myeloid leukemia, Philadelphia chromosome-positive acute lymphoblastic leukemia, Parkinson's disease, Alzheimer's disease, neurofibromatosis, gastrointestinal stromal tumor, systemic mastocytosis, hypereosinophilic syndrome, liver fibrosis, renal fibrosis, or scleroderma.

**48.** The use or compound and/or inhibitor for use or method according to any one of claims 39-47, wherein the mutation of the mutant BCR-ABL1 kinase is one or more of T315I, T315M, F317L, E355G, V299L, M244V, G250E, Q252H, Y253H, E255K, E255V, H396P, H396R, H396A, A397P, P223S, K294E, M351T, F359V, E459K, P465S, V468F, I502L, A337V or A424T;

alternatively, the mutation of the mutant BCR-ABL1 kinase is one or more of T315I, T315M, Y253H/T315I, E255V/T315I, Q252H/T315I, F317L/T315I, F359V/T315I, Y253H/E255K, Y253H/F359V;
alternatively, the mutation of the mutant BCR-ABL1 kinase is T315I, T315M, Y253H/T315I, E255V/T315I or Q252H/T315I;
alternatively, the mutation of the mutant BCR-ABL1 kinase is T315M, Y253H/T315I, E255V/T315I or Q252H/T315I.

**49.** The use or compound and/or inhibitor for use or method according to any one of claims 39-48, wherein the compound of formula (I) is (R)-N-(4-(chlorodifluoromethoxy)phenyl)-6-(3-fluoropyrrolidin-1-yl)-5-(pyrazin-2-yl)nicotinamide (Compound A) or a pharmaceutically acceptable salt, crystal form, solvate, or hydrate thereof.

**50.** The use or compound and/or inhibitor for use or method according to any one of claims 39-49, wherein the ATP-competitive BCR-ABL1 inhibitor(s) is selected from imatinib, dasatinib, nilotinib, bosutinib, flumatinib, ponatinib, olverembatinib, radotinib, bafetinib, vodobatinib, CHMFL-ABL-039, CHMFL-ABL-053, CHMFL-ABL-KIT-155, PF-114, pacritinib, rebasinib, danusertib, tozasertib, AT9283, KW-2449 or XL228;

alternatively, the ATP-competitive BCR-ABL1 inhibitor(s) is selected from imatinib, dasatinib, nilotinib, bosutinib, flumatinib, ponatinib, olverembatinib or PF-114;
alternatively, the ATP-competitive BCR-ABL1 inhibitor(s) is selected from imatinib, nilotinib, radotinib, flumatinib, ponatinib, bafetinib, CHMFL-ABL-039, CHMFL-ABL-053, CHMFL-ABL-KIT-155, vodobatinib, PF-114, olverembatinib or rebastinib;
alternatively, the ATP-competitive BCR-ABL1 inhibitor(s) is selected from ponatinib, PF-114, olverembatinib or rebastinib;
alternatively, the ATP-competitive BCR-ABL1 inhibitor(s) is selected from ponatinib, olverembatinib or PF-114;
alternatively, the ATP-competitive BCR-ABL1 inhibitor(s) is selected from ponatinib or olverembatinib;
alternatively, the ATP-competitive BCR-ABL1 inhibitor(s) is selected from ponatinib.

**51.** The use or compound and/or inhibitor for use or method according to any one of claims 39-50, wherein the dose of the compound of formula (I) or pharmaceutically acceptable salt, crystal form, solvate, or hydrate thereof (alternatively compound A) is about 1-500 mg;

alternatively, wherein the dose of the compound of formula (I) or pharmaceutically acceptable salt, crystal form, solvate, or hydrate thereof (alternatively compound A) is about 1-250 mg;
alternatively, wherein the dose of the compound of formula (I) or pharmaceutically acceptable salt, crystal form, solvate, or hydrate thereof (alternatively compound A) is about 1 mg, 2 mg, 3 mg, 4 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg or 250 mg;
alternatively, wherein the dose of the compound of formula (I) or pharmaceutically acceptable salt, crystal form, solvate, or hydrate thereof (alternatively compound A) is about 1 mg, 2 mg, 3 mg, 4 mg, 5 mg, 10 mg, 15 mg, 20 mg, 25 mg, 30 mg, 35 mg, 40 mg, 45 mg, 50 mg, 55 mg, 60 mg, 80 mg, 160 mg or 240 mg;

alternatively, wherein the compound of formula (I) or a pharmaceutically acceptable salt, crystal form, solvate, or hydrate thereof (alternatively compound A) is administered once daily or twice daily.

**52.** The use or compound and/or inhibitor for use or method according to any one of claims 39-51, wherein the dose of the ATP-competitive BCR-ABL1 inhibitor is about 0.1-200 mg, alternatively about 0.1-60 mg.

**53.** The use or compound and/or inhibitor for use or method according to claim 52, wherein the ATP-competitive BCR-ABL1 inhibitor is ponatinib at a dose of about 0.1-200 mg, alternatively about 0.1-60 mg;

alternatively, the dose of ponatinib is 0.1 mg, 0.5 mg, 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 20.5 mg, 21 mg, 21.5 mg, 22 mg, 22.5 mg, 23 mg, 23.5 mg, 24 mg, 24.5 mg, 25 mg, 25.5 mg, 26 mg, 26.5 mg, 27 mg, 27.5 mg, 28 mg, 28.5 mg, 29 mg, 29.5 mg, 30 mg, 30.5 mg, 31 mg, 31.5 mg, 32 mg, 32.5 mg, 33 mg, 33.5 mg, 34 mg, 34.5 mg, 35 mg, 35.5 mg, 36 mg, 36.5 mg, 37 mg, 37.5 mg, 38 mg, 38.5 mg, 39 mg, 39.5 mg, 40 mg, 40.5 mg, 41 mg, 41.5 mg, 42 mg, 42.5 mg, 43 mg, 43.5 mg, 44 mg, 44.5 mg, 45 mg, 45.5 mg, 46 mg, 46.5 mg, 47 mg, 47.5 mg, 48 mg, 48.5 mg, 49 mg, 49.5 mg, 50 mg, 50.5 mg, 51 mg, 51.5 mg, 52 mg, 52.5 mg, 53 mg, 53.5 mg, 54 mg, 54.5 mg, 55 mg, 55.5 mg, 56 mg, 56.5 mg, 57 mg, 57.5 mg, 58 mg, 58.5 mg, 59 mg, 59.5 mg or 60 mg;
alternatively, the dose of ponatinib is 2 mg, 4 mg, 8 mg, 15 mg, 30 mg, 45 mg or 60 mg;
alternatively, ponatinib is administered once daily or twice daily.

**54.** The use or compound and/or inhibitor for use or method according to claim 52, wherein the ATP-competitive BCR-ABL1 inhibitor is olverembatinib at a dose of about 0.1-200 mg, alternatively about 0.1-60 mg;

alternatively, the dose of olverembatinib is 0.1 mg, 0.5 mg, 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 20.5 mg, 21 mg, 21.5 mg, 22 mg, 22.5 mg, 23 mg, 23.5 mg, 24 mg, 24.5 mg, 25 mg, 25.5 mg, 26 mg, 26.5 mg, 27 mg, 27.5 mg, 28 mg, 28.5 mg, 29 mg, 29.5 mg, 30 mg, 30.5 mg, 31 mg, 31.5 mg, 32 mg, 32.5 mg, 33 mg, 33.5 mg, 34 mg, 34.5 mg, 35 mg, 35.5 mg, 36 mg, 36.5 mg, 37 mg, 37.5 mg, 38 mg, 38.5 mg, 39 mg, 39.5 mg, 40 mg, 40.5 mg, 41 mg, 41.5 mg, 42 mg, 42.5 mg, 43 mg, 43.5 mg, 44 mg, 44.5 mg, 45 mg, 45.5 mg, 46 mg, 46.5 mg, 47 mg, 47.5 mg, 48 mg, 48.5 mg, 49 mg, 49.5 mg, 50 mg, 50.5 mg, 51 mg, 51.5 mg, 52 mg, 52.5 mg, 53 mg, 53.5 mg, 54 mg, 54.5 mg, 55 mg, 55.5 mg, 56 mg, 56.5 mg, 57 mg, 57.5 mg, 58 mg, 58.5 mg, 59 mg, 59.5 mg or 60 mg;
alternatively, the dose of olverembatinib is 1 mg, 2 mg, 4 mg, 8 mg, 12 mg, 20 mg, 30 mg, 40 mg, 45 mg, 50 mg or 60 mg;
alternatively, olverembatinib is administered once daily or twice daily.

**55.** The use or compound and/or inhibitor for use or method according to any one of claims 39-54, wherein the (a) compound of formula (I) or a pharmaceutically acceptable salt, crystal form, solvate, or hydrate thereof; and (b) ATP-competitive BCR-ABL1 inhibitor or a pharmaceutically acceptable salt, crystal form, solvate, or hydrate thereof are present in the same dosage form.

**56.** The use or compound and/or inhibitor for use or method according to any one of claims 39-54, wherein the (a) compound of formula (I) or a pharmaceutically acceptable salt, crystal form, solvate, or hydrate thereof; and (b) ATP-competitive BCR-ABL1 inhibitor or a pharmaceutically acceptable salt, crystal form, solvate, or hydrate thereof are present in different dosage forms.

**57.** A method for inhibiting a mutant BCR-ABL1 kinase, comprising administering:

(a) a compound of formula (I) according to claim 1, or a pharmaceutically acceptable salt, crystal form, solvate, or hydrate thereof; and
(b) an ATP-competitive BCR-ABL1 inhibitor, or a pharmaceutically acceptable salt, crystal form, solvate, or hydrate thereof.

**58.** The method according to claim 57, wherein the mutant BCR-ABL1 kinase is selected from one or more of T315I, T315M, F317L, E355G, V299L, M244V, G250E, Q252H, Y253H, E255K, E255V, H396P, H396R, H396A, A397P,

P223S, K294E, M351T, F359V, E459K, P465S, V468F, I502L, A337V or A424T;

alternatively, the mutant BCR-ABL1 kinase is T315I, T315M, Y253H/T315I, E255V/T315I, Q252H/T315I, F317L/T315I, F359V/T315I, Y253H/E255K or Y253H/F359V;
alternatively, the mutant BCR-ABL1 kinase is T315I, T315M, Y253H/T315I, E255V/T315I or Q252H/T315I;
alternatively, the mutant BCR-ABL1 kinase is T315M, Y253H/T315I, E255V/T315I or Q252H/T315I.

**59.** A commercial package, comprising:

(a) Compound A of claim 4 or a pharmaceutically acceptable salt, crystal form, solvate, or hydrate thereof; and
(b) an ATP-competitive BCR-ABL1 inhibitor or a pharmaceutically acceptable salt, crystal form, solvate, or hydrate thereof;

wherein (a) and (b) are present in a single unit dosage form or in two separate unit dosage forms.

**60.** A commercial package comprising:

(a) Compound A of claim 4 or a pharmaceutically acceptable salt, crystal form, solvate, or hydrate thereof; and
(b) Ponatinib or a pharmaceutically acceptable salt, crystal form, solvate, or hydrate thereof;

wherein (a) and (b) are present in a single unit dosage form or in two separate unit dosage forms.

**61.** A commercial package comprising:

(a) Compound A of claim 4 or a pharmaceutically acceptable salt, crystal form, solvate, or hydrate thereof; and
(b) Olverembatinib or a pharmaceutically acceptable salt, crystal form, solvate, or hydrate thereof;

wherein (a) and (b) are present in a single unit dosage form or in two separate unit dosage forms.

**62.** A commercial package comprising:

(a) Compound A of claim 4 or a pharmaceutically acceptable salt, crystal form, solvate, or hydrate thereof; and
(b) PF-114 or a pharmaceutically acceptable salt, crystal form, solvate, or hydrate thereof;

wherein (a) and (b) are present in a single unit dosage form or in two separate unit dosage forms.

**63.** The commercial package according to any one of claims 59-62, wherein the unit dosage form is a fixed combination.

**64.** The commercial packaging according to any one of claims 59-63, for use in treating a disease regulated by wild-type or mutant BCR-ABL1 kinase.

Ba/F3 BCR-ABL1$^{Y253H/T315I}$

Inhibition%

120
100
80
60
40
20
0
-20

1
10
100
1000

Conc.(nM) of Cmpd A

Cmpd A
Cmpd A+Pona.

Fig. 1a

Ba/F3 BCR-ABL1Y253H/T315I
Inhibition%
120
100
80
60
40
20
0
-20
0.1
1
10
100
Conc.(nM) of Ponatinib
Pona.
Cmpd A+Pona.

Fig. 1b

Ba/F3 BCR-ABL1 $^{Y253H/T315I}$

Inhibition%

120
100
80
60
40
20
0
-20

1
10
100
1000

Conc.(nM) of Cmpd A

Cmpd A
Cmpd A+Olv.

Fig. 1c

Ba/F3 BCR-ABL1Y253H/T315I
Inhibition%
120
100
80
60
40
20
0
-20
0.1
1
10
100
Conc.(nM) of Olverembatinib
Olv.
Cmpd A+Olv.

Fig. 1d

Cmpd A + Ponatinib
CI
3.0
2.0
1.0
0
0
0.2
0.4
0.6
0.8
1.0
Fractional Effect

**Fig. 1e**

Cmpd A + olverembatinib
CI
1.5
1.0
0.5
0
0
0.2
0.4
0.6
0.8
1.0
Fractional Effect

**Fig. 1f**

Ba/F3 BCR-ABL1 Y253H/T315I
Cell viability of control (%)
120.00
100.00
80.00
60.00
40.00
20.00
0.00
0
30
100
300
500
1000
3000
5000
10000
15000
Cmpd A Conc.(nM)
Ponatinib Conc.(nM)
0
0.3
1
3
5
10
30
50
100


Fig. 2a

Ba/F3 BCR-ABL1 Y253H/T315I
Cell viability of control (%)
120.00
100.00
80.00
60.00
40.00
20.00
0.00
0
30
100
300
500
1000
3000
5000
10000
15000
Cmpd A Conc.(nM)
0
0.3
1
3
5
10
30
50
100
Olverembatinib Conc.(nM)


Fig. 2b

Ba/F3 BCR-ABL1 Y253H/T315I
IC90 for ponatinib (nM)
2000
1500
1000
200
150
100
50
0
1489nM
60.9nM
165nM
@Pona. 45mg QD
CmaxSS=145.4nM
CminSS=64.3nM
Pona. alone
Cmpd A+Pona.
ABL001+Pona.


Fig. 3

Ba/F3 BCR-ABL1 E255V/T315I
Cell viability (% of control)
Conc. of ponatinib (nM)
60
48
24
19.2
ns
**
****
Cmpd A (720nM) +Pona.
Asciminib (600nM) + Pona.
Asciminib (6000nM) + Pona.
Ponatinib alone

Fig. 4

Ba/F3 BCR-ABL1 Q252H/T315I
Cell viability (% of control)
Concentration of ponatinib (nM)
60
48
24
19.2
ns
**
****
Cmpd A (720nM) +Pona.
Asciminib (600nM) + ponatinib
Asciminib (6000nM) + ponatinib
Ponatinib alone

Fig. 5

Ba/F3 BCR-ABL1 T315M
****
ns
ns
****
ns
ns
****
ns
ns
****
ns
****
Cell viability (% of control)
120
80
40
30
20
10
0
60
48
24
19.2
Concentration of ponatinib (nM)
Cmpd A (720nM) + Pona.
Asciminib (600nM) + Pona.
Asciminib (6000nM) + Pona.
Ponatinib alone

Fig. 6

Cell viability(%)
120
65
10
10
8
6
4
2
0
T315I
Y253H/F359V
Y253H/E255K
F359V
Ba/F3 BCR-ABL1 mutants
Cmpd A(720nM) +Pona.(19.2nM)
Cmpd A(720nM)
Pona.(19.2nM)

Fig. 7

1.E+05
1.E+04
1.E+03
1.E+02
1.E+01
BLI value (photons/s×10^5)
Vehicle control
Compound A 50 mg/kg
9
15
21
27
33
Days post-tumor inoculation


Fig. 8

**INTERNATIONAL SEARCH REPORT**

International application No. **PCT/CN2024/128825**

**A. CLASSIFICATION OF SUBJECT MATTER**

A61K31/4439(2006.01)i； A61K31/444(2006.01)i； A61K31/501(2006.01)i； C07D401/14(2006.01)i； C07D403/14(2006.01)i； A61P35/02(2006.01)i； A61P35/00(2006.01)i

According to International Patent Classification (IPC) or to both national classification and IPC

**B. FIELDS SEARCHED**

Minimum documentation searched (classification system followed by classification symbols)

IPC： A61K C07D A61P

Documentation searched other than minimum documentation to the extent that such documents are included in the fields searched

Electronic data base consulted during the international search (name of data base and, where practicable, search terms used)

CNABS, CNTXT, WPABS, ENTXT, CNKI, PUBMED, ISI WEB OF SCIENCE, STNext: 深圳市塔吉瑞生物医药有限公司, 王义汉, BCR-ABL, 抑制剂, 突变, 耐药, T315I, 阿思尼布, 伊马替尼, 达沙替尼, 尼洛替尼, 帕纳替尼, 奥雷巴替尼, PF-114, 白血病, 结构式检索, structural formula search, asciminib, ABL001, imatinib, dasatinib, nilotinib, ponatinib, olverembatinib, vamotinib, CML, AML, 2240190-29-6, 1492952-76-7, 943319-70-8, 1257628-77-5, 1416241-23-0

**C. DOCUMENTS CONSIDERED TO BE RELEVANT**

| Category* | Citation of document, with indication, where appropriate, of the relevant passages | Relevant to claim No. |
|---|---|---|
| PX | CN 117402142 A (SHENZHEN TARGETRX, INC.) 16 January 2024 (2024-01-16) claims 1-7, and description, paragraphs 0008, 0039-0043 and 0083, and embodiments 1-2 | 5-28 |
| X | WO 2018133827 A1 (SHENZHEN TARGETRX, INC.) 26 July 2018 (2018-07-26) claims 1-13, and description, page 3, lines 5-19, page 7, lines 5-9, page 24, lines 10-11 and 25-34, and page 26, lines 15-16, and embodiments 7, 13 and 15 | 1-28, 40, 43, 47-56 |
| X | CN 104379574 A (NOVARTIS AG) 25 February 2015 (2015-02-25) description, paragraph [0006] | 41, 44, 47-56 |
| Y | WO 2018133827 A1 (SHENZHEN TARGETRX, INC.) 26 July 2018 (2018-07-26) claims 1-13, and description, page 3, lines 5-19, page 7, lines 5-9, page 24, lines 10-11 and 25-34, and page 26, lines 15-16, and embodiments 7, 13 and 15 | 29-39, 42, 45-64 |
| Y | CN 104379574 A (NOVARTIS AG) 25 February 2015 (2015-02-25) claims 34 and 38-39, and description, paragraph 0006 | 29-39, 42, 45-64 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 February 2025** | **24 February 2025** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No. **PCT/CN2024/128825**

**C. DOCUMENTS CONSIDERED TO BE RELEVANT**

| Category* | Citation of document, with indication, where appropriate, of the relevant passages | Relevant to claim No. |
|---|---|---|
| X | WO 2021213380 A1 (SHENZHEN TARGETRX, INC.) 28 October 2021 (2021-10-28) claims 1-77 and 164-169, and description, page 1, lines 4-7 and 10-14 | 1-28, 40, 43, 47-54 |
| Y | WO 2021213380 A1 (SHENZHEN TARGETRX, INC.) 28 October 2021 (2021-10-28) claims 1-77 and 164-169, and description, page 1, lines 4-7 and 10-14 | 29-39, 42, 45-64 |
| Y | SAGLIO, G. et al. "Randomized, Open-Label, Multicenter, Phase 2 Study of Asciminib (ABL001) As an Add-on to Imatinib Versus Continued Imatinib Versus Switch to Nilotinib in Patients with Chronic Myeloid Leukemia in Chronic Phase who have not Achieved a Deep Molecular Response with Frontline Imatinib" *Blood,* Vol. vol. 134 (Supplement_1), 31 December 2019 (2019-12-31), p. 5910 | 29-39, 42, 45-64 |
| Y | WO 2022238884 A1 (NOVARTIS AG) 17 November 2022 (2022-11-17) claims 1-7, and description, paragraphs 0004-0005 | 29-39, 42, 45-64 |
| Y | WYLIE, A.A. et al. "The Allosteric Inhibitor ABL001 Enables Dual Targeting of BCR-ABL1" *Nature,* Vol. vol. 543, 30 March 2017 (2017-03-30), pp. 733-737 | 29-39, 42, 45-64 |

Form PCT/ISA/210 (second sheet) (July 2022)

**INTERNATIONAL SEARCH REPORT**

International application No.
**PCT/CN2024/128825**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **2, 4-28, 45-58**
because they relate to subject matter not required to be searched by this Authority, namely:
The subject matter of claims 2, 4-28 and 45-58 falls within methods for treating diseases of a human body as described in PCT Rule 39.1(iv). A search report is provided on the basis of the use of the compound or composition in the preparation of a drug for treating a corresponding disease.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/128825**

| Patent document cited in search report | | | Publication date (day/month/year) | Patent family member(s) | | | Publication date (day/month/year) |
|---|---|---|---|---|---|---|---|
| CN | 117402142 | A | 16 January 2024 | None | | | |
| WO | 2018133827 | A1 | 26 July 2018 | EP | 3553056 | A1 | 16 October 2019 |
| | | | | EP | 3553056 | A4 | 08 April 2020 |
| | | | | EP | 3553056 | B1 | 17 February 2021 |
| | | | | US | 2019382374 | A1 | 19 December 2019 |
| | | | | US | 2020377473 | A9 | 03 December 2020 |
| | | | | US | 11091462 | B2 | 17 August 2021 |
| | | | | ES | 2864405 | T3 | 13 October 2021 |
| | | | | JP | 2020506965 | A | 05 March 2020 |
| | | | | JP | 6840411 | B2 | 10 March 2021 |
| | | | | EP | 3838897 | A2 | 23 June 2021 |
| | | | | EP | 3838897 | A3 | 07 July 2021 |
| CN | 104379574 | A | 25 February 2015 | WO | 2013171640 | A1 | 21 November 2013 |
| | | | | KR | 20150020169 | A | 25 February 2015 |
| | | | | US | 2015183801 | A1 | 02 July 2015 |
| | | | | US | 9278981 | B2 | 08 March 2016 |
| | | | | AU | 2013261128 | A1 | 23 October 2014 |
| | | | | AU | 2013261128 | B2 | 12 November 2015 |
| | | | | EA | 201492005 | A1 | 30 April 2015 |
| | | | | JP | 2015516460 | A | 11 June 2015 |
| | | | | JP | 6078640 | B2 | 08 February 2017 |
| | | | | CA | 2871332 | A1 | 21 November 2013 |
| | | | | BR | 112014027181 | A2 | 27 June 2017 |
| | | | | MX | 2014013373 | A | 14 August 2015 |
| | | | | EP | 2858988 | A1 | 15 April 2015 |
| | | | | EP | 2858988 | B1 | 28 February 2018 |
| | | | | ES | 2670667 | T3 | 31 May 2018 |
| WO | 2021213380 | A1 | 28 October 2021 | US | 2023322717 | A1 | 12 October 2023 |
| | | | | EP | 4122928 | A1 | 25 January 2023 |
| | | | | EP | 4122928 | A4 | 13 December 2023 |
| | | | | JP | 2023522110 | A | 26 May 2023 |
| | | | | JP | 7612229 | B2 | 14 January 2025 |
| WO | 2022238884 | A1 | 17 November 2022 | IL | 308095 | A | 01 December 2023 |
| | | | | KR | 20240006600 | A | 15 January 2024 |
| | | | | CA | 3218550 | A1 | 17 November 2022 |
| | | | | US | 2024245666 | A1 | 25 July 2024 |
| | | | | EP | 4337205 | A1 | 20 March 2024 |
| | | | | JP | 2024518426 | A | 01 May 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021213380 A1
- WO 2013171640 A1
- WO 2021143927 A1
- CN 202311337298
- WO 2018133827 A1


### Non-patent literature cited in the description


- **BERGE et al.** *J. Pharmaceutical Sciences*, 1977, vol. 66, 1-19
- **BACCARANI et al.** European LeukemiaNet recommendations for the management of chronic myeloid leukemia: 2013. *Blood*, 2013, vol. 122 (6)
- **Y.-L PAN et al.** The progress of small-molecules and degraders against BCR-ABL1 for the treatment of CML. *European Journal of Medicinal Chemistry*, 2022, vol. 238, 114442
- Diagnosis and Treatment Guidelines for Adult Chronic Myeloid Leukemia (2018 Edition. *National Health Commission of the People's Republic of China in*, December 2018
- *Diagnosis and Treatment Guidelines for Adult Chronic Myeloid Leukemia*, 2018